# EUROPEAN PATENT SPECIFICATION

(11) **EP 4 013 877 B1**
(45) Date of publication and mention of the grant of the patent: **14.10.2026**
(21) Application number: 20765126.6
(22) Date of filing: 14.08.2020
(51) Int. Cl.: C12N 15/113, A61K 31/7125, A61K 9/127, A61P 11/00, A61P 35/00

(54) **EXTRACELLULAR VESICLE-ASO CONSTRUCTS TARGETING STAT6**
EXTRAZELLULÄRE VESIKEL MIT ASO-KONSTRUKTEN GEGEN STAT6
CONSTRUCTIONS DE VÉSICULES EXTRACELLULAIRES - ASO CIBLANT STAT6

(30) Priority: 14.08.2019 US 201962886944 P; 13.09.2019 US 201962900138 P; 20.09.2019 US 201962903518 P; 15.11.2019 US 201962936216 P; 13.03.2020 US 202062989477 P; 05.06.2020 US 202063035392 P
(43) Date of publication of application: 22.06.2022
(60) Divisional application: 24204511.0 / 4 470 619
(73) Proprietor: Lonza Sales AG, 4052 Basel (CH)
(72) Inventor: BURZYN, Dalia, Cambridge, Massachusetts 02140 (US); KAMERKAR, Sushrut, Cambridge, Massachusetts 02140 (US); BOUTIN, Adam T., Cambridge, Massachusetts 02140 (US); BROOM, Wendy, Cambridge, Massachusetts 02140 (US); SATHYANARAYANAN, Sriram, Cambrdige, Massachusetts 02140 (US); VERMA, Ajay, Cambridge, Massachusetts 02140 (US)
(74) Representative: Mathys & Squire
(86) International application number: PCT/US2020/046559
(87) International publication number: WO 2021/030776

(56) References cited:
- WO-A1-2017/161010
- WO-A1-2018/102397
- WO-A1-2019/099942
- WO-A1-2019/122282
- WO-A1-2019/157535
- WO-A1-2020/101740
- WO-A2-2004/052309
- XUE-JIA LIN ET AL: "Hepatocellular Carcinoma Cell-Secreted Exosomal MicroRNA-210 Promotes Angiogenesis In Vitro and In Vivo", MOLECULAR THERAPY-NUCLEIC ACIDS, vol. 11, 6 March 2018 (2018-03-06), US, pages 243 - 252, XP055740900, ISSN: 2162-2531, DOI: 10.1016/j.omtn.2018.02.014
- XIN-RUI TIAN ET AL: "Inhibition of allergic airway inflammation by antisense-induced blockade of STAT6 expression", CHINESE MEDICAL JOURNAL / ZHONGHUA YIXUE ZAZHI YINGWEN BAN., vol. 124, no. 1, January 2011 (2011-01-01), CN, pages 26 - 31, XP055741028, ISSN: 0366-6999, DOI: 10.3760/cma.j.issn.0366-6999.2010.01.006
- PURNA KRISHNAMURTHY ET AL: "STAT6 and PARP Family Members in the Development of T Cell-dependent Allergic Inflammation", IMMUNE NETWORK, vol. 16, no. 4, 23 August 2016 (2016-08-23), pages 201, XP055741053, ISSN: 1598-2629, DOI: 10.4110/in.2016.16.4.201
- HANNAH H WALFORD ET AL: "STAT6 and lung inflammation", JAK-STAT, vol. 2, no. 4, 10 June 2013 (2013-06-10), pages e25301, XP055741051, DOI: 10.4161/jkst.25301

## Description

### FIELD OF DISCLOSURE

The present disclosure relates to extracellular vesicles (EVs), e.g., exosomes, comprising an antisense oligonucleotide (ASO), wherein the ASO comprises a contiguous nucleotide sequence of 10 to 30 nucleotides in length that is complementary to a nucleic acid sequence within a *STAT6* transcript, as defined in the appended claims. In certain aspects of the disclosure, the extracellular vesicle further comprises a scaffold protein.

### BACKGROUND

Exosomes are small extracellular vesicles that are naturally produced by every eukaryotic cell. Exosomes comprise a membrane that encloses an internal space *(i.e.,* lumen). As drug delivery vehicles, EVs, e.g., exosomes, offer many advantages over traditional drug delivery methods as a new treatment modality in many therapeutic areas. In particular, exosomes have intrinsically low immunogenicity, even when administered to a different species.

Antisense oligonucleotides have emerged as a powerful means of regulating target gene expression *in vitro* or *in vivo.* However, there remains a need to improve the stability and targeting of ASOs *in vivo.* Accordingly, new and more effective engineered-EVs (*e.g.,* exosomes), particularly those that can be used to deliver therapeutic agents that can reduce the expression of a gene associated with a disease (*e.g.,* N for cancer), are necessary to better enable therapeutic use and other applications of EV-based technologies. WO 2018/102397 describes Exosomes for Delivery of Therapeutic Agents; Lin et al., (2018) 6(11):243-252 reports Hepatocellular Carcinoma Cell-Secreted Exosomal MicroRNA-210 Promotes Angiogenesis *In Vitro* and *In Vivo;* WO 2017/161010 describes Therapeutic Membrane Vesicles; WO 2019/099942 describes Compositions of Engineered Exosomes and Methods of Loading Luminal Exosomes Payloads; and WO 2004/052309 describes the modulation of STAT6 expression.

### SUMMARY OF DISCLOSURE

The present invention and embodiments thereof are set out in the appended claims. In so far as the terms "invention", "example", "aspect", and "embodiment" are used herein, these terms will be interpreted in such a way that protection does not extend beyond the scope of the claims.

The present invention provides an extracellular vesicle capable of reducing STAT6 protein or mRNA expression in a human cell expressing STAT6, wherein the extracellular vesicle comprises an antisense oligonucleotide (ASO) which comprises a contiguous nucleotide sequence of 10 to 30 nucleotides in length that is complementary to a nucleic acid sequence within a *STAT6* transcript (SEQ ID NO: 1 or SEQ ID NO: 3), wherein the ASO comprises the nucleotide sequence set forth in SEQ ID NO: 91.

A further aspect of the invention is a pharmaceutical composition comprising the extracellular vesicle of the invention and a pharmaceutically acceptable diluent, carrier, salt, or adjuvant.

Certain aspects of the invention are directed to a extracellular vesicle or a pharmaceutical composition as defined in the claims, for use in the treatment of a cancer.

Certain aspects of the present disclosure are directed to an extracellular vesicle comprising an antisense oligonucleotide (ASO) which comprises a contiguous nucleotide sequence of 10 to 30 nucleotides in length that is complementary to a nucleic acid sequence within a *STAT6* transcript (SEQ ID NO: 1 or SEQ ID NO: 3) as defined in the claims. The ASO is not TGAGCGAATGGACAGGTCTT (SEQ ID NO: 89).

In some aspects, the extracellular vesicle targets a cell selected from the group consisting of a macrophage, a myeloid-derived suppressor cell (MDSC), a monocyte, a basophil, a neutrophil, an eosinophil, and any combination thereof.

In some aspects, the ASO comprises a contiguous nucleotide sequence of 10 to 30 nucleotides in length that is complementary to a nucleic acid sequence within nucleotides 1 to 2056 of a *STAT6* transcript corresponding to a nucleotide sequence as set forth in SEQ ID NO: 3 as defined in the claims. In some aspects, the contiguous nucleotide sequence is at least about 80%, at least about 85%, at least about 90%, at least about 95%, or about 100% complementary to the nucleic acid sequence within the *STAT6* transcript. In some aspects, the ASO is capable of reducing STAT6 protein expression in a human cell (*e.g.,* an immune cell), wherein the human cell expresses the STAT6 protein. In some aspects, the STAT6 protein expression is reduced by at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100% compared to STAT6 protein expression in a human cell that is not exposed to the ASO.

In some aspects, the ASO is capable of reducing a level of *STAT6* mRNA in a human cell (*e.g.,* an immune cell), wherein the human cell expresses the *STAT6* mRNA. In some aspects, the level of *STAT6* mRNA is reduced by at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100% compared to the level of the *STAT6* mRNA in a human cell that is not exposed to the ASO.

In some aspects, the ASO is a gapmer, a mixmer, or a totalmer. In some aspects, the ASO comprises one or more nucleoside analogs. In some aspects, one or more of the nucleoside analogs comprises a 2'-O-alkyl-RNA; 2'-O-methyl RNA (2'-OMe); 2'-alkoxy-RNA; 2'-O-methoxyethyl-RNA (2'-MOE); 2'-amino-DNA; 2'-fluro-RNA; 2'-fluoro-DNA; arabino nucleic acid (ANA); 2'-fluoro-ANA; or bicyclic nucleoside analog. In some aspects, one or more of the nucleoside analogs is a sugar modified nucleoside. In some aspects, the sugar modified nucleoside is an affinity enhancing 2' sugar modified nucleoside. In some aspects, one or more of the nucleoside analogs comprises a nucleoside comprising a bicyclic sugar. In some aspects, one or more of the nucleoside analogs comprises an LNA.

In some aspects, one or more of the nucleotide analogs is selected from the group consisting of constrained ethyl nucleoside (cEt), 2',4'-constrained 2'-O-methoxyethyl (cMOE), α-L-LNA, β-D-LNA, 2'-O,4'-C-ethylene-bridged nucleic acids (ENA), amino-LNA, oxy-LNA, thio-LNA, and any combination thereof. In some aspects, the ASO comprises one or more 5'-methyl-cytosine nucleobases.

The contiguous nucleotide sequence defined in the claims is complementary to a nucleic acid sequence comprising (i) nucleotides 1000-1500 of SEQ ID NO: 3; (ii) nucleotides 952-1688 of SEQ ID NO: 3; (iii) nucleotides 1002-1638 of SEQ ID NO: 3; or (iv) nucleotides 1042-1598 of SEQ ID NO: 3. The contiguous nucleotide sequence defined in the claims is complementary to a nucleic acid sequence within nucleotides 1052 - 1588 of SEQ ID NO: 3.

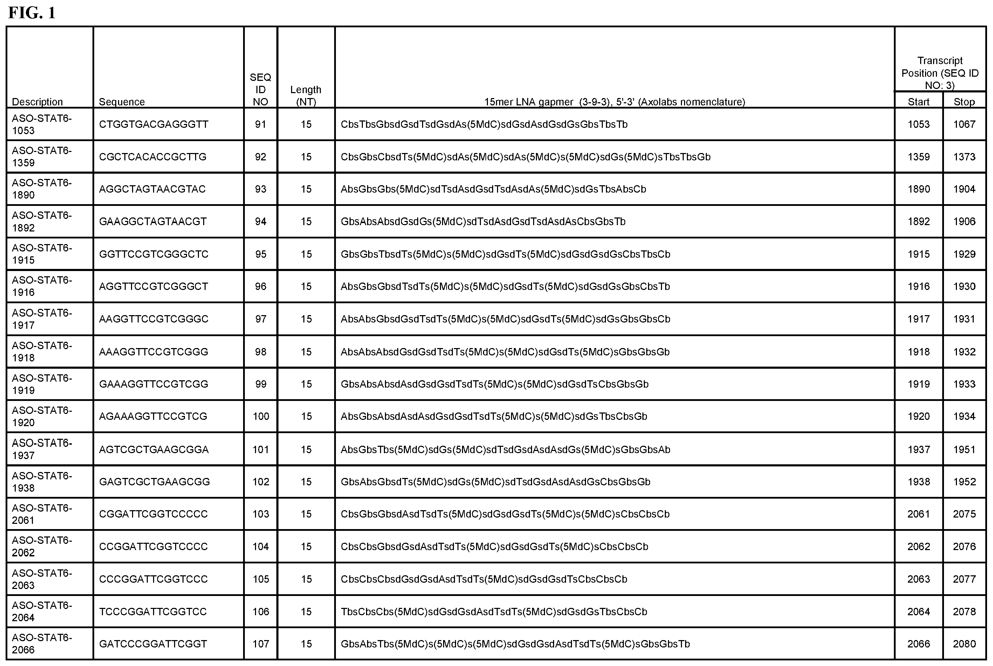

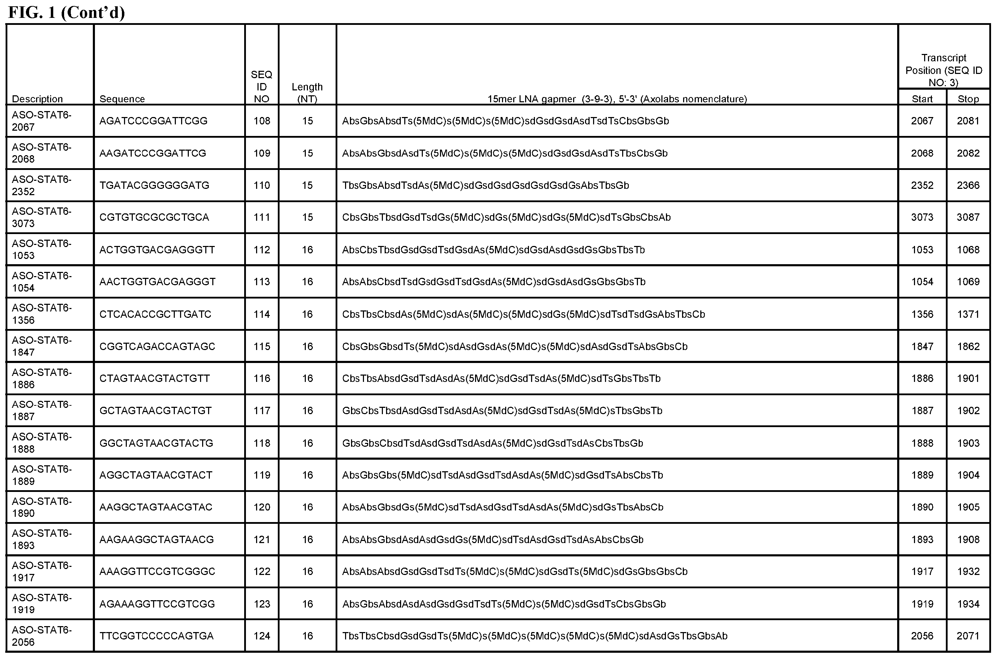

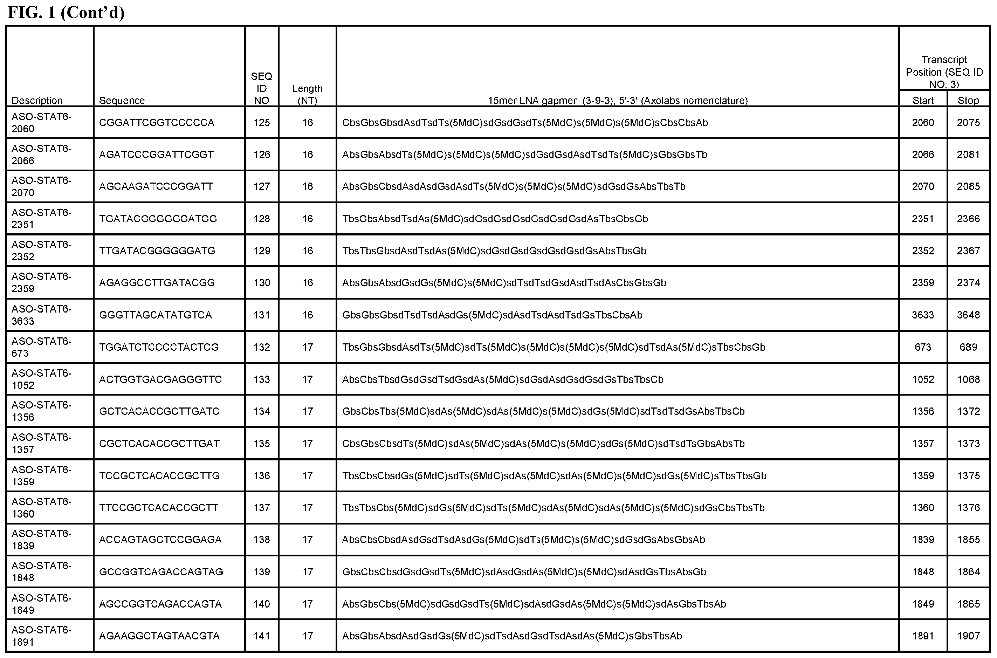

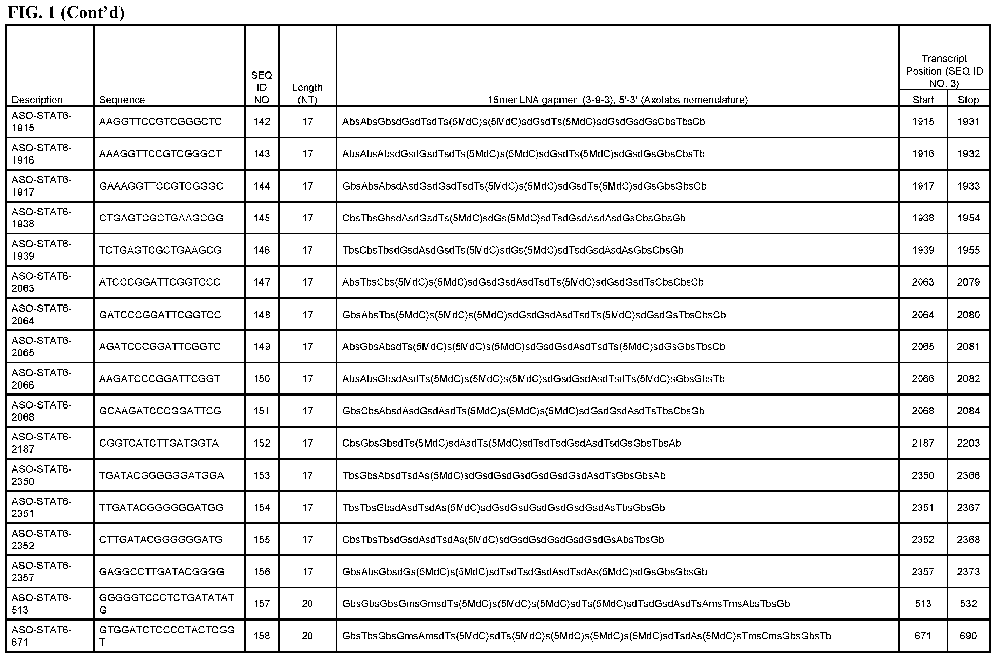

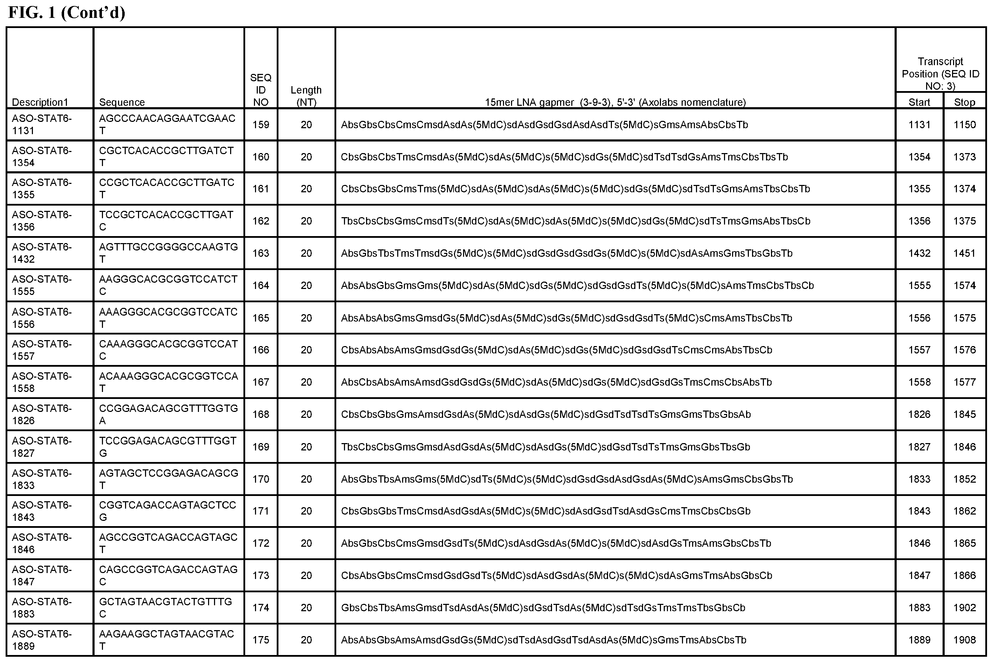

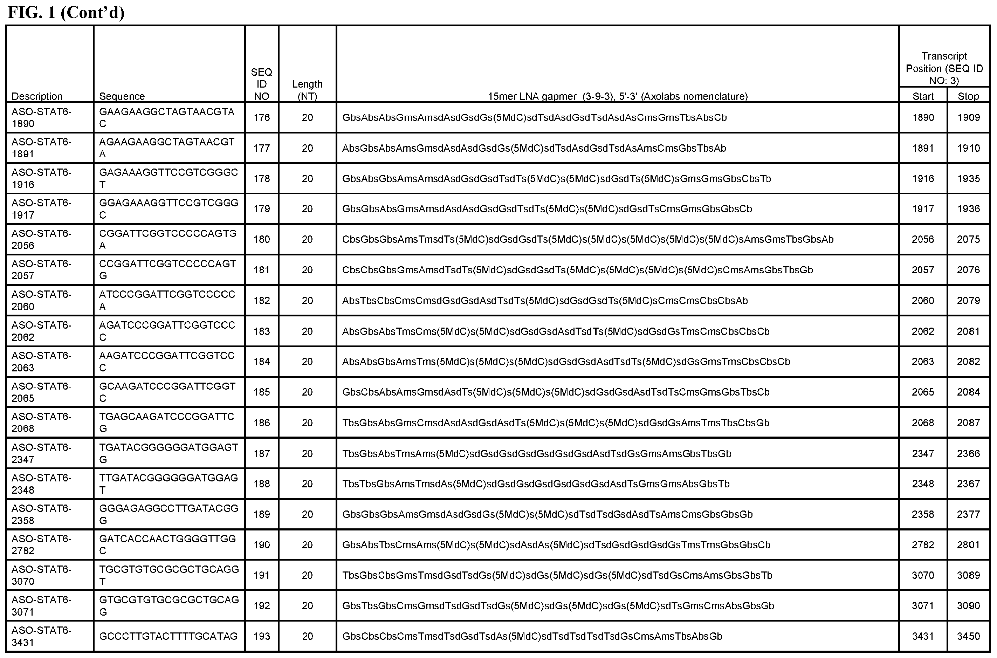

The contiguous nucleotide sequence defined in the claims comprises a nucleotide sequence complementary to a sequence selected from the sequences in FIG. 1.

In some aspects, the continuous nucleotide sequence is fully complementary to a nucleotide sequence within the *STAT6* transcript. The ASO comprises the nucleotide sequence of SEQ ID NO: 91. In some aspects, the ASO has a design selected from the group consisting of the designs in FIG. 1, as defined in the claims, wherein the upper case letter is a sugar modified nucleoside and the lower case letter is DNA. In some aspects, the ASO defined in the claims is from 14 to 20 nucleotides in length.

In some aspects, the contiguous nucleotide sequence comprises one or more modified internucleoside linkages. In some aspects, the one or more modified internucleoside linkages is a phosphorothioate linkage. In some aspects, at least 75%, at least 80%, at least 85%, at least 90%, at least 95%, or 100% of internucleoside linkages are modified. In some aspects, each of the internucleoside linkages in the ASO is a phosphorothioate linkage.

In some aspects, the extracellular vesicle further comprises an anchoring moiety. In some aspects, the ASO is linked to the anchoring moiety. In some aspects, the extracellular vesicle further comprises an exogenous targeting moiety. In some aspects, the exogenous targeting moiety comprises a peptide, an antibody or an antigen-binding fragment thereof, a chemical compound, an RNA aptamer, or any combination thereof. In some aspects, the exogenous targeting moiety comprises a peptide. In some aspects, the exogenous targeting moiety comprises a microprotein, a designed ankyrin repeat protein (darpin), an anticalin, an adnectin, an aptamer, a peptide mimetic molecule, a natural ligand for a receptor, a camelid nanobody, or any combination thereof.

In some aspects, the exogenous targeting moiety comprises a full-length antibody, a single domain antibody, a heavy chain only antibody (VHH), a single chain antibody, a shark heavy chain only antibody (VNAR), an scFv, a Fv, a Fab, a Fab', a F(ab')2, or any combination thereof.

In some aspects, the antibody is a single chain antibody.

In some aspects, the exogenous targeting moiety targets the exosome to the liver, heart, lungs, brain, kidneys, central nervous system, peripheral nervous system, muscle, bone, joint, skin, intestine, bladder, pancreas, lymph nodes, spleen, blood, bone marrow, or any combination thereof. In some aspects, the exogenous targeting moiety targets the exosome to a tumor cell, dendritic cell, T cell, B cell, macrophage, neuron, hepatocyte, Kupffer cell, myeloid-lineage cell *(e.g.,* a neutrophils, monocytes, macrophages, hematopoietic stem cell, an MDSC *(e.g.,* a monocytic MDSC or a granulocytic MDSC)), or any combination thereof.

In some aspects, the EV comprises a scaffold moiety linking the exogenous targeting moiety to the EV. In some aspects, the anchoring moiety and/or the scaffold moiety is a Scaffold X. In some aspects, the anchoring moiety and/or the scaffold moiety is a Scaffold Y.

In some aspects, the Scaffold X is a scaffold protein that is capable of anchoring the ASO on the luminal surface of the EV and/or on the exterior surface of the EV.

In some aspects, the Scaffold Y is a scaffold protein that is capable of anchoring the ASO on the luminal surface of the EV and/or on the exterior surface of the EV.

In some aspects, the ASO is linked to the anchoring moiety and/or the scaffold moiety on the exterior surface of the EV. In some aspects, the ASO is linked to the anchoring moiety and/or the scaffold moiety on the luminal surface of the EV. In some aspects, the anchoring moiety comprises sterol, GM1, a lipid, a vitamin, a small molecule, a peptide, or a combination thereof. In some aspects, the anchoring moiety comprises cholesterol. In some aspects, the anchoring moiety comprises a phospholipid, a lysophospholipid, a fatty acid, a vitamin (e.g., vitamin D and/or vitamin E), or any combination thereof. In some aspects, the ASO is linked to the anchoring moiety and/or the scaffold moiety by a linker.

In some aspects, the ASO is linked to the EV by a linker. In some aspects, the linker is a polypeptide. In some aspects, the linker is a non-polypeptide moiety. In some aspects, the linker comprise ethylene glycol. In some aspects, the linker comprises HEG, TEG, PEG, or any combination thereof.

In some aspects, the linker comprises acrylic phosphoramidite (e.g., ACRYDITE^{™}), adenylation, azide (NHS Ester), digoxigenin (NHS Ester), cholesterol-TEG, I-LINKER^{™}, an amino modifier (e.g., amino modifier C6, amino modifier C12, amino modifier C6 dT, or Uni-Link^{™} amino modifier), alkyne, 5' Hexynyl, 5-Octadiynyl dU, biotinylation (e.g., biotin, biotin (Azide), biotin dT, biotin-TEG, dual biotin, PC biotin, or desthiobiotin), thiol modification (thiol modifier C3 S-S, dithiol or thiol modifier C6 S-S), or any combination thereof. In some aspects, the linker is a cleavable linker. In some aspects, the linker comprises valine-alanine-p-aminobenzylcarbamate or valine-citrulline-p-aminobenzylcarbamate. In some aspects, the linker comprises (i) a maleimide moiety and (ii) valine-alanine-p-aminobenzylcarbamate or valine-citrulline-p-aminobenzylcarbamate.

**In** some aspects, the EV is an exosome.

Certain aspects of the present disclosure, falling outside the scope of the appended claims, are directed to an antisense oligonucleotide (ASO) comprising comprises a contiguous nucleotide sequence of 10 to 30 nucleotides in length that is complementary to a nucleic acid sequence within a *STAT6* transcript (SEQ ID NO: 1 or SEQ ID NO: 3). In some aspects, the ASO is not TGAGCGAATGGACAGGTCTT (SEQ ID NO: 89). In some aspects, the ASO comprises a contiguous nucleotide sequence of 10 to 30 nucleotides in length that is complementary to a nucleic acid sequence within nucleotides 1 to 2056 of a *STAT6* transcript corresponding to a nucleotide sequence as set forth in SEQ ID NO: 3, nucleotides 2059 to 3963 of a *STAT6* transcript corresponding to a nucleotide sequence as set forth in SEQ ID NO: 3. In some aspects, the contiguous nucleotide sequence thereof is at least about 80%, at least about 85%, at least about 90%, at least about 95%, or about 100% complementary to the nucleic acid sequence within the *STAT6* transcript. In some aspects, the ASO is capable of reducing STAT6 protein expression in a human cell (*e.g.,* an immune cell), wherein the human cell expresses the STAT6 protein. In some aspects, the STAT6 protein expression is reduced by at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100% compared to STAT6 protein expression in a human cell that is not exposed to the ASO. In some aspects, the ASO is capable of reducing a level of *STAT6* mRNA in a human cell (*e.g.,* an immune cell), wherein the human cell expresses the *STAT6* mRNA. In some aspects, the level of *STAT6* mRNA is reduced by at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100% compared to the level of the *STAT6* mRNA in a human cell that is not exposed to the ASO.

In some aspects, the ASO is a gapmer, a mixmer, or a totalmer. In some aspects, the ASO comprises one or more nucleoside analogs. In some aspects, one or more of the nucleoside analogs comprises a 2'-O-alkyl-RNA; 2'-O-methyl RNA (2'-OMe); 2'-alkoxy-RNA; 2'-O-methoxyethyl-RNA (2'-MOE); 2'-amino-DNA; 2'-fluro-RNA; 2'-fluoro-DNA; arabino nucleic acid (ANA); 2'-fluoro-ANA; or bicyclic nucleoside analog (LNA). In some aspects, one or more of the nucleoside analogs is a sugar modified nucleoside. In some aspects, the sugar modified nucleoside is an affinity enhancing 2' sugar modified nucleoside. In some aspects, one or more of the nucleoside analogs comprises a nucleoside comprising a bicyclic sugar. In some aspects, one or more of the nucleoside analogs comprises an LNA. In some aspects, the LNA is selected from the group consisting of constrained ethyl nucleoside (cEt), 2',4'-constrained 2'-O-methoxyethyl (cMOE), α-L-LNA, β-D-LNA, 2'-O,4'-C-ethylene-bridged nucleic acids (ENA), amino-LNA, oxy-LNA, thio-LNA, and any combination thereof. In some aspects, the ASO comprises one or more 5'-methyl-cytosine nucleobases.

In some aspects, the ASO comprises any one of SEQ ID NO: 91 to SEQ ID NO: 193. In some aspects, the ASO has a design selected from the group consisting of the designs in FIG. 1, wherein the upper letter is a sugar modified nucleoside and the lower case letter is DNA. In some aspects, the ASO is from 14 to 20 nucleotides in length.

In some aspects, the contiguous nucleotide sequence comprises one or more modified internucleoside linkages. In some aspects, the one or more modified internucleoside linkages is a phosphorothioate linkage. In some aspects, at least 75%, at least 80%, at least 85%, at least 90%, at least 95%, or 100% of internucleoside linkages are modified. In some aspects, each of the internucleoside linkages in the ASO is a phosphorothioate linkage.

Certain aspects of the present disclosure, falling outside of the scope of the appended claims, are directed to a conjugate comprising an ASO described herein covalently attached to at least one non-nucleotide or non-polynucleotide moiety. In some aspects, the non-nucleotide or non-polynucleotide moiety comprises a protein, a fatty acid chain, a sugar residue, a glycoprotein, a polymer, or any combinations thereof.

Certain aspects of the present disclosure are directed to an extracellular vesicle comprising an ASO as defined in the claims.

Certain aspects of the present disclosure are directed to a pharmaceutical composition comprising an extracellular vesicle , and a pharmaceutically acceptable diluent, carrier, salt, or adjuvant as defined in the claims.

In some aspects, the pharmaceutically acceptable salt comprises a sodium salt, a potassium salt, an ammonium salt, or any combination thereof. In some aspects, the pharmaceutical composition further comprises at least one additional therapeutic agent.

In some aspects, the additional therapeutic agent is an STAT6 antagonist. In some aspects, the STAT6 antagonist is a chemical compound, an siRNA, an shRNA, an antisense oligonucleotide, a protein, or any combination thereof. In some aspects, the STAT6 antagonist is an anti-STAT6 antibody, or a fragment thereof. In some aspects, the STAT6 antagonist comprises an antisense oligonucleotide (ASO).

Certain aspects of the present disclosure, falling outside of the scope of the appended claims, are directed to a kit comprising an extracellular vesicle disclosed herein, an ASO disclosed herein, a conjugate disclosed herein, or a pharmaceutical composition disclosed herein, and instructions for use.

Certain aspects of the present disclosure, falling outside of the scope of the appended claims, are directed to a diagnostic kit comprising an extracellular vesicle disclosed herein, an ASO disclosed herein, a conjugate disclosed herein, or a pharmaceutical composition disclosed herein, and instructions for use.

Certain aspects of the present disclosure, falling outside of the scope of the appended claims, are directed to a method of inhibiting or reducing STAT6 protein expression in a cell, comprising administering an extracellular vesicle disclosed herein, an ASO disclosed herein, a conjugate disclosed herein, or a pharmaceutical composition disclosed herein to the cell expressing STAT6 protein, wherein the STAT6 protein expression in the cell is inhibited or reduced after the administration.

Certain aspects of the present disclosure are directed to an extracellular vesicle or a pharmaceutical composition disclosed as defined in the claims, for use in the treatment of a cancer.

Certain aspects of the present disclosure, falling outside the scope of the appended claims are directed to a method of treating a disease or disorder in a subject in need thereof, comprising administering an effective amount of an extracellular vesicle disclosed herein, an ASO disclosed herein, a conjugate disclosed herein, or a pharmaceutical composition disclosed herein to the subject, wherein the disease or disorder is selected from a fibrosis, an inflammation, a neurodegenerative disease, a metabolic disorder/CVD, and any combination thereof.

Certain aspects of the present disclosure, falling outside the scope of the appended claims are directed to a use of an extracellular vesicle disclosed herein, an ASO disclosed herein, a conjugate disclosed herein, or a pharmaceutical composition disclosed herein in the manufacture of a medicament for the treatment of a disease or disorder in a subject in need thereof, wherein the disease or disorder is selected from a fibrosis, an inflammation, a neurodegenerative disease, a metabolic disorder/CVD, and any combination thereof.

In some aspects, the ASO inhibits or reduces expression of *STAT6* mRNA in the cell after the administration as defined in the claims. In some aspects, a level of *STAT6* mRNA is reduced by at least about 20%, at least about 30%, at least about 40%, at least about 50%, at least about 60%, at least about 70%, at least about 80%, at least about 90%, or about 100% after the administration compared to the level of *STAT6* mRNA in a cell not exposed to the ASO. In some aspects, the expression of STAT6 protein is reduced by at least about 60%, at least about 70%, at least about 75%, at least about 80%, at least about 85%, at least about 90%, at least about 95%, at least about 96%, at least about 97%, at least about 98%, at least about 99%, or about 100% after the administration compared to the expression of STAT6 protein in a cell not exposed to the ASO.

In some aspects, the extracellular vesicle or the pharmaceutical composition is administered intracardially, orally, parenterally, intrathecally, intra-cerebroventricularly, pulmorarily, topically, or intraventricularly.

In some aspects, the cancer is selected from the group consisting of fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell cancer, squamous cell cancer of the head and neck cancer, colorectal cancer, lymphoma, leukemia, liver cancer, glioblastoma, melanoma, myeloma basal cell cancer, adenocarcinoma, sweat gland cancer, sebaceous gland cancer, papillary cancer, papillary adenocarcinomas, cystadenocarcinoma, medullary cancer, bronchogenic cancer, renal cell cancer, hepatoma, bile duct cancer, choriocarcinoma, seminoma, embryonal cancer, Wilms' tumor, cervical cancer, testicular cancer, lung cancer, small cell lung cancer, bladder cancer, epithelial cancer, glioma, glioblastoma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, follicular lymphoma, Hodgkin's lymphoma, B cell lymphoma, colon adenocarcinoma, rectum adenocarcinoma, pancreatic adenocarcinoma, ovarian serous cystadenocarcinoma, acute myelid leukemia, testicular germ cell tumors, lung adenocarcinoma, brain lower grade glioma, glioblastoma multiforme, uveal melanoma, thyroid carcinoma, uterine corpus endometrial carcinoma, uterine carcinosarcoma, pheochromocytoma, paraganglioma, and any combination thereof.

In some aspects falling outside the scope of the appended claims, the disease or disorder comprises a fibrosis. In some aspects, the disease or disorder comprises a fibrosis selected from the group consisting of liver fibrosis (NASH), cirrhosis, pulmonary fibrosis, cystic fibrosis, chronic ulcerative colitis/IBD, bladder fibrosis, kidney fibrosis, CAPS (Muckle-Wells syndrome), atrial fibrosis, endomyocardial fibrosis, old myocardial infarction, glial scar, arterial stiffness, arthrofibrosis, Crohn's disease, Dupuytren's contracture, keloid fibrosis, mediastinal fibrosis, myelofibrosis, Peyronie's disease, nephrogenic systemic fibrosis, progressive massive fibrosis, retroperitoneal fibrosis, scleroderma/systemic sclerosis, adhesive capsulitis, and any combination thereof.

Certain aspects of the present disclosure, falling outside the scope of the appended claims, are directed to a method of activating meningeal macrophages, treating a cancer of the central nervous system, inducing M1 polarization of meningeal macrophages, inducing meningeal macrophage infiltration of a tumor, and/or in a subject in need thereof, comprising administering an extracellular vesicle described herein, an ASO described herein, a conjugate described herein, or a pharmaceutical composition described herein.

### BRIEF DESCRIPTION OF FIGURES

FIG. 1 is a table listing various ASO sequences that target the *STAT6* transcript. The tables include the following information (from left to right): (i) description of the ASO, (ii) the ASO sequence without any particular design or chemical structure, (iii) SEQ ID number designated for the ASO sequence only, (iv) the ASO length, (v) the ASO sequence with a chemical structure, and (vi) the target start and end positions on the target transcript sequence (SEQ ID NO: 3). The ASOs are from 5' to 3'. The symbols in the chemical structures are as follows: Nb means LNA; dN means DNA; 5MdC means 5-Methyl-dC; Nm means MOE; and s means phosphorothioate.

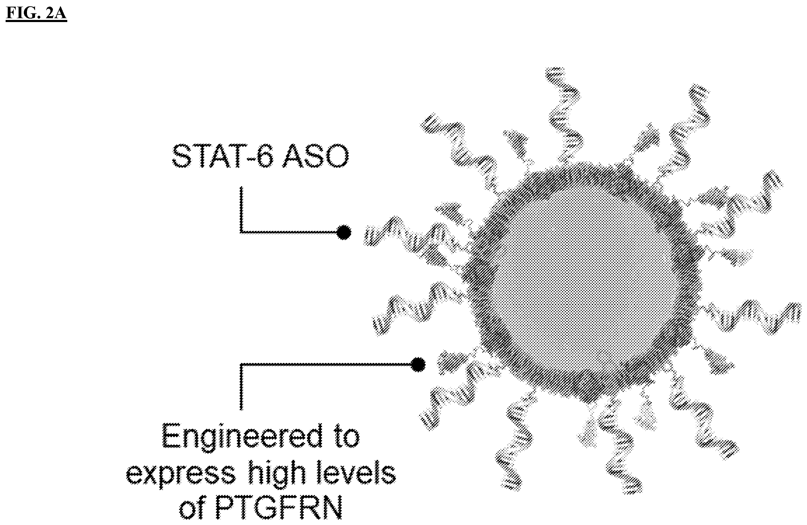

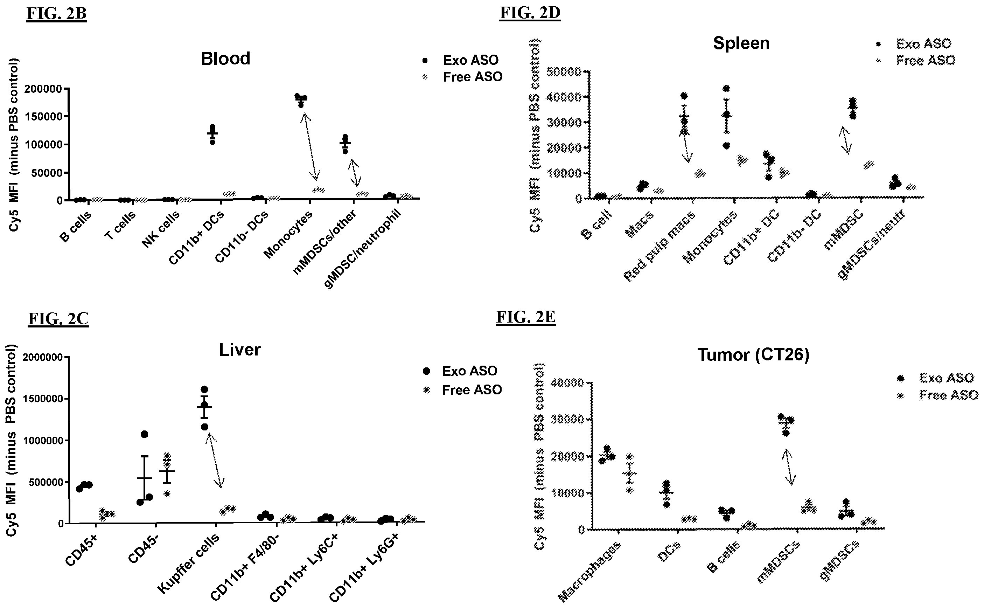

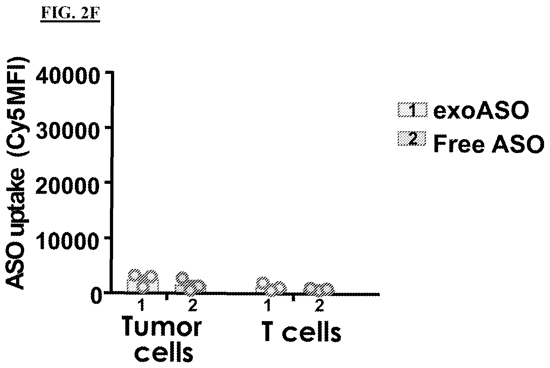

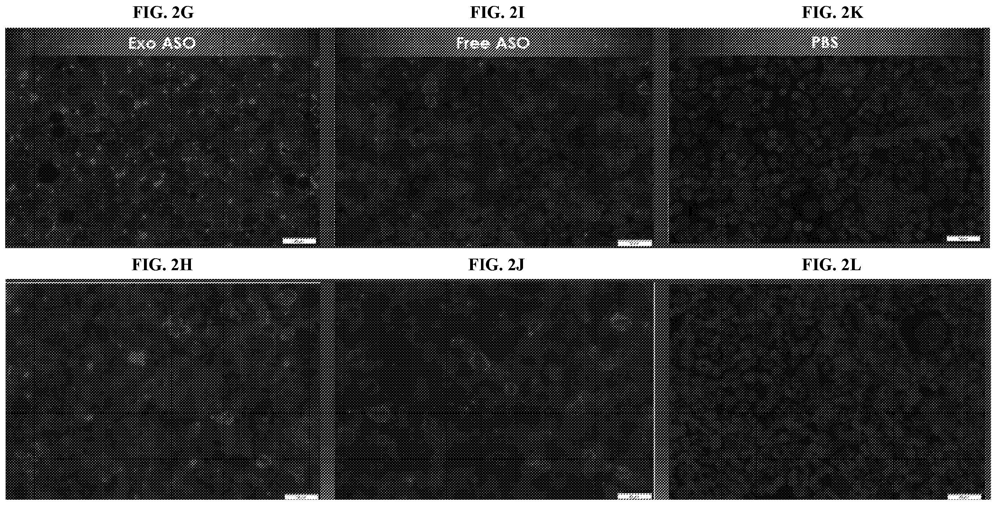

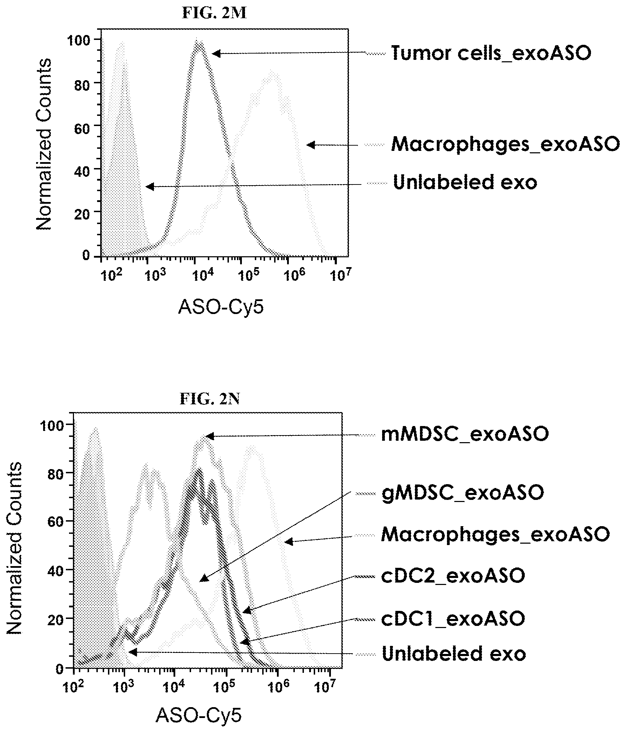

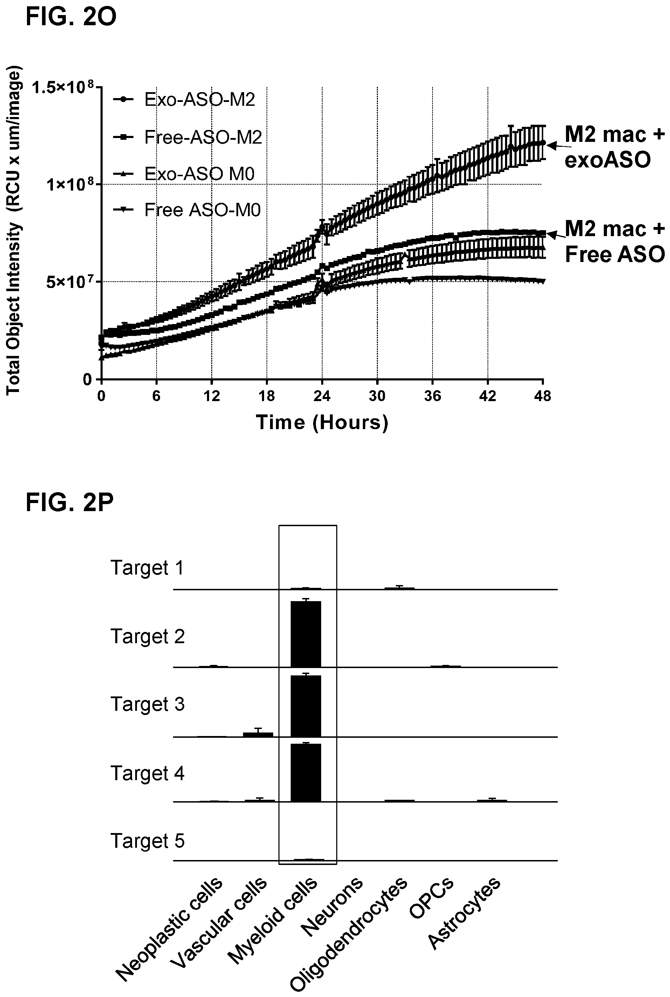

FIG. 2A is a schematic drawing of an exosome overexpressing PTGFRN loaded with ASOs that target STAT6. FIGs. 2B-2E are graphical representations of Cy5 levels, as detected by fluorescence (MFI) and normalized to PBS controls. Cy5 is used as a marker of uptake of exosomes comprising ASOs ("Exo ASO"; left) or free ASOs (right), as indicated, in various cell types isolated from the blood (FIG. 2B), liver (FIG. 2C), spleen (FIG. 2D), and a tumor (CT26; FIGs. 2E-2F). Horizontal lines indicate the average MFI. FIGs. 2G-2L are fluorescent images of bone marrow tissue samples taken from two mice each, showing uptake of exosomes comprising ASOs (FIGs. 2G-2H) or free ASO (FIGs. 2I-2J), as compared to PBS negative controls (FIGs. 2K-2L). FIG. 2M is a graphical representation of the results of flow cytometry to quantify the number of Cy5 labeled tumor cells and macrophages after intratumoral administration of fluorescently labeled exoASO. FIG. 2N is a graphical representation of the results of flow cytometry to quantify the number of Cy5 labeled myeloid-derived suppressor cells, macrophages, and dendritic cells after intratumoral administration of fluorescently labeled exoASO. MDSC = myeloid-derived suppressor cells; mMDSC = monocytic MDSC; gMDSC = granulocytic MDSC; cDC1 = type 1 conventional dendritic cells; cDC2 = type 2 conventional dendritic cells. FIG. 2O is a graphical representation of the uptake of Cy5 labeled ASO, either free, or loaded on exosomes (exoASO) in M2 or M0 primary human macrophages. FIG. 2P is graphical representation of the expression level of PTGFRN cognate receptors in glioblastoma (GBM) in various cell types across five targets.

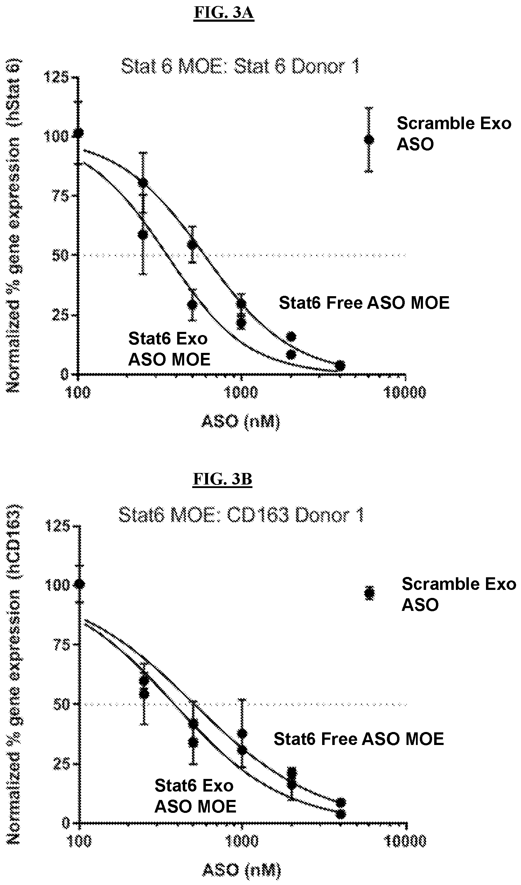

FIGs. 3A and 3B are graphical representations of the normalized gene expression (%) of *STAT6* (FIG. 3A) and *CD163* (FIG. 3B) in polarized macrophages following treatment with STAT6 Exo ASO, STAT6 free ASO, or a scrambled Exo ASO (negative control), as indicated (FIGs. 3A-3B).

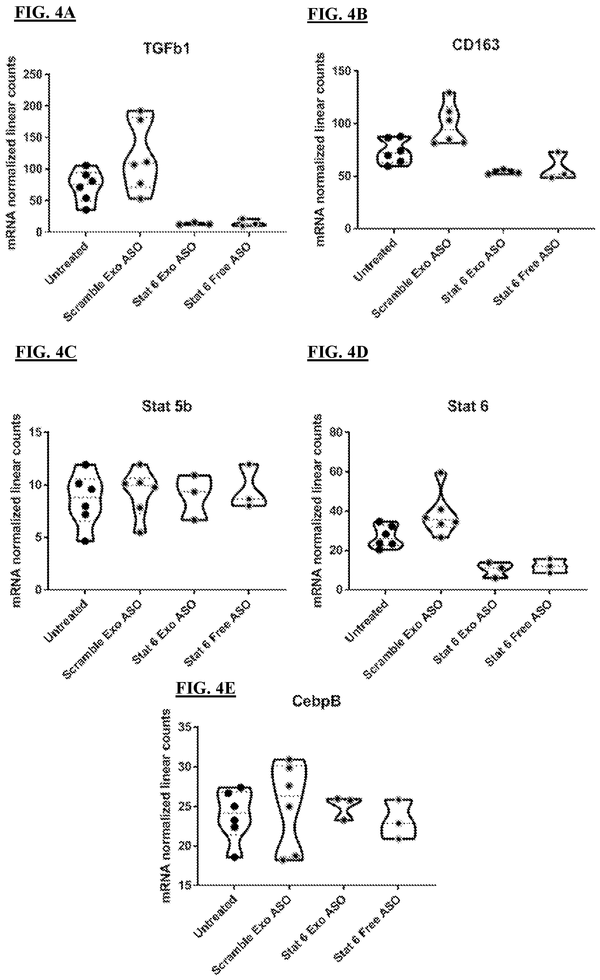

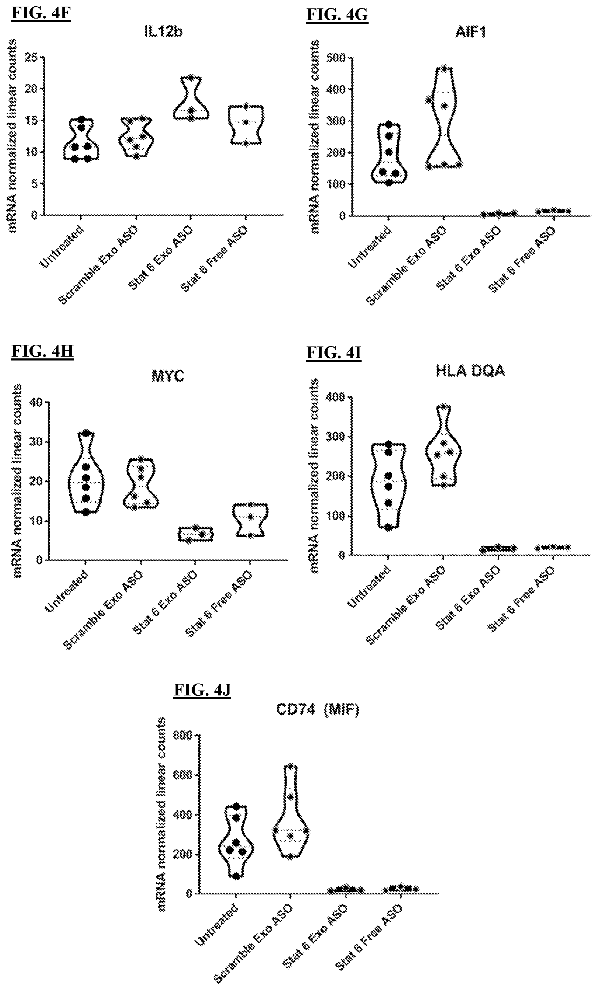

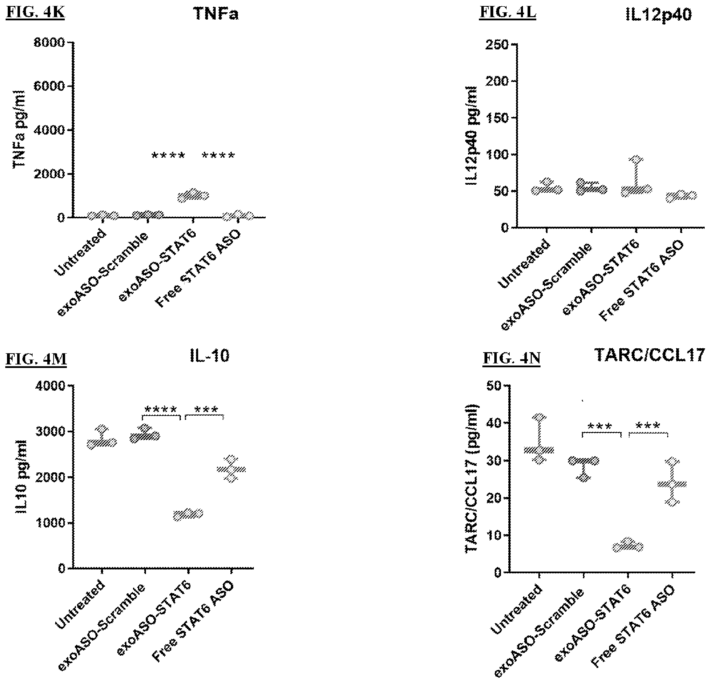

FIGs. 4A-4J are graphical representations of the expression of *TGFβ1* (FIG. 4A), *CD163* (FIG. 4B), *STAT5b* (FIG. 4C), *STAT6* (FIG. 4D), *CEBP*/*β* (FIG. 4E), *IL12β* (FIG. 4F),*AIF1* (FIG. 4G), *MYC* (FIG. 4H), *HLA DQA* (FIG. 4I), *CD74 (MIF)* (FIG. 4J), TNF-α (FIG. 4K), IL12p40 (FIG. 4L), IL-10 (FIG. 4M), and TARC/CCL17 (FIG. 4N) in primary human macrophages untreated or treated with scramble Exo ASO, STAT6-Exo-ASO, or STAT-6 free ASO, as indicated. *** = p<0.001; and **** = P< 0.0001.

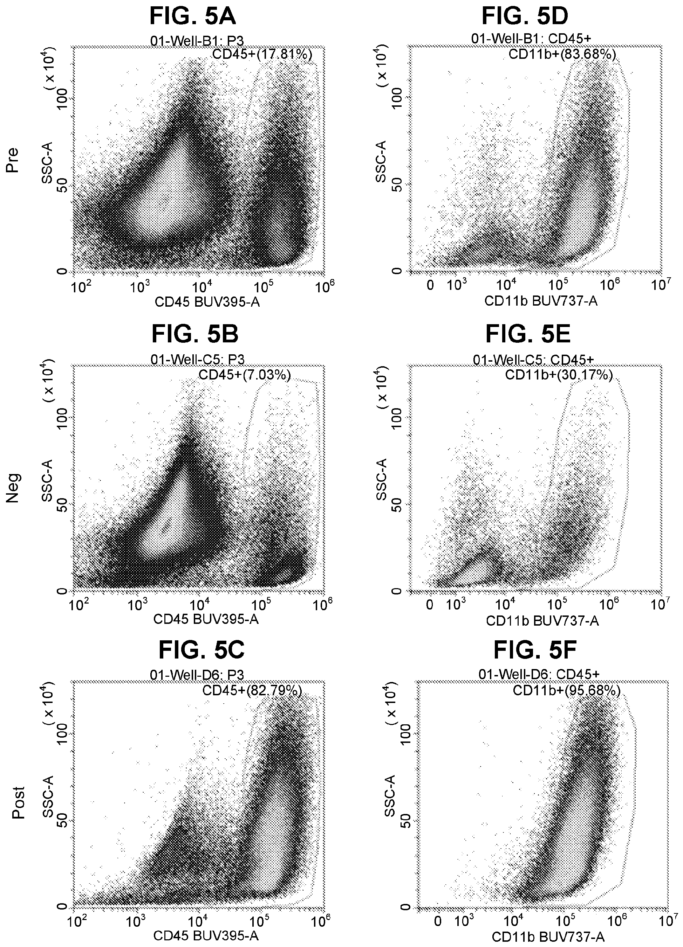

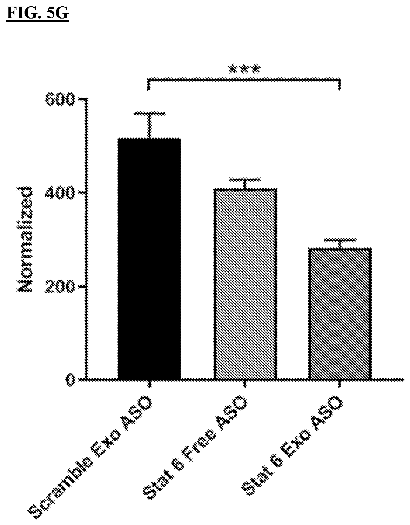

FIGs. 5A-5F are graphical representations of the results of flow cytometry to isolate CD11b cells. FIGs. 5A-5C show CD45 expression pre-treatment (FIG. 5A), following treatment with a negative control (scramble Exo ASO; FIG. 5B), or post-treatment with an Exo-ASO (FIG. 5C). FIGs. 5D-5F show CD11b expression pre-treatment (FIG. 5D), following treatment with a negative control (scramble Exo ASO; FIG. 5E), or post-treatment with an Exo-ASO (FIG. 5F). FIG. 5G is a graphical representation of tumor volume in mice following treatment with a negative control (scramble Exo ASO), or post-treatment with an Exo-ASO.

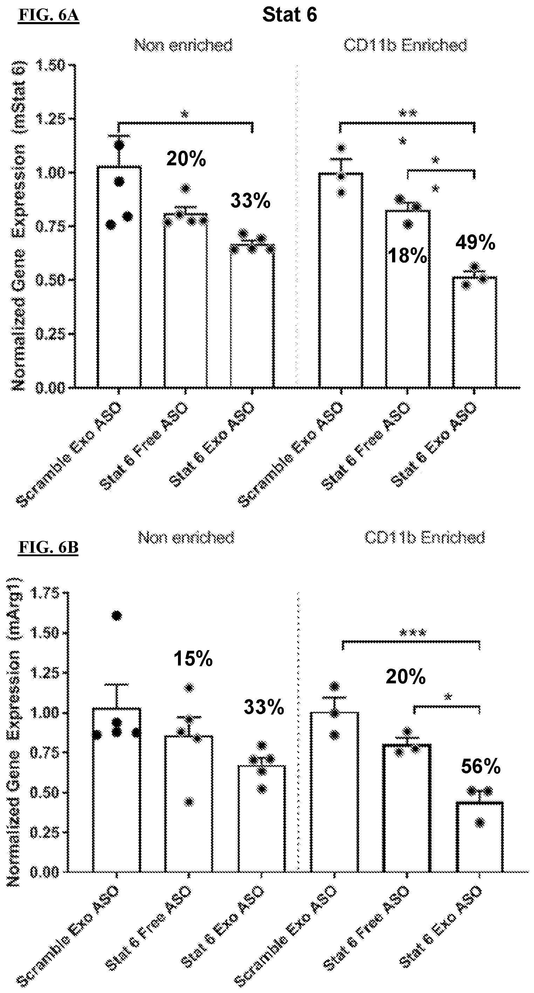

FIGs. 6A and 6B are graphical representations of the expression of *STAT6* (FIG. 6A) and *ARG1* (FIG. 6B) in CD11b-enriched cells as compared to non-enriched cells following exposure to scramble Exo-ASO, STAT6 free ASO, or STAT6-Exo-ASO (FIGs. 6A and 6B, as indicated).

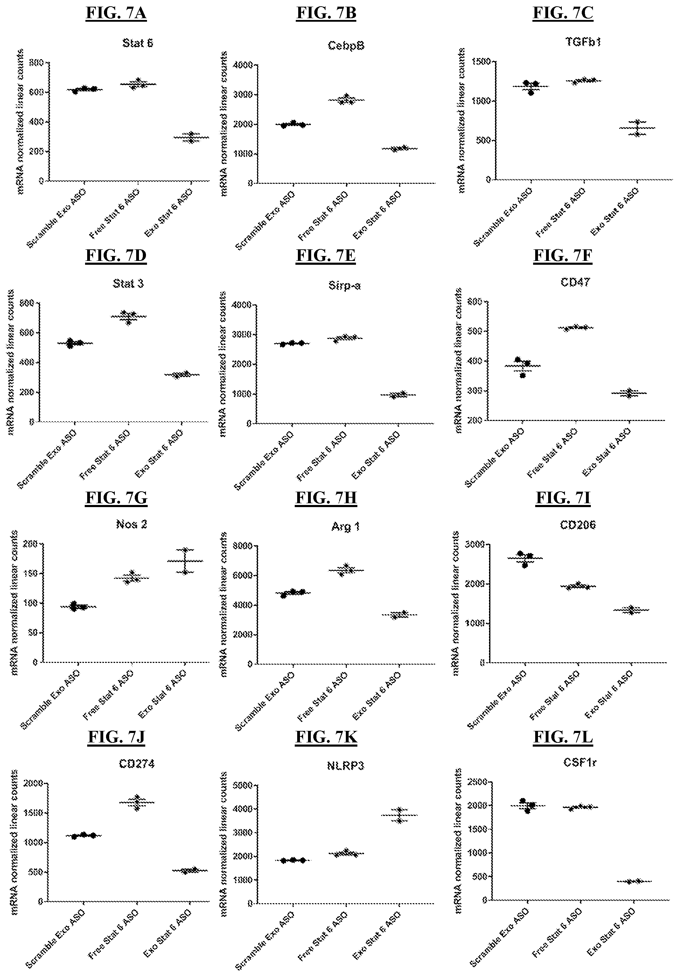

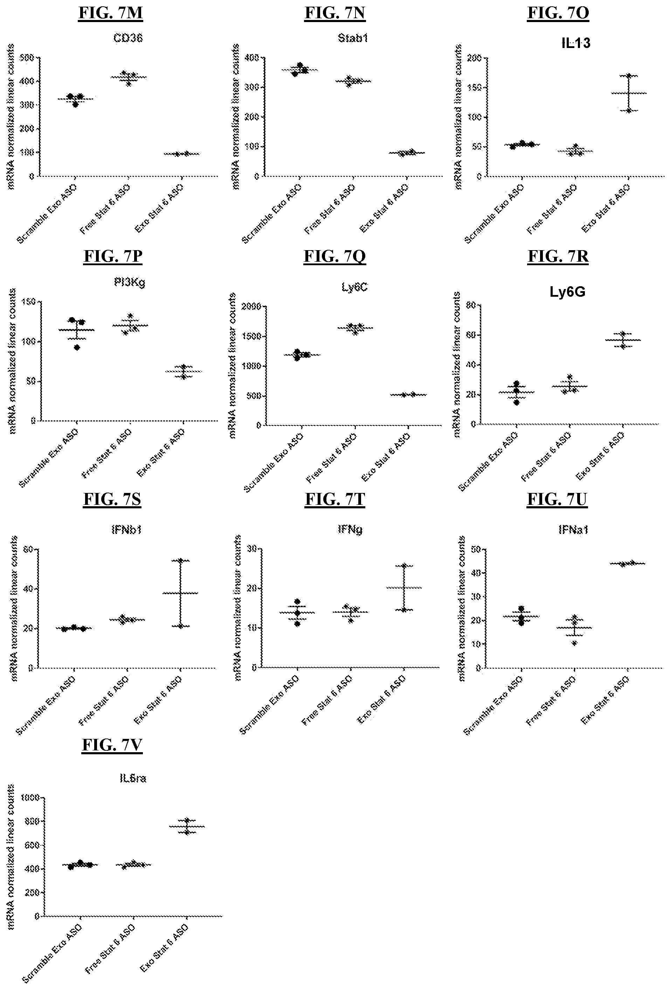

FIGs. 7A-7V are graphical representations of the expression of *STAT6* (FIG. 7A), *CEBP*/*β* (FIG. 7B), *TGFβ1* (FIG. 7C), *STAT3* (FIG. 7D), *SIRP-α* (FIG. 7E), *CD47* (FIG. 7F), *NOS2* (FIG. 7G), *ARG1* (FIG. 7H), *CD206* (FIG. 7I), *CD274* (FIG. 7J), *NLRP3* (FIG. 7K), *CSF1R* (FIG. *7L**), CD36* (FIG. 7M), *STAB1* (FIG. 7N), *IL13* (FIG. 7O), *PI3KG* (FIG. 7P), *LY6C* (FIG. 7Q), *LY6G* (FIG. 7R), *IFNβ1* (FIG. 7S), *IFNγ* (FIG. 7T), *IFNα1* (FIG. 7U), and *IL6Rα* (FIG. 7V) in CD11b-enriched cells treated with scramble Exo ASO, STAT6-Exo-ASO, or STAT-6 free ASO, as indicated.

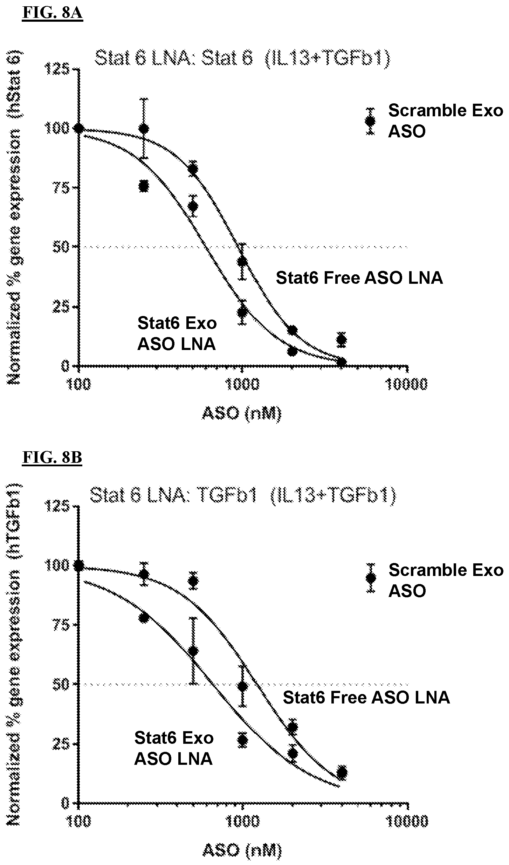

FIGs. 8A and 8B are graphical representations of the normalized gene expression (%) of *STAT6* (FIG. 8A) and *TGFβ1* (FIG. 8B) in primary human M2 macrophages that were polarized with IL-13/TGFβ treatment subsequently treated with STAT6 Exo ASO, STAT6 free ASO, or a scrambled Exo ASO (negative control), as indicated.

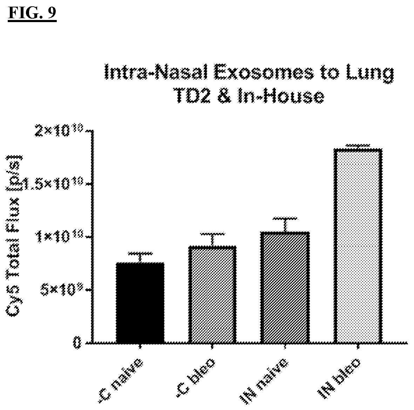

FIG. 9 is a graphical representation of exosome uptake, as evidenced by Cy5 levels, in Lung TD2 following nasal administration of a negative control (-C) or Exo-ASO-Cy5 ("IN") to naïve mice or mice were treated with bleomycin to induce pulmonary fibrosis ("bleo").

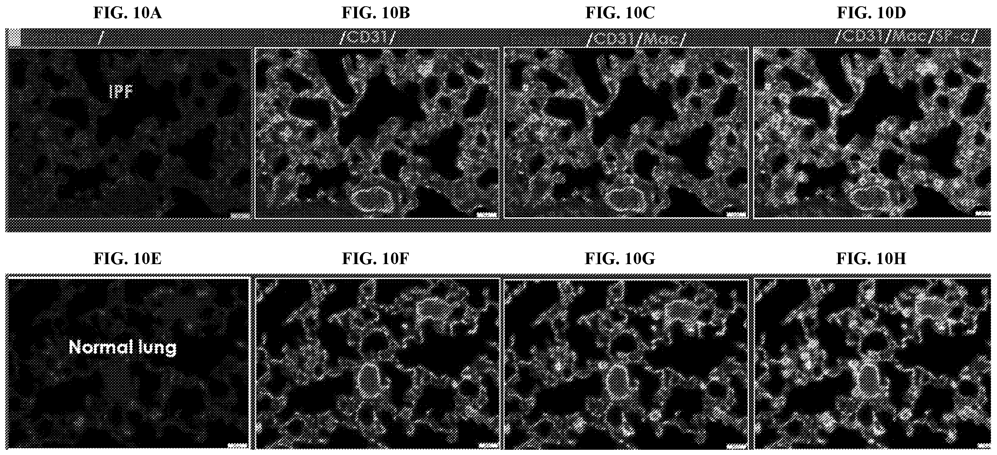

FIGs. 10A-10H are images of fluorescent *in situ* hybridization to detect exosome uptake by normal and induced fibrotic lung tissue.

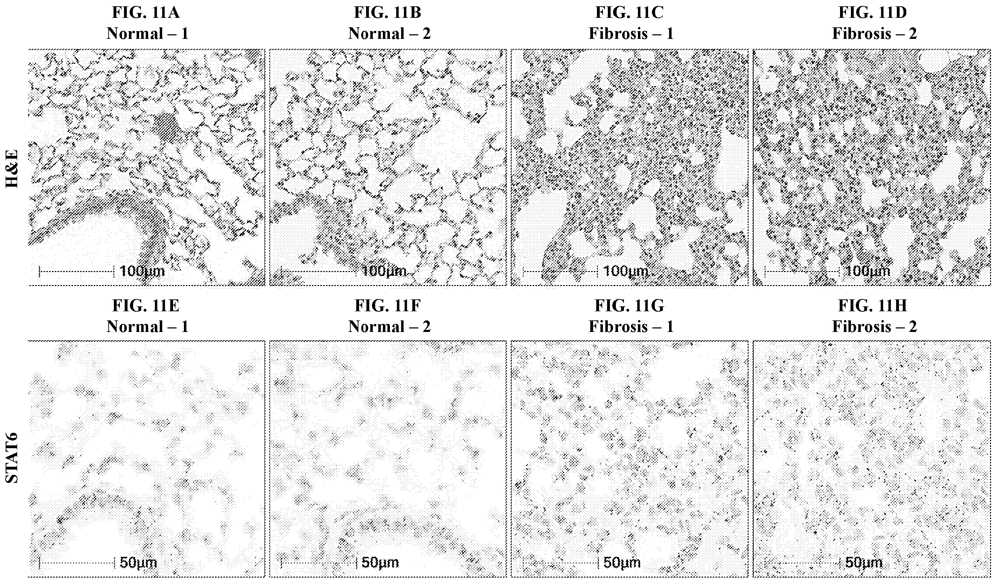

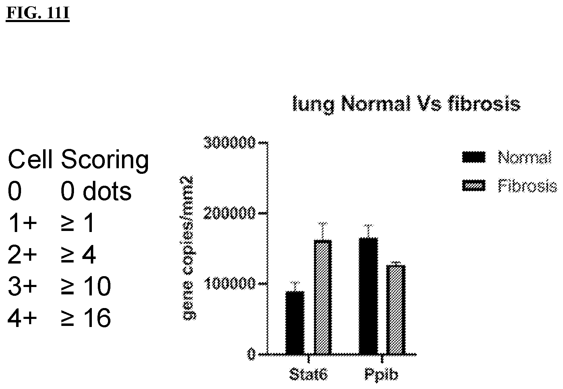

FIGs. 11A-11H are images of *in situ* hybridization to detect exosome uptake by normal and induced fibrotic lung tissue. FIG. 11I is a graphical representation showing the level of saturation in the *in situ* hybridization images, indicating the level of exosome uptake in normal and fibrotic tissue.

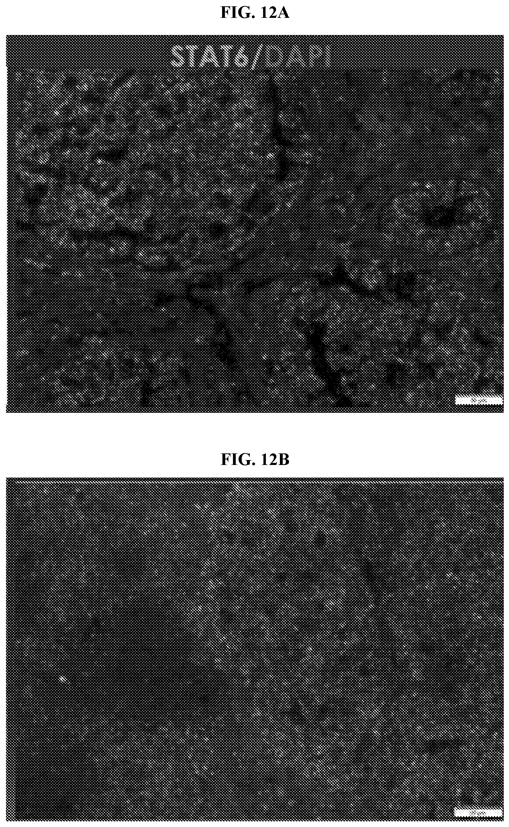

FIGs. 12A and 12B are images of fluorescent *in situ* hybridization to detect exosome uptake by lung tissue in Hepa1-6 mice.

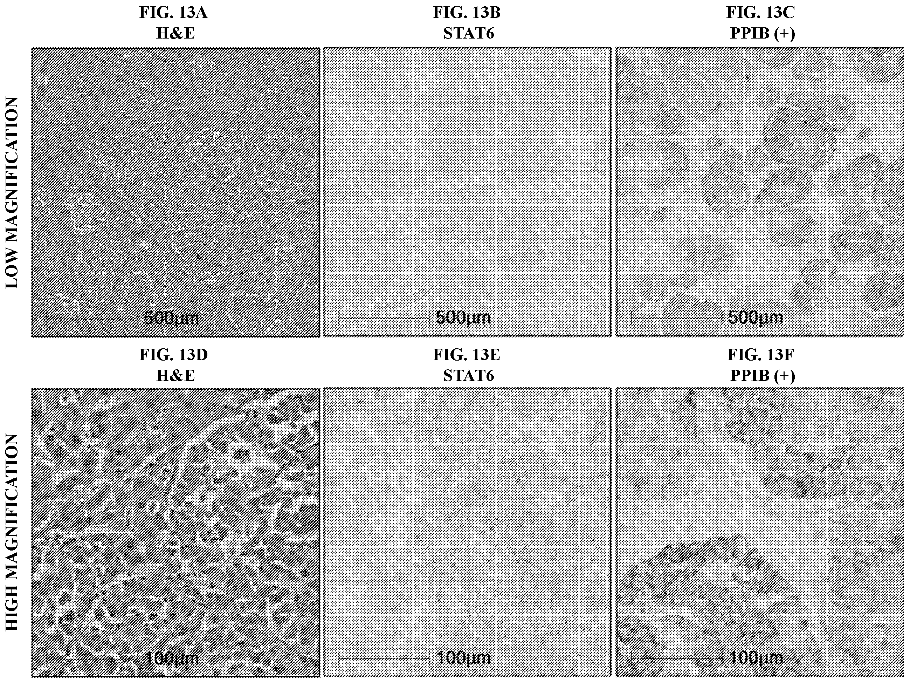

FIGs. 13A-13F are images of *in situ* hybridization to detect exosome uptake by lung tissue in Hepa1-6 mice.

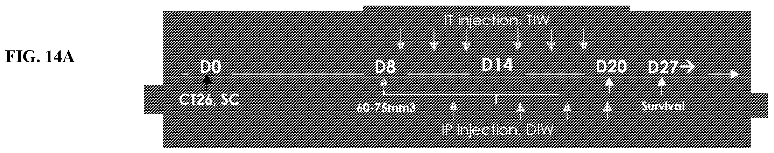

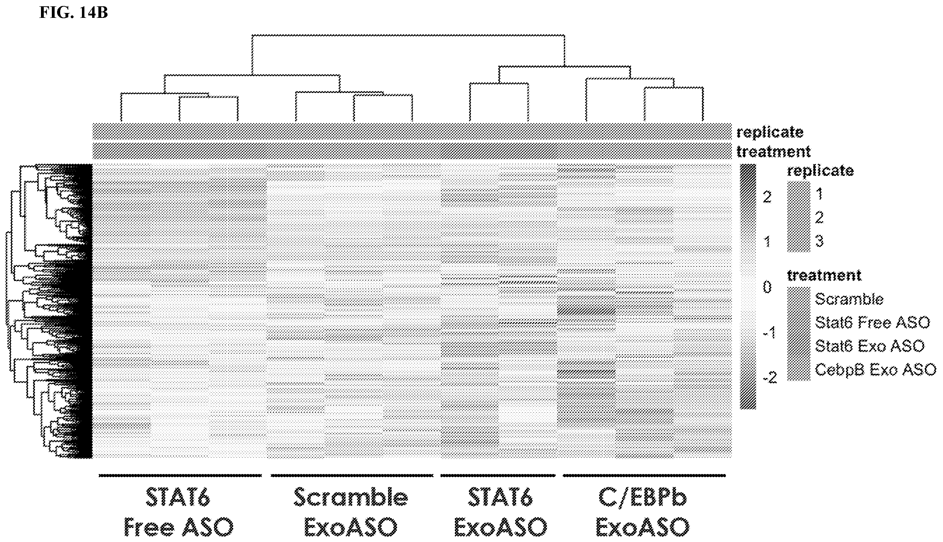

FIGs. 14A and 14B show that STAT6 exoASO repolarizes M2 macrophages and has anti-tumor activity. FIG. 14A shows the timeline of the STAT6 exoASO administration. FIG. 14B shows that administration of the STAT6 exoASOs decreases expression of M2 genes and increases expression of M1 genes.

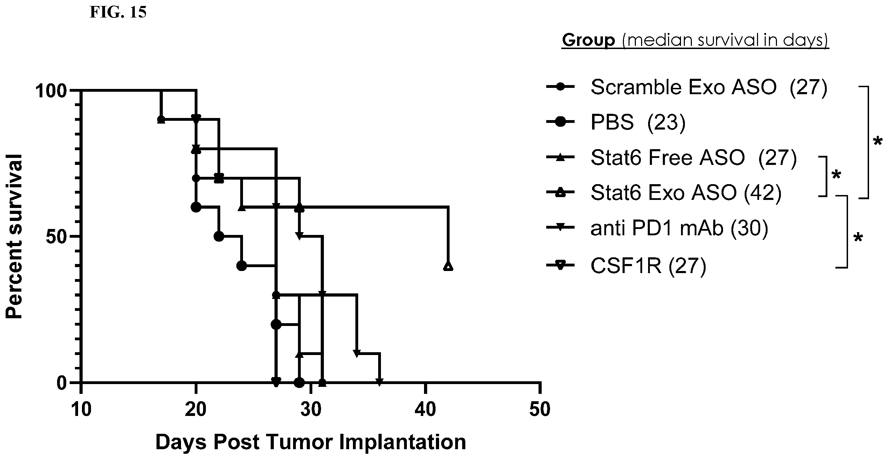

FIG. 15 shows that STAT6 exoASO monotherapy results in 50% complete response and 80% tumor growth inhibition. STAT6 free ASO did not have any effect on tumor growth. Anti-CSF1R antibody (macrophage depleting therapy) did not induce any anti tumor efficacy.

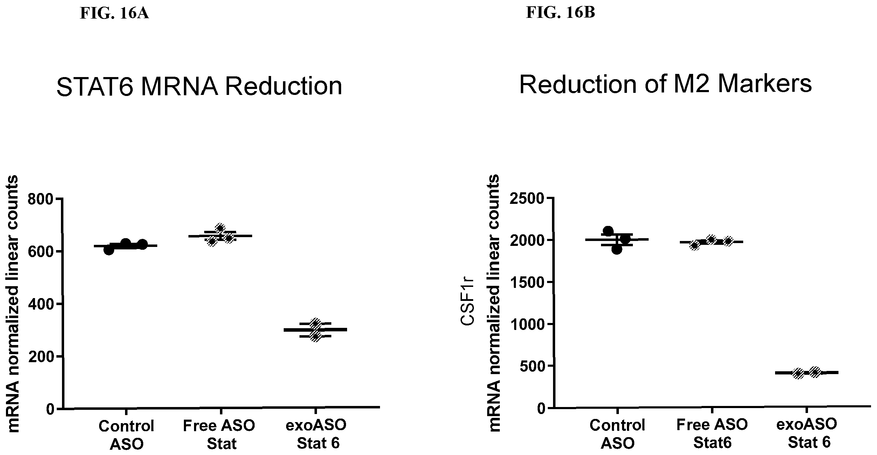

FIG. 16A shows STAT6 mRNA reduction by control ASO, free ASO STAT, and STAT6 ExoASO.
FIG. 16B shows reduction of M2 markers, i.e., CSF1R, by control ASO, free ASO STAT6, and STAT6 ExoASO.

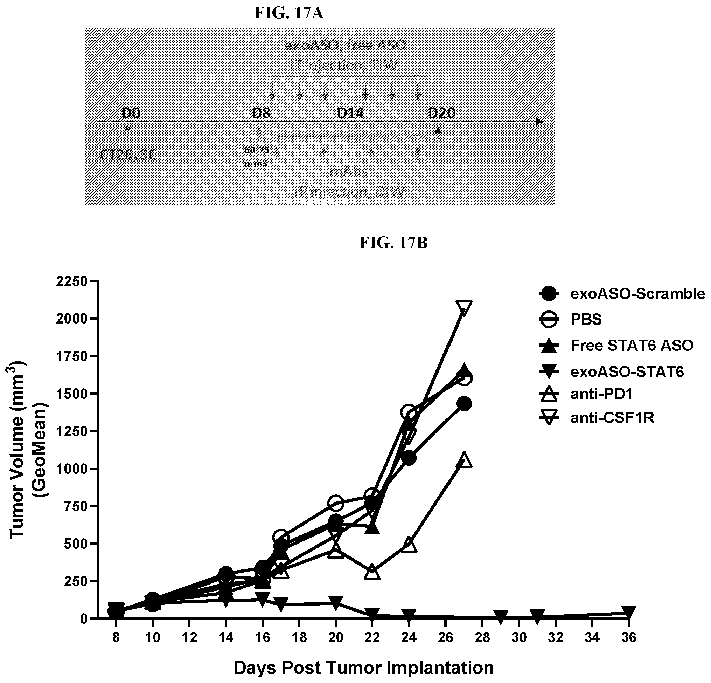

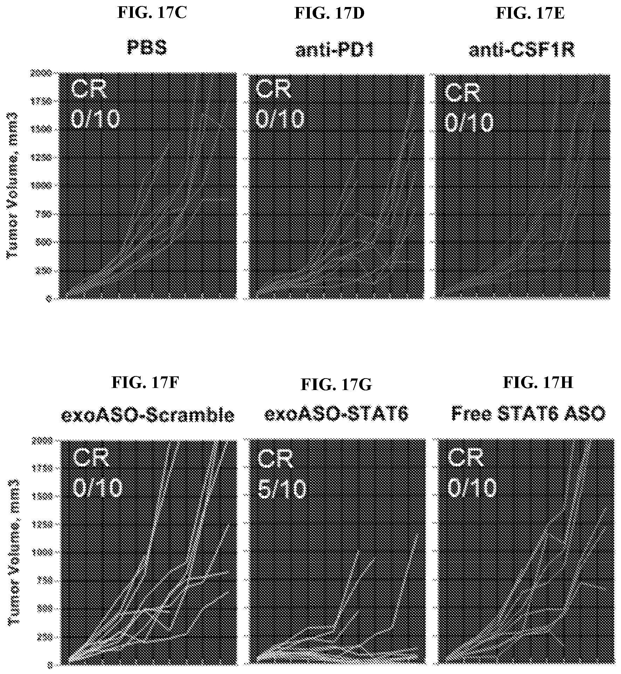

FIGs. 17A-17H show the anti-tumor activity of STAT6 exoASO compared to free STAT6 ASO, anti-PD1 antibodies, or anti-CSF1R antibodies. FIG. 17A shows the timeline of STAT6 exoASO, free STAT ASO, anti-PD1 antibody, and anti-CSF1R antibody administration. FIG. 17B shows the average tumor growth in mice treated with either STAT6 exoASO, free STAT6 ASO, an anti-PD1 antibody, or an anti-CSF1R antibody. FIGs. 17C-17H show individual tumor growth in mice treated with STAT6 exoASO (FIG. 17G), free STAT6 ASO (FIG. 17H), a PBS control (FIG. 17C), an anti-PD1 antibody (FIG. 17D), an anti-CSF1R antibody (FIG. 17E), or an exoASO-scramble negative control (FIG. 17F). CR = number of complete responders.

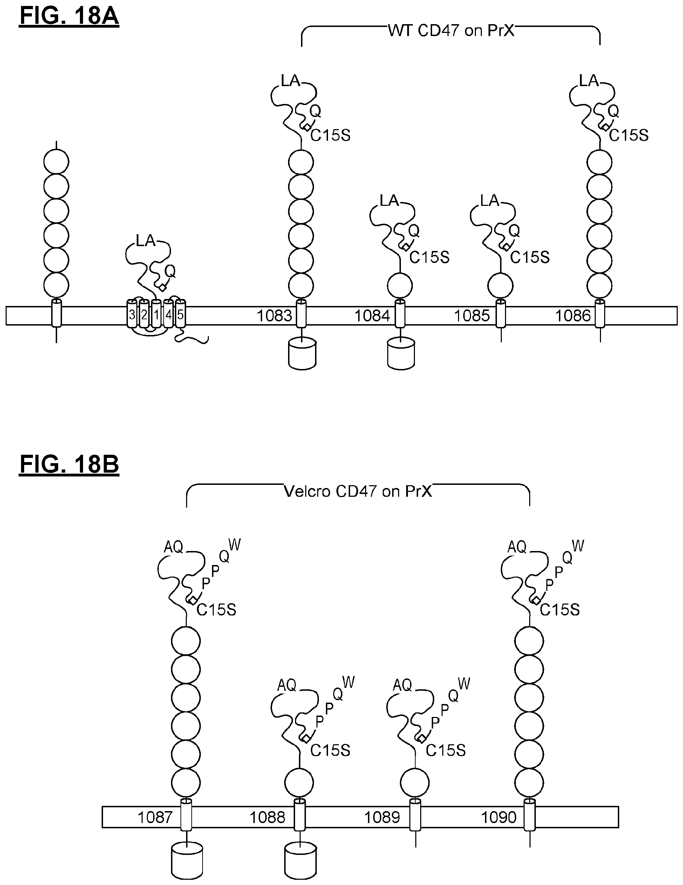

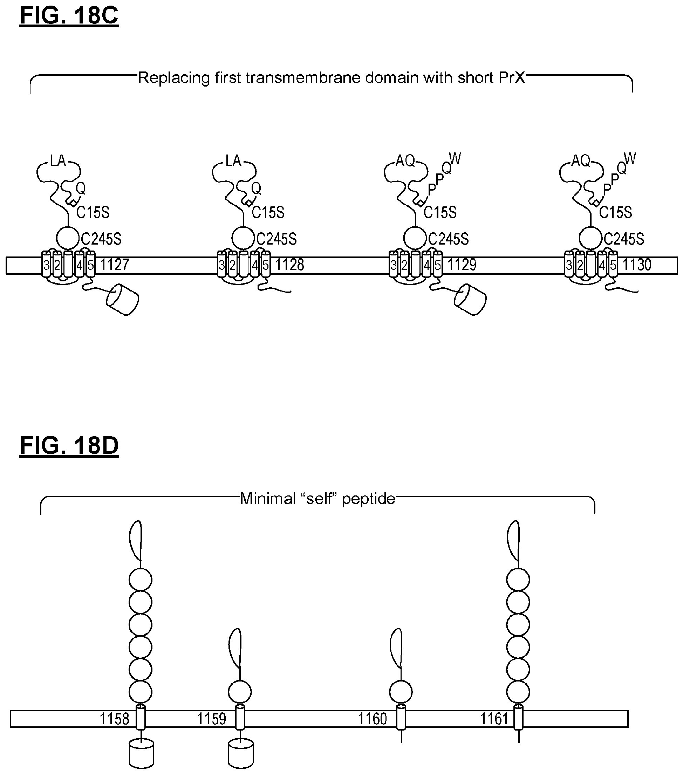

FIGs. 18A-18D are schematic drawings of exemplary CD47-Scaffold X fusion constructs that can be expressed on the extracellular vesicles described herein, along with an ASO targeting a *STAT6* transcript. FIG. 18A shows constructs comprising the extracellular domain of wild-type CD47 (with a C15S substitution) fused to either a flag-tagged (1083 and 1084) or non-flag-tagged (1085 and 1086) full length Scaffold X (1083 and 1086) or a truncated Scaffold X (1084 and 1085). FIG. 18B shows constructs comprising the extracellular domain of Velcro-CD47 fused to either a flag-tagged (1087 and 1088) or non-flag-tagged (1089 and 1090) full length Scaffold X (1087 and 1090) or a truncated Scaffold X (1088 and 1089). FIG. 18C shows constructs wherein the first transmembrane domain of wild-type CD47 (with a C15S substitution; 1127 and 1128) or Velcro-CD47 (1129 and 1130) is replaced with a fragment of Scaffold X, comprising the transmembrane domain and the first extracellular motif of Scaffold X. FIG. 18D shows various constructs comprising a minimal "self" peptide (GNYTCEVTELTREGETIIELK; SEQ ID NO: 628) fused to either a flag-tagged (1158 and 1159) or non-flag-tagged (1160 and 1161) full length Scaffold X (1158 and 1161) or a truncated Scaffold X (1159 and 1160).

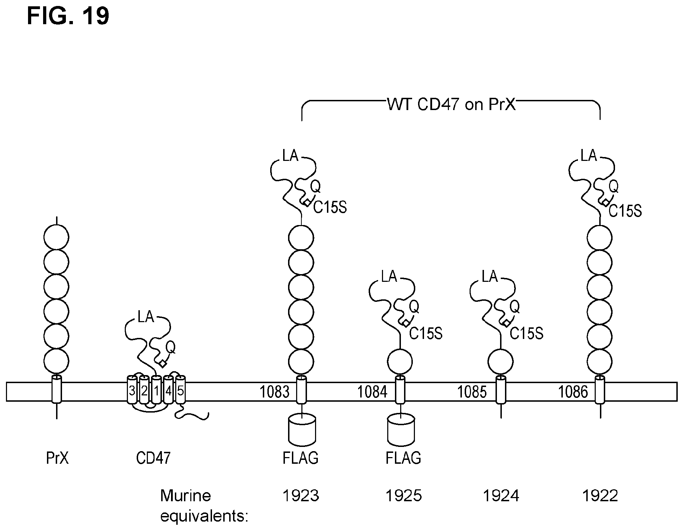

FIG. 19 shows the expression of exemplary mouse CD47-Scaffold X fusion constructs that can be expressed on the surface of modified exosomes, along with an ASO targeting *a STAT6* transcript. The constructs comprises the extracellular domain of wild-type murine CD47 (with a C15S substitution) fused to either a flag-tagged (1923 and 1925) or non-flag-tagged (1924 and 1922) full length Scaffold X (1923 and 1922) or a truncated Scaffold X (1925 and 1924).

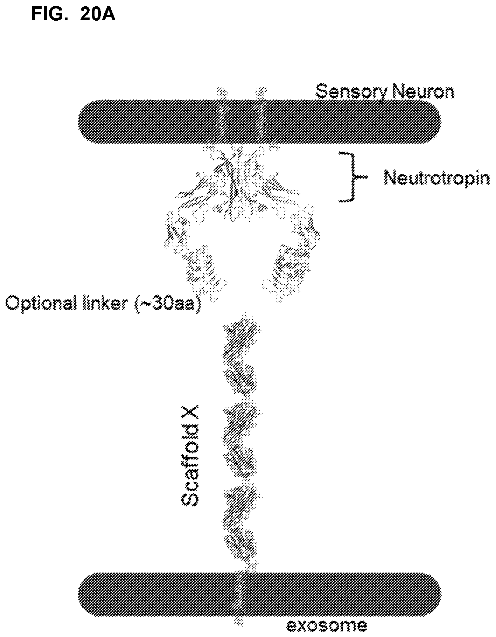

FIG. 20A shows a schematic diagram of exemplary extracellular vesicle (e.g., exosome) targeting Trks using neurotrophin-Scaffold X fusion construct that can be expressed along with an ASO targeting a *STAT6* transcript. Neurotrophins bind to Trk receptors as a homo dimer and allow the EV to target a sensory neuron.

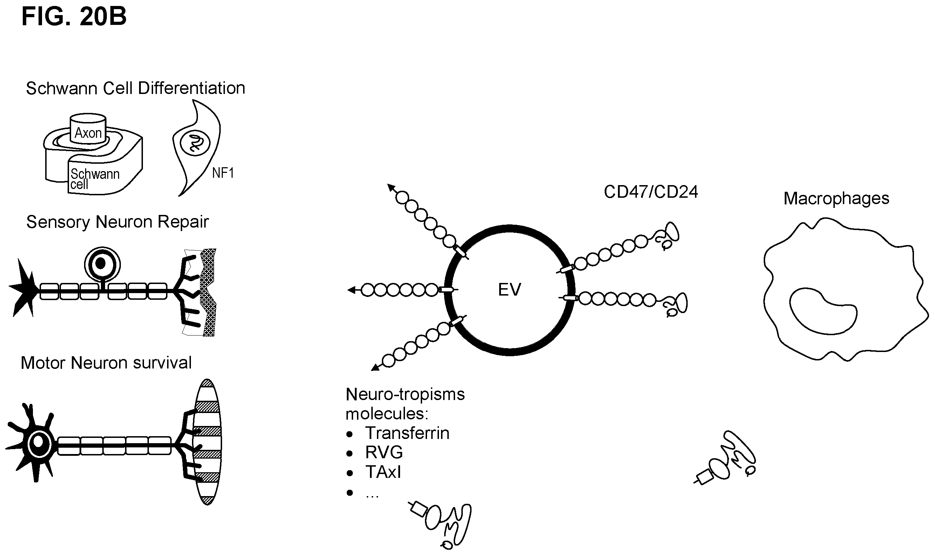

FIG. 20B shows a schematic diagram of exemplary extracellular vesicle (e.g., exosome) having (i) neuro-tropism as well as (ii) an anti-phagocytic signal, e.g., CD47 and/or CD24, on the exterior surface of the EV that can be expressed along with (iii) an ASO targeting a *STAT6* transcript.

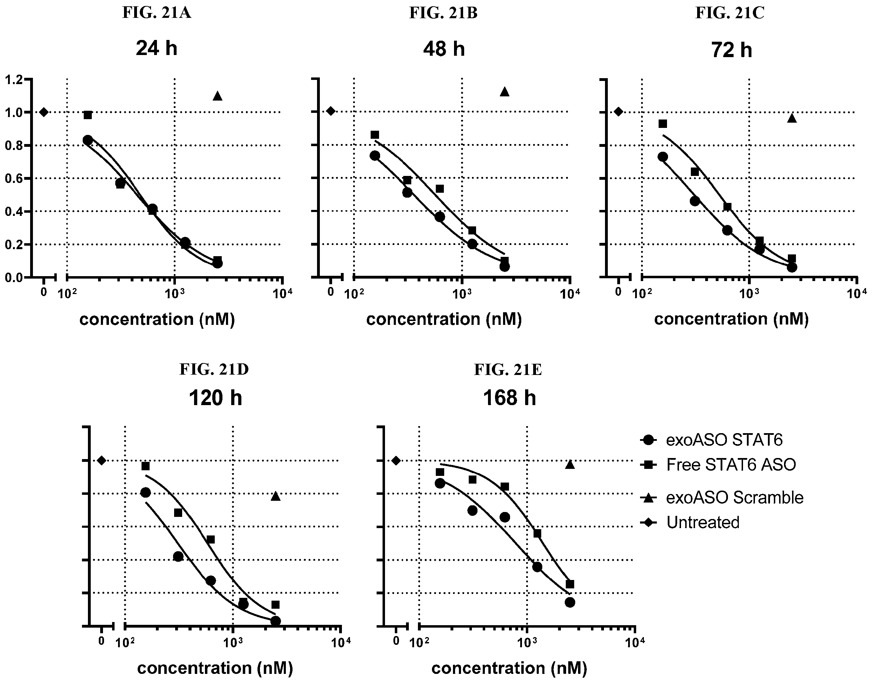

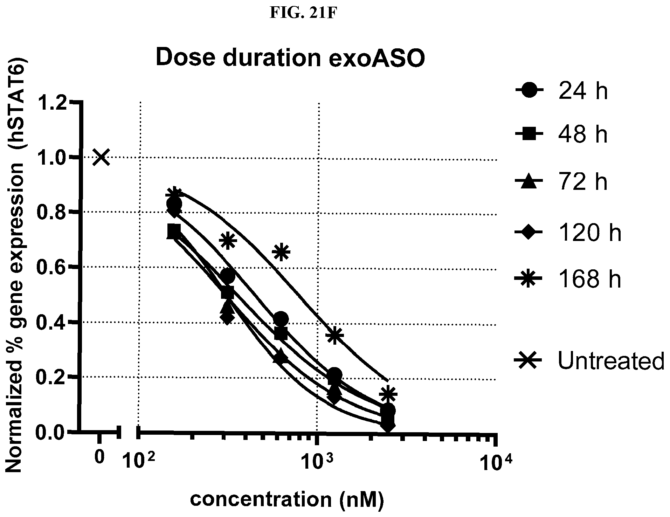

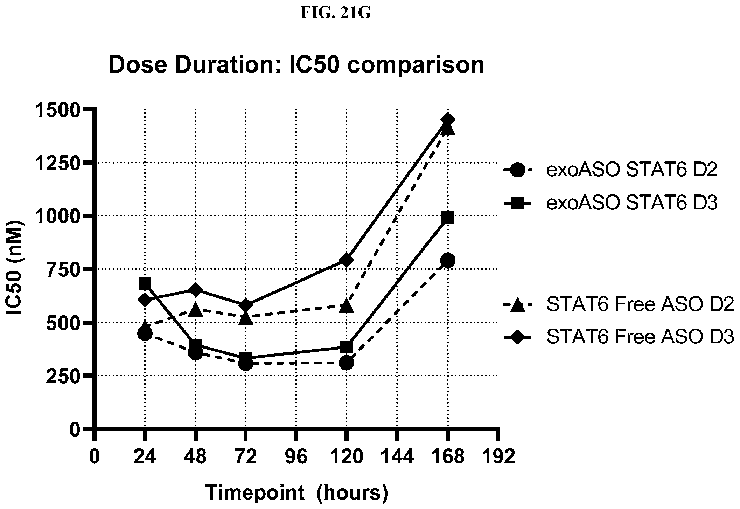

FIGs. 21A-21F are graphical representations of the dose dependent knock down of human STAT6 mRNA, as normalized to a housekeeping gene, in human macrophages following administration of increasing doses of exo-STAT6-ASO at 24 hours (FIG. 21A), 48 hours (FIG. 21B), 72 hours (FIG. 21C), 120 hours (FIG. 21D), and 168 (FIG. 21E) hours post administration (FIG. 21F). FIG. 21G is a graphical representation of the IC50 dose duration in human macrophages following treatment with exo-STAT6-ASO or a STAT6 free ASO in two donors over time.

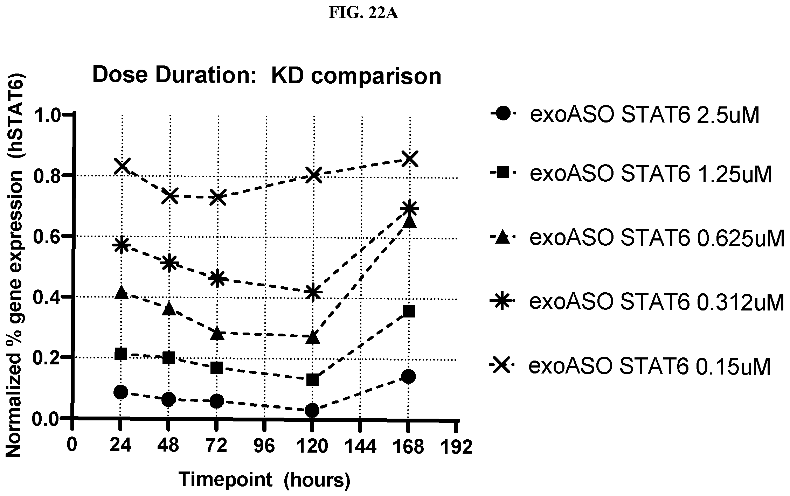

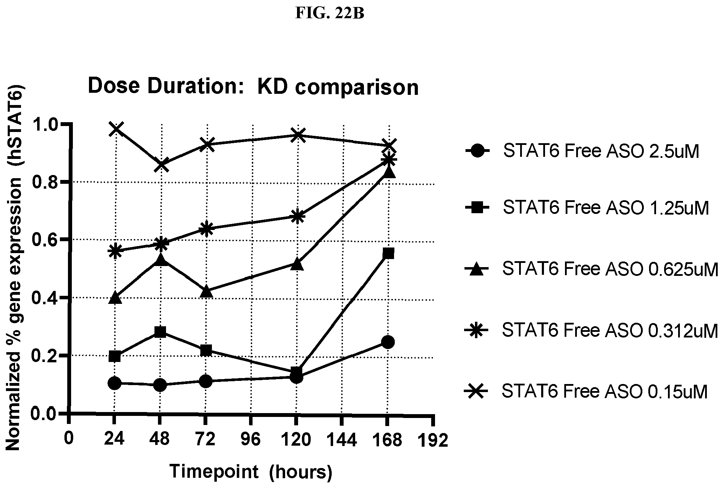

FIGs. 22A-22B are graphical representations illustrating the knock down of human STAT6 mRNA levels in human macrophages from a first donor (FIG. 22A) and a second donor (FIG. 22B) at 24 hours, 48 hours, 72 hours, 120 hours, and 168 hours post administration of 0.15 µM, 0.312 µM, 0.624 µM, 1.25 µM, or 2.5 µM exo-STAT6-ASO.

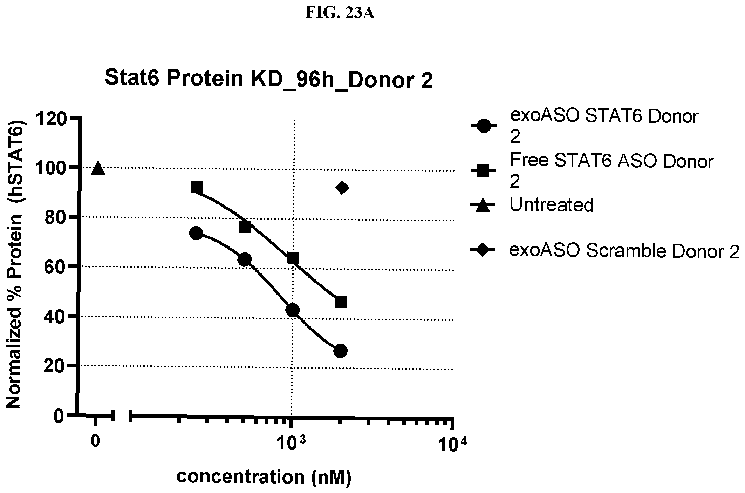

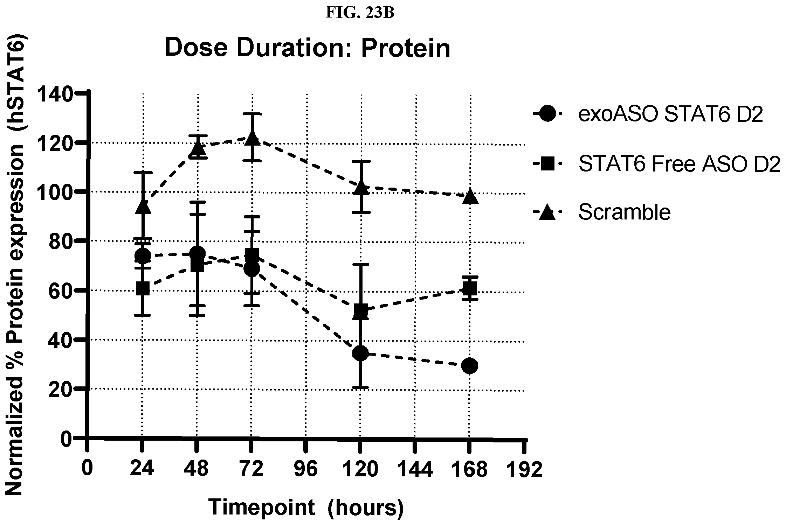

FIG. 23A is a graphical representation of the knock down of Stat6 protein levels in untreated donor samples or donor samples 96 hours after administration of increasing doses of exo-STAT6-ASO, free STAT6 ASO, or a scramble exo-ASO. FIG. 23B is a scatter plot illustrating the duration of Stat6 protein knock down at 24 hours, 48 hours, 72 hours, 120 hours, and 168 hours post administration of exo-STAT6-ASO, free STAT6 ASO, or a scramble exo-ASO.

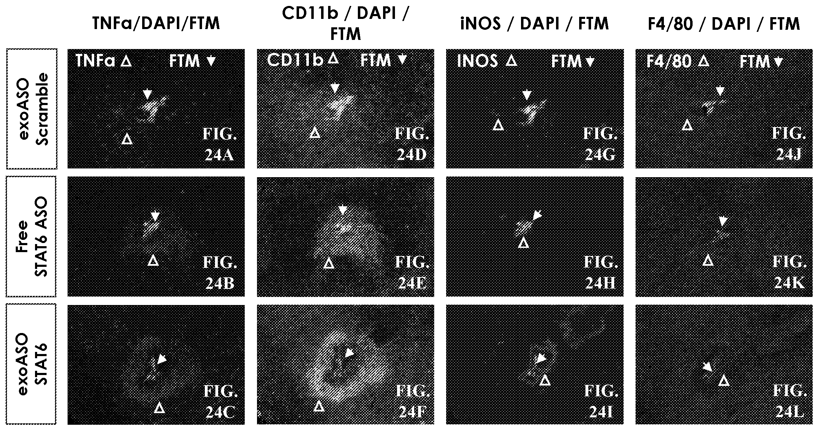

FIGs. 24A-24L are immunohistochemistry images showing the expression of TNFα (FIGs. 23A-23C), CD11b (FIGs. 23D-23F), iNOS (FIGs. 23G-23H), and F4/80 (FIGs. 23J-23L), following intratumoral injection (by CIVO^{®}) of an exo-ASO scramble (FIGs. 23A, 23D, 23G, and 23J); free STAT6 ASO (FIGs. 23B, 23E, 23H, and 23K); or exo-STAT6-ASO (FIGs. 23C, 23F, 23I, and 23L). Closed arrows indicate FTM staining. Open triangles indicate TNFα (FIGs. 23A-23C), CD11b (FIGs. 23D-23F), iNOS (FIGs. 23G-23H), and F4/80 (FIGs. 23J-23L) staining.

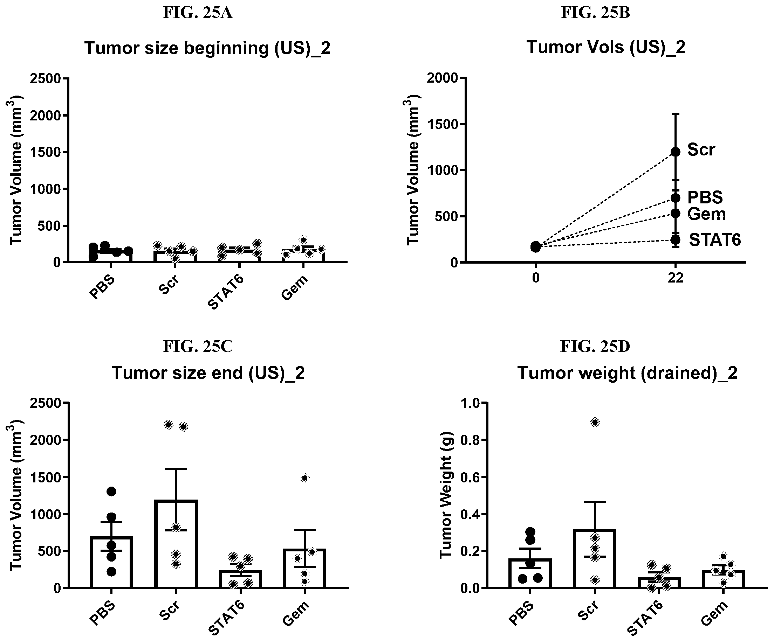

FIGs. 25A-25D are graphical representations showing starting tumor size (FIG. 25A) and tumor volume (FIG. 25B), tumor size (FIG. 25C), and tumor weight (FIG. 25D) in a prostate cancer mouse model at 22 days following intraperitoneal injection of PBS, a scrambles exo-ASO ("Scr"), exo-STAT6-ASO, or gemcitabine ("Gem").

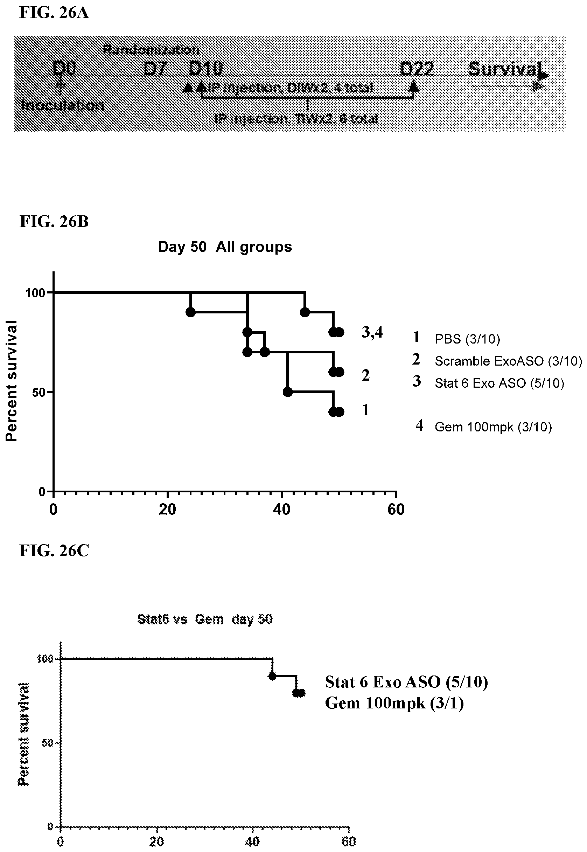

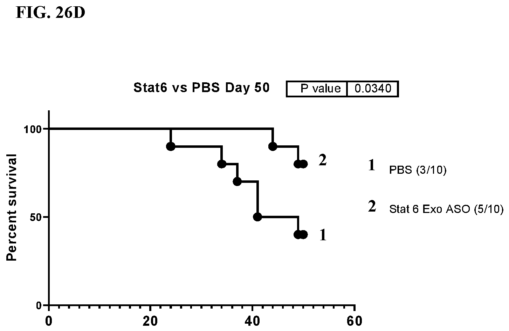

FIGs. 26A-26D show the survival response in a prostate cancer mouse model following administration of PBS, a scrambles exo-ASO ("Scr"; FIG. 26B), exo-STAT6-ASO (FIGs. FIG. 26B-26D, gemcitabine ("Gem"; FIGs. 26B-26C), or PBS (FIGs. 26B and 26D), as indicated, according to the timeline presented in FIG. 26A.

### DETAILED DESCRIPTION OF DISCLOSURE

The technical information set out below may in some respects go beyond the scope of the invention, which is defined exclusively by the appended claims. The additional technical information is provided to place the actual invention in a broader technical context and to illustrate possible related technical developments. Any additional technical information which does not fall within the scope of the appended claims, is not as such claimed or to be construed as being part of the invention.

In so far as the terms "invention", "example", "aspect", and "embodiment" are used herein, these terms will be interpreted in such a way that protection does not extend beyond the scope of the claims.

Incidental references to methods for treatment of the human or animal body by surgery or therapy and diagnostic methods practiced on the human or animal body are not to be construed as claiming protection for such method as such, but are instead to be construed as claims to products, in particular substances or compositions, for use in any of these methods.

Certain aspects of the present disclosure are directed to an extracellular vesicle (EV), e.g., an exosome, comprising an antisense oligonucleotide (ASO), wherein the ASO comprises a contiguous nucleotide sequence of 10 to 30 nucleotides in length that is complementary to a nucleic acid sequence within a *STAT6* transcript, as defined in the claims. In certain aspects, the EVs, *e.g*., exosomes, disclosed herein are loaded with one or more ASOs that target and reduce the level of STAT6 expression in target macrophages. Without being bound by any particular mechanism or theory, reducing the level of STAT6 in M2 macrophages promotes the repolarizing of M2 macrophages to an M1 macrophage phenotype. By repolarizing the macrophages to an M1 immune stimulatory phenotype, the EVs disclose herein promote an influx of T cells and immune effector cells to control tumor growth.

### I. Definitions

In order that the present description can be more readily understood, certain terms are first defined. Additional definitions are set forth throughout the detailed description.

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Unless otherwise indicated, nucleotide sequences are written left to right in 5' to 3' orientation. Amino acid sequences are written left to right in amino to carboxy orientation. The headings provided herein are not limitations of the various aspects of the disclosure, which can be had by reference to the specification as a whole. Accordingly, the terms defined immediately below are more fully defined by reference to the specification in its entirety.

The term "about" is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, *e.g.,* 10 percent, up or down (higher or lower). For example, if it is stated that "the ASO reduces expression of STAT6 protein in a cell following administration of the ASO by at least about 60%," it is implied that the STAT6 levels are reduced by a range of 50% to 70%.

The term "antisense oligonucleotide" (ASO) refers to an oligomer or polymer of nucleosides, such as naturally-occurring nucleosides or modified forms thereof, that are covalently linked to each other through internucleotide linkages. The ASO useful for the disclosure includes at least one non-naturally occurring nucleoside. An ASO is at least partially complementary to a target nucleic acid, such that the ASO hybridizes to the target nucleic acid sequence.

The term "nucleic acids" or "nucleotides" is intended to encompass plural nucleic acids. In some aspects, the term "nucleic acids" or "nucleotides" refers to a target sequence, e.g., pre-mRNAs, mRNAs, or DNAs *in vivo* or *in vitro.* When the term refers to the nucleic acids or nucleotides in a target sequence, the nucleic acids or nucleotides can be naturally occurring sequences within a cell. In other aspects, "nucleic acids" or "nucleotides" refer to a sequence in the ASOs of the disclosure. When the term refers to a sequence in the ASOs, the nucleic acids or nucleotides can be non-naturally occurring, *i.e.,* chemically synthesized, enzymatically produced, recombinantly produced, or any combination thereof. In some aspects, the nucleic acids or nucleotides in the ASOs are produced synthetically or recombinantly, but are not a naturally occurring sequence or a fragment thereof. In some aspects, the nucleic acids or nucleotides in the ASOs are not naturally occurring because they contain at least one nucleoside analog that is not naturally occurring in nature.

The term "nucleotide" as used herein, refers to a glycoside comprising a sugar moiety, a base moiety and a covalently linked group (linkage group), such as a phosphate or phosphorothioate intemucleotide linkage group, and covers both naturally occurring nucleotides, such as DNA or RNA, and non-naturally occurring nucleotides comprising modified sugar and/or base moieties, which are also referred to as "nucleotide analogs" herein. Herein, a single nucleotide can be referred to as a monomer or unit. In certain aspects, the term "nucleotide analogs" refers to nucleotides having modified sugar moieties. Non-limiting examples of the nucleotides having modified sugar moieties (e.g., LNA) are disclosed elsewhere herein. In other aspects, the term "nucleotide analogs" refers to nucleotides having modified nucleobase moieties. The nucleotides having modified nucleobase moieties include, but are not limited to, 5-methyl-cytosine, isocytosine, pseudoisocytosine, 5-bromouracil, 5-propynyluracil, 6-aminopurine, 2-aminopurine, inosine, diaminopurine, and 2-chloro-6-aminopurine. In some aspects, the terms "nucleotide", "unit" and "monomer" are used interchangeably. It will be recognized that when referring to a sequence of nucleotides or monomers, what is referred to is the sequence of bases, such as A, T, G, C or U, and analogs thereof.

The term "nucleoside" as used herein is used to refer to a glycoside comprising a sugar moiety and a base moiety, and can therefore be used when referring to the nucleotide units, which are covalently linked by the intemucleotide linkages between the nucleotides of the ASO. In the field of biotechnology, the term "nucleotide" is often used to refer to a nucleic acid monomer or unit. In the context of an ASO, the term "nucleotide" can refer to the base alone, *i.e.,* a nucleobase sequence comprising cytosine (DNA and RNA), guanine (DNA and RNA), adenine (DNA and RNA), thymine (DNA) and uracil (RNA), in which the presence of the sugar backbone and intemucleotide linkages are implicit. Likewise, particularly in the case of oligonucleotides where one or more of the internucleotide linkage groups are modified, the term "nucleotide" can refer to a "nucleoside." For example the term "nucleotide" can be used, even when specifying the presence or nature of the linkages between the nucleosides.

The term "nucleotide length" as used herein means the total number of the nucleotides (monomers) in a given sequence. For example, the sequence of ASO-STAT6-1053 (SEQ ID NO: 91) has 15 nucleotides; thus the nucleotide length of the sequence is 15. The term "nucleotide length" is therefore used herein interchangeably with "nucleotide number."

As one of ordinary skill in the art would recognize, the 5' terminal nucleotide of an oligonucleotide does not comprise a 5' internucleotide linkage group, although it can comprise a 5' terminal group.

The compounds described herein can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates. In some aspects, the asymmetric center can be an asymmetric carbon atom. The term "asymmetric carbon atom" means a carbon atom with four different substituents. According to the Cahn-Ingold-Prelog Convention an asymmetric carbon atom can be of the "R" or "S" configuration.

As used herein, the term "bicyclic sugar" refers to a modified sugar moiety comprising a 4 to 7 membered ring comprising a bridge connecting two atoms of the 4 to 7 membered ring to form a second ring, resulting in a bicyclic structure. In some aspects, the bridge connects the C2' and C4' of the ribose sugar ring of a nucleoside (*i.e.,* 2'-4' bridge), as observed in LNA nucleosides.

As used herein, a "coding region" or "coding sequence" is a portion of polynucleotide which consists of codons translatable into amino acids. Although a "stop codon" (TAG, TGA, or TAA) is typically not translated into an amino acid, it can be considered to be part of a coding region, but any flanking sequences, for example promoters, ribosome binding sites, transcriptional terminators, introns, untranslated regions ("UTRs"), and the like, are not part of a coding region. The boundaries of a coding region are typically determined by a start codon at the 5' terminus, encoding the amino terminus of the resultant polypeptide, and a translation stop codon at the 3' terminus, encoding the carboxyl terminus of the resulting polypeptide.

The term "non-coding region" as used herein means a nucleotide sequence that is not a coding region. Examples of non-coding regions include, but are not limited to, promoters, ribosome binding sites, transcriptional terminators, introns, untranslated regions ("UTRs"), non-coding exons and the like. Some of the exons can be wholly or part of the 5' untranslated region (5' UTR) or the 3' untranslated region (3' UTR) of each transcript. The untranslated regions are important for efficient translation of the transcript and for controlling the rate of translation and half-life of the transcript.

The term "region" when used in the context of a nucleotide sequence refers to a section of that sequence. For example, the phrase "region within a nucleotide sequence" or "region within the complement of a nucleotide sequence" refers to a sequence shorter than the nucleotide sequence, but longer than at least 10 nucleotides located within the particular nucleotide sequence or the complement of the nucleotides sequence, respectively. The term "sub-sequence" or "subsequence" can also refer to a region of a nucleotide sequence.

The term "downstream," when referring to a nucleotide sequence, means that a nucleic acid or a nucleotide sequence is located 3' to a reference nucleotide sequence. In certain aspects, downstream nucleotide sequences relate to sequences that follow the starting point of transcription. For example, the translation initiation codon of a gene is located downstream of the start site of transcription.

The term "upstream" refers to a nucleotide sequence that is located 5' to a reference nucleotide sequence.

As used herein, the term "regulatory region" refers to nucleotide sequences located upstream (5' non-coding sequences), within, or downstream (3' non-coding sequences) of a coding region, and which influence the transcription, RNA processing, stability, or translation of the associated coding region. Regulatory regions can include promoters, translation leader sequences, introns, polyadenylation recognition sequences, RNA processing sites, effector binding sites, UTRs, and stem-loop structures. If a coding region is intended for expression in a eukaryotic cell, a polyadenylation signal and transcription termination sequence will usually be located 3' to the coding sequence.

The term "transcript" as used herein can refer to a primary transcript that is synthesized by transcription of DNA and becomes a messenger RNA (mRNA) after processing, *i.e.,* a precursor messenger RNA (pre-mRNA), and the processed mRNA itself. The term "transcript" can be interchangeably used with "pre-mRNA" and "mRNA." After DNA strands are transcribed to primary transcripts, the newly synthesized primary transcripts are modified in several ways to be converted to their mature, functional forms to produce different proteins and RNAs, such as mRNA, tRNA, rRNA, lncRNA, miRNA and others. Thus, the term "transcript" can include exons, introns, 5' UTRs, and 3' UTRs.

The term "expression" as used herein refers to a process by which a polynucleotide produces a gene product, for example, a RNA or a polypeptide. It includes, without limitation, transcription of the polynucleotide into messenger RNA (mRNA) and the translation of an mRNA into a polypeptide. Expression produces a "gene product." As used herein, a gene product can be either a nucleic acid, *e.g.,* a messenger RNA produced by transcription of a gene, or a polypeptide which is translated from a transcript. Gene products described herein further include nucleic acids with post transcriptional modifications, e.g., polyadenylation or splicing, or polypeptides with post translational modifications, e.g., methylation, glycosylation, the addition of lipids, association with other protein subunits, or proteolytic cleavage.

The terms "identical" or percent "identity" in the context of two or more nucleic acids refer to two or more sequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences.

One such non-limiting example of a sequence alignment algorithm is the algorithm described in Karlin et al., 1990, Proc. Natl. Acad. Sci., 87:2264-2268, as modified in Karlin et al., 1993, Proc. Natl. Acad. Sci., 90:5873-5877, and incorporated into the NBLAST and XBLAST programs (Altschul et al., 1991, Nucleic Acids Res., 25:3389-3402). In certain aspects, Gapped BLAST can be used as described in Altschul et al., 1997, Nucleic Acids Res. 25:3389-3402. BLAST-2, WU-BLAST-2 (Altschul et al., 1996, Methods in Enzymology, 266:460-480), ALIGN, ALIGN-2 (Genentech, South San Francisco, California) or Megalign (DNASTAR) are additional publicly available software programs that can be used to align sequences. In certain aspects, the percent identity between two nucleotide sequences is determined using the GAP program in the GCG software package (e.g., using a NWSgapdna.CMP matrix and a gap weight of 40, 50, 60, 70, or 90 and a length weight of 1, 2, 3, 4, 5, or 6). In certain alternative aspects, the GAP program in the GCG software package, which incorporates the algorithm of Needleman and Wunsch (J. Mol. Biol. (48):444-453 (1970)) can be used to determine the percent identity between two amino acid sequences (e.g., using either a BLOSUM 62 matrix or a PAM250 matrix, and a gap weight of 16, 14, 12, 10, 8, 6, or 4 and a length weight of 1, 2, 3, 4, 5). Alternatively, in certain aspects, the percent identity between nucleotide or amino acid sequences is determined using the algorithm of Myers and Miller (CABIOS, 4:11-17 (1989)). For example, the percent identity can be determined using the ALIGN program (version 2.0) and using a PAM120 with residue table, a gap length penalty of 12 and a gap penalty of 4. One skilled in the art can determine appropriate parameters for maximal alignment by particular alignment software. In certain aspects, the default parameters of the alignment software are used.

In certain aspects, the percentage identity "X" of a first nucleotide sequence to a second nucleotide sequence is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the length of a first sequence is longer than the second sequence, the percent identity of the first sequence to the second sequence will be higher than the percent identity of the second sequence to the first sequence.

Different regions within a single polynucleotide target sequence that align with a polynucleotide reference sequence can each have their own percent sequence identity. It is noted that the percent sequence identity value is rounded to the nearest tenth. For example, 80.11, 80.12, 80.13, and 80.14 are rounded down to 80.1, while 80.15, 80.16, 80.17, 80.18, and 80.19 are rounded up to 80.2. It also is noted that the length value will always be an integer.

As used herein, the terms "homologous" and "homology" are interchangeable with the terms "identity" and "identical."

The term "naturally occurring variant thereof" refers to variants of the STAT6 polypeptide sequence or *STAT6* nucleic acid sequence (*e.g.,* transcript) which exist naturally within the defined taxonomic group, such as mammalian, such as mouse, monkey, and human. Typically, when referring to "naturally occurring variants" of a polynucleotide the term also can encompass any allelic variant of the *STAT6*-encoding genomic DNA which is found at Chromosomal position 1q44 at 247,416,156-247,449,108 (*i.e*., nucleotides 247,416,156-247,449,108 of GenBank Accession No. NC_000001.11) by chromosomal translocation or duplication, and the RNA, such as mRNA derived therefrom. "Naturally occurring variants" can also include variants derived from alternative splicing of the *STAT6* mRNA. When referenced to a specific polypeptide sequence, *e.g.,* the term also includes naturally occurring forms of the protein, which can therefore be processed, *e.g*., by co- or post-translational modifications, such as signal peptide cleavage, proteolytic cleavage, glycosylation, *etc.*

In determining the degree of "complementarity" between the ASOs of the disclosure (or regions thereof) and the target region of the nucleic acid which encodes mammalian STAT6 *(e.g.,* the *STAT6* gene), such as those disclosed herein, the degree of "complementarity" (also, "homology" or "identity") is expressed as the percentage identity (or percentage homology) between the sequence of the ASO (or region thereof) and the sequence of the target region (or the reverse complement of the target region) that best aligns therewith. The percentage is calculated by counting the number of aligned bases that are identical between the two sequences, dividing by the total number of contiguous monomers in the ASO, and multiplying by 100. In such a comparison, if gaps exist, it is preferable that such gaps are merely mismatches rather than areas where the number of monomers within the gap differs between the ASO of the disclosure and the target region.

The term "complement" as used herein indicates a sequence that is complementary to a reference sequence. It is well known that complementarity is the base principle of DNA replication and transcription as it is a property shared between two DNA or RNA sequences, such that when they are aligned antiparallel to each other, the nucleotide bases at each position in the sequences will be complementary, much like looking in the mirror and seeing the reverse of things. Therefore, for example, the complement of a sequence of 5‴ATGC''3' can be written as 3‴TACG"5' or 5‴GCAT"3'. The terms "reverse complement", "reverse complementary", and "reverse complementarity" as used herein are interchangeable with the terms "complement", "complementary", and "complementarity." In some aspects, the term "complementary" refers to 100% match or complementarity (*i.e.,* fully complementary) to a contiguous nucleic acid sequence within a *STAT6* transcript. In some aspects, the term "complementary" refers to at least about 80%, at least about 85%, at least about 90%, at least about 91%, at least about 92%, at least about 93%, at least about 94%, at least about 95%, at least about 96%, at least about 97%, at least about 98%, or at least about 99% match or complementarity to a contiguous nucleic acid sequence within a *STAT6* transcript.

The terms "corresponding to" and "corresponds to," when referencing two separate nucleic acid or nucleotide sequences can be used to clarify regions of the sequences that correspond or are similar to each other based on homology and/or functionality, although the nucleotides of the specific sequences can be numbered differently. For example, different isoforms of a gene transcript can have similar or conserved portions of nucleotide sequences whose numbering can differ in the respective isoforms based on alternative splicing and/or other modifications. In addition, it is recognized that different numbering systems can be employed when characterizing a nucleic acid or nucleotide sequence (*e.g*., a gene transcript and whether to begin numbering the sequence from the translation start codon or to include the 5'UTR). Further, it is recognized that the nucleic acid or nucleotide sequence of different variants of a gene or gene transcript can vary. As used herein, however, the regions of the variants that share nucleic acid or nucleotide sequence homology and/or functionality are deemed to "correspond" to one another. For example, a nucleotide sequence of a *STAT6* transcript corresponding to nucleotides X to Y of SEQ ID NO: 1 ("reference sequence") refers to an *STAT6* transcript sequence (*e.g., STAT6* pre-mRNA or mRNA) that has an identical sequence or a similar sequence to nucleotides X to Y of SEQ ID NO: 1, wherein X is the start site and Y is the end site (as shown in FIG. 1). A person of ordinary skill in the art can identify the corresponding X and Y residues in the *STAT6* transcript sequence by aligning the *STAT6* transcript sequence with SEQ ID NO: 1.

The terms "corresponding nucleotide analog" and "corresponding nucleotide" are intended to indicate that the nucleobase in the nucleotide analog and the naturally occurring nucleotide have the same pairing, or hybridizing, ability. For example, when the 2-deoxyribose unit of the nucleotide is linked to an adenine, the "corresponding nucleotide analog" contains a pentose unit (different from 2-deoxyribose) linked to an adenine.

The annotation of ASO chemistry is as follows Beta-D-oxy LNA nucleotides are designated by OxyB where B designates a nucleotide base such as thymine (T), uridine (U), cytosine (C), 5-methylcytosine (MC), adenine (A) or guanine (G), and thus include OxyA, OxyT, OxyMC, OxyC and OxyG. DNA nucleotides are designated by DNAb, where the lower case b designates a nucleotide base such as thymine (T), uridine (U), cytosine (C), 5-methylcytosine (Mc), adenine (A) or guanine (G), and thus include DNAa, DNAt, DNA and DNAg. The letter M before C or c indicates 5-methylcytosine. The letter "s" indicates a phosphorothioate intemucleotide linkage.

The term "ASO Number" or "ASO No." as used herein refers to a unique number given to a nucleotide sequence having the detailed chemical structure of the components, *e.g.*, nucleosides (*e.g.,* DNA), nucleoside analogs (*e.g.,* beta-D-oxy-LNA), nucleobase (*e.g.,* A, T, G, C, U, or MC), and backbone structure (*e.g*., phosphorothioate or phosphorodiester). For example, ASO-STAT6-1053 can refer to STAT6-1053 (SEQ ID NO: 91).

"Potency" is normally expressed as an IC₅₀ or EC₅₀ value, in µM, nM or pM unless otherwise stated. Potency can also be expressed in terms of percent inhibition. IC₅₀ is the median inhibitory concentration of a therapeutic molecule. EC₅₀ is the median effective concentration of a therapeutic molecule relative to a vehicle or control (*e.g*., saline). In functional assays, IC₅₀ is the concentration of a therapeutic molecule that reduces a biological response, *e.g.*, transcription of mRNA or protein expression, by 50% of the biological response that is achieved by the therapeutic molecule. In functional assays, EC₅₀ is the concentration of a therapeutic molecule that produces 50% of the biological response, *e.g*., transcription of mRNA or protein expression. IC₅₀ or EC₅₀ can be calculated by any number of means known in the art.

As used herein, the term "inhibiting," *e.g.,* the expression of *STAT6* gene transcript and/or STAT6 protein refers to the ASO reducing the expression of the *STAT6* gene transcript and/or STAT6 protein in a cell or a tissue. In some aspects, the term "inhibiting" refers to complete inhibition (100% inhibition or non-detectable level) of *STAT6* gene transcript or STAT6 protein. In other aspects, the term "inhibiting" refers to at least 5%, at least 10%, at least 15%, at least 20%, at least 25%, at least 30%, at least 35%, at least 40%, at least 45%, at least 50%, at least 60%, at least 70%, at least 80%, at least 90%, at least 95% or at least 99% inhibition of *STAT6* gene transcript and/or STAT6 protein expression in a cell or a tissue.

As used herein, the term "extracellular vesicle" or "EV" refers to a cell-derived vesicle comprising a membrane that encloses an internal space. Extracellular vesicles comprise all membrane-bound vesicles (*e.g*., exosomes, nanovesicles) that have a smaller diameter than the cell from which they are derived. In some aspects, extracellular vesicles range in diameter from 20 nm to 1000 nm, and can comprise various macromolecular payload either within the internal space (*i.e.,* lumen), displayed on the external surface of the extracellular vesicle, and/or spanning the membrane. In some aspects, the payload can comprise nucleic acids, proteins, carbohydrates, lipids, small molecules, and/or combinations thereof. In certain aspects, an extracellular vehicle comprises a scaffold moiety. By way of example and without limitation, extracellular vesicles include apoptotic bodies, fragments of cells, vesicles derived from cells by direct or indirect manipulation (*e.g*., by serial extrusion or treatment with alkaline solutions), vesiculated organelles, and vesicles produced by living cells (*e.g*., by direct plasma membrane budding or fusion of the late endosome with the plasma membrane). Extracellular vesicles can be derived from a living or dead organism, explanted tissues or organs, prokaryotic or eukaryotic cells, and/or cultured cells. In some aspects, the extracellular vesicles are produced by cells that express one or more transgene products.

As used herein, the term "exosome" refers to an extracellular vesicle with a diameter between 20-300 nm (*e.g*., between 40-200 nm). Exosomes comprise a membrane that encloses an internal space (*i.e*., lumen), and, in some aspects, can be generated from a cell (*e.g*., producer cell) by direct plasma membrane budding or by fusion of the late endosome with the plasma membrane. In certain aspects, an exosome comprises a scaffold moiety. As described *infra,* exosome can be derived from a producer cell, and isolated from the producer cell based on its size, density, biochemical parameters, or a combination thereof. In some aspects, the EVs, *e.g*., exosomes, of the present disclosure are produced by cells that express one or more transgene products.

As used herein, the term "nanovesicle" refers to an extracellular vesicle with a diameter between 20-250 nm (*e.g.,* between 30-150 nm) and is generated from a cell (*e.g.,* producer cell) by direct or indirect manipulation such that the nanovesicle would not be produced by the cell without the manipulation. Appropriate manipulations of the cell to produce the nanovesicles include but are not limited to serial extrusion, treatment with alkaline solutions, sonication, or combinations thereof. In some aspects, production of nanovesicles can result in the destruction of the producer cell. In some aspects, population of nanovesicles described herein are substantially free of vesicles that are derived from cells by way of direct budding from the plasma membrane or fusion of the late endosome with the plasma membrane. In certain aspects, a nanovesicle comprises a scaffold moiety. Nanovesicles, once derived from a producer cell, can be isolated from the producer cell based on its size, density, biochemical parameters, or a combination thereof.

As used herein the term "surface-engineered EVs, *e.g.,* exosomes" (*e.g.,* Scaffold X-engineered EVs, *e.g.,* exosomes) refers to an EV, *e.g.,* exosome, with the membrane or the surface of the EV, *e.g*., exosome, modified in its composition so that the surface of the engineered EV, *e.g.,* exosome, is different from that of the EV, *e.g.,* exosome, prior to the modification or of the naturally occurring EV, *e.g.,* exosome. The engineering can be on the surface of the EV, *e.g.,* exosome, or in the membrane of the EV, *e.g.,* exosome, so that the surface of the EV, *e.g.,* exosome, is changed. For example, the membrane is modified in its composition of a protein, a lipid, a small molecule, a carbohydrate, *etc.* The composition can be changed by a chemical, a physical, or a biological method or by being produced from a cell previously or concurrently modified by a chemical, a physical, or a biological method. Specifically, the composition can be changed by a genetic engineering or by being produced from a cell previously modified by genetic engineering. In some aspects, a surface-engineered EV, *e.g.,* exosome, comprises an exogenous protein *(i.e.,* a protein that the EV, e.g., exosome, does not naturally express) or a fragment or variant thereof that can be exposed to the surface of the EV, e.g., exosome, or can be an anchoring point (attachment) for a moiety exposed on the surface of the EV, *e.g.,* exosome. In other aspects, a surface-engineered EV, *e.g.,* exosome, comprises a higher expression *(e.g.,* higher number) of a natural exosome protein (*e.g.,* Scaffold X) or a fragment or variant thereof that can be exposed to the surface of the EV, *e.g.,* exosome, or can be an anchoring point (attachment) for a moiety exposed on the surface of the EV, *e.g.,* exosome.

As used herein the term "lumen-engineered exosome" (*e.g.,* Scaffold Y-engineered exosome) refers to an EV, *e.g.,* exosome, with the membrane or the lumen of the EV, *e.g.,* exosome, modified in its composition so that the lumen of the engineered EV, *e.g.,* exosome, is different from that of the EV, *e.g.,* exosome, prior to the modification or of the naturally occurring EV, *e.g.,* exosome. The engineering can be directly in the lumen or in the membrane of the EV, *e.g.,* exosome so that the lumen of the EV, *e.g.,* exosome is changed. For example, the membrane is modified in its composition of a protein, a lipid, a small molecule, a carbohydrate, *etc.* so that the lumen of the EV, *e.g.,* exosome is modified. The composition can be changed by a chemical, a physical, or a biological method or by being produced from a cell previously modified by a chemical, a physical, or a biological method. Specifically, the composition can be changed by a genetic engineering or by being produced from a cell previously modified by genetic engineering. In some aspects, a lumen-engineered exosome comprises an exogenous protein *(i.e.,* a protein that the EV, *e.g.,* exosome does not naturally express) or a fragment or variant thereof that can be exposed in the lumen of the EV, *e.g.,* exosome or can be an anchoring point (attachment) for a moiety exposed on the inner layer of the EV, *e.g.,* exosome. In other aspects, a lumen-engineered EV, *e.g.,* exosome, comprises a higher expression of a natural exosome protein (*e.g.,* Scaffold X or Scaffold Y) or a fragment or variant thereof that can be exposed to the lumen of the exosome or can be an anchoring point (attachment) for a moiety exposed in the lumen of the exosome.

The term "modified," when used in the context of EVs, *e.g.,* exosomes described herein, refers to an alteration or engineering of an EV, *e.g.,* exosome and/or its producer cell, such that the modified EV, *e.g.,* exosome is different from a naturally-occurring EV, *e.g.,* exosome. In some aspects, a modified EV, *e.g.,* exosome described herein comprises a membrane that differs in composition of a protein, a lipid, a small molecular, a carbohydrate, *etc.* compared to the membrane of a naturally-occurring EV, *e.g.,* exosome (*e.g.,* membrane comprises higher density or number of natural exosome proteins and/or membrane comprises proteins that are not naturally found in exosomes (*e.g.,* an ASO). In certain aspects, such modifications to the membrane changes the exterior surface of the EV, *e.g.,* exosome *(e.g.,* surface-engineered EVs, *e.g.,* exosomes described herein). In certain aspects, such modifications to the membrane changes the lumen of the EV, *e.g.,* exosome (*e.g.,* lumen-engineered EVs, *e.g.,* exosomes described herein).

As used herein, the term "scaffold moiety" refers to a molecule that can be used to anchor a payload or any other compound of interest (*e.g.,* an ASO) to the EV, *e.g.,* exosome either on the luminal surface or on the exterior surface of the EV, *e.g.,* exosome. In certain aspects, a scaffold moiety comprises a synthetic molecule. In some aspects, a scaffold moiety comprises a non-polypeptide moiety. In other aspects, a scaffold moiety comprises a lipid, carbohydrate, or protein that naturally exists in the EV, *e.g.,* exosome. In some aspects, a scaffold moiety comprises a lipid, carbohydrate, or protein that does not naturally exist in the EV, *e.g.,* exosome. In certain aspects, a scaffold moiety is Scaffold X. In some aspects, a scaffold moiety is Scaffold Y. In further aspects, a scaffold moiety comprises both Scaffold X and Scaffold Y. Non-limiting examples of other scaffold moieties that can be used with the present disclosure include: aminopeptidase N (CD13); Neprilysin, AKA membrane metalloendopeptidase (MME); ectonucleotide pyrophosphatase/phosphodiesterase family member 1 (ENPP1); Neuropilin-1 (NRP1); CD9, CD63, CD81, PDGFR, GPI anchor proteins, lactadherin (MFGE8), LAMP2, and LAMP2B.

As used herein, the term "Scaffold X" refers to exosome proteins that have recently been identified on the surface of exosomes. *See, e.g.,* U.S. Pat. No. 10,195,290. Non-limiting examples of Scaffold X proteins include: prostaglandin F2 receptor negative regulator ("the PTGFRN protein"); basigin ("the BSG protein"); immunoglobulin superfamily member 2 ("the IGSF2 protein"); immunoglobulin superfamily member 3 ("the IGSF3 protein"); immunoglobulin superfamily member 8 ("the IGSF8 protein"); integrin beta-1 ("the ITGB1 protein); integrin alpha-4 ("the ITGA4 protein"); 4F2 cell-surface antigen heavy chain ("the SLC3A2 protein"); a class of ATP transporter proteins ("the ATP1A1 protein," "the ATP1A2 protein," "the ATP1A3 protein," "the ATP1A4 protein," "the ATP1B3 protein," "the ATP2B1 protein," "the ATP2B2 protein," "the ATP2B3 protein," "the ATP2B protein"); and a functional fragment thereof. In some aspects, a Scaffold X protein can be a whole protein or a fragment thereof (*e.g.,* functional fragment, *e.g.,* the smallest fragment that is capable of anchoring another moiety on the exterior surface or on the luminal surface of the EV, *e.g.,* exosome). In some aspects, a Scaffold X can anchor a moiety (*e.g.,* an ASO) to the external surface or the luminal surface of the exosome.

As used herein, the term "Scaffold Y" refers to exosome proteins that were newly identified within the lumen of exosomes. *See, e.g.,* International Publ. No. WO/2019/099942. Non-limiting examples of Scaffold Y proteins include: myristoylated alanine rich Protein Kinase C substrate ("the MARCKS protein"); myristoylated alanine rich Protein Kinase C substrate like 1 ("the MARCKSL1 protein"); and brain acid soluble protein 1 ("the BASP1 protein"). In some aspects, a Scaffold Y protein can be a whole protein or a fragment thereof (*e.g.,* functional fragment, *e.g.,* the smallest fragment that is capable of anchoring a moiety to the luminal surface of the exosome). In some aspects, a Scaffold Y can anchor a moiety (*e.g.,* an ASO) to the luminal surface of the EV, *e.g.,* exosome. In some aspects, a Scaffold Y can anchor a moiety *(e.g.,* an ASO) to the exterior surface of the EV, *e.g.,* exosome.

As used herein, the term "fragment" of a protein (*e.g.,* therapeutic protein, Scaffold X, or Scaffold Y) refers to an amino acid sequence of a protein that is shorter than the naturally-occurring sequence, N- and/or C-terminally deleted or any part of the protein deleted in comparison to the naturally occurring protein. As used herein, the term "functional fragment" refers to a protein fragment that retains protein function. Accordingly, in some aspects, a functional fragment of a Scaffold X protein retains the ability to anchor a moiety on the luminal surface or on the exterior surface of the EV, *e.g.,* exosome. Similarly, in certain aspects, a functional fragment of a Scaffold Y protein retains the ability to anchor a moiety on the luminal surface or exterior surface of the EV, *e.g.,* exosome. Whether a fragment is a functional fragment can be assessed by any art known methods to determine the protein content of EVs, *e.g.,* exosomes including Western Blots, FACS analysis and fusions of the fragments with autofluorescent proteins like, *e.g.,* GFP. In certain aspects, a functional fragment of a Scaffold X protein retains at least about 50%, at least about 60%, at least about 70%, at least about 80%, at least about 90% or at least about 100% of the ability, *e.g.,* an ability to anchor a moiety, of the naturally occurring Scaffold X protein. In some aspects, a functional fragment of a Scaffold Y protein retains at least about 50%, at least about 60%, at least about 70%, at least about 80%, at least about 90% or at least about 100% of the ability, *e.g.,* an ability to anchor another molecule, of the naturally occurring Scaffold Y protein.

As used herein, the term "variant" of a molecule (*e.g.,* functional molecule, antigen, Scaffold X and/or Scaffold Y) refers to a molecule that shares certain structural and functional identities with another molecule upon comparison by a method known in the art. For example, a variant of a protein can include a substitution, insertion, deletion, frameshift or rearrangement in another protein.

In some aspects, a variant of a Scaffold X comprises a variant having at least about 70% identity to the full-length, mature PTGFRN, BSG, IGSF2, IGSF3, IGSF8, ITGB1, ITGA4, SLC3A2, or ATP transporter proteins or a fragment (*e.g.,* functional fragment) of the PTGFRN, BSG, IGSF2, IGSF3, IGSF8, ITGB1, ITGA4, SLC3A2, or ATP transporter proteins. In some aspects, variants or variants of fragments of PTGFRN share at least about 70%, at least about 80%, at least about 85%, at least about 90%, at least about 95%, at least about 96%, at least about 97%, at least about 98%, or at least about 99% sequence identity with PTGFRN according to SEQ ID NO: 301 or with a functional fragment thereof. In some aspects variants or variants of fragments of BSG share at least about 70%, at least about 80%, at least about 85%, at least about 90%, at least about 95%, at least about 96%, at least about 97%, at least about 98%, or at least about 99% sequence identity with BSG according to SEQ ID NO: 303 or with a functional fragment thereof. In some aspects variants or variants of fragments of IGSF2 share at least about 70%, at least about 80%, at least about 85%, at least about 90%, at least about 95%, at least about 96%, at least about 97%, at least about 98%, or at least about 99% sequence identity with IGSF2 according to SEQ ID NO: 308 or with a functional fragment thereof. In some aspects variants or variants of fragments of IGSF3 share at least about 70%, at least about 80%, at least about 85%, at least about 90%, at least about 95%, at least about 96%, at least about 97%, at least about 98%, or at least about 99% sequence identity with IGSF3 according to SEQ ID NO: 309 or with a functional fragment thereof. In some aspects variants or variants of fragments of IGSF8 share at least about 70%, at least about 80%, at least about 85%, at least about 90%, at least about 95%, at least about 96%, at least about 97%, at least about 98%, or at least about 99% sequence identity with IGSF8 according to SEQ ID NO: 304 or with a functional fragment thereof. In some aspects variants or variants of fragments of ITGB1 share at least about 70%, at least about 80%, at least about 85%, at least about 90%, at least about 95%, at least about 96%, at least about 97%, at least about 98%, or at least about 99% sequence identity with ITGB1 according to SEQ ID NO: 305 or with a functional fragment thereof. In some aspects variants or variants of fragments of ITGA4 share at least about 70%, at least about 80%, at least about 85%, at least about 90%, at least about 95%, at least about 96%, at least about 97%, at least about 98%, or at least about 99% sequence identity with ITGA4 according to SEQ ID NO: 306 or with a functional fragment thereof. In some aspects variants or variants of fragments of SLC3A2 share at least about 70%, at least about 80%, at least about 85%, at least about 90%, at least about 95%, at least about 96%, at least about 97%, at least about 98%, or at least about 99% sequence identity with SLC3A2 according to SEQ ID NO: 307 or with a functional fragment thereof. In some aspects variants or variants of fragments of ATP1A1 share at least about 70%, at least about 80%, at least about 85%, at least about 90%, at least about 95%, at least about 96%, at least about 97%, at least about 98%, or at least about 99% sequence identity with ATP1A1 according to SEQ ID NO: 310 or with a functional fragment thereof. In some aspects variants or variants of fragments of ATP1A2 share at least about 70%, at least about 80%, at least about 85%, at least about 90%, at least about 95%, at least about 96%, at least about 97%, at least about 98%, or at least about 99% sequence identity with ATP1A2 according to SEQ ID NO: 311 or with a functional fragment thereof. In some aspects variants or variants of fragments of ATP1A3 share at least about 70%, at least about 80%, at least about 85%, at least about 90%, at least about 95%, at least about 96%, at least about 97%, at least about 98%, or at least about 99% sequence identity with ATP1A3 according to SEQ ID NO: 312 or with a functional fragment thereof. In some aspects variants or variants of fragments of ATP1A4 share at least about 70%, at least about 80%, at least about 85%, at least about 90%, at least about 95%, at least about 96%, at least about 97%, at least about 98%, or at least about 99% sequence identity with ATP1A4 according to SEQ ID NO: 313 or with a functional fragment thereof. In some aspects variants or variants of fragments of ATP1B3 share at least about 70%, at least about 80%, at least about 85%, at least about 90%, at least about 95%, at least about 96%, at least about 97%, at least about 98%, or at least about 99% sequence identity with ATP1B3 according to SEQ ID NO: 314 or with a functional fragment thereof. In some aspects variants or variants of fragments of ATP2B1 share at least about 70%, at least about 80%, at least about 85%, at least about 90%, at least about 95%, at least about 96%, at least about 97%, at least about 98%, or at least about 99% sequence identity with ATP2B1 according to SEQ ID NO: 315 or with a functional fragment thereof. In some aspects variants or variants of fragments of ATP2B2 share at least about 70%, at least about 80%, at least about 85%, at least about 90%, at least about 95%, at least about 96%, at least about 97%, at least about 98%, or at least about 99% sequence identity with ATP2B2 according to SEQ ID NO: 316 or with a functional fragment thereof. In some aspects variants or variants of fragments of ATP2B3 share at least about 70%, at least about 80%, at least about 85%, at least about 90%, at least about 95%, at least about 96%, at least about 97%, at least about 98%, or at least about 99% sequence identity with ATP2B3 according to SEQ ID NO: 317 or with a functional fragment thereof. In some aspects variants or variants of fragments of ATP2B4 share at least about 70%, at least about 80%, at least about 85%, at least about 90%, at least about 95%, at least about 96%, at least about 97%, at least about 98%, or at least about 99% sequence identity with ATP2B4 according to SEQ ID NO: 318 or with a functional fragment thereof. In some aspects, the variant or variant of a fragment of Scaffold X protein disclosed herein retains the ability to be specifically targeted to EVs, *e.g.,* exosomes. In some aspects, the Scaffold X includes one or more mutations, for example, conservative amino acid substitutions.

In some aspects, a variant of a Scaffold Y comprises a variant having at least 70% identity to MARCKS, MARCKSL1, BASP1, or a fragment of MARCKS, MARCKSL1, or BASP1. In some aspects variants or variants of fragments of MARCKS share at least about 70%, at least about 80%, at least about 85%, at least about 90%, at least about 95%, at least about 96%, at least about 97%, at least about 98%, or at least about 99% sequence identity with MARCKS according to SEQ ID NO: 401 or with a functional fragment thereof. In some aspects variants or variants of fragments of MARCKSL1 share at least about 70%, at least about 80%, at least about 85%, at least about 90%, at least about 95%, at least about 96%, at least about 97%, at least about 98%, or at least about 99% sequence identity with MARCKSL1 according to SEQ ID NO: 402 or with a functional fragment thereof. In some aspects variants or variants of fragments of BASP1 share at least about 70%, at least about 80%, at least about 85%, at least about 90%, at least about 95%, at least about 96%, at least about 97%, at least about 98%, or at least about 99% sequence identity with BASP1 according to SEQ ID NO: 403 or with a functional fragment thereof. In some aspects, the variant or variant of a fragment of Scaffold Y protein retains the ability to be specifically targeted to the luminal surface of EVs, *e.g.,* exosomes. In some aspects, the Scaffold Y includes one or more mutations, *e.g.,* conservative amino acid substitutions.

A "conservative amino acid substitution" is one in which the amino acid residue is replaced with an amino acid residue having a similar side chain. Families of amino acid residues having similar side chains have been defined in the art, including basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g.,* alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine). Thus, if an amino acid in a polypeptide is replaced with another amino acid from the same side chain family, the substitution is considered to be conservative. In another aspect, a string of amino acids can be conservatively replaced with a structurally similar string that differs in order and/or composition of side chain family members.

The term "percent sequence identity" or "percent identity" between two polynucleotide or polypeptide sequences refers to the number of identical matched positions shared by the sequences over a comparison window, taking into account additions or deletions (*i.e.,* gaps) that must be introduced for optimal alignment of the two sequences. A matched position is any position where an identical nucleotide or amino acid is presented in both the target and reference sequence. Gaps presented in the target sequence are not counted since gaps are not nucleotides or amino acids. Likewise, gaps presented in the reference sequence are not counted since target sequence nucleotides or amino acids are counted, not nucleotides or amino acids from the reference sequence.

The percentage of sequence identity is calculated by determining the number of positions at which the identical amino-acid residue or nucleic acid base occurs in both sequences to yield the number of matched positions, dividing the number of matched positions by the total number of positions in the window of comparison and multiplying the result by 100 to yield the percentage of sequence identity. The comparison of sequences and determination of percent sequence identity between two sequences may be accomplished using readily available software both for online use and for download. Suitable software programs are available from various sources, and for alignment of both protein and nucleotide sequences. One suitable program to determine percent sequence identity is bl2seq, part of the BLAST suite of programs available from the U.S. government's National Center for Biotechnology Information BLAST web site (blast.ncbi.nlm.nih.gov). Bl2seq performs a comparison between two sequences using either the BLASTN or BLASTP algorithm. BLASTN is used to compare nucleic acid sequences, while BLASTP is used to compare amino acid sequences. Other suitable programs are, *e.g.,* Needle, Stretcher, Water, or Matcher, part of the EMBOSS suite of bioinformatics programs and also available from the European Bioinformatics Institute (EBI) at www.ebi.ac.uk/Tools/psa.

Different regions within a single polynucleotide or polypeptide target sequence that aligns with a polynucleotide or polypeptide reference sequence can each have their own percent sequence identity. It is noted that the percent sequence identity value is rounded to the nearest tenth. For example, 80.11, 80.12, 80.13, and 80.14 are rounded down to 80.1, while 80.15, 80.16, 80.17, 80.18, and 80.19 are rounded up to 80.2. It also is noted that the length value will always be an integer.

One skilled in the art will appreciate that the generation of a sequence alignment for the calculation of a percent sequence identity is not limited to binary sequence-sequence comparisons exclusively driven by primary sequence data. Sequence alignments can be derived from multiple sequence alignments. One suitable program to generate multiple sequence alignments is ClustalW2, available from www.clustal.org. Another suitable program is MUSCLE, available from www.drive5.com/muscle/. ClustalW2 and MUSCLE are alternatively available, *e.g.,* from the EBI.

It will also be appreciated that sequence alignments can be generated by integrating sequence data with data from heterogeneous sources such as structural data (*e.g.,* crystallographic protein structures), functional data (*e.g.,* location of mutations), or phylogenetic data. A suitable program that integrates heterogeneous data to generate a multiple sequence alignment is T-Coffee, available at www.tcoffee.org, and alternatively available, *e.g.,* from the EBI. It will also be appreciated that the final alignment used to calculate percent sequence identity may be curated either automatically or manually.

The polynucleotide variants can contain alterations in the coding regions, non-coding regions, or both. In one aspect, the polynucleotide variants contain alterations which produce silent substitutions, additions, or deletions, but do not alter the properties or activities of the encoded polypeptide. In another aspect, nucleotide variants are produced by silent substitutions due to the degeneracy of the genetic code. In other aspects, variants in which 5-10, 1-5, or 1-2 amino acids are substituted, deleted, or added in any combination. Polynucleotide variants can be produced for a variety of reasons, *e.g.,* to optimize codon expression for a particular host (change codons in the human mRNA to others, *e.g.,* a bacterial host such as *E. coli*).

Naturally occurring variants are called "allelic variants," and refer to one of several alternate forms of a gene occupying a given locus on a chromosome of an organism (Genes II, Lewin, B., ed., John Wiley & Sons, New York (1985)). These allelic variants can vary at either the polynucleotide and/or polypeptide level and are included in the present disclosure. Alternatively, non-naturally occurring variants can be produced by mutagenesis techniques or by direct synthesis.

Using known methods of protein engineering and recombinant DNA technology, variants can be generated to improve or alter the characteristics of the polypeptides. For instance, one or more amino acids can be deleted from the N-terminus or C-terminus of the secreted protein without substantial loss of biological function. Ron et al., J. Biol. Chem. 268: 2984-2988 (1993) reported variant KGF proteins having heparin binding activity even after deleting 3, 8, or 27 amino-terminal amino acid residues. Similarly, interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See Abstract.) In fact, only 23 unique amino acid sequences, out of more than 3,500 nucleotide sequences examined, produced a protein that significantly differed in activity from wild-type.

As stated above, polypeptide variants include, e.g., modified polypeptides. Modifications include, *e.g.,* acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation (Mei et al., Blood 116:270-79 (2010)), proteolytic processing, phosphorylation, prenylation, racemization, selenoylation, sulfation, transfer-RNA mediated addition of amino acids to proteins such as arginylation, and ubiquitination. In some aspects, Scaffold X and/or Scaffold Y is modified at any convenient location.

As used herein the term "linked to" or "conjugated to" are used interchangeably and refer to a covalent or non-covalent bond formed between a first moiety and a second moiety, *e.g.,* Scaffold X and an ASO, respectively, *e.g.,* a scaffold moiety expressed in or on the extracellular vesicle and an ASO, *e.g.,* Scaffold X (*e.g.,* a PTGFRN protein), respectively, in the luminal surface of or on the external surface of the extracellular vesicle.

The term "encapsulated", or grammatically different forms of the term (*e.g.,* encapsulation, or encapsulating) refers to a status or process of having a first moiety (*e.g*., an ASO) inside a second moiety (*e.g.,* an EV, *e.g.,* exosome) without chemically or physically linking the two moieties. In some aspects, the term "encapsulated" can be used interchangeably with "in the lumen of." Non-limiting examples of encapsulating a first moiety (*e.g.,* an ASO) into a second moiety (*e.g.,* EVs, *e.g.,* exosomes) are disclosed elsewhere herein.

As used herein, the term "producer cell" refers to a cell used for generating an EV, *e.g.,* exosome. A producer cell can be a cell cultured *in vitro,* or a cell *in vivo.* A producer cell includes, but not limited to, a cell known to be effective in generating EVs, *e.g.,* exosomes, *e.g.,* HEK293 cells, Chinese hamster ovary (CHO) cells, mesenchymal stem cells (MSCs), BJ human foreskin fibroblast cells, fHDF fibroblast cells, AGE.HN^{®} neuronal precursor cells, CAP^{®} amniocyte cells, adipose mesenchymal stem cells, RPTEC/TERT1 cells. In certain aspects, a producer cell is not an antigen-presenting cell. In some aspects, a producer cell is not a dendritic cell, a B cell, a mast cell, a macrophage, a neutrophil, Kupffer-Browicz cell, cell derived from any of these cells, or any combination thereof. In some aspects, the EVs, *e.g.,* exosomes useful in the present disclosure do not carry an antigen on MHC class I or class II molecule exposed on the surface of the EV, *e.g.,* exosome, but instead can carry an antigen in the lumen of the EV, *e.g.,* exosome or on the surface of the EV, *e.g.,* exosome by attachment to Scaffold X and/or Scaffold Y.

As used herein, the terms "isolate," "isolated," and "isolating" or "purify," "purified," and "purifying" as well as "extracted" and "extracting" are used interchangeably and refer to the state of a preparation (*e.g.,* a plurality of known or unknown amount and/or concentration) of desired EVs, that have undergone one or more processes of purification, *e.g.,* a selection or an enrichment of the desired EV preparation. In some aspects, isolating or purifying as used herein is the process of removing, partially removing (e.g., a fraction) of the EVs from a sample containing producer cells. In some aspects, an isolated EV composition has no detectable undesired activity or, alternatively, the level or amount of the undesired activity is at or below an acceptable level or amount. In other aspects, an isolated EV composition has an amount and/or concentration of desired EVs at or above an acceptable amount and/or concentration. In other aspects, the isolated EV composition is enriched as compared to the starting material (*e.g.,* producer cell preparations) from which the composition is obtained. This enrichment can be by 10%, 20%, 30%, 40%, 50%, 60%, 70%, 80%, 90%, 95%, 96%, 97%, 98%, 99%, 99.9%, 99.99%, 99.999%, 99.9999%, or greater than 99.9999% as compared to the starting material. In some aspects, isolated EV preparations are substantially free of residual biological products. In some aspects, the isolated EV preparations are 100% free, 99% free, 98% free, 97% free, 96% free, 95% free, 94% free, 93% free, 92% free, 91% free, or 90% free of any contaminating biological matter. Residual biological products can include abiotic materials (including chemicals) or unwanted nucleic acids, proteins, lipids, or metabolites. Substantially free of residual biological products can also mean that the EV composition contains no detectable producer cells and that only EVs are detectable.

As used herein, the term "payload" refers to an agent that acts on a target (*e.g.,* a target cell) that is contacted with the EV. A non-limiting examples of payload that can be included on the EV, *e.g.,* exosome, is an ASO. Payloads that can be introduced into an EV, *e.g.,* exosome, and/or a producer cell include agents such as, nucleotides (*e.g*., nucleotides comprising a detectable moiety or a toxin or that disrupt transcription), nucleic acids (*e.g.,* DNA or mRNA molecules that encode a polypeptide such as an enzyme, or RNA molecules that have regulatory function such as miRNA, dsDNA, lncRNA, and siRNA), amino acids (*e.g.,* amino acids comprising a detectable moiety or a toxin or that disrupt translation), polypeptides (*e.g.,* enzymes), lipids, carbohydrates, and small molecules (e.g., small molecule drugs and toxins). In certain aspects, a payload comprises an ASO. As used herein, the term "antibody" encompasses an immunoglobulin whether natural or partly or wholly synthetically produced, and fragments thereof. The term also covers any protein having a binding domain that is homologous to an immunoglobulin binding domain. "Antibody" further includes a polypeptide comprising a framework region from an immunoglobulin gene or fragments thereof that specifically binds and recognizes an antigen. As used herein, the term "antigen" refers to any agent that when introduced into a subject elicits an immune response (cellular or humoral) to itself. Use of the term antibody is meant to include whole antibodies, polyclonal, monoclonal and recombinant antibodies, fragments thereof, and further includes single-chain antibodies, humanized antibodies, murine antibodies, chimeric, mouse-human, mouse-primate, primate-human monoclonal antibodies, anti-idiotype antibodies, antibody fragments, such as, *e.g.,* scFv, (scFv)₂, Fab, Fab', and F(ab')₂, F(ab1)₂, Fv, dAb, and Fd fragments, diabodies, and antibody-related polypeptides. Antibody includes bispecific antibodies and multispecific antibodies so long as they exhibit the desired biological activity or function.

The terms "individual," "subject," "host," and "patient," are used interchangeably herein and refer to any mammalian subject for whom diagnosis, treatment, or therapy is desired, particularly humans. The compositions and methods described herein are applicable to both human therapy and veterinary applications. In some aspects, the subject is a mammal, and in other aspects the subject is a human. As used herein, a "mammalian subject" includes all mammals, including without limitation, humans, domestic animals (*e.g.,* dogs, cats and the like), farm animals (*e.g.,* cows, sheep, pigs, horses and the like) and laboratory animals (*e.g.,* monkey, rats, mice, rabbits, guinea pigs and the like).

The term "pharmaceutical composition" refers to a preparation which is in such form as to permit the biological activity of the active ingredient to be effective, and which contains no additional components which are unacceptably toxic to a subject to which the composition would be administered. Such composition can be sterile.

As used herein, the term "substantially free" means that the sample comprising EVs, *e.g.,* exosomes, comprise less than 10% of macromolecules by mass/volume (m/v) percentage concentration. Some fractions may contain less than 0.001%, less than 0.01%, less than 0.05%, less than 0.1%, less than 0.2%, less than 0.3%, less than 0.4%, less than 0.5%, less than 0.6%, less than 0.7%, less than 0.8%, less than 0.9%, less than 1%, less than 2%, less than 3%, less than 4%, less than 5%, less than 6%, less than 7%, less than 8%, less than 9%, or less than 10% (m/v) of macromolecules.

As used herein, the term "macromolecule" means nucleic acids, contaminant proteins, lipids, carbohydrates, metabolites, or a combination thereof.

As used herein, the term "conventional exosome protein" means a protein previously known to be enriched in exosomes, including but is not limited to CD9, CD63, CD81, PDGFR, GPI anchor proteins, lactadherin (MFGE8), LAMP2, and LAMP2B, a fragment thereof, or a peptide that binds thereto.

"Administering," as used herein, means to give a composition comprising an EV, e.g., exosome, disclosed herein to a subject via a pharmaceutically acceptable route. Routes of administration can be intravenous, e.g., intravenous injection and intravenous infusion. Additional routes of administration include, *e.g.,* subcutaneous, intramuscular, oral, nasal, and pulmonary administration. EVs, *e.g.,* exosomes can be administered as part of a pharmaceutical composition comprising at least one excipient.

An "effective amount" of, *e.g.,* an ASO or an extracellular vesicle as disclosed herein, is an amount sufficient to carry out a specifically stated purpose. An "effective amount" can be determined empirically and in a routine manner, in relation to the stated purpose.

"Treat," "treatment," or "treating," as used herein refers to, *e.g.,* the reduction in severity of a disease or condition; the reduction in the duration of a disease course; the amelioration or elimination of one or more symptoms associated with a disease or condition; the provision of beneficial effects to a subject with a disease or condition, without necessarily curing the disease or condition. The term also includes prophylaxis or prevention of a disease or condition or its symptoms thereof. In one aspect, the "treating" or "treatment" includes inducing hematopoiesis in a subject in need thereof. In some aspects, the disease or condition is associated with a hematopoiesis or a deficiency thereof. In certain aspects, the disease or condition is a cancer. In some aspects, the treating enhances hematopoiesis in a subject having a cancer, wherein the enhanced hematopoiesis comprises increased proliferation and/or differentiation of one or more immune cell in the subject

"Prevent" or "preventing," as used herein, refers to decreasing or reducing the occurrence or severity of a particular outcome. In some aspects, preventing an outcome is achieved through prophylactic treatment. In some aspects, an EV, *e.g.,* an exosome, comprising an ASO, described herein, is administered to a subject prophylactically. In some aspects, the subject is at risk of developing cancer. In some aspects, the subject is at risk of developing a hematopoietic disorder.

### II. Antisense Oligonucleotides (ASOs)

The present disclosure employs antisense oligonucleotides (ASOs) as defined in the claims for use in modulating the function of nucleic acid molecules encoding mammalian STAT6, such as the *STAT6* nucleic acid, *e.g., STAT6* transcript, including *STAT6* pre-mRNA, and *STAT6* mRNA, or naturally occurring variants of such nucleic acid molecules encoding mammalian STAT6. The term "ASO" in the context of the present disclosure, refers to a molecule formed by covalent linkage of two or more nucleotides (*i.e*., an oligonucleotide).

The EV, *e.g.,* the exosome, comprises at least one ASO. In some aspects, the EV, *e.g.,* the exosome, comprises at least two ASOs, *e.g.,* a first ASO comprising a first nucleotide sequence and a second ASO comprising a second nucleotide sequence. In some aspects, the EV, *e.g.,* the exosome, comprises at least three ASOs, at least four ASOs, at least five ASOs, at least six ASOs, or more than six ASOs. In some aspects, each of the first ASO, the second ASO, the third ASO, the fourth ASO, the fifth ASO, the sixth ASO, and/or the ninth ASO is different.

In some aspects, the EV, *e.g.* the exosome, comprises a first ASO and a second ASO, wherein the first ASO comprises a first nucleotide sequence that is complementary to a first target sequence in a first transcript, and wherein the second ASO comprises a second nucleotide sequence that is complementary to a second target sequence in the first transcript. In some aspects, the first target sequence does not overlap with the second target sequence. In some aspects, the first target sequence comprises at least one nucleotide that is within the 5'UTR of the transcript, and the second target sequence does not comprise a nucleotide that is within the 5'UTR. In some aspects, the first target sequence comprises at least one nucleotide that is within the 3'UTR of the transcript, and the second target sequence does not comprise a nucleotide that is within the 3'UTR. In some aspects that fall outside the scope of the appended claims, the first target sequence comprises at least one nucleotide that is within the 5'UTR of the transcript, and the second target sequence comprises at least one nucleotide that is within the 3'UTR.

In some aspects, the first ASO targets a sequence within an exon-intron junction, and the second ASO targets a sequence within an exon-intron junction. In some aspects, the first ASO targets a sequence within an exon-intron junction, and the second ASO targets a sequence within an exon. In some aspects, the first ASO targets a sequence within an exon-intron junction, and the second ASO targets a sequence within an intron. In some aspects, the first ASO targets a sequence within an exon, and the second ASO targets a sequence within an exon. In some aspects, the first ASO targets a sequence within an intron, and the second ASO targets a sequence within an exon. In some aspects, the first ASO targets a sequence within an intron, and the second ASO targets a sequence within an intron.

In some aspects, the EV, *e.g.* the exosome, comprises a first ASO and a second ASO, wherein the first ASO comprises a first nucleotide sequence that is complementary to a first target sequence in a first transcript, and wherein the second ASO comprises a second nucleotide sequence that is complementary to a second target sequence in a second transcript, wherein the first transcript is not the product of the same gene as the second transcript.

The ASO comprises a contiguous nucleotide sequence of from about 10 to about 30, such as 10-20, 14-20, 16-20, or 15-25, nucleotides in length. In certain aspects, the ASO is 20 nucleotides in length. In certain aspects, the ASO is 18 nucleotides in length. In certain aspects, the ASO is 19 nucleotides in length. In certain aspects, the ASO is 17 nucleotides in length. In certain aspects, the ASO is 16 nucleotides in length. In certain aspects, the ASO is 15 nucleotides in length.

The ASO comprises a contiguous nucleotide sequence of from about 10 to about 30 nucleotides in length. In certain aspects, the ASO is 21 nucleotides in length. In certain aspects, the ASO is 22 nucleotides in length. In certain aspects, the ASO is 23 nucleotides in length. In certain aspects, the ASO is 24 nucleotides in length. In certain aspects, the ASO is 25 nucleotides in length. In certain aspects, the ASO is 26 nucleotides in length. In certain aspects, the ASO is 27 nucleotides in length. In certain aspects, the ASO is 28 nucleotides in length. In certain aspects, the ASO is 29 nucleotides in length. In certain aspects, the ASO is 30 nucleotides in length. In certain aspects, the ASO is 31 nucleotides in length. In certain aspects, the ASO is 32 nucleotides in length. In certain aspects, the ASO is 33 nucleotides in length. In certain aspects, the ASO is 34 nucleotides in length. In certain aspects, the ASO is 35 nucleotides in length. In certain aspects, the ASO is 36 nucleotides in length. In certain aspects, the ASO is 37 nucleotides in length. In certain aspects, the ASO is 38 nucleotides in length. In certain aspects, the ASO is 39 nucleotides in length. In certain aspects, the ASO is 40 nucleotides in length. In certain aspects, the ASO is 41 nucleotides in length. In certain aspects, the ASO is 42 nucleotides in length. In certain aspects, the ASO is 43 nucleotides in length. In certain aspects, the ASO is 44 nucleotides in length. In certain aspects, the ASO is 45 nucleotides in length. In certain aspects, the ASO is 46 nucleotides in length. In certain aspects, the ASO is 47 nucleotides in length. In certain aspects, the ASO is 48 nucleotides in length. In certain aspects, the ASO is 49 nucleotides in length. In certain aspects, the ASO is 50 nucleotides in length.

The terms "antisense ASO," "antisense oligonucleotide," and "oligomer" as used herein are interchangeable with the term "ASO."

A reference to a SEQ ID number includes a particular nucleobase sequence, but does not include any design or full chemical structure. Furthermore, the ASOs disclosed in the figures herein show a representative design, but are not limited to the specific design shown in the figures unless otherwise indicated. For example, when a claim (or this specification) refers to SEQ ID NO: 101, it includes the nucleotide sequence of SEQ ID NO: 101 only. The design of any ASO disclosed herein can be written as SEQ ID NO: XX, wherein each of the first nucleotide, the second nucleotide, the third nucleotide, the first nucleotide, the second nucleotide, and the Nth nucleotide from the 5' end is a modified nucleotide, e.g., LNA, and each of the other nucleotides is a non-modified nucleotide (e.g., DNA).

In various aspects, the ASO of the disclosure does not comprise RNA (units). In some aspects, the ASO comprises one or more DNA units. In one aspect, the ASO according to the disclosure is a linear molecule or is synthesized as a linear molecule. In some aspects, the ASO is a single stranded molecule, and does not comprise short regions of, for example, at least 3, 4 or 5 contiguous nucleotides, which are complementary to equivalent regions within the same ASO (*i.e.* duplexes) - in this regard, the ASO is not (essentially) double stranded. In some aspects, the ASO is essentially not double stranded. In some aspects, the ASO is not a siRNA. In various aspects, the ASO of the disclosure can consist entirely of the contiguous nucleotide region. Thus, in some aspects the ASO is not substantially self-complementary.

In other aspects, falling outside the scope of the appended claims, the present disclosure includes fragments of ASOs. For example, the disclosure includes at least one nucleotide, at least two contiguous nucleotides, at least three contiguous nucleotides, at least four contiguous nucleotides, at least five contiguous nucleotides, at least six contiguous nucleotides, at least seven contiguous nucleotides, at least eight contiguous nucleotides, or at least nine contiguous nucleotides of the ASOs disclosed herein.

In some aspects, the ASOs for the present disclosure include a phosphorodiamidate Morpholino oligomer (PMO) or a peptide-conjugated phosphorodiamidate morpholino oligomer (PPMO).

### II.A. The Target

Suitably the ASO of the disclosure is capable of down-regulating (*e.g.,* reducing or removing) expression of the *STAT6* mRNA or STAT6 protein. In this regard, the ASO of the disclosure can promote differentiation of M2 macrophages and/or decrease the differentiation of M1 macrophages. In particular, the present disclosure is directed to ASOs as defined in the claims that target one or more regions of the *STAT6* pre-mRNA *(e.g.,* intron regions, exon regions, and/or exon-intron junction regions).

Unless indicated otherwise, the term "STAT6," as used herein, can refer to STAT6 from one or more species (*e.g.,* humans, non-human primates, dogs, cats, guinea pigs, rabbits, rats, mice, horses, cattle, and bears).

STAT6 (*STAT6*) is also known as signal transducer and activator of transcription 6. Synonyms of STAT6/STAT6 are known and include IL-4 STAT; STAT, Interleukin4-Induced; Transcription Factor IL-4 STAT; STAT6B; STAT6C; and D12S1644. The sequence for the human *STAT6* gene can be found under publicly available GenBank Accession Number NC_000012.12:c57111413-57095404. The human *STAT6* gene is found at chromosome location 12q13.3 at 57111413-57095404, complement.

The sequence for the human *STAT6* pre-mRNA transcript (SEQ ID NO: 1) corresponds to the reverse complement of residues 57111413-57095404, complement, of chromosome 12q13.3. The *STAT6* mRNA sequence (GenBank Accession No. NM_001178078.1) is provided in SEQ ID NO: 3 (Table 1), except that the nucleotide "t" in SEQ ID NO: 3 is shown as "u" in the mRNA. The sequence for human STAT6 protein can be found under publicly available Accession Numbers: P42226-1, (canonical sequence, SEQ ID NO: 2; Table 1), P42226-2 (SEQ ID NO: 4), and P42226-3 (SEQ ID NO: 5).

**Table 1. STAT6 mRNA and Protein Sequences**

| |
|---|
| ***STAT6* mRNA Sequence** |
| GGGGCAGCCACTGCTTACACTGAAGAGGGAGGACGGAGAGGAGTGTGTGTGTGTGTGTGT GTGTGTGTGTATGTATGTGTGTGCTTTATCTTATTTTTCTTTTTGGTGGTGGTGGTGGAAGGG GGGAGGTGCTAGCAGGGCCAGCCTTGAACTCGCTGGACAGAGCTACAGACCTATGGGGCCTGG |
| AAGTGCCCGCTGAGAAAGGAGAAGACAGCAGAGGGGTTGCCGAGGCAACCTCCAAGTCCCAG ATCATGTCTCTGTGGGGTCTGGTCTCCAAGATGCCCCCAGAAAAAGTGCAGCGGCTCTATGTC GACTTTCCCCAACACCTGCGGCATCTTCTGGGTGACTGGCTGGAGAGCCAGCCCTGGGAGTTC CTGGTCGGCTCCGACGCCTTCTGCTGCAACTTGGCTAGTGCCCTACTTTCAGACACTGTCCAG CACCTTCAGGCCTCGGTGGGAGAGCAGGGGGAGGGGAGCACCATCTTGCAACACATCAGCACC CTTGAGAGCATATATCAGAGGGACCCCCTGAAGCTGGTGGCCACTTTCAGACAAATACTTCAA GGAGAGAAAAAAGCTGTTATGGAACAGTTCCGCCACTTGCCAATGCCTTTCCACTGGAAGCAG GAAGAACTCAAGTTTAAGACAGGCTTGCGGAGGCTGCAGCACCGAGTAGGGGAGATCCACCTT CTCCGAGAAGCCCTGCAGAAGGGGGCTGAGGCTGGCCAAGTGTCTCTGCACAGCTTGATAGAA ACTCCTGCTAATGGGACTGGGCCAAGTGAGGCCCTGGCCATGCTACTGCAGGAGACCACTGGA GAGCTAGAGGCAGCCAAAGCCCTAGTGCTGAAGAGGATCCAGATTTGGAAACGGCAGCAGCAG CTGGCAGGGAATGGCGCACCGTTTGAGGAGAGCCTGGCCCCACTCCAGGAGAGGTGTGAAAGC CTGGTGGACATTTATTCCCAGCTACAGCAGGAGGTAGGGGCGGCTGGTGGGGAGCTTGAGCCC AAGACCCGGGCATCGCTGACTGGCCGGCTGGATGAAGTCCTGAGAACCCTCGTCACCAGTTGC TTCCTGGTGGAGAAGCAGCCCCCCCAGGTACTGAAGACTCAGACCAAGTTCCAGGCTGGAGTT CGATTCCTGTTGGGCTTGAGGTTCCTGGGGGCCCCAGCCAAGCCTCCGCTGGTCAGGGCCGAC ATGGTGACAGAGAAGCAGGCGCGGGAGCTGAGTGTGCCTCAGGGTCCTGGGGCTGGAGCAGAA AGCACTGGAGAAATCATCAACAACACTGTGCCCTTGGAGAACAGCATTCCTGGGAACTGCTGC TCTGCCCTGTTCAAGAACCTGCTTCTCAAGAAGATCAAGCGGTGTGAGCGGAAGGGCACTGAG TCTGTCACAGAGGAGAAGTGCGCTGTGCTCTTCTCTGCCAGCTTCACACTTGGCCCCGGCAAA CTCCCCATCCAGCTCCAGGCCCTGTCTCTGCCCCTGGTGGTCATCGTCCATGGCAACCAAGAC AACAATGCCAAAGCCACTATCCTGTGGGACAATGCCTTCTCTGAGATGGACCGCGTGCCCTTT GTGGTGGCTGAGCGGGTGCCCTGGGAGAAGATGTGTGAAACTCTGAACCTGAAGTTCATGGCT GAGGTGGGGACCAACCGGGGGCTGCTCCCAGAGCACTTCCTCTTCCTGGCCCAGAAGATCTTC AATGACAACAGCCTCAGTATGGAGGCCTTCCAGCACCGTTCTGTGTCCTGGTCGCAGTTCAAC AAGGAGATCCTGCTGGGCCGTGGCTTCACCTTTTGGCAGTGGTTTGATGGTGTCCTGGACCTC ACCAAACGCTGTCTCCGGAGCTACTGGTCTGACCGGCTGATCATTGGCTTCATCAGCAAACAG TACGTTACTAGCCTTCTTCTCAATGAGCCCGACGGAACCTTTCTCCTCCGCTTCAGCGACTCA GAGATTGGGGGCATCACCATTGCCCATGTCATCCGGGGCCAGGATGGCTCTCCACAGATAGAG AACATCCAGCCATTCTCTGCCAAAGACCTGTCCATTCGCTCACTGGGGGACCGAATCCGGGAT CTTGCTCAGCTCAAAAATCTCTATCCCAAGAAGCCCAAGGATGAGGCTTTCCGGAGCCACTAC AAGCCTGAACAGATGGGTAAGGATGGCAGGGGTTATGTCCCAGCTACCATCAAGATGACCGTG GAAAGGGACCAACCACTTCCTACCCCAGAGCTCCAGATGCCTACCATGGTGCCTTCTTATGAC CTTGGAATGGCCCCTGATTCCTCCATGAGCATGCAGCTTGGCCCAGATATGGTGCCCCAGGTG TACCCACCACACTCTCACTCCATCCCCCCGTATCAAGGCCTCTCCCCAGAAGAATCAGTCAAC GTGTTGTCAGCCTTCCAGGAGCCTCACCTGCAGATGCCCCCCAGCCTGGGCCAGATGAGCCTG CCCTTTGACCAGCCTCACCCCCAGGGCCTGCTGCCGTGCCAGCCTCAGGAGCATGCTGTGTCC AGCCCTGACCCCCTGCTCTGCTCAGATGTGACCATGGTGGAAGACAGCTGCCTGAGCCAGCCA GTGACAGCGTTTCCTCAGGGCACTTGGATTGGTGAAGACATATTCCCTCCTCTGCTGCCTCCC ACTGAACAGGACCTCACTAAGCTTCTCCTGGAGGGGCAAGGGGAGTCGGGGGGAGGGTCCTTG GGGGCACAGCCCCTCCTGCAGCCCTCCCACTATGGGCAATCTGGGATCTCAATGTCCCACATG GACCTAAGGGCCAACCCCAGTTGGTGATCCCAGCTGGAGGGAGAACCCAAAGAGACAGCTCTT CTACTACCCCCACAGACCTGCTCTGGACACTTGCTCATGCCCTGCCAAGCAGCAGATGGGGAG GGTGCCCTCCTATCCCCACCTACTCCTGGGTCAGGAGGAAAAGACTAACAGGAGAATGCACAG TGGGTGGAGCCAATCCACTCCTTCCTTTCTATCATTCCCCTGCCCACCTCCTTCCAGCACTGA CTGGAAGGGAAGTTCAGGCTCTGAGACACACCCCAACATGCCTGCACCTGCAGCGCGCACACG CACGCACACACACACATACAGAGCTCTCTGAGGGTGATGGGGCTGAGCAGGAGGGGGGCTGGGTA AGAGCACAGGTTAGGGCATGGAAGGCTTCTCCGCCCATTCTGACCCAGGGCCTAGGACGGATA GGCAGGAACATACAGACACATTTACACTAGAGGCCAGGGATAGAGGATATTGGGTCTCAGCCC TAGGGGAATGGGAAGCAGCTCAAGGGACCCTGGGTGGGAGCATAGGAGGGGTCTGGACATGTG |
| GTTACTAGTACAGGTTTTGCCCTGATTAAAAAATCTCCCAAAGCCCCAAATTCCTGTTAGCCA GGTGGAGGCTTCTGATACGTGTATGAGACTATGCAAAAGTACAAGGGCTGAGATTCTTCGTGT ATAGCTGTGTGAACGTGTATGTACCTAGGATATGTTAAATGTATAGCTGGCACCTTAGTTGCA TGACCACATAGAACATGTGTCTATCTGCTTTTGCCTACGTGACAACACAAATTTGGGAGGGTG AGACACTGCACAGAAGACAGCAGCAAGTGTGCTGGCCTCTCTGACATATGCTAACCCCCAAAT ACTCTGAATTTGGAGTCTGACTGTGCCCAAGTGGGTCCAAGTGGCTGTGACATCTACGTATGG CTCCACACCTCCAATGCTGCCTGGGAGCCAGGGTGAGAGTCTGGGTCCAGGCCTGGCCATGTG GCCCTCCAGTGTATGAGAGGGCCCTGCCTGCTGCATCTTTTCTGTTGCCCCATCCACCGCCAG CTTCCCTTCACTCCCCTATCCCATTCTCCCTCTCAAGGCAGGGGTCATAGATCCTAAGCCATA AAATAAATTTTATTCCAAAATAACAAAATAAATAATCTACTGTACACAATCTGAAAA   (SEQ ID NO: 3) |
| **STAT6 Protein Sequence** |
| MSLWGLVSKMPPEKVQRLYVDFPQHLRHLLGDWLESQPWEFLVGSDAFCCNLASALLSDTVQH LQASVGEQGEGSTILQHISTLESIYQRDPLKLVATFRQILQGEKKAVMEQFRHLPMPFHWKQE ELKFKTGLRRLQHRVGEIHLLREALQKGAEAGQVSLHSLIETPANGTGPSEALAMLLQETTGE LEAAKALVLKRIQIWKRQQQLAGNGAPFEESLAPLQERCESLVDIYSQLQQEVGAAGGELEPK TRASLTGRLDEVLRTLVTSCFLVEKQPPQVLKTQTKFQAGVRFLLGLRFLGAPAKPPLVRADM VTEKQARELSVPQGPGAGAESTGEIINNTVPLENSIPGNCCSALFKNLLLKKIKRCERKGTES VTEEKCAVLFSASFTLGPGKLPIQLQALSLPLVVIVHGNQDNNAKATILWDNAFSEMDRVPFV VAERVPWEKMCETLNLKFMAEVGTNRGLLPEHFLFLAQKIFNDNSLSMEAFQHRSVSWSQFNK EILLGRGFTFWQWFDGVLDLTKRCLRSYWSDRLIIGFISKQYVTSLLLNEPDGTFLLRFSDSE IGGITIAHVIRGQDGSPQIENIQPFSAKDLSIRSLGDRIRDLAQLKNLYPKKPKDEAFRSHYK PEQMGKDGRGYVPATIKMTVERDQPLPTPELQMPTMVPSYDLGMAPDSSMSMQLGPDMVPQVY PPHSHSIPPYQGLSPEESVNVLSAFQEPHLQMPPSLGQMSLPFDQPHPQGLLPCQPQEHAVSS PDPLLCSDVTMVEDSCLSQPVTAFPQGTWIGEDIFPPLLPPTEQDLTKLLLEGQGESGGGSLG AQPLLQPSHYGQSGISMSHMDLRANPSW (SEQ ID NO: 2) |

Natural variants of the human *STAT6* gene product are known. For example, natural variants of human STAT6 protein can contain one or more amino acid substitutions selected from: M118R, D419N, and any combination thereof. Additional variants of human STAT6 protein resulting from alternative splicing are also known in the art. STAT6 Isoform 2 (identifier: P42226-2 at UniProt) differs from the canonical sequence (SEQ ID NO: 3) as follows: deletion of residues 1-174 and substitution of ₁₇₅PSE₁₇₇ with ₁₇₅MEQ₁₇₇ relative to SEQ ID NO: 3. The sequence of STAT6 Isoform 3 (identifier: P42226-3) differs from the canonical sequence (SEQ ID NO: 3) as follows: deletion of residues 1-110 relative to SEQ ID NO: 3. Therefore, the ASOs of the present disclosure can be designed to reduce or inhibit expression of the natural variants of the STAT6 protein.

An example of a target nucleic acid sequence of the ASOs is *STAT6* pre-mRNA. SEQ ID NO: 1 represents a human *STAT6* genomic sequence *(i.e.,* reverse complement of nucleotides 57111413-57095404, complement, of chromosome 12q13.3). SEQ ID NO: 1 is identical to a *STAT6* pre-mRNA sequence except that nucleotide "t" in SEQ ID NO: 1 is shown as "u" in pre-mRNA. In certain aspects, the "target nucleic acid" comprises an intron of a STAT6 protein-encoding nucleic acids or naturally occurring variants thereof, and RNA nucleic acids derived therefrom, *e.g.,* pre-mRNA. In other aspects, the target nucleic acid comprises an exon region of a STAT6 protein-encoding nucleic acids or naturally occurring variants thereof, and RNA nucleic acids derived therefrom, *e.g.,* pre-mRNA. In yet other aspects, the target nucleic acid comprises an exon-intron junction of a STAT6 protein-encoding nucleic acids or naturally occurring variants thereof, and RNA nucleic acids derived therefrom, *e.g.,* pre-mRNA. In some aspects, for example when used in research or diagnostics the "target nucleic acid" can be a cDNA or a synthetic oligonucleotide derived from the above DNA or RNA nucleic acid targets. The human STAT6 protein sequence encoded by the *STAT6* pre-mRNA is shown as SEQ ID NO: 3. In other aspects, the target nucleic acid comprises an untranslated region of a STAT6 protein-encoding nucleic acids or naturally occurring variants thereof, *e.g.,* 5' UTR, 3' UTR, or both.

In some aspects falling outside the scope of the appended claims, an ASO of the disclosure hybridizes to a region within the introns of a *STAT6* transcript, *e.g.,* SEQ ID NO: 1. In certain aspects, an ASO of the disclosure hybridizes to a region within the exons of a *STAT6* transcript, *e.g.,* SEQ ID NO: 1. In other aspects, an ASO of the disclosure hybridizes to a region within the exon-intron junction of a *STAT6* transcript, *e.g.,* SEQ ID NO: 1. In some aspects, an ASO of the disclosure hybridizes to a region within a *STAT6* transcript *(e.g.,* an intron, exon, or exon-intron junction), *e.g.,* SEQ ID NO: 1, wherein the ASO has a design according to formula: 5' A-B-C 3' as described elsewhere herein.

In some aspects, the ASO targets a mRNA encoding a particular isoform of STAT6 protein (*e.g.,* Isoform 1). In some aspects, the ASO targets all isoforms of STAT6 protein. In other aspects, the ASO targets two isoforms (*e.g.,* Isoform 1 and Isoform 2, Isoform 1 and Isoform 3, or Isoform 2 and Isoform 3) of STAT6 protein.

The ASO comprises a contiguous nucleotide sequence (*e.g.,* 10 to 30 nucleotides in length, *e.g.,* 20 nucleotides in length) that are complementary to a nucleic acid sequence within a *STAT6* transcript, *e.g.,* a region corresponding to SEQ ID NO: 1 or SEQ ID NO: 3, as defined in the claims. In some aspects, the contiguous nucleotide sequence defined in the claims hybridizes to a nucleic acid sequence, or a region within the sequence, of a *STAT6* transcript ("target region"), wherein the nucleic acid sequence corresponds to nucleotides 1000-1500 of SEQ ID NO: 3 and wherein, optionally, the ASO has one of the designs described herein or a chemical structure shown elsewhere herein.

In some aspects, the contiguous nucleotide sequence defined in the claims hybridizes to a nucleic acid sequence, or a region within the sequence, of a *STAT6* transcript ("target region"), wherein the nucleic acid sequence corresponds to nucleotides 952-1688 of SEQ ID NO: 3 and wherein, optionally, the ASO has one of the designs described herein or a chemical structure shown elsewhere herein.

In some aspects, the contiguous nucleotide sequence defined in the claims hybridizes to a nucleic acid sequence, or a region within the sequence, of a *STAT6* transcript ("target region"), wherein the nucleic acid sequence corresponds to nucleotides 1002-1638 of SEQ ID NO: 3 and wherein, optionally, the ASO has one of the designs described herein or a chemical structure shown elsewhere herein.

In some aspects, the contiguous nucleotide sequence defined in the claims hybridizes to a nucleic acid sequence, or a region within the sequence, of a *STAT6* transcript ("target region"), wherein the nucleic acid sequence corresponds to nucleotides 1042-1598 of SEQ ID NO: 3 and wherein, optionally, the ASO has one of the designs described herein or a chemical structure shown elsewhere herein.

In some aspects, the target region corresponds to nucleotides 1053-1067 of SEQ ID NO: 3 (e.g., ASO-STAT6-1053; SEQ ID NO: 91).

The target regions listed in this paragraph fall outside the scope of the appended claims. In some aspects, the target region corresponds to nucleotides 1359-1373 of SEQ ID NO: 3 (e.g., ASO-STAT6-1359; SEQ ID NO: 92). In some aspects, the target region corresponds to nucleotides 1890-1904 of SEQ ID NO: 3 (e.g., ASO-STAT6-1890; SEQ ID NO: 93). In some aspects, the target region corresponds to nucleotides 1892-1906 of SEQ ID NO: 3 (e.g., ASO-STAT6-1892; SEQ ID NO: 94). In some aspects, the target region corresponds to nucleotides 1915-1929 of SEQ ID NO: 3 (e.g., ASO-STAT6-1915; SEQ ID NO: 95). In some aspects, the target region corresponds to nucleotides 1916-1930 of SEQ ID NO: 3 (e.g., ASO-STAT6-1916; SEQ ID NO: 96). In some aspects, the target region corresponds to nucleotides 1917-1931 of SEQ ID NO: 3 (e.g., ASO-STAT6-1917; SEQ ID NO: 97). In some aspects, the target region corresponds to nucleotides 1918-1932 of SEQ ID NO: 3 (e.g., ASO-STAT6-1918; SEQ ID NO: 98). In some aspects, the target region corresponds to nucleotides 1919-1933 of SEQ ID NO: 3 (e.g., ASO-STAT6-1919; SEQ ID NO: 99). In some aspects, the target region corresponds to nucleotides 1920-1934 of SEQ ID NO: 3 (e.g., ASO-STAT6-1920; SEQ ID NO: 100). In some aspects, the target region corresponds to nucleotides 1937-1951 of SEQ ID NO: 3 (e.g., ASO-STAT6-1937; SEQ ID NO: 101). In some aspects, the target region corresponds to nucleotides 1938-1952 of SEQ ID NO: 3 (e.g., ASO-STAT6-1938; SEQ ID NO: 102). In some aspects, the target region corresponds to nucleotides 2061-2075 of SEQ ID NO: 3 (e.g., ASO-STAT6-2061; SEQ ID NO: 103). In some aspects, the target region corresponds to nucleotides 2062-2076 of SEQ ID NO: 3 (e.g., ASO-STAT6-2062; SEQ ID NO: 104). In some aspects, the target region corresponds to nucleotides 2063-2077 of SEQ ID NO: 3 (e.g., ASO-STAT6-2063; SEQ ID NO: 105). In some aspects, the target region corresponds to nucleotides 2064-2078 of SEQ ID NO: 3 (e.g., ASO-STAT6-2064; SEQ ID NO: 106). In some aspects, the target region corresponds to nucleotides 2066-2080 of SEQ ID NO: 3 (e.g., ASO-STAT6-2066; SEQ ID NO: 107). In some aspects, the target region corresponds to nucleotides 2067-2081 of SEQ ID NO: 3 (e.g., ASO-STAT6-2067; SEQ ID NO: 108). In some aspects, the target region corresponds to nucleotides 2068-2082 of SEQ ID NO: 3 (e.g., ASO-STAT6-2068; SEQ ID NO: 109). In some aspects, the target region corresponds to nucleotides 2352-2366 of SEQ ID NO: 3 (e.g., ASO-STAT6-2352; SEQ ID NO: 110). In some aspects, the target region corresponds to nucleotides 3073-3087 of SEQ ID NO: 3 (e.g., ASO-STAT6-3073; SEQ ID NO: 111). In some aspects, the target region corresponds to nucleotides 1053-1068 of SEQ ID NO: 3 (e.g., ASO-STAT6-1053; SEQ ID NO: 112). In some aspects, the target region corresponds to nucleotides 1054-1069 of SEQ ID NO: 3 (e.g., ASO-STAT6-1054; SEQ ID NO: 113). In some aspects, the target region corresponds to nucleotides 1356-1371 of SEQ ID NO: 3 (e.g., ASO-STAT6-1356; SEQ ID NO: 114). In some aspects, the target region corresponds to nucleotides 1847-1862 of SEQ ID NO: 3 (e.g., ASO-STAT6-1847; SEQ ID NO: 115). In some aspects, the target region corresponds to nucleotides 1886-1901 of SEQ ID NO: 3 (e.g., ASO-STAT6-1886; SEQ ID NO: 116). In some aspects, the target region corresponds to nucleotides 1887-1902 of SEQ ID NO: 3 (e.g., ASO-STAT6-1887; SEQ ID NO: 117). In some aspects, the target region corresponds to nucleotides 1888-1903 of SEQ ID NO: 3 (e.g., ASO-STAT6-1888; SEQ ID NO: 118). In some aspects, the target region corresponds to nucleotides 1889-1904 of SEQ ID NO: 3 (e.g., ASO-STAT6-1889; SEQ ID NO: 119). In some aspects, the target region corresponds to nucleotides 1890-1905 of SEQ ID NO: 3 (e.g., ASO-STAT6-1890; SEQ ID NO: 120). In some aspects, the target region corresponds to nucleotides 1893-1908 of SEQ ID NO: 3 (e.g., ASO-STAT6-1893; SEQ ID NO: 121). In some aspects, the target region corresponds to nucleotides 1917-1932 of SEQ ID NO: 3 (e.g., ASO-STAT6-1917; SEQ ID NO: 122). In some aspects, the target region corresponds to nucleotides 1919-1934 of SEQ ID NO: 3 (e.g., ASO-STAT6-1919; SEQ ID NO: 123). In some aspects, the target region corresponds to nucleotides 2056-2071 of SEQ ID NO: 3 (e.g., ASO-STAT6-2056; SEQ ID NO: 124). In some aspects, the target region corresponds to nucleotides 2060-2075 of SEQ ID NO: 3 (e.g., ASO-STAT6-2060; SEQ ID NO: 125). In some aspects, the target region corresponds to nucleotides 2066-2081 of SEQ ID NO: 3 (e.g., ASO-STAT6-2066; SEQ ID NO: 126). In some aspects, the target region corresponds to nucleotides 2070-2085 of SEQ ID NO: 3 (e.g., ASO-STAT6-2070; SEQ ID NO: 127). In some aspects, the target region corresponds to nucleotides 2351-2366 of SEQ ID NO: 3 (e.g., ASO-STAT6-2351; SEQ ID NO: 128). In some aspects, the target region corresponds to nucleotides 2352-2367 of SEQ ID NO: 3 (e.g., ASO-STAT6-2352; SEQ ID NO: 129). In some aspects, the target region corresponds to nucleotides 2359-2374 of SEQ ID NO: 3 (e.g., ASO-STAT6-2359; SEQ ID NO: 130). In some aspects, the target region corresponds to nucleotides 3633-3648 of SEQ ID NO: 3 (e.g., ASO-STAT6-3633; SEQ ID NO: 131). In some aspects, the target region corresponds to nucleotides 673-689 of SEQ ID NO: 3 (e.g., ASO-STAT6-673; SEQ ID NO: 132). In some aspects, the target region corresponds to nucleotides 1052-1068 of SEQ ID NO: 3 (e.g., ASO-STAT6-1052; SEQ ID NO: 133). In some aspects, the target region corresponds to nucleotides 1356-1372 of SEQ ID NO: 3 (e.g., ASO-STAT6-1356; SEQ ID NO: 134). In some aspects, the target region corresponds to nucleotides 1357-1373 of SEQ ID NO: 3 (e.g., ASO-STAT6-1357; SEQ ID NO: 135). In some aspects, the target region corresponds to nucleotides 1359-1375 of SEQ ID NO: 3 (e.g., ASO-STAT6-1359; SEQ ID NO: 136). In some aspects, the target region corresponds to nucleotides 1360-1376 of SEQ ID NO: 3 (e.g., ASO-STAT6-1360; SEQ ID NO: 137). In some aspects, the target region corresponds to nucleotides 1839-1855 of SEQ ID NO: 3 (e.g., ASO-STAT6-1839; SEQ ID NO: 138). In some aspects, the target region corresponds to nucleotides 1848-1864 of SEQ ID NO: 3 (e.g., ASO-STAT6-1848; SEQ ID NO: 139). In some aspects, the target region corresponds to nucleotides 1849-1865 of SEQ ID NO: 3 (e.g., ASO-STAT6-1849; SEQ ID NO: 140). In some aspects, the target region corresponds to nucleotides 1891-1907 of SEQ ID NO: 3 (e.g., ASO-STAT6-1891; SEQ ID NO: 141). In some aspects, the target region corresponds to nucleotides 1915-1931 of SEQ ID NO: 3 (e.g., ASO-STAT6-1915; SEQ ID NO: 142). In some aspects, the target region corresponds to nucleotides 1916-1932 of SEQ ID NO: 3 (e.g., ASO-STAT6-1916; SEQ ID NO: 143). In some aspects, the target region corresponds to nucleotides 1917-1933 of SEQ ID NO: 3 (e.g., ASO-STAT6-1917; SEQ ID NO: 144). In some aspects, the target region corresponds to nucleotides 1938-1954 of SEQ ID NO: 3 (e.g., ASO-STAT6-1938; SEQ ID NO: 145). In some aspects, the target region corresponds to nucleotides 1939-1955 of SEQ ID NO: 3 (e.g., ASO-STAT6-1939; SEQ ID NO: 146). In some aspects, the target region corresponds to nucleotides 2063-2079 of SEQ ID NO: 3 (e.g., ASO-STAT6-2063; SEQ ID NO: 147). In some aspects, the target region corresponds to nucleotides 2064-2080 of SEQ ID NO: 3 (e.g., ASO-STAT6-2064; SEQ ID NO: 148). In some aspects, the target region corresponds to nucleotides 2065-2081 of SEQ ID NO: 3 (e.g., ASO-STAT6-2065; SEQ ID NO: 149). In some aspects, the target region corresponds to nucleotides 2066-2082 of SEQ ID NO: 3 (e.g., ASO-STAT6-2066; SEQ ID NO: 150). In some aspects, the target region corresponds to nucleotides 2068-2084 of SEQ ID NO: 3 (e.g., ASO-STAT6-2068; SEQ ID NO: 151). In some aspects, the target region corresponds to nucleotides 2187-2203 of SEQ ID NO: 3 (e.g., ASO-STAT6-2187; SEQ ID NO: 152). In some aspects, the target region corresponds to nucleotides 2350-2366 of SEQ ID NO: 3 (e.g., ASO-STAT6-2350; SEQ ID NO: 153). In some aspects, the target region corresponds to nucleotides 2351-2367 of SEQ ID NO: 3 (e.g., ASO-STAT6-2351; SEQ ID NO: 154). In some aspects, the target region corresponds to nucleotides 2352-2368 of SEQ ID NO: 3 (e.g., ASO-STAT6-2352; SEQ ID NO: 155). In some aspects, the target region corresponds to nucleotides 2357-2373 of SEQ ID NO: 3 (e.g., ASO-STAT6-2357; SEQ ID NO: 156). In some aspects, the target region corresponds to nucleotides 513-532 of SEQ ID NO: 3 (e.g., ASO-STAT6-513; SEQ ID NO: 157). In some aspects, the target region corresponds to nucleotides 671-690 of SEQ ID NO: 3 (e.g., ASO-STAT6-671; SEQ ID NO: 158). In some aspects, the target region corresponds to nucleotides 1131-1150 of SEQ ID NO: 3 (e.g., ASO-STAT6-1131; SEQ ID NO: 159). In some aspects, the target region corresponds to nucleotides 1354-1373 of SEQ ID NO: 3 (e.g., ASO-STAT6-1354; SEQ ID NO: 160). In some aspects, the target region corresponds to nucleotides 1355-1374 of SEQ ID NO: 3 (e.g., ASO-STAT6-1355; SEQ ID NO: 161). In some aspects, the target region corresponds to nucleotides 1356-1375 of SEQ ID NO: 3 (e.g., ASO-STAT6-1356; SEQ ID NO: 162). In some aspects, the target region corresponds to nucleotides 1432-1451 of SEQ ID NO: 3 (e.g., ASO-STAT6-1432; SEQ ID NO: 163). In some aspects, the target region corresponds to nucleotides 1555-1574 of SEQ ID NO: 3 (e.g., ASO-STAT6-1555; SEQ ID NO: 164). In some aspects, the target region corresponds to nucleotides 1556-1575 of SEQ ID NO: 3 (e.g., ASO-STAT6-1556; SEQ ID NO: 165). In some aspects, the target region corresponds to nucleotides 1557-1576 of SEQ ID NO: 3 (e.g., ASO-STAT6-1557; SEQ ID NO: 166). In some aspects, the target region corresponds to nucleotides 1558-1577 of SEQ ID NO: 3 (e.g., ASO-STAT6-1558; SEQ ID NO: 167). In some aspects, the target region corresponds to nucleotides 1826-1845 of SEQ ID NO: 3 (e.g., ASO-STAT6-1826; SEQ ID NO: 168). In some aspects, the target region corresponds to nucleotides 1827-1846 of SEQ ID NO: 3 (e.g., ASO-STAT6-1827; SEQ ID NO: 169). In some aspects, the target region corresponds to nucleotides 1833-1852 of SEQ ID NO: 3 (e.g., ASO-STAT6-1833; SEQ ID NO: 170). In some aspects, the target region corresponds to nucleotides 1843-1862 of SEQ ID NO: 3 (e.g., ASO-STAT6-1843; SEQ ID NO: 171). In some aspects, the target region corresponds to nucleotides 1846-1865 of SEQ ID NO: 3 (e.g., ASO-STAT6-1846; SEQ ID NO: 172). In some aspects, the target region corresponds to nucleotides 1847-1866 of SEQ ID NO: 3 (e.g., ASO-STAT6-1847; SEQ ID NO: 173). In some aspects, the target region corresponds to nucleotides 1883-1902 of SEQ ID NO: 3 (e.g., ASO-STAT6-1883; SEQ ID NO: 174). In some aspects, the target region corresponds to nucleotides 1889-1908 of SEQ ID NO: 3 (e.g., ASO-STAT6-1889; SEQ ID NO: 175). In some aspects, the target region corresponds to nucleotides 1890-1909 of SEQ ID NO: 3 (e.g., ASO-STAT6-1890; SEQ ID NO: 176). In some aspects, the target region corresponds to nucleotides 1891-1910 of SEQ ID NO: 3 (e.g., ASO-STAT6-1891; SEQ ID NO: 177). In some aspects, the target region corresponds to nucleotides 1916-1935 of SEQ ID NO: 3 (e.g., ASO-STAT6-1916; SEQ ID NO: 178). In some aspects, the target region corresponds to nucleotides 1917-1936 of SEQ ID NO: 3 (e.g., ASO-STAT6-1917; SEQ ID NO: 179). In some aspects, the target region corresponds to nucleotides 2056-2075 of SEQ ID NO: 3 (e.g., ASO-STAT6-2056; SEQ ID NO: 180). In some aspects, the target region corresponds to nucleotides 2057-2076 of SEQ ID NO: 3 (e.g., ASO-STAT6-2057; SEQ ID NO: 181). In some aspects, the target region corresponds to nucleotides 2060-2079 of SEQ ID NO: 3 (e.g., ASO-STAT6-2060; SEQ ID NO: 182). In some aspects, the target region corresponds to nucleotides 2062-2081 of SEQ ID NO: 3 (e.g., ASO-STAT6-2062; SEQ ID NO: 183). In some aspects, the target region corresponds to nucleotides 2063-2082 of SEQ ID NO: 3 (e.g., ASO-STAT6-2063; SEQ ID NO: 184). In some aspects, the target region corresponds to nucleotides 2065-2084 of SEQ ID NO: 3 (e.g., ASO-STAT6-2065; SEQ ID NO: 185). In some aspects, the target region corresponds to nucleotides 2068-2087 of SEQ ID NO: 3 (e.g., ASO-STAT6-2068; SEQ ID NO: 186). In some aspects, the target region corresponds to nucleotides 2347-2366 of SEQ ID NO: 3 (e.g., ASO-STAT6-2347; SEQ ID NO: 187). In some aspects, the target region corresponds to nucleotides 2348-2367 of SEQ ID NO: 3 (e.g., ASO-STAT6-2348; SEQ ID NO: 188). In some aspects, the target region corresponds to nucleotides 2358-2377 of SEQ ID NO: 3 (e.g., ASO-STAT6-2358; SEQ ID NO: 189). In some aspects, the target region corresponds to nucleotides 2782-2801 of SEQ ID NO: 3 (e.g., ASO-STAT6-2782; SEQ ID NO: 190). In some aspects, the target region corresponds to nucleotides 3070-3089 of SEQ ID NO: 3 (e.g., ASO-STAT6-3070; SEQ ID NO: 191). In some aspects, the target region corresponds to nucleotides 3071-3090 of SEQ ID NO: 3 (e.g., ASO-STAT6-3071; SEQ ID NO: 192). In some aspects, the target region corresponds to nucleotides 3431-3450 of SEQ ID NO: 3 (e.g., ASO-STAT6-3431; SEQ ID NO: 193).

In some aspects, the target region corresponds to nucleotides 1053-1067 of SEQ ID NO: 3 (e.g., ASO-STAT6-1053; SEQ ID NO: 91) ± 10nucleotides at the 3' end and/or the 5' end, within the scope of the claims.

The target regions listed in this paragraph fall outside the scope of the appended claims. In some aspects, the target region corresponds to nucleotides 1359-1373 of SEQ ID NO: 3 (e.g., ASO-STAT6-1359; SEQ ID NO: 92) ± 10, ± 20, ± 30, ± 40, ± 50, ± 60, ± 70, ± 80, or ± 90 nucleotides at the 3' end and/or the 5' end. In some aspects, the target region corresponds to nucleotides 1890-1904 of SEQ ID NO: 3 (e.g., ASO-STAT6-1890; SEQ ID NO: 93) ± 10, ± 20, ± 30, ± 40, ± 50, ± 60, ± 70, ± 80, or ± 90 nucleotides at the 3' end and/or the 5' end. In some aspects, the target region corresponds to nucleotides 1892-1906 of SEQ ID NO: 3 (e.g., ASO-STAT6-1892; SEQ ID NO: 94) ± 10, ± 20, ± 30, ± 40, ± 50, ± 60, ± 70, ± 80, or ± 90 nucleotides at the 3' end and/or the 5' end. In some aspects, the target region corresponds to nucleotides 1915-1929 of SEQ ID NO: 3 (e.g., ASO-STAT6-1915; SEQ ID NO: 95) ± 10, ± 20, ± 30, ± 40, ± 50, ± 60, ± 70, ± 80, or ± 90 nucleotides at the 3' end and/or the 5' end. In some aspects, the target region corresponds to nucleotides 1916-1930 of SEQ ID NO: 3 (e.g., ASO-STAT6-1916; SEQ ID NO: 96) ± 10, ± 20, ± 30, ± 40, ± 50, ± 60, ± 70, ± 80, or ± 90 nucleotides at the 3' end and/or the 5' end. In some aspects, the target region corresponds to nucleotides 1917-1931 of SEQ ID NO: 3 (e.g., ASO-STAT6-1917; SEQ ID NO: 97) ± 10, ± 20, ± 30, ± 40, ± 50, ± 60, ± 70, ± 80, or ± 90 nucleotides at the 3' end and/or the 5' end. In some aspects, the target region corresponds to nucleotides 1918-1932 of SEQ ID NO: 3 (e.g., ASO-STAT6-1918; SEQ ID NO: 98) ± 10, ± 20, ± 30, ± 40, ± 50, ± 60, ± 70, ± 80, or ± 90 nucleotides at the 3' end and/or the 5' end. In some aspects, the target region corresponds to nucleotides 1919-1933 of SEQ ID NO: 3 (e.g., ASO-STAT6-1919; SEQ ID NO: 99) ± 10, ± 20, ± 30, ± 40, ± 50, ± 60, ± 70, ± 80, or ± 90 nucleotides at the 3' end and/or the 5' end. In some aspects, the target region corresponds to nucleotides 1920-1934 of SEQ ID NO: 3 (e.g., ASO-STAT6-1920; SEQ ID NO: 100) ± 10, ± 20, ± 30, ± 40, ± 50, ± 60, ± 70, ± 80, or ± 90 nucleotides at the 3' end and/or the 5' end. In some aspects, the target region corresponds to nucleotides 1937-1951 of SEQ ID NO: 3 (e.g., ASO-STAT6-1937; SEQ ID NO: 101) ± 10, ± 20, ± 30, ± 40, ± 50, ± 60, ± 70, ± 80, or ± 90 nucleotides at the 3' end and/or the 5' end. In some aspects, the target region corresponds to nucleotides 1938-1952 of SEQ ID NO: 3 (e.g., ASO-STAT6-1938; SEQ ID NO: 102) ± 10, ± 20, ± 30, ± 40, ± 50, ± 60, ± 70, ± 80, or ± 90 nucleotides at the 3' end and/or the 5' end. In some aspects, the target region corresponds to nucleotides 2061-2075 of SEQ ID NO: 3 (e.g., ASO-STAT6-2061; SEQ ID NO: 103) ± 10, ± 20, ± 30, ± 40, ± 50, ± 60, ± 70, ± 80, or ± 90 nucleotides at the 3' end and/or the 5' end. In some aspects, the target region corresponds to nucleotides 2062-2076 of SEQ ID NO: 3 (e.g., ASO-STAT6-2062; SEQ ID NO: 104) ± 10, ± 20, ± 30, ± 40, ± 50, ± 60, ± 70, ± 80, or ± 90 nucleotides at the 3' end and/or the 5' end. In some aspects, the target region corresponds to nucleotides 2063-2077 of SEQ ID NO: 3 (e.g., ASO-STAT6-2063; SEQ ID NO: 105) ± 10, ± 20, ± 30, ± 40, ± 50, ± 60, ± 70, ± 80, or ± 90 nucleotides at the 3' end and/or the 5' end. In some aspects, the target region corresponds to nucleotides 2064-2078 of SEQ ID NO: 3 (e.g., ASO-STAT6-2064; SEQ ID NO: 106) ± 10, ± 20, ± 30, ± 40, ± 50, ± 60, ± 70, ± 80, or ± 90 nucleotides at the 3' end and/or the 5' end. In some aspects, the target region corresponds to nucleotides 2066-2080 of SEQ ID NO: 3 (e.g., ASO-STAT6-2066; SEQ ID NO: 107) ± 10, ± 20, ± 30, ± 40, ± 50, ± 60, ± 70, ± 80, or ± 90 nucleotides at the 3' end and/or the 5' end. In some aspects, the target region corresponds to nucleotides 2067-2081 of SEQ ID NO: 3 (e.g., ASO-STAT6-2067; SEQ ID NO: 108) ± 10, ± 20, ± 30, ± 40, ± 50, ± 60, ± 70, ± 80, or ± 90 nucleotides at the 3' end and/or the 5' end. In some aspects, the target region corresponds to nucleotides 2068-2082 of SEQ ID NO: 3 (e.g., ASO-STAT6-2068; SEQ ID NO: 109) ± 10, ± 20, ± 30, ± 40, ± 50, ± 60, ± 70, ± 80, or ± 90 nucleotides at the 3' end and/or the 5' end. In some aspects, the target region corresponds to nucleotides 2352-2366 of SEQ ID NO: 3 (e.g., ASO-STAT6-2352; SEQ ID NO: 110) ± 10, ± 20, ± 30, ± 40, ± 50, ± 60, ± 70, ± 80, or ± 90 nucleotides at the 3' end and/or the 5' end. In some aspects, the target region corresponds to nucleotides 3073-3087 of SEQ ID NO: 3 (e.g., ASO-STAT6-3073; SEQ ID NO: 111) ± 10, ± 20, ± 30, ± 40, ± 50, ± 60, ± 70, ± 80, or ± 90 nucleotides at the 3' end and/or the 5' end. In some aspects, the target region corresponds to nucleotides 1053-1068 of SEQ ID NO: 3 (e.g., ASO-STAT6-1053; SEQ ID NO: 112) ± 10, ± 20, ± 30, ± 40, ± 50, ± 60, ± 70, ± 80, or ± 90 nucleotides at the 3' end and/or the 5' end. In some aspects, the target region corresponds to nucleotides 1054-1069 of SEQ ID NO: 3 (e.g., ASO-STAT6-1054; SEQ ID NO: 113) ± 10, ± 20, ± 30, ± 40, ± 50, ± 60, ± 70, ± 80, or ± 90 nucleotides at the 3' end and/or the 5' end. In some aspects, the target region corresponds to nucleotides 1356-1371 of SEQ ID NO: 3 (e.g., ASO-STAT6-1356; SEQ ID NO: 114) ± 10, ± 20, ± 30, ± 40, ± 50, ± 60, ± 70, ± 80, or ± 90 nucleotides at the 3' end and/or the 5' end. In some aspects, the target region corresponds to nucleotides 1847-1862 of SEQ ID NO: 3 (e.g., ASO-STAT6-1847; SEQ ID NO: 115) ± 10, ± 20, ± 30, ± 40, ± 50, ± 60, ± 70, ± 80, or ± 90 nucleotides at the 3' end and/or the 5' end. In some aspects, the target region corresponds to nucleotides 1886-1901 of SEQ ID NO: 3 (e.g., ASO-STAT6-1886; SEQ ID NO: 116) ± 10, ± 20, ± 30, ± 40, ± 50, ± 60, ± 70, ± 80, or ± 90 nucleotides at the 3' end and/or the 5' end. In some aspects, the target region corresponds to nucleotides 1887-1902 of SEQ ID NO: 3 (e.g., ASO-STAT6-1887; SEQ ID NO: 117) ± 10, ± 20, ± 30, ± 40, ± 50, ± 60, ± 70, ± 80, or ± 90 nucleotides at the 3' end and/or the 5' end. In some aspects, the target region corresponds to nucleotides 1888-1903 of SEQ ID NO: 3 (e.g., ASO-STAT6-1888; SEQ ID NO: 118) ± 10, ± 20, ± 30, ± 40, ± 50, ± 60, ± 70, ± 80, or ± 90 nucleotides at the 3' end and/or the 5' end. In some aspects, the target region corresponds to nucleotides 1889-1904 of SEQ ID NO: 3 (e.g., ASO-STAT6-1889; SEQ ID NO: 119) ± 10, ± 20, ± 30, ± 40, ± 50, ± 60, ± 70, ± 80, or ± 90 nucleotides at the 3' end and/or the 5' end. In some aspects, the target region corresponds to nucleotides 1890-1905 of SEQ ID NO: 3 (e.g., ASO-STAT6-1890; SEQ ID NO: 120) ± 10, ± 20, ± 30, ± 40, ± 50, ± 60, ± 70, ± 80, or ± 90 nucleotides at the 3' end and/or the 5' end. In some aspects, the target region corresponds to nucleotides 1893-1908 of SEQ ID NO: 3 (e.g., ASO-STAT6-1893; SEQ ID NO: 121) ± 10, ± 20, ± 30, ± 40, ± 50, ± 60, ± 70, ± 80, or ± 90 nucleotides at the 3' end and/or the 5' end. In some aspects, the target region corresponds to nucleotides 1917-1932 of SEQ ID NO: 3 (e.g., ASO-STAT6-1917; SEQ ID NO: 122) ± 10, ± 20, ± 30, ± 40, ± 50, ± 60, ± 70, ± 80, or ± 90 nucleotides at the 3' end and/or the 5' end. In some aspects, the target region corresponds to nucleotides 1919-1934 of SEQ ID NO: 3 (e.g., ASO-STAT6-1919; SEQ ID NO: 123) ± 10, ± 20, ± 30, ± 40, ± 50, ± 60, ± 70, ± 80, or ± 90 nucleotides at the 3' end and/or the 5' end. In some aspects, the target region corresponds to nucleotides 2056-2071 of SEQ ID NO: 3 (e.g., ASO-STAT6-2056; SEQ ID NO: 124) ± 10, ± 20, ± 30, ± 40, ± 50, ± 60, ± 70, ± 80, or ± 90 nucleotides at the 3' end and/or the 5' end. In some aspects, the target region corresponds to nucleotides 2060-2075 of SEQ ID NO: 3 (e.g., ASO-STAT6-2060; SEQ ID NO: 125) ± 10, ± 20, ± 30, ± 40, ± 50, ± 60, ± 70, ± 80, or ± 90 nucleotides at the 3' end and/or the 5' end. In some aspects, the target region corresponds to nucleotides 2066-2081 of SEQ ID NO: 3 (e.g., ASO-STAT6-2066; SEQ ID NO: 126) ± 10, ± 20, ± 30, ± 40, ± 50, ± 60, ± 70, ± 80, or ± 90 nucleotides at the 3' end and/or the 5' end. In some aspects, the target region corresponds to nucleotides 2070-2085 of SEQ ID NO: 3 (e.g., ASO-STAT6-2070; SEQ ID NO: 127) ± 10, ± 20, ± 30, ± 40, ± 50, ± 60, ± 70, ± 80, or ± 90 nucleotides at the 3' end and/or the 5' end. In some aspects, the target region corresponds to nucleotides 2351-2366 of SEQ ID NO: 3 (e.g., ASO-STAT6-2351; SEQ ID NO: 128) ± 10, ± 20, ± 30, ± 40, ± 50, ± 60, ± 70, ± 80, or ± 90 nucleotides at the 3' end and/or the 5' end. In some aspects, the target region corresponds to nucleotides 2352-2367 of SEQ ID NO: 3 (e.g., ASO-STAT6-2352; SEQ ID NO: 129) ± 10, ± 20, ± 30, ± 40, ± 50, ± 60, ± 70, ± 80, or ± 90 nucleotides at the 3' end and/or the 5' end. In some aspects, the target region corresponds to nucleotides 2359-2374 of SEQ ID NO: 3 (e.g., ASO-STAT6-2359; SEQ ID NO: 130) ± 10, ± 20, ± 30, ± 40, ± 50, ± 60, ± 70, ± 80, or ± 90 nucleotides at the 3' end and/or the 5' end. In some aspects, the target region corresponds to nucleotides 3633-3648 of SEQ ID NO: 3 (e.g., ASO-STAT6-3633; SEQ ID NO: 131) ± 10, ± 20, ± 30, ± 40, ± 50, ± 60, ± 70, ± 80, or ± 90 nucleotides at the 3' end and/or the 5' end. In some aspects, the target region corresponds to nucleotides 673-689 of SEQ ID NO: 3 (e.g., ASO-STAT6-673; SEQ ID NO: 132) ± 10, ± 20, ± 30, ± 40, ± 50, ± 60, ± 70, ± 80, or ± 90 nucleotides at the 3' end and/or the 5' end. In some aspects, the target region corresponds to nucleotides 1052-1068 of SEQ ID NO: 3 (e.g., ASO-STAT6-1052; SEQ ID NO: 133) ± 10, ± 20, ± 30, ± 40, ± 50, ± 60, ± 70, ± 80, or ± 90 nucleotides at the 3' end and/or the 5' end. In some aspects, the target region corresponds to nucleotides 1356-1372 of SEQ ID NO: 3 (e.g., ASO-STAT6-1356; SEQ ID NO: 134) ± 10, ± 20, ± 30, ± 40, ± 50, ± 60, ± 70, ± 80, or ± 90 nucleotides at the 3' end and/or the 5' end. In some aspects, the target region corresponds to nucleotides 1357-1373 of SEQ ID NO: 3 (e.g., ASO-STAT6-1357; SEQ ID NO: 135) ± 10, ± 20, ± 30, ± 40, ± 50, ± 60, ± 70, ± 80, or ± 90 nucleotides at the 3' end and/or the 5' end. In some aspects, the target region corresponds to nucleotides 1359-1375 of SEQ ID NO: 3 (e.g., ASO-STAT6-1359; SEQ ID NO: 136) ± 10, ± 20, ± 30, ± 40, ± 50, ± 60, ± 70, ± 80, or ± 90 nucleotides at the 3' end and/or the 5' end. In some aspects, the target region corresponds to nucleotides 1360-1376 of SEQ ID NO: 3 (e.g., ASO-STAT6-1360; SEQ ID NO: 137) ± 10, ± 20, ± 30, ± 40, ± 50, ± 60, ± 70, ± 80, or ± 90 nucleotides at the 3' end and/or the 5' end. In some aspects, the target region corresponds to nucleotides 1839-1855 of SEQ ID NO: 3 (e.g., ASO-STAT6-1839; SEQ ID NO: 138) ± 10, ± 20, ± 30, ± 40, ± 50, ± 60, ± 70, ± 80, or ± 90 nucleotides at the 3' end and/or the 5' end. In some aspects, the target region corresponds to nucleotides 1848-1864 of SEQ ID NO: 3 (e.g., ASO-STAT6-1848; SEQ ID NO: 139) ± 10, ± 20, ± 30, ± 40, ± 50, ± 60, ± 70, ± 80, or ± 90 nucleotides at the 3' end and/or the 5' end. In some aspects, the target region corresponds to nucleotides 1849-1865 of SEQ ID NO: 3 (e.g., ASO-STAT6-1849; SEQ ID NO: 140) ± 10, ± 20, ± 30, ± 40, ± 50, ± 60, ± 70, ± 80, or ± 90 nucleotides at the 3' end and/or the 5' end. In some aspects, the target region corresponds to nucleotides 1891-1907 of SEQ ID NO: 3 (e.g., ASO-STAT6-1891; SEQ ID NO: 141) ± 10, ± 20, ± 30, ± 40, ± 50, ± 60, ± 70, ± 80, or ± 90 nucleotides at the 3' end and/or the 5' end. In some aspects, the target region corresponds to nucleotides 1915-1931 of SEQ ID NO: 3 (e.g., ASO-STAT6-1915; SEQ ID NO: 142) ± 10, ± 20, ± 30, ± 40, ± 50, ± 60, ± 70, ± 80, or ± 90 nucleotides at the 3' end and/or the 5' end. In some aspects, the target region corresponds to nucleotides 1916-1932 of SEQ ID NO: 3 (e.g., ASO-STAT6-1916; SEQ ID NO: 143) ± 10, ± 20, ± 30, ± 40, ± 50, ± 60, ± 70, ± 80, or ± 90 nucleotides at the 3' end and/or the 5' end. In some aspects, the target region corresponds to nucleotides 1917-1933 of SEQ ID NO: 3 (e.g., ASO-STAT6-1917; SEQ ID NO: 144) ± 10, ± 20, ± 30, ± 40, ± 50, ± 60, ± 70, ± 80, or ± 90 nucleotides at the 3' end and/or the 5' end. In some aspects, the target region corresponds to nucleotides 1938-1954 of SEQ ID NO: 3 (e.g., ASO-STAT6-1938; SEQ ID NO: 145) ± 10, ± 20, ± 30, ± 40, ± 50, ± 60, ± 70, ± 80, or ± 90 nucleotides at the 3' end and/or the 5' end. In some aspects, the target region corresponds to nucleotides 1939-1955 of SEQ ID NO: 3 (e.g., ASO-STAT6-1939; SEQ ID NO: 146) ± 10, ± 20, ± 30, ± 40, ± 50, ± 60, ± 70, ± 80, or ± 90 nucleotides at the 3' end and/or the 5' end. In some aspects, the target region corresponds to nucleotides 2063-2079 of SEQ ID NO: 3 (e.g., ASO-STAT6-2063; SEQ ID NO: 147) ± 10, ± 20, ± 30, ± 40, ± 50, ± 60, ± 70, ± 80, or ± 90 nucleotides at the 3' end and/or the 5' end. In some aspects, the target region corresponds to nucleotides 2064-2080 of SEQ ID NO: 3 (e.g., ASO-STAT6-2064; SEQ ID NO: 148) ± 10, ± 20, ± 30, ± 40, ± 50, ± 60, ± 70, ± 80, or ± 90 nucleotides at the 3' end and/or the 5' end. In some aspects, the target region corresponds to nucleotides 2065-2081 of SEQ ID NO: 3 (e.g., ASO-STAT6-2065; SEQ ID NO: 149) ± 10, ± 20, ± 30, ± 40, ± 50, ± 60, ± 70, ± 80, or ± 90 nucleotides at the 3' end and/or the 5' end. In some aspects, the target region corresponds to nucleotides 2066-2082 of SEQ ID NO: 3 (e.g., ASO-STAT6-2066; SEQ ID NO: 150) ± 10, ± 20, ± 30, ± 40, ± 50, ± 60, ± 70, ± 80, or ± 90 nucleotides at the 3' end and/or the 5' end. In some aspects, the target region corresponds to nucleotides 2068-2084 of SEQ ID NO: 3 (e.g., ASO-STAT6-2068; SEQ ID NO: 151) ± 10, ± 20, ± 30, ± 40, ± 50, ± 60, ± 70, ± 80, or ± 90 nucleotides at the 3' end and/or the 5' end. In some aspects, the target region corresponds to nucleotides 2187-2203 of SEQ ID NO: 3 (e.g., ASO-STAT6-2187; SEQ ID NO: 152) ± 10, ± 20, ± 30, ± 40, ± 50, ± 60, ± 70, ± 80, or ± 90 nucleotides at the 3' end and/or the 5' end. In some aspects, the target region corresponds to nucleotides 2350-2366 of SEQ ID NO: 3 (e.g., ASO-STAT6-2350; SEQ ID NO: 153) ± 10, ± 20, ± 30, ± 40, ± 50, ± 60, ± 70, ± 80, or ± 90 nucleotides at the 3' end and/or the 5' end. In some aspects, the target region corresponds to nucleotides 2351-2367 of SEQ ID NO: 3 (e.g., ASO-STAT6-2351; SEQ ID NO: 154) ± 10, ± 20, ± 30, ± 40, ± 50, ± 60, ± 70, ± 80, or ± 90 nucleotides at the 3' end and/or the 5' end. In some aspects, the target region corresponds to nucleotides 2352-2368 of SEQ ID NO: 3 (e.g., ASO-STAT6-2352; SEQ ID NO: 155) ± 10, ± 20, ± 30, ± 40, ± 50, ± 60, ± 70, ± 80, or ± 90 nucleotides at the 3' end and/or the 5' end. In some aspects, the target region corresponds to nucleotides 2357-2373 of SEQ ID NO: 3 (e.g., ASO-STAT6-2357; SEQ ID NO: 156) ± 10, ± 20, ± 30, ± 40, ± 50, ± 60, ± 70, ± 80, or ± 90 nucleotides at the 3' end and/or the 5' end. In some aspects, the target region corresponds to nucleotides 513-532 of SEQ ID NO: 3 (e.g., ASO-STAT6-513; SEQ ID NO: 157) ± 10, ± 20, ± 30, ± 40, ± 50, ± 60, ± 70, ± 80, or ± 90 nucleotides at the 3' end and/or the 5' end. In some aspects, the target region corresponds to nucleotides 671-690 of SEQ ID NO: 3 (e.g., ASO-STAT6-671; SEQ ID NO: 158) ± 10, ± 20, ± 30, ± 40, ± 50, ± 60, ± 70, ± 80, or ± 90 nucleotides at the 3' end and/or the 5' end. In some aspects, the target region corresponds to nucleotides 1131-1150 of SEQ ID NO: 3 (e.g., ASO-STAT6-1131; SEQ ID NO: 159) ± 10, ± 20, ± 30, ± 40, ± 50, ± 60, ± 70, ± 80, or ± 90 nucleotides at the 3' end and/or the 5' end. In some aspects, the target region corresponds to nucleotides 1354-1373 of SEQ ID NO: 3 (e.g., ASO-STAT6-1354; SEQ ID NO: 160) ± 10, ± 20, ± 30, ± 40, ± 50, ± 60, ± 70, ± 80, or ± 90 nucleotides at the 3' end and/or the 5' end. In some aspects, the target region corresponds to nucleotides 1355-1374 of SEQ ID NO: 3 (e.g., ASO-STAT6-1355; SEQ ID NO: 161) ± 10, ± 20, ± 30, ± 40, ± 50, ± 60, ± 70, ± 80, or ± 90 nucleotides at the 3' end and/or the 5' end. In some aspects, the target region corresponds to nucleotides 1356-1375 of SEQ ID NO: 3 (e.g., ASO-STAT6-1356; SEQ ID NO: 162) ± 10, ± 20, ± 30, ± 40, ± 50, ± 60, ± 70, ± 80, or ± 90 nucleotides at the 3' end and/or the 5' end. In some aspects, the target region corresponds to nucleotides 1432-1451 of SEQ ID NO: 3 (e.g., ASO-STAT6-1432; SEQ ID NO: 163) ± 10, ± 20, ± 30, ± 40, ± 50, ± 60, ± 70, ± 80, or ± 90 nucleotides at the 3' end and/or the 5' end. In some aspects, the target region corresponds to nucleotides 1555-1574 of SEQ ID NO: 3 (e.g., ASO-STAT6-1555; SEQ ID NO: 164) ± 10, ± 20, ± 30, ± 40, ± 50, ± 60, ± 70, ± 80, or ± 90 nucleotides at the 3' end and/or the 5' end. In some aspects, the target region corresponds to nucleotides 1556-1575 of SEQ ID NO: 3 (e.g., ASO-STAT6-1556; SEQ ID NO: 165) ± 10, ± 20, ± 30, ± 40, ± 50, ± 60, ± 70, ± 80, or ± 90 nucleotides at the 3' end and/or the 5' end. In some aspects, the target region corresponds to nucleotides 1557-1576 of SEQ ID NO: 3 (e.g., ASO-STAT6-1557; SEQ ID NO: 166) ± 10, ± 20, ± 30, ± 40, ± 50, ± 60, ± 70, ± 80, or ± 90 nucleotides at the 3' end and/or the 5' end. In some aspects, the target region corresponds to nucleotides 1558-1577 of SEQ ID NO: 3 (e.g., ASO-STAT6-1558; SEQ ID NO: 167) ± 10, ± 20, ± 30, ± 40, ± 50, ± 60, ± 70, ± 80, or ± 90 nucleotides at the 3' end and/or the 5' end. In some aspects, the target region corresponds to nucleotides 1826-1845 of SEQ ID NO: 3 (e.g., ASO-STAT6-1826; SEQ ID NO: 168) ± 10, ± 20, ± 30, ± 40, ± 50, ± 60, ± 70, ± 80, or ± 90 nucleotides at the 3' end and/or the 5' end. In some aspects, the target region corresponds to nucleotides 1827-1846 of SEQ ID NO: 3 (e.g., ASO-STAT6-1827; SEQ ID NO: 169) ± 10, ± 20, ± 30, ± 40, ± 50, ± 60, ± 70, ± 80, or ± 90 nucleotides at the 3' end and/or the 5' end. In some aspects, the target region corresponds to nucleotides 1833-1852 of SEQ ID NO: 3 (e.g., ASO-STAT6-1833; SEQ ID NO: 170) ± 10, ± 20, ± 30, ± 40, ± 50, ± 60, ± 70, ± 80, or ± 90 nucleotides at the 3' end and/or the 5' end. In some aspects, the target region corresponds to nucleotides 1843-1862 of SEQ ID NO: 3 (e.g., ASO-STAT6-1843; SEQ ID NO: 171) ± 10, ± 20, ± 30, ± 40, ± 50, ± 60, ± 70, ± 80, or ± 90 nucleotides at the 3' end and/or the 5' end. In some aspects, the target region corresponds to nucleotides 1846-1865 of SEQ ID NO: 3 (e.g., ASO-STAT6-1846; SEQ ID NO: 172) ± 10, ± 20, ± 30, ± 40, ± 50, ± 60, ± 70, ± 80, or ± 90 nucleotides at the 3' end and/or the 5' end. In some aspects, the target region corresponds to nucleotides 1847-1866 of SEQ ID NO: 3 (e.g., ASO-STAT6-1847; SEQ ID NO: 173) ± 10, ± 20, ± 30, ± 40, ± 50, ± 60, ± 70, ± 80, or ± 90 nucleotides at the 3' end and/or the 5' end. In some aspects, the target region corresponds to nucleotides 1883-1902 of SEQ ID NO: 3 (e.g., ASO-STAT6-1883; SEQ ID NO: 174) ± 10, ± 20, ± 30, ± 40, ± 50, ± 60, ± 70, ± 80, or ± 90 nucleotides at the 3' end and/or the 5' end. In some aspects, the target region corresponds to nucleotides 1889-1908 of SEQ ID NO: 3 (e.g., ASO-STAT6-1889; SEQ ID NO: 175) ± 10, ± 20, ± 30, ± 40, ± 50, ± 60, ± 70, ± 80, or ± 90 nucleotides at the 3' end and/or the 5' end. In some aspects, the target region corresponds to nucleotides 1890-1909 of SEQ ID NO: 3 (e.g., ASO-STAT6-1890; SEQ ID NO: 176) ± 10, ± 20, ± 30, ± 40, ± 50, ± 60, ± 70, ± 80, or ± 90 nucleotides at the 3' end and/or the 5' end. In some aspects, the target region corresponds to nucleotides 1891-1910 of SEQ ID NO: 3 (e.g., ASO-STAT6-1891; SEQ ID NO: 177) ± 10, ± 20, ± 30, ± 40, ± 50, ± 60, ± 70, ± 80, or ± 90 nucleotides at the 3' end and/or the 5' end. In some aspects, the target region corresponds to nucleotides 1916-1935 of SEQ ID NO: 3 (e.g., ASO-STAT6-1916; SEQ ID NO: 178) ± 10, ± 20, ± 30, ± 40, ± 50, ± 60, ± 70, ± 80, or ± 90 nucleotides at the 3' end and/or the 5' end. In some aspects, the target region corresponds to nucleotides 1917-1936 of SEQ ID NO: 3 (e.g., ASO-STAT6-1917; SEQ ID NO: 179) ± 10, ± 20, ± 30, ± 40, ± 50, ± 60, ± 70, ± 80, or ± 90 nucleotides at the 3' end and/or the 5' end. In some aspects, the target region corresponds to nucleotides 2056-2075 of SEQ ID NO: 3 (e.g., ASO-STAT6-2056; SEQ ID NO: 180) ± 10, ± 20, ± 30, ± 40, ± 50, ± 60, ± 70, ± 80, or ± 90 nucleotides at the 3' end and/or the 5' end. In some aspects, the target region corresponds to nucleotides 2057-2076 of SEQ ID NO: 3 (e.g., ASO-STAT6-2057; SEQ ID NO: 181) ± 10, ± 20, ± 30, ± 40, ± 50, ± 60, ± 70, ± 80, or ± 90 nucleotides at the 3' end and/or the 5' end. In some aspects, the target region corresponds to nucleotides 2060-2079 of SEQ ID NO: 3 (e.g., ASO-STAT6-2060; SEQ ID NO: 182) ± 10, ± 20, ± 30, ± 40, ± 50, ± 60, ± 70, ± 80, or ± 90 nucleotides at the 3' end and/or the 5' end. In some aspects, the target region corresponds to nucleotides 2062-2081 of SEQ ID NO: 3 (e.g., ASO-STAT6-2062; SEQ ID NO: 183) ± 10, ± 20, ± 30, ± 40, ± 50, ± 60, ± 70, ± 80, or ± 90 nucleotides at the 3' end and/or the 5' end. In some aspects, the target region corresponds to nucleotides 2063-2082 of SEQ ID NO: 3 (e.g., ASO-STAT6-2063; SEQ ID NO: 184) ± 10, ± 20, ± 30, ± 40, ± 50, ± 60, ± 70, ± 80, or ± 90 nucleotides at the 3' end and/or the 5' end. In some aspects, the target region corresponds to nucleotides 2065-2084 of SEQ ID NO: 3 (e.g., ASO-STAT6-2065; SEQ ID NO: 185) ± 10, ± 20, ± 30, ± 40, ± 50, ± 60, ± 70, ± 80, or ± 90 nucleotides at the 3' end and/or the 5' end. In some aspects, the target region corresponds to nucleotides 2068-2087 of SEQ ID NO: 3 (e.g., ASO-STAT6-2068; SEQ ID NO: 186) ± 10, ± 20, ± 30, ± 40, ± 50, ± 60, ± 70, ± 80, or ± 90 nucleotides at the 3' end and/or the 5' end. In some aspects, the target region corresponds to nucleotides 2347-2366 of SEQ ID NO: 3 (e.g., ASO-STAT6-2347; SEQ ID NO: 187) ± 10, ± 20, ± 30, ± 40, ± 50, ± 60, ± 70, ± 80, or ± 90 nucleotides at the 3' end and/or the 5' end. In some aspects, the target region corresponds to nucleotides 2348-2367 of SEQ ID NO: 3 (e.g., ASO-STAT6-2348; SEQ ID NO: 188) ± 10, ± 20, ± 30, ± 40, ± 50, ± 60, ± 70, ± 80, or ± 90 nucleotides at the 3' end and/or the 5' end. In some aspects, the target region corresponds to nucleotides 2358-2377 of SEQ ID NO: 3 (e.g., ASO-STAT6-2358; SEQ ID NO: 189) ± 10, ± 20, ± 30, ± 40, ± 50, ± 60, ± 70, ± 80, or ± 90 nucleotides at the 3' end and/or the 5' end. In some aspects, the target region corresponds to nucleotides 2782-2801 of SEQ ID NO: 3 (e.g., ASO-STAT6-2782; SEQ ID NO: 190) ± 10, ± 20, ± 30, ± 40, ± 50, ± 60, ± 70, ± 80, or ± 90 nucleotides at the 3' end and/or the 5' end. In some aspects, the target region corresponds to nucleotides 3070-3089 of SEQ ID NO: 3 (e.g., ASO-STAT6-3070; SEQ ID NO: 191) ± 10, ± 20, ± 30, ± 40, ± 50, ± 60, ± 70, ± 80, or ± 90 nucleotides at the 3' end and/or the 5' end. In some aspects, the target region corresponds to nucleotides 3071-3090 of SEQ ID NO: 3 (e.g., ASO-STAT6-3071; SEQ ID NO: 192) ± 10, ± 20, ± 30, ± 40, ± 50, ± 60, ± 70, ± 80, or ± 90 nucleotides at the 3' end and/or the 5' end. In some aspects, the target region corresponds to nucleotides 3431-3450 of SEQ ID NO: 3 (e.g., ASO-STAT6-3431; SEQ ID NO: 193) ± 10, ± 20, ± 30, ± 40, ± 50, ± 60, ± 70, ± 80, or ± 90 nucleotides at the 3' end and/or the 5' end).

The ASO is not TGAGCGAATGGACAGGTCTT (SEQ ID NO: 89). In some aspects, the target region corresponds to a contiguous nucleotide sequence of 10 to 30 nucleotides in length that is complementary to a nucleic acid sequence within nucleotides 1-2056 of SEQ ID NO: 3. In some aspects, the target region corresponds to a contiguous nucleotide sequence of 10 to 30 nucleotides in length that is complementary to a nucleic acid sequence within nucleotides 1-2055 of SEQ ID NO: 3. In some aspects, the target region corresponds to a contiguous nucleotide sequence of 10 to 30 nucleotides in length that is complementary to a nucleic acid sequence within nucleotides 1-2054 of SEQ ID NO: 3. In some aspects, the target region corresponds to a contiguous nucleotide sequence of 10 to 30 nucleotides in length that is complementary to a nucleic acid sequence within nucleotides 1-2053 of SEQ ID NO: 3. In some aspects, the target region corresponds to a contiguous nucleotide sequence of 10 to 30 nucleotides in length that is complementary to a nucleic acid sequence within nucleotides 1-2052 of SEQ ID NO: 3. In some aspects, the target region corresponds to a contiguous nucleotide sequence of 10 to 30 nucleotides in length that is complementary to a nucleic acid sequence within nucleotides 1-2051 of SEQ ID NO: 3. In some aspects, the target region corresponds to a contiguous nucleotide sequence of 10 to 30 nucleotides in length that is complementary to a nucleic acid sequence within nucleotides 1-2050 of SEQ ID NO: 3. In some aspects, the target region corresponds to a contiguous nucleotide sequence of 10 to 30 nucleotides in length that is complementary to a nucleic acid sequence within nucleotides 1-2049 of SEQ ID NO: 3. In some aspects, the target region corresponds to a contiguous nucleotide sequence of 10 to 30 nucleotides in length that is complementary to a nucleic acid sequence within nucleotides 1-2048 of SEQ ID NO: 3. In some aspects, the target region corresponds to a contiguous nucleotide sequence of 10 to 30 nucleotides in length that is complementary to a nucleic acid sequence within nucleotides 1-2047 of SEQ ID NO: 3. In some aspects, the target region corresponds to a contiguous nucleotide sequence of 10 to 30 nucleotides in length that is complementary to a nucleic acid sequence within nucleotides 1-2046 of SEQ ID NO: 3. In some aspects, the target region corresponds to a contiguous nucleotide sequence of 10 to 30 nucleotides in length that is complementary to a nucleic acid sequence within nucleotides 1-2045 of SEQ ID NO: 3. In some aspects, the target region corresponds to a contiguous nucleotide sequence of 10 to 30 nucleotides in length that is complementary to a nucleic acid sequence within nucleotides 1-2044 of SEQ ID NO: 3. In some aspects, the target region corresponds to a contiguous nucleotide sequence of 10 to 30 nucleotides in length that is complementary to a nucleic acid sequence within nucleotides 1-2043 of SEQ ID NO: 3. In some aspects, the target region corresponds to a contiguous nucleotide sequence of 10 to 30 nucleotides in length that is complementary to a nucleic acid sequence within nucleotides 1-2042 of SEQ ID NO: 3. In some aspects, the target region corresponds to a contiguous nucleotide sequence of 10 to 30 nucleotides in length that is complementary to a nucleic acid sequence within nucleotides 1-2041 of SEQ ID NO: 3. In some aspects, the target region corresponds to a contiguous nucleotide sequence of 10 to 30 nucleotides in length that is complementary to a nucleic acid sequence within nucleotides 1-2040 of SEQ ID NO: 3. In some aspects, the target region corresponds to a contiguous nucleotide sequence of 10 to 30 nucleotides in length that is complementary to a nucleic acid sequence within nucleotides 1-2039 of SEQ ID NO: 3. In some aspects, the target region corresponds to a contiguous nucleotide sequence of 10 to 30 nucleotides in length that is complementary to a nucleic acid sequence within nucleotides 1-2038 of SEQ ID NO: 3.

The target regions listed in this paragraph fall outside the scope of the appended claims. In some aspects, the target region corresponds to a contiguous nucleotide sequence of 10 to 30 nucleotides in length that is complementary to a nucleic acid sequence within nucleotides 2041-3963 of SEQ ID NO: 3. In some aspects, the target region corresponds to a contiguous nucleotide sequence of 10 to 30 nucleotides in length that is complementary to a nucleic acid sequence within nucleotides 2042-3963 of SEQ ID NO: 3. In some aspects, the target region corresponds to a contiguous nucleotide sequence of 10 to 30 nucleotides in length that is complementary to a nucleic acid sequence within nucleotides 2043-3963 of SEQ ID NO: 3. In some aspects, the target region corresponds to a contiguous nucleotide sequence of 10 to 30 nucleotides in length that is complementary to a nucleic acid sequence within nucleotides 2044-3963 of SEQ ID NO: 3. In some aspects, the target region corresponds to a contiguous nucleotide sequence of 10 to 30 nucleotides in length that is complementary to a nucleic acid sequence within nucleotides 2045-3963 of SEQ ID NO: 3. In some aspects, the target region corresponds to a contiguous nucleotide sequence of 10 to 30 nucleotides in length that is complementary to a nucleic acid sequence within nucleotides 2046-3963 of SEQ ID NO: 3. In some aspects, the target region corresponds to a contiguous nucleotide sequence of 10 to 30 nucleotides in length that is complementary to a nucleic acid sequence within nucleotides 2047-3963 of SEQ ID NO: 3. In some aspects, the target region corresponds to a contiguous nucleotide sequence of 10 to 30 nucleotides in length that is complementary to a nucleic acid sequence within nucleotides 2048-3963 of SEQ ID NO: 3. In some aspects, the target region corresponds to a contiguous nucleotide sequence of 10 to 30 nucleotides in length that is complementary to a nucleic acid sequence within nucleotides 2049-3963 of SEQ ID NO: 3. In some aspects, the target region corresponds to a contiguous nucleotide sequence of 10 to 30 nucleotides in length that is complementary to a nucleic acid sequence within nucleotides 2050-3963 of SEQ ID NO: 3. In some aspects, the target region corresponds to a contiguous nucleotide sequence of 10 to 30 nucleotides in length that is complementary to a nucleic acid sequence within nucleotides 2051-3963 of SEQ ID NO: 3. In some aspects, the target region corresponds to a contiguous nucleotide sequence of 10 to 30 nucleotides in length that is complementary to a nucleic acid sequence within nucleotides 2052-3963 of SEQ ID NO: 3. In some aspects, the target region corresponds to a contiguous nucleotide sequence of 10 to 30 nucleotides in length that is complementary to a nucleic acid sequence within nucleotides 2053-3963 of SEQ ID NO: 3. In some aspects, the target region corresponds to a contiguous nucleotide sequence of 10 to 30 nucleotides in length that is complementary to a nucleic acid sequence within nucleotides 2054-3963 of SEQ ID NO: 3. In some aspects, the target region corresponds to a contiguous nucleotide sequence of 10 to 30 nucleotides in length that is complementary to a nucleic acid sequence within nucleotides 2055-3963 of SEQ ID NO: 3. In some aspects, the target region corresponds to a contiguous nucleotide sequence of 10 to 30 nucleotides in length that is complementary to a nucleic acid sequence within nucleotides 2056-3963 of SEQ ID NO: 3. In some aspects, the target region corresponds to a contiguous nucleotide sequence of 10 to 30 nucleotides in length that is complementary to a nucleic acid sequence within nucleotides 2057-3963 of SEQ ID NO: 3. In some aspects, the target region corresponds to a contiguous nucleotide sequence of 10 to 30 nucleotides in length that is complementary to a nucleic acid sequence within nucleotides 2058-3963 of SEQ ID NO: 3. In some aspects, the target region corresponds to a contiguous nucleotide sequence of 10 to 30 nucleotides in length that is complementary to a nucleic acid sequence within nucleotides 2059-3963 of SEQ ID NO: 3.

In some aspects, the ASO of the present disclosure hybridizes to multiple target regions within the *STAT6* transcript (*e.g.,* genomic sequence, SEQ ID NO: 1 or SEQ ID NO: 11, respectively). In some aspects, the ASO hybridizes to two different target regions within the *STAT6* transcript. In some aspects, the ASO hybridizes to three different target regions within the *STAT6* transcript. The sequences of exemplary ASOs that hybridizes to multiple target regions, and the start/end sites of the different target regions are provided in FIG. 1. In some aspects, the ASOs that hybridizes to multiple regions within the *STAT6* transcript (*e.g.,* genomic sequence, SEQ ID NO: 1 or SEQ ID NO: 11, respectively) are more potent (*e.g.,* having lower EC50) at reducing *STAT6* expression compared to ASOs that hybridizes to a single region within the *STAT6* transcript (*e.g.,* genomic sequence, SEQ ID NO: 1 or SEQ ID NO: 11, respectively).

In some aspects, the ASO of the disclosure is capable of hybridizing to the target nucleic acid (*e.g., STAT6* transcript) under physiological condition, *i.e., in vivo* condition. In some aspects, the ASO of the disclosure is capable of hybridizing to the target nucleic acid (*e.g., STAT6* transcript) *in vitro.* In some aspects, the ASO of the disclosure is capable of hybridizing to the target nucleic acid (*e.g., STAT6* transcript) *in vitro* under stringent conditions. Stringency conditions for hybridization *in vitro* are dependent on, *inter alia,* productive cell uptake, RNA accessibility, temperature, free energy of association, salt concentration, and time (*see, e.g.,* Stanley T Crooke, Antisense Drug Technology: Principles, Strategies and Applications, 2nd Edition, CRC Press (2007)). Generally, conditions of high to moderate stringency are used for *in vitro* hybridization to enable hybridization between substantially similar nucleic acids, but not between dissimilar nucleic acids. An example of stringent hybridization conditions includes hybridization in 5X saline-sodium citrate (SSC) buffer (0.75 M sodium chloride/0.075 M sodium citrate) for 1 hour at 40°C, followed by washing the sample 10 times in 1X SSC at 40°C and 5 times in 1X SSC buffer at room temperature. *In vivo* hybridization conditions consist of intracellular conditions (*e.g.,* physiological pH and intracellular ionic conditions) that govern the hybridization of antisense oligonucleotides with target sequences. *In vivo* conditions can be mimicked *in vitro* by relatively low stringency conditions. For example, hybridization can be carried out *in vitro* in 2X SSC (0.3 M sodium chloride/0.03 M sodium citrate), 0.1% SDS at 37°C. A wash solution containing 4X SSC, 0.1% SDS can be used at 37°C, with a final wash in 1X SSC at 45°C.

In some aspects, the ASO of the present disclosure is capable of targeting a *STAT6* transcript from one or more species (*e.g.,* humans, non-human primates, dogs, cats, guinea pigs, rabbits, rats, mice, horses, cattle, and bears). In certain aspects, the ASO disclosed herein is capable of targeting both human and rodent (*e.g.,* mice or rats) *STAT6* transcript. Accordingly, in some aspects, the ASO is capable of down-regulating (*e.g.,* reducing or removing) expression of the *STAT6* mRNA or protein both in humans and in rodents (*e.g.,* mice or rats). In some aspects, any ASO described herein is part of a conjugate, comprising the ASO covalently linked to at least one non-nucleotide or non-polynucleotide.

Certain aspects of the present disclosure not falling within the scope of the appended claims are directed to a conjugate comprising an ASO described herein. In certain aspects, the conjugate comprises an ASO covalently attached to at least one non-nucleotide. In certain aspects, the conjugate comprises an ASO covalently attached to at least non-polynucleotide moiety. In some aspects, the non-nucleotide or non-polynucleotide moiety comprises a protein, a fatty acid chain, a sugar residue, a glycoprotein, a polymer, or any combinations thereof.

### II.B. ASO Sequences

The ASOs of the disclosure comprise a contiguous nucleotide sequence which corresponds to the complement of a region of *STAT6* transcript, *e.g.,* a nucleotide sequence corresponding to SEQ ID NO: 1 or SEQ ID NO: 3, as defined in the claims.

In certain aspects, the disclosure provides an ASO from 10 - 30, such as 10 - 15 nucleotides, 10 - 20 nucleotides, 10 - 25 nucleotides in length, or about 20 nucleotides in length, wherein the contiguous nucleotide sequence has at least about 80%, at least about 85%, at least about 90%, at least about 95%, at least about 96%, at least about 97%, at least about 98%, at least about 99%, or about 100% sequence identity to a region within the complement of a *STAT6* transcript, such as SEQ ID NO: 1 or SEQ ID NO: 3 or naturally occurring variant thereof. Thus, for example, the ASO hybridizes to a single stranded nucleic acid molecule having the sequence of SEQ ID NO: 1 or SEQ ID NO: 3 or a portion thereof, as defined in the claims.

The ASO can comprise a contiguous nucleotide sequence which is fully complementary (perfectly complementary) to the equivalent region of a nucleic acid which encodes a mammalian STAT6 protein (*e.g.,* SEQ ID NO: 1 or SEQ ID NO: 3), as defined in the claims. The ASO can comprise a contiguous nucleotide sequence which is fully complementary (perfectly complementary) to a nucleic acid sequence, or a region within the sequence, corresponding to nucleotides X-Y of SEQ ID NO: 1 or SEQ ID NO: 3, wherein X and Y are the start site and the end site, respectively, as shown in FIG. 1A, as defined in the claims.

The ASO can comprise a contiguous nucleotide sequence which is fully complementary (perfectly complementary) to the equivalent region of a mRNA which encodes a mammalian STAT6 protein (*e.g.,* SEQ ID NO: 3), as defined in the claims. The ASO can comprise a contiguous nucleotide sequence which is fully complementary (perfectly complementary) to a mRNA sequence, or a region within the sequence, corresponding to nucleotides X-Y of SEQ ID NO: 3, wherein X and Y are the start site and the end site, respectively, as defined in the claims.

The ASO defined in the claims comprises the nucleotide sequence set forth in SEQ ID NO: 91. In some aspects, the nucleotide sequence of the ASOs of the disclosure or the contiguous nucleotide sequence has at least about 80% sequence identity to a sequence selected from SEQ ID NOs: 91 to 193 (*i.e.,* the sequences in FIG. 1A), such as at least about 80%, at least about 85%, at least about 90%, at least about 91%, at least about 92%, at least about 93%, at least about 94%, at least about 95%, at least about 96% sequence identity, at least about 97% sequence identity, at least about 98% sequence identity, at least about 99% sequence identity, such as about 100% sequence identity (homologous). In some aspects, the ASO has a design described elsewhere herein or a chemical structure shown elsewhere herein (*e.g.,* FIG. 1A).

In some aspects the ASO (or contiguous nucleotide portion thereof) comprises SEQ ID NO: 91, wherein the ASO (or contiguous nucleotide portion thereof) can optionally comprise one, two, three, or four mismatches when compared to the corresponding *STAT6* transcript, within the scope of the appended claims.

The ASO defined in the appended claims comprises the sequence of SEQ ID NO: 91 (e.g., ASO-STAT6-1053).

In other aspects falling outside the scope of the appended claims, the ASO comprises a sequence selected from the group consisting of SEQ ID NO: 92 (e.g., ASO-STAT6-1359), 93 (e.g., ASO-STAT6-1890), 94 (e.g., ASO-STAT6-1892), 95 (e.g., ASO-STAT6-1915), 96 (e.g., ASO-STAT6-1916), 97 (e.g., ASO-STAT6-1917), 98 (e.g., ASO-STAT6-1918), 99 (e.g., ASO-STAT6-1919), 100 (e.g., ASO-STAT6-1920), 101 (e.g., ASO-STAT6-1937), 102 (e.g., ASO-STAT6-1938), 103 (e.g., ASO-STAT6-2061), 104 (e.g., ASO-STAT6-2062), 105 (e.g., ASO-STAT6-2063), 106 (e.g., ASO-STAT6-2064), 107 (e.g., ASO-STAT6-2066), 108 (e.g., ASO-STAT6-2067), 109 (e.g., ASO-STAT6-2068), 110 (e.g., ASO-STAT6-2352), 111 (e.g., ASO-STAT6-3073), 112 (e.g., ASO-STAT6-1053), 113 (e.g., ASO-STAT6-1054), 114 (e.g., ASO-STAT6-1356), 115 (e.g., ASO-STAT6-1847), 116 (e.g., ASO-STAT6-1886), 117 (e.g., ASO-STAT6-1887), 118 (e.g., ASO-STAT6-1888), 119 (e.g., ASO-STAT6-1889), 120 (e.g., ASO-STAT6-1890), 121 (e.g., ASO-STAT6-1893), 122 (e.g., ASO-STAT6-1917), 123 (e.g., ASO-STAT6-1919), 124 (e.g., ASO-STAT6-2056), 125 (e.g., ASO-STAT6-2060), 126 (e.g., ASO-STAT6-2066), 127 (e.g., ASO-STAT6-2070), 128 (e.g., ASO-STAT6-2351), 129 (e.g., ASO-STAT6-2352), 130 (e.g., ASO-STAT6-2359), 131 (e.g., ASO-STAT6-3633), 132 (e.g., ASO-STAT6-673), 133 (e.g., ASO-STAT6-1052), 134 (e.g., ASO-STAT6-1356), 135 (e.g., ASO-STAT6-1357), 136 (e.g., ASO-STAT6-1359), 137 (e.g., ASO-STAT6-1360), 138 (e.g., ASO-STAT6-1839), 139 (e.g., ASO-STAT6-1848), 140 (e.g., ASO-STAT6-1849), 141 (e.g., ASO-STAT6-1891), 142 (e.g., ASO-STAT6-1915), 143 (e.g., ASO-STAT6-1916), 144 (e.g., ASO-STAT6-1917), 145 (e.g., ASO-STAT6-1938), 146 (e.g., ASO-STAT6-1939), 147 (e.g., ASO-STAT6-2063), 148 (e.g., ASO-STAT6-2064), 149 (e.g., ASO-STAT6-2065), 150 (e.g., ASO-STAT6-2066), 151 (e.g., ASO-STAT6-2068), 152 (e.g., ASO-STAT6-2187), 153 (e.g., ASO-STAT6-2350), 154 (e.g., ASO-STAT6-2351), 155 (e.g., ASO-STAT6-2352), 156 (e.g., ASO-STAT6-2357), 157 (e.g., ASO-STAT6-513), 158 (e.g., ASO-STAT6-671), 159 (e.g., ASO-STAT6-1131), 160 (e.g., ASO-STAT6-1354), 161 (e.g., ASO-STAT6-1355), 162 (e.g., ASO-STAT6-1356), 163 (e.g., ASO-STAT6-1432), 164 (e.g., ASO-STAT6-1555), 165 (e.g., ASO-STAT6-1556), 166 (e.g., ASO-STAT6-1557), 167 (e.g., ASO-STAT6-1558), 168 (e.g., ASO-STAT6-1826), 169 (e.g., ASO-STAT6-1827), 170 (e.g., ASO-STAT6-1833), 171 (e.g., ASO-STAT6-1843), 172 (e.g., ASO-STAT6-1846), 173 (e.g., ASO-STAT6-1847), 174 (e.g., ASO-STAT6-1883), 175 (e.g., ASO-STAT6-1889), 176 (e.g., ASO-STAT6-1890), 177 (e.g., ASO-STAT6-1891), 178 (e.g., ASO-STAT6-1916), 179 (e.g., ASO-STAT6-1917), 180 (e.g., ASO-STAT6-2056), 181 (e.g., ASO-STAT6-2057), 182 (e.g., ASO-STAT6-2060), 183 (e.g., ASO-STAT6-2062), 184 (e.g., ASO-STAT6-2063), 185 (e.g., ASO-STAT6-2065), 186 (e.g., ASO-STAT6-2068), 187 (e.g., ASO-STAT6-2347), 188 (e.g., ASO-STAT6-2348), 189 (e.g., ASO-STAT6-2358), 190 (e.g., ASO-STAT6-2782), 191 (e.g., ASO-STAT6-3070), 192 (e.g., ASO-STAT6-3071), and 193 (e.g., ASO-STAT6-3431).

The ASO defined in the appended claims comprises the sequence as set forth in SEQ ID NO: 91 (e.g., ASO-STAT6-1053).

In some aspects, the ASOs of the disclosure bind to the target nucleic acid sequence *(STAT6* transcript) and are capable of inhibiting or reducing expression of the *STAT6* transcript by at least 10% or 20% compared to the normal (*i.e.,* control) expression level in the cell, *e.g.,* at least about 30%, at least about 40%, at least about 50%, at least about 60%, at least about 70%, at least about 80%, at least about 90%, at least about 95%, at least about 96%, at least about 97%, at least about 98%, at least about 99%, or about 100% compared to the normal expression level (*e.g.,* expression level in cells that have not been exposed to the ASO).

In some aspects, the ASOs of the disclosure are capable of reducing expression of *STAT6* mRNA *in vitro* by at least about 20%, at least about 30%, at least about 40%, at least about 50%, at least about 60%, at least about 70%, at least about 80%, at least about 90%, at least about 95%, at least about 96%, at least about 97%, at least about 98%, at least about 99%, or about 100% in target cells when the cells are in contact with the ASO compared to cells that are not in contact with the ASO (*e.g.,* contact with saline).

In some aspects, the ASO can tolerate 1, 2, 3, or 4 (or more) mismatches, when hybridizing to the target sequence and still sufficiently bind to the target to show the desired effect, *i.e.,* down-regulation of the target mRNA and/or protein. Mismatches can, for example, be compensated by increased length of the ASO nucleotide sequence and/or an increased number of nucleotide analogs, which are disclosed elsewhere herein.

In some aspects, the ASO of the disclosure comprises no more than three mismatches when hybridizing to the target sequence. In other aspects, the contiguous nucleotide sequence comprises no more than two mismatches when hybridizing to the target sequence. In other aspects, the contiguous nucleotide sequence comprises no more than one mismatch when hybridizing to the target sequence.

### II.C. ASO Length

The ASOs can comprise a contiguous nucleotide sequence of a total of 15, 16, 17, 18, 19, 20, 21, 22, 23, 24, 25, 26, 27, 28, 29, or 30 contiguous nucleotides in length, as defined in the claims. It should be understood that when a range is given for an ASO, or contiguous nucleotide sequence length, the range includes the lower and upper lengths provided in the range, for example from (or between) 10-30, includes both 10 and 30.

In some aspects, the ASOs comprise a contiguous nucleotide sequence of a total of about 15, 16, 17, 18, 19, or 20 contiguous nucleotides in length, as defined in the claims. In certain aspects, the ASOs comprise a contiguous nucleotide sequence of a total of about 20 contiguous nucleotides in length. In certain aspects, ASOs of the present disclosure are 15 nucleotides in length. In certain aspects, ASOs of the present disclosure are 16 nucleotides in length. In certain aspects, ASOs of the present disclosure are 17 nucleotides in length. In certain aspects, ASOs of the present disclosure are 18 nucleotides in length. In certain aspects, ASOs of the present disclosure are 19 nucleotides in length.

### II.D. Nucleosides and Nucleoside analogs

In one aspect of the disclosure, the ASOs comprise one or more non-naturally occurring nucleoside analogs. "Nucleoside analogs" as used herein are variants of natural nucleosides, such as DNA or RNA nucleosides, by virtue of modifications in the sugar and/or base moieties. Analogs could in principle be merely "silent" or "equivalent" to the natural nucleosides in the context of the oligonucleotide, *i.e.* have no functional effect on the way the oligonucleotide works to inhibit target gene expression. Such "equivalent" analogs can nevertheless be useful if, for example, they are easier or cheaper to manufacture, or are more stable to storage or manufacturing conditions, or represent a tag or label. In some aspects, however, the analogs will have a functional effect on the way in which the ASO works to inhibit expression; for example by producing increased binding affinity to the target and/or increased resistance to intracellular nucleases and/or increased ease of transport into the cell. Specific examples of nucleoside analogs are described by *e.g.* Freier & Altmann; Nucl. Acid Res., 1997, 25, 4429-4443 and Uhlmann; Curr. Opinion in Drug Development, 2000, 3(2), 293-213, and in Scheme 1. The ASOs of the present disclosure can contain more than one, more than two, more than three, more than four, more than five, more than six, more than seven, more than eight, more than nine, more than 10, more than 11, more than 12, more than 13, more than 14, more than 15, more than 16, more than 18, more than 19, or more than 20 nucleoside analogs. In some aspects, the nucleoside analogs in the ASOs are the same. In other aspects, the nucleoside analogs in the ASOs are different. The nucleotide analogs in the ASOs can be any one of or combination of the following nucleoside analogs.

In some aspects, the nucleoside analog comprises a 2'-O-alkyl-RNA; 2'-O-methyl RNA (2'-OMe); 2'-alkoxy-RNA; 2'-O-methoxyethyl-RNA (2'-MOE); 2'-amino-DNA; 2'-fluro-RNA; 2'-fluoro-DNA; arabino nucleic acid (ANA); 2'-fluoro-ANA; bicyclic nucleoside analog; or any combination thereof. In some aspects, the nucleoside analog comprises a sugar modified nucleoside. In some aspects, the nucleoside analog comprises a nucleoside comprising a bicyclic sugar. In some aspects, the nucleoside analog comprises an LNA.

In some aspects, the nucleoside analog is selected from the group consisting of constrained ethyl nucleoside (cEt), 2',4'-constrained 2'-O-methoxyethyl (cMOE), α-L-LNA, β-D-LNA, 2'-O,4'-C-ethylene-bridged nucleic acids (ENA), amino-LNA, oxy-LNA, thio-LNA, and any combination thereof. In some aspects, the ASO comprises one or more 5'-methyl-cytosine nucleobases.

### II.D.1. Nucleobase

The term nucleobase includes the purine (*e.g.,* adenine and guanine) and pyrimidine (*e.g.,* uracil, thymine and cytosine) moiety present in nucleosides and nucleotides which form hydrogen bonds in nucleic acid hybridization. In the context of the present disclosure, the term nucleobase also encompasses modified nucleobases which may differ from naturally occurring nucleobases, but are functional during nucleic acid hybridization. In some aspects, the nucleobase moiety is modified by modifying or replacing the nucleobase. In this context, "nucleobase" refers to both naturally occurring nucleobases such as adenine, guanine, cytosine, thymidine, uracil, xanthine and hypoxanthine, as well as non-naturally occurring variants. Such variants are for example described in Hirao et al., (2012) Accounts of Chemical Research vol 45 page 2055 and Bergstrom (2009) Current Protocols in Nucleic Acid Chemistry Suppl. 37 1.4.1.

In a some aspects, the nucleobase moiety is modified by changing the purine or pyrimidine into a modified purine or pyrimidine, such as substituted purine or substituted pyrimidine, such as a nucleobase selected from isocytosine, pseudoisocytosine, 5-methyl-cytosine, 5-thiozolo-cytosine, 5-propynyl-cytosine, 5-propynyl-uracil, 5-bromouracil, 5-thiazolo-uracil, 2-thio-uracil, 2'thio-thymine, inosine, diaminopurine, 6-aminopurine, 2-aminopurine, 2,6-diaminopurine, and 2-chloro-6-aminopurine.

The nucleobase moieties may be indicated by the letter code for each corresponding nucleobase, *e.g.,* A, T, G, C, or U, wherein each letter may optionally include modified nucleobases of equivalent function. For example, in the exemplified oligonucleotides, the nucleobase moieties are selected from A, T, G, C, and 5-methyl-cytosine. Optionally, for LNA gapmers, 5-methyl-cytosine LNA nucleosides may be used.

### II.D.2. Sugar Modification

The ASO of the disclosure can comprise one or more nucleosides which have a modified sugar moiety, *i.e.* a modification of the sugar moiety when compared to the ribose sugar moiety found in DNA and RNA. Numerous nucleosides with modification of the ribose sugar moiety have been made, primarily with the aim of improving certain properties of oligonucleotides, such as affinity and/or nuclease resistance.

Such modifications include those where the ribose ring structure is modified, *e.g.* by replacement with a hexose ring (HNA), or a bicyclic ring, which typically have a biradical bridge between the C2' and C4' carbons on the ribose ring (LNA), or an unlinked ribose ring which typically lacks a bond between the C2' and C3' carbons (*e.g.,* UNA). Other sugar modified nucleosides include, for example, bicyclohexose nucleic acids (WO2011/017521) or tricyclic nucleic acids (WO2013/154798). Modified nucleosides also include nucleosides where the sugar moiety is replaced with a non-sugar moiety, for example in the case of peptide nucleic acids (PNA), or morpholino nucleic acids.

Sugar modifications also include modifications made via altering the substituent groups on the ribose ring to groups other than hydrogen, or the 2'-OH group naturally found in RNA nucleosides. Substituents may, for example be introduced at the 2', 3', 4', or 5' positions. Nucleosides with modified sugar moieties also include 2' modified nucleosides, such as 2' substituted nucleosides. Indeed, much focus has been spent on developing 2' substituted nucleosides, and numerous 2' substituted nucleosides have been found to have beneficial properties when incorporated into oligonucleotides, such as enhanced nucleoside resistance and enhanced affinity.

### II.D.2.a 2' modified nucleosides

A 2' sugar modified nucleoside is a nucleoside which has a substituent other than H or -OH at the 2' position (2' substituted nucleoside) or comprises a 2' linked biradical, and includes 2' substituted nucleosides and LNA (2' - 4' biradical bridged) nucleosides. For example, the 2' modified sugar may provide enhanced binding affinity (*e.g.,* affinity enhancing 2' sugar modified nucleoside) and/or increased nuclease resistance to the oligonucleotide. Examples of 2' substituted modified nucleosides are 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, 2'-Fluro-DNA, arabino nucleic acids (ANA), and 2'-Fluoro-ANA nucleoside. For further examples, please *see, e.g.,* Freier & Altmann; Nucl. Acid Res., 1997, 25, 4429-4443; Uhlmann, Curr. Opinion in Drug Development, 2000, 3(2), 293-213; and Deleavey and Damha, Chemistry and Biology 2012, 19, 937. Below are illustrations of some 2' substituted modified nucleosides.

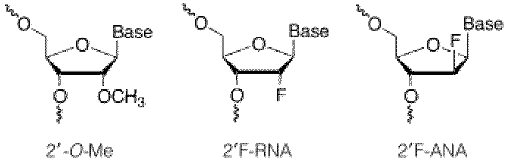

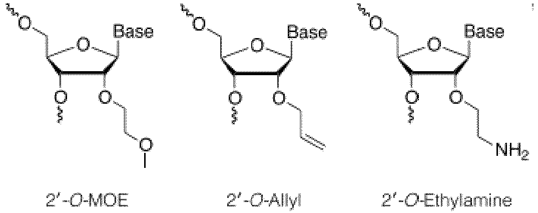

### II.D.2.b Locked Nucleic Acid Nucleosides (LNA).

LNA nucleosides are modified nucleosides which comprise a linker group (referred to as a biradical or a bridge) between C2' and C4' of the ribose sugar ring of a nucleoside (*i.e., 2'-*4' bridge), which restricts or locks the conformation of the ribose ring. These nucleosides are also termed bridged nucleic acid or bicyclic nucleic acid (BNA) in the literature. The locking of the conformation of the ribose is associated with an enhanced affinity of hybridization (duplex stabilization) when the LNA is incorporated into an oligonucleotide for a complementary RNA or DNA molecule. This can be routinely determined by measuring the melting temperature of the oligonucleotide/complement duplex.

Non limiting, exemplary LNA nucleosides are disclosed in WO 99/014226, WO 00/66604, WO 98/039352 , WO 2004/046160, WO 00/047599, WO 2007/134181, WO 2010/077578, WO 2010/036698, WO 2007/090071, WO 2009/006478, WO 2011/156202, WO 2008/154401, WO 2009/067647, WO 2008/150729, Morita et al., Bioorganic & Med.Chem. Lett. 12, 73-76, Seth et al., J. Org. Chem. 2010, Vol 75(5) pp. 1569-81, and Mitsuoka et al., Nucleic Acids Research 2009, 37(4), 1225-1238.

In some aspects, the modified nucleoside or the LNA nucleosides of the ASO of the disclosure has a general structure of the formula I or II: 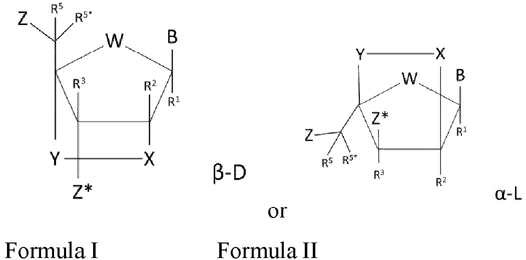 wherein
W is selected from -O-, -S-, -N(R^{a})-, -C(R^{a}R^{b})-, in particular -O-;
B is a nucleobase or a modified nucleobase moiety;
Z is an intemucleoside linkage to an adjacent nucleoside or a 5'-terminal group;
Z* is an intemucleoside linkage to an adjacent nucleoside or a 3'-terminal group;
R¹, R², R³, R⁵ and R⁵* are independently selected from hydrogen, halogen, alkyl, alkenyl, alkynyl, hydroxy, alkoxy, alkoxyalkyl, alkenyloxy, carboxyl, alkoxycarbonyl, alkylcarbonyl, formyl, azide, heterocycle and aryl; and
X, Y, R^{a} and R^{b} are as defined herein.

In some aspects, -X-Y-, R^{a} is hydrogen or alkyl, in particular hydrogen or methyl. In some aspects of -X-Y-, R^{b} is hydrogen or alkyl, in particular hydrogen or methyl. In other aspects of -X-Y-, one or both of R^{a} and R^{b} are hydrogen. In further aspects of -X-Y-, only one of R^{a} and R^{b} is hydrogen. In some aspects of -X-Y-, one of R^{a} and R^{b} is methyl and the other one is hydrogen. In certain aspects of -X-Y-, R^{a} and R^{b} are both methyl at the same time.

In some aspects, -X-, R^{a} is hydrogen or alkyl, in particular hydrogen or methyl. In some aspects of -X-, R^{b} is hydrogen or alkyl, in particular hydrogen or methyl. In other aspects of -X-, one or both of R^{a} and R^{b} are hydrogen. In certain aspects of -X-, only one of R^{a} and R^{b} is hydrogen. In certain aspects of -X-, one of R^{a} and R^{b} is methyl and the other one is hydrogen. In other aspects of -X-, R^{a} and R^{b} are both methyl at the same time.

In some aspects, -Y-, R^{a} is hydrogen or alkyl, in particular hydrogen or methyl. In certain aspects of -Y-, R^{b} is hydrogen or alkyl, in particular hydrogen or methyl. In other aspects of -Y-, one or both of R^{a} and R^{b} are hydrogen. In some aspects of -Y-, only one of R^{a} and R^{b} is hydrogen. In other aspects of -Y-, one of R^{a} and R^{b} is methyl and the other one is hydrogen. In some aspects of -Y-, R^{a} and R^{b} are both methyl at the same time.

In some aspects, R¹, R², R³, R⁵ and R⁵* are independently selected from hydrogen and alkyl, in particular hydrogen and methyl.

In some aspects, R¹, R², R³, R⁵ and R⁵* are all hydrogen at the same time.

In some aspects, R¹, R², R³, are all hydrogen at the same time, one of R⁵ and R⁵* is hydrogen and the other one is as defined above, in particular alkyl, more particularly methyl.

In some aspects, R¹, R², R³, are all hydrogen at the same time, one of R⁵ and R⁵* is hydrogen and the other one is azide..

In some aspects, -X-Y- is -O-CH₂-, W is oxygen and R¹, R², R³, R⁵ and R⁵* are all hydrogen at the same time. Such LNA nucleosides are disclosed in WO 99/014226, WO 00/66604, WO 98/039352 and WO 2004/046160 and include what are commonly known in the art as beta-D-oxy LNA and alpha-L-oxy LNA nucleosides.

In some aspects, -X-Y- is -S-CH₂-, W is oxygen and R¹, R², R³, R⁵ and R⁵* are all hydrogen at the same time. Such thio LNA nucleosides are disclosed in WO 99/014226 and WO 2004/046160.

In some aspects, -X-Y- is -NH-CH₂-, W is oxygen and R¹, R², R³, R⁵ and R⁵* are all hydrogen at the same time. Such amino LNA nucleosides are disclosed in WO 99/014226 and WO 2004/046160.

In some aspects, -X-Y- is -O-CH₂CH₂- or -OCH₂CH₂CH₂-, W is oxygen, and R¹, R², R³, R⁵ and R⁵* are all hydrogen at the same time. Such LNA nucleosides are disclosed in WO 00/047599 and Morita et al., Bioorganic & Med.Chem. Lett. 12, 73-76, and include what are commonly known in the art as 2'-O-4'C-ethylene bridged nucleic acids (ENA).

In some aspects, -X-Y- is -O-CH₂-, W is oxygen, R¹, R², R³ are all hydrogen at the same time, one of R⁵ and R⁵* is hydrogen and the other one is not hydrogen, such as alkyl, for example methyl. Such 5' substituted LNA nucleosides are disclosed in WO 2007/134181.

In some aspects, -X-Y- is -O-CR^{a}R^{b}-, wherein one or both of R^{a} and R^{b} are not hydrogen, in particular alkyl such as methyl, W is oxygen, R¹, R², R³ are all hydrogen at the same time, one of R⁵ and R⁵* is hydrogen and the other one is not hydrogen, in particular alkyl, for example methyl. Such bis modified LNA nucleosides are disclosed in WO 2010/077578.

In some aspects, -X-Y- is -O-CH(CH₂-O-CH₃)- ("2' O-methoxyethyl bicyclic nucleic acid", Seth et al., J. Org. Chem. 2010, Vol 75(5) pp. 1569-81).

In some aspects, -X-Y- is -O-CHR^{a}-, W is oxygen and R¹, R², R³, R⁵ and R⁵* are all hydrogen at the same time. Such 6'-substituted LNA nucleosides are disclosed in WO 2010/036698 and WO 2007/090071. In such 6'-substituted LNA nucleosides, R^{a} is in particular C1-C6 alkyl, such as methyl.

In some aspects, -X-Y- is -O-CH(CH₂-O-CH₃)-, W is oxygen and R¹, R², R³, R⁵ and R⁵* are all hydrogen at the same time. Such LNA nucleosides are also known in the art as cyclic MOEs (cMOE) and are disclosed in WO 2007/090071.

In some aspects, -X-Y- is -O-CH(CH₃)-.

In some aspects, -X-Y- is -O-CH₂-O-CH₂- (Seth et al., J. Org. Chem 2010 op. cit.)

In some aspects, -X-Y- is -O-CH(CH₃)-, W is oxygen and R¹, R², R³, R⁵ and R^{5*} are all hydrogen at the same time. Such 6'-methyl LNA nucleosides are also known in the art as cET nucleosides, and may be either (S)-cET or (R)-cET diastereoisomers, as disclosed in WO 2007/090071 (beta-D) and WO 2010/036698 (alpha-L).

In some aspects, -X-Y- is -O-CR^{a}R^{b}-, wherein neither R^{a} nor R^{b} is hydrogen, W is oxygen, and R¹, R², R³, R⁵ and R^{5*} are all hydrogen at the same time. In certain aspects, R^{a} and R^{b} are both alkyl at the same time, in particular both methyl at the same time. Such 6'-di-substituted LNA nucleosides are disclosed in WO 2009/006478.

In some aspects, -X-Y- is -S-CHR^{a}-, W is oxygen, and R¹, R², R³, R⁵ and R^{5*} are all hydrogen at the same time. Such 6'-substituted thio LNA nucleosides are disclosed in WO 2011/156202. In certain aspects of such 6'-substituted thio LNA, R^{a} is alkyl, in particular methyl.

In some aspects, -X-Y- is -C(=CH₂)C(R^{a}R^{b})-, such as, W is oxygen, and R¹, R², R³, R⁵ and R^{5*} are all hydrogen at the same time. Such vinyl carbo LNA nucleosides are disclosed in WO 2008/154401 and WO 2009/067647.

In some aspects, -X-Y- is -N(OR^{a})-CH₂-, W is oxygen and R¹, R², R³, R⁵ and R^{5*} are all hydrogen at the same time. In some aspects, R^{a} is alkyl such as methyl. Such LNA nucleosides are also known as N substituted LNAs and are disclosed in WO 2008/150729.

In some aspects, -X-Y- is -O-NCH₃- (Seth et al., J. Org. Chem 2010 op. cit.).

In some aspects, -X-Y- is ON(R^{a})- -N(R^{a})-O-,-NR^{a}-CR^{a}R^{b}-CR^{a}R^{b}-, or -NR^{a}-CR^{a}R^{b}-, W is oxygen, and R¹, R², R³, R⁵ and R^{5*} are all hydrogen at the same time. In certain aspects, R^{a} is alkyl, such as methyl. (Seth et al., J. Org. Chem 2010 op. cit.).

In some aspects, R⁵ and R^{5*} are both hydrogen at the same time. In other aspects, one of R⁵ and R^{5*} is hydrogen and the other one is alkyl, such as methyl. In such aspects, R¹, R² and R³ can be in particular hydrogen and -X-Y- can be in particular -O-CH₂- or -O-CHC(R^{a})₃-, such as -O-CH(CH₃)-.

In some aspects, -X-Y- is -CR^{a}R^{b}-O-CR^{a}R^{b}-, such as -CH₂-O-CH₂-, W is oxygen and R¹, R², R³, R⁵ and R^{5*} are all hydrogen at the same time. In such aspects, R^{a} can be in particular alkyl such as methyl. Such LNA nucleosides are also known as conformationally restricted nucleotides (CRNs) and are disclosed in WO 2013/036868.

In some aspects, -X-Y- is -O-CR^{a}R^{b}-O-CR^{a}R^{b}-, such as -O-CH₂-O-CH₂-, W is oxygen and R¹, R², R³, R⁵ and R^{5*} are all hydrogen at the same time. In certain aspects, R^{a} can be in particular alkyl such as methyl. Such LNA nucleosides are also known as COC nucleotides and are disclosed in Mitsuoka et al., Nucleic Acids Research 2009, 37(4), 1225-1238.

It will be recognized than, unless specified, the LNA nucleosides may be in the beta-D or alpha-L stereoisoform.

Certain examples of LNA nucleosides are presented in Scheme 1.

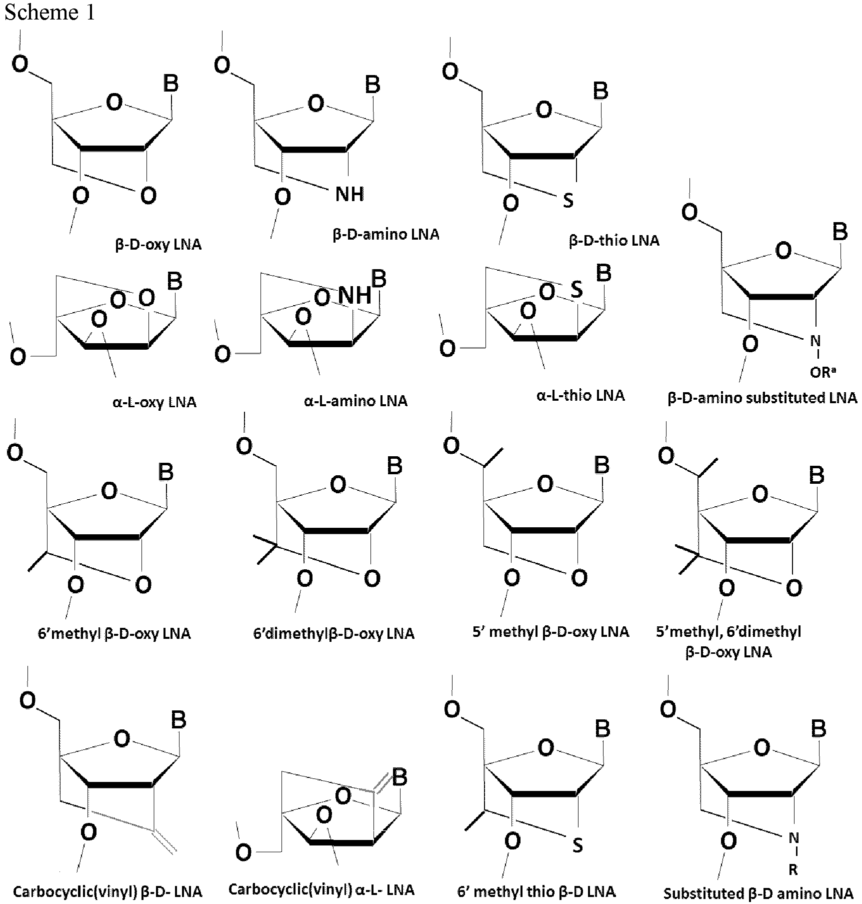

As illustrated elsewhere, in some aspects of the disclosure the LNA nucleosides in the oligonucleotides are beta-D-oxy-LNA nucleosides.

### III.E. Nuclease mediated degradation

Nuclease mediated degradation refers to an oligonucleotide capable of mediating degradation of a complementary nucleotide sequence when forming a duplex with such a sequence.

In some aspects, the oligonucleotide may function via nuclease mediated degradation of the target nucleic acid, where the oligonucleotides of the disclosure are capable of recruiting a nuclease, particularly and endonuclease, preferably endoribonuclease (RNase), such as RNase H. Examples of oligonucleotide designs which operate via nuclease mediated mechanisms are oligonucleotides which typically comprise a region of at least 5 or 6 DNA nucleosides and are flanked on one side or both sides by affinity enhancing nucleosides, for example gapmers.

### II.F. RNase H Activity and Recruitment

The RNase H activity of an antisense oligonucleotide refers to its ability to recruit RNase H when in a duplex with a complementary RNA molecule and induce degradation of the complementary RNA molecule. WO01/23613 provides *in vitro* methods for determining RNaseH activity, which may be used to determine the ability to recruit RNaseH. Typically, an oligonucleotide is deemed capable of recruiting RNase H if, when provided with a complementary target nucleic acid sequence, it has an initial rate, as measured in pmol/l/min, of at least 5%, such as at least 10% or more than 20% of the of the initial rate determined when using a oligonucleotide having the same base sequence as the modified oligonucleotide being tested, but containing only DNA monomers, with phosphorothioate linkages between all monomers in the oligonucleotide, and using the methodology provided by Example 91 - 95 of WO01/23613.

In some aspects, an oligonucleotide is deemed essentially incapable of recruiting RNaseH if, when provided with the complementary target nucleic acid, the RNaseH initial rate, as measured in pmol/l/min, is less than 20%, such as less than 10%,such as less than 5% of the initial rate determined when using a oligonucleotide having the same base sequence as the oligonucleotide being tested, but containing only DNA monomers, with no 2' substitutions, with phosphorothioate linkages between all monomers in the oligonucleotide, and using the methodology provided by Example 91 - 95 of WO01/23613.

### II.G. ASO Design

The ASO of the disclosure can comprise a nucleotide sequence which comprises both nucleosides and nucleoside analogs, and can be in the form of a gapmer. Examples of configurations of a gapmer that can be used with the ASO of the disclosure are described in U.S. Patent Appl. Publ. No. 2012/0322851.

The term "gapmer" as used herein refers to an antisense oligonucleotide which comprises a region of RNase H recruiting oligonucleotides (gap) which is flanked 5' and 3' by one or more affinity enhancing modified nucleosides (flanks). The term "LNA gapmer" is a gapmer oligonucleotide wherein at least one of the affinity enhancing modified nucleosides is an LNA nucleoside. The term "mixed wing gapmer" refers to an LNA gapmer wherein the flank regions comprise at least one LNA nucleoside and at least one DNA nucleoside or non-LNA modified nucleoside, such as at least one 2' substituted modified nucleoside, such as, for example, 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, 2'-Fluro-DNA, arabino nucleic acid (ANA), and 2'-Fluoro-ANA nucleoside(s).

In some aspects, the ASO of the disclosure can be in the form of a mixmer. In some aspects, the ASO of the disclosure can be in the form of a totalmer. In some aspects, in addition to enhancing affinity of the ASO for the target region, some nucleoside analogs also mediate RNase (*e.g.,* RNaseH) binding and cleavage. Since α-L-LNA monomers recruit RNaseH activity to a certain extent, in some aspects, gap regions (*e.g.,* region B as referred to herein) of ASOs containing α-L-LNA monomers consist of fewer monomers recognizable and cleavable by the RNaseH, and more flexibility in the mixmer construction is introduced.

### II.G.1. Gapmer Design

In some aspects, the ASO of the disclosure is a gapmer and comprises a contiguous stretch of nucleotides (*e.g.,* one or more DNA) which is capable of recruiting an RNase, such as RNaseH, referred to herein in as region B (B), wherein region B is flanked at both 5' and 3' by regions of nucleoside analogs 5' and 3' to the contiguous stretch of nucleotides of region B- these regions are referred to as regions A (A) and C (C), respectively. In some aspects, the nucleoside analogs are sugar modified nucleosides (*e.g.,* high affinity sugar modified nucleosides). In certain aspects, the sugar modified nucleosides of regions A and C enhance the affinity of the ASO for the target nucleic acid (*i.e.,* affinity enhancing 2' sugar modified nucleosides). In some aspects, the sugar modified nucleosides are 2' sugar modified nucleosides, such as high affinity 2' sugar modifications, such as LNA and/or 2'-MOE.

In a gapmer, the 5' and 3' most nucleosides of region B are DNA nucleosides, and are positioned adjacent to nucleoside analogs (*e.g.,* high affinity sugar modified nucleosides) of regions A and C, respectively. In some aspects, regions A and C can be further defined by having nucleoside analogs at the end most distant from region B (*i.e.,* at the 5' end of region A and at the 3' end of region C).

**In** some aspects, the ASOs of the present disclosure comprise a nucleotide sequence of formula (5' to 3') A-B-C, wherein: (A) (5' region or a first wing sequence) comprises at least one nucleoside analog (*e.g.,* 3-5 LNA units); (B) comprises at least four consecutive nucleosides (*e.g.,* 4-24 DNA units), which are capable of recruiting RNase (when formed in a duplex with a complementary RNA molecule, such as the pre-mRNA or mRNA target); and (C) (3' region or a second wing sequence) comprises at least one nucleoside analog (*e.g.,* 3-5 LNA units).

In some aspects, region A comprises 3-5 nucleoside analogs, such as LNA, region B consists of 6-24 (*e.g.,* 6, 7, 8, 9, 10, 11, 12, 13, or 14) DNA units, and region C consists of 3 or 4 nucleoside analogs, such as LNA. Such designs include (A-B-C) 3-14-3, 3-11-3, 3-12-3, 3-13-3, 4-9-4, 4-10-4, 4-11-4, 4-12-4, and 5-10-5 . In some aspects, the ASO has a design of LLLDₙLLL, LLLLDₙLLLL, or LLLLLDₙLLLLL, wherein the L is a nucleoside analog, the D is DNA, and n can be any integer between 4 and 24. In some aspects, n can be any integer between 6 and 14. In some aspects, n can be any integer between 8 and 12. In some aspects, the ASO has a design of LLLMMDnMMLLL, LLLMDnMLLL, LLLLMMDnMMLLLL, LLLLMDnMLLLL, LLLLLLMMDnMMLLLLL, or LLLLLLMDnMLLLLL, wherein the D is DNA, n can be any integer between 3 and 15, the L is LNA, and the M is 2'MOE.

Further gapmer designs are disclosed in WO2004/046160, WO 2007/146511, and WO2008/113832.

### II.H. Internucleotide Linkages

The monomers of the ASOs described herein are coupled together via linkage groups. Suitably, each monomer is linked to the 3' adjacent monomer via a linkage group.

The person having ordinary skill in the art would understand that, in the context of the present disclosure, the 5' monomer at the end of an ASO does not comprise a 5' linkage group, although it may or may not comprise a 5' terminal group.

In some aspects, the contiguous nucleotide sequence comprises one or more modified intemucleoside linkages. The terms "linkage group" or "intemucleoside linkage" are intended to mean a group capable of covalently coupling together two nucleosides. Non-limiting examples include phosphate groups and phosphorothioate groups.

The nucleosides of the ASO of the disclosure or contiguous nucleosides sequence thereof are coupled together via linkage groups. Suitably, each nucleoside is linked to the 3' adjacent nucleoside via a linkage group.

In some aspects, the intemucleoside linkage is modified from its normal phosphodiester to one that is more resistant to nuclease attack, such as phosphorothioate, which is cleavable by RNaseH, also allows that route of antisense inhibition in reducing the expression of the target gene. In some aspects, at least 75%, at least 80%, at least 85%, at least 90%, at least 91%, at least 92%, at least 93%, at least 94%, at least 95%, at least 96%, at least 97%, at least 98%, at least 99%, or 100% of intemucleoside linkages are modified.

### III. Extracellular Vesicles, e.g., Exosomes

Disclosed herein are EVs, *e.g.,* exosomes, comprising an ASO. The ASO can be any ASO described herein or a functional fragment thereof. In certain aspects, the ASO reduces the level of an *STAT6* mRNA or an STAT6 protein in a target cell.

In some aspects, the EV, *e.g.,* the exosome, targets a tumor cell, dendritic cell, T cell, B cell, macrophage, monocyte, , neuron, hepatocyte, Kupffer cell, myeloid-lineage cell (*e.g.,* a neutrophil, myeloid-derived suppressor cell (MDSC, *e.g.,* a monocytic MDSC or a granulocytic MDSC), monocyte, macrophage, hematopoietic stem cell, basophil, neutrophil, or eosinophil), or any combination thereof. In some aspects, the EV, *e.g.,* the exosome, targets a myeloid-lineage cell. In some aspects, the EV, *e.g.,* the exosome, targets a macrophage. In certain aspects, the EV, *e.g.,* the exosome, targets the liver, heart, lungs, brain, kidneys, central nervous system, peripheral nervous system, muscle, bone, joint, skin, intestine, bladder, pancreas, lymph nodes, spleen, blood, bone marrow, or any combination thereof.

In some aspects, the EV, *e.g.,* the exosome, reduces the expression of one or more gene that is upregulated by the STAT6. In some aspects, the EV, *e.g.,* the exosome, promotes differentiation of M2 macrophages. In some aspects, the EV, *e.g.,* the exosome, reduces differentiation of M1 macrophages.

In some aspects, the EV, *e.g.,* the exosome, treats a cancer in a subject in need thereof. In some aspects, the cancer is selected from the group consisting of fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, colon adenocarcinoma, rectum adenocarcinoma, pancreatic cancer, pancreatic adenocarcinoma, breast cancer, ovarian cancer, ovarian serous cystadenocarcinoma, prostate cancer, squamous cell cancer, squamous cell cancer of the head and neck cancer, colorectal cancer, lymphoma, leukemia (*e.g.,* acute myelid leukemia), liver cancer, glioblastoma, melanoma, myeloma basal cell cancer, adenocarcinoma, sweat gland cancer, sebaceous gland cancer, papillary cancer, papillary adenocarcinomas, cystadenocarcinoma, medullary cancer, bronchogenic cancer, renal cell cancer, hepatoma, bile duct cancer, choriocarcinoma, seminoma, embryonal cancer, Wilms' tumor, cervical cancer, testicular cancer, testicular germ cell tumors, lung cancer, small cell lung cancer, lung adenocarcinoma, bladder cancer, epithelial cancer, glioma (*e.g.,* brain lower grade glioma), glioblastoma (*e.g.,* glioblastoma multiforme), astrocytoma, medulloblastoma, craniopharyngioma, ependymoma, pinealoma, hemangioblastoma, acoustic neuroma, oligodendroglioma, meningioma, melanoma, uveal melanoma, neuroblastoma, retinoblastoma, thyroid carcinoma, uterine corpus endometrial carcinoma, uterine carcinosarcoma, pheochromocytoma, paraganglioma, and any combination thereof. In some aspects, the EV, *e.g.,* the exosome, increases immune cell, *e.g.,* macrophage, infiltration of a tumor. In certain aspects, the cancer is a myeloid-rich cancer. In certain aspects, the cancer comprises an M2-rich cancer. In some aspects, the cancer comprises a liver cancer. In some aspects, the cancer comprises hepatocellular cancer (HCC). In some aspects, the cancer comprises pancreatic ductal adenocarcinoma (PDAC), in some aspects, the cancer comprises colorectal carcinoma (CRC). In some aspects, the cancer comprises ovarian cancer. In some aspects, the cancer comprises leptomeningeal cancer. In certain aspects, the cancer comprises an intractable tumor of the central nervous system.

In some aspects, the EV, *e.g.,* the exosome, treats a tumor of the central nervous system in a subject. In some aspects, the EV, *e.g.,* the exosome, treats a brain tumor in a subject. In some aspects, the EV, *e.g.,* the exosome, treats a glioblastoma in a subject. In some aspects, the glioblastoma is a glioblastoma multiforme (GBM). In some aspects, the EV, *e.g.,* the exosome, treats a leptomeningeal cancer disease in a subject. In some aspects, the EV, *e.g.,* the exosome, comprising the ASO activates macrophages within the central nervous system. In some aspects, the EV, *e.g.,* the exosome, comprising the ASO induces M1 polarization of macrophages within the central nervous system. In some aspects, the EV, *e.g.,* the exosome, comprising the ASO activates meningeal macrophages. In some aspects, the EV, *e.g.,* the exosome, comprising the ASO induces M1 polarization of meningeal macrophages. In some aspects, the EV, *e.g.,* the exosome, comprising the ASO induces tumor infiltration of meningeal macrophages.

In some aspects, the EV, *e.g.,* the exosome, treats a fibrosis in a subject in need thereof. Excessive M2 macrophage activation leads to the continuous production of TGFβ and growth factors that promote proliferation of myofibroblasts, activation of EMT/EndoMT, and extracellular matrix deposition. M2 macrophages represent a break point between wound healing and exacerbation of pro-fibrotic process. In some aspects, the fibrosis is selected from liver fibrosis (NASH), cirrhosis, pulmonary fibrosis, cystic fibrosis, chronic ulcerative colitis/IBD, bladder fibrosis, kidney fibrosis, CAPS (Muckle-Wells syndrome), atrial fibrosis, endomyocardial fibrosis, old myocardial infarction, glial scar, arterial stiffness, arthrofibrosis, Crohn's disease, Dupuytren's contracture, keloid fibrosis, mediastinal fibrosis, myelofibrosis, Peyronie's disease, nephrogenic systemic fibrosis, progressive massive fibrosis, retroperitoneal fibrosis, scleroderma/systemic sclerosis, adhesive capsulitis, and any combination thereof. In some aspects, the EV, *e.g.,* the exosome, treats liver fibrosis (NASH). In some aspects, the EV, *e.g.,* the exosome, treats CAPS (Muckle-Wells syndrome).

In some aspects, the EV, *e.g.,* the exosome, treats a neurodegenerative disease. In some aspects, the neurodegenerative disease is selected from Alzheimer's disease, Parkinson's disease, prion disease, motor neuron disease, Huntington's disease, spinocerebellar ataxia, spinal muscular atrophy, and any combination thereof.

In some aspects, the EV, *e.g.,* the exosome, treats a metabolic disorder/CVD. In some aspects, the metabolic disorder/CVD is selected from an acid-base imbalance, metabolic brain disease, disorder of calcium metabolism, DNA repair-deficiency disorder, glucose metabolism disorder, hyperlactatemia, iron metabolism disorder, lipid metabolism disorder, malabsorption syndrome, metabolic syndrome X, inborn error of metabolism, mitochondrial disease, phosphorus metabolism disorder, porphyrias, proteostasis deficiency, metabolic skin disease, wasting syndrome, water-electrolyte imbalance, and any combination thereof.

As described *supra,* EVs, *e.g.,* exosomes, described herein are extracellular vesicles with a diameter between about 20-300 nm. The size of the EV, *e.g.,* exosome, described herein can be measured according to methods described, *infra.*

In some aspects, an EV, *e.g.,* exosome, of the present disclosure comprises a bi-lipid membrane ("EV, *e.g.,* exosome, membrane"), comprising an interior (luminal) surface and an exterior surface. In certain aspects, the interior (luminal) surface faces the inner core *(i.e.,* lumen) of the EV, *e.g.,* exosome. In certain aspects, the exterior surface can be in contact with the endosome, the multivesicular bodies, or the membrane/cytoplasm of a producer cell or a target cell

In some aspects, the EV, *e.g.,* exosome, membrane comprises lipids and fatty acids. In some aspects, the EV, *e.g.,* exosome, membrane comprises phospholipids, glycolipids, fatty acids, sphingolipids, phosphoglycerides, sterols, cholesterols, and phosphatidylserines.

In some aspects, the EV, *e.g.,* exosome, membrane comprises an inner leaflet and an outer leaflet. The composition of the inner and outer leaflet can be determined by transbilayer distribution assays known in the art, *see, e.g.,* Kuypers et al., Biohim Biophys Acta 1985 819:170. In some aspects, the composition of the outer leaflet is between approximately 70-90% choline phospholipids, between approximately 0-15% acidic phospholipids, and between approximately 5-30% phosphatidylethanolamine. In some aspects, the composition of the inner leaflet is between approximately 15-40% choline phospholipids, between approximately 10-50% acidic phospholipids, and between approximately 30-60% phosphatidylethanolamine.

In some aspects, the EV, *e.g.,* exosome, membrane comprises one or more polysaccharide, such as glycan.

In some aspects, the EV, *e.g.,* exosome, of the present disclosure comprises an ASO, wherein the ASO is linked to the EV via a scaffold moiety, either on the exterior surface of the EV or on the luminal surface of the EV.

In some aspects, the EV, e.g., exosome, comprising an ASO comprises an anchoring moiety, which optionally comprising a linker, between the ASO and the exosome membrane. Non-limiting examples of the linkers are disclosed elsewhere herein.

### III.A. Anchoring moieties (AM)

One or more anchoring moieties (AMs) can be used to anchor an ASO to the EV of the present disclosure. In some aspects, the ASO is linked directly to the anchoring moiety or via a linker. In some aspects, the ASO can be attached to an anchoring moiety or linker combination via reaction between a "reactive group" (RG; e.g., amine, thiol, hydroxy, carboxylic acid, or azide) with a "reactive moiety" (RM; e.g., maleimide, succinate, NHS). Several potential synthetic routes are envisioned, for example: $\left[AM\right] - / Reactive moiety / + / Reactive group / - \left[ASO\right]$ $\left[AM\right] - \left[Linker\right] n - / Reactive moiety / + / Reactive group / - \left[ASO\right]$ $\left[AM\right] - / Reactive moiety / + / Reactive group / - \left[Linker\right] n - \left[ASO\right]$ $\left[AM\right] - \left[Linker\right] n - / Reactive moiety / + / Reactive group / - \left[Linker\right] n - \left[ASO\right]$

The anchoring moiety can insert into the lipid bilayer of an EV, e.g., an exosome, allowing the loading of the exosome with an ASO. Currently, a predominant obstacle to the commercialization of exosomes as a delivery vehicle for polar ASOs, is highly inefficient loading. This obstacle can be overcome by modifying polar ASOs, prior to loading them into exosomes. Thus, as described herein, modification of ASOs facilitates their loading into exosomes.

The methods of loading exosomes with modified polar ASOs set forth herein significantly improve loading efficiency as compared to the loading efficiency previously reported for introducing unmodified ASOs into exosomes by, for example, electroporation or cationic lipid transfection.

In some aspects, the modifications increase the hydrophobicity of the an ASO by at least about 1, at least about 2, at least about 3, at least about 4, at least about 5, at least about 6, at least about 7, at least about 8, at least about 9, or at least about 10 fold relative to native (non-modified) ASO. In some aspects, the modifications increase the hydrophobicity of the ASO by at least about 1, at least about 2, at least about 3, at least about 4, at least about 5, at least about 6, at least about 7, at least about 8, at least about 9, or at least about 10 orders of magnitude relative to native (non-modified) ASO.

In some aspects, the modifications increase the hydrophobicity of the ASO by at least about 10%, at least about 20%, at least about 30%, at least about 40%, at least about 50%, at least about 60%, at least about 70%, at least about 80%, at least about 90%, at least about 100%, at least about 125%, at least about 150%, at least about 175%, at least about 200%, at least about 250%, at least about 300%, at least about 350%, at least about 400%, at least about 450%, at least about 500%, at least about 600%, at least about 700%, at least about 800%, at least about 900%, or at least about 1000% relative to native (non-modified) ASO, e.g., the corresponding unmodified ASO. Increases in hydrophobicity can be assessed using any suitable method. For example, hydrophobicity can be determined by measuring the percentage solubility in an organic solvent, such as octanol, as compared to solubility in an aqueous solvent, such as water.

In some aspect, an anchoring moiety can be chemically conjugated to an ASO to enhance its hydrophobic character. In exemplary aspects, the anchoring moiety is a sterol (e.g., cholesterol), GM1, a lipid, a vitamin, a small molecule, a peptide, or a combination thereof. In some aspects, the moiety is a lipid. In some aspects, the anchoring moiety is a sterol, e.g., cholesterol. Additional hydrophobic moieties include, for example, phospholipids, lysophospholipids, fatty acids, or vitamins (e.g., vitamin D or vitamin E).

In some aspects, the anchoring moiety is conjugated at the termini of the ASO either directly or via one or more linkers (i.e., "terminal modification"). In other aspects, the anchoring moiety is conjugated to other portions of the ASO.

In some aspects, the ASO can include a detectable label. Exemplary labels include fluorescent labels and/or radioactive labels. In some aspects, where ASOs are fluorescently labeled, the detectable label can be, for example, Cy3. Adding a detectable label to ASOs can be used as a way of labeling exosomes, and following their biodistribution. In other aspects, a detectable label can be attached to exosomes directly, for example, by way of labeling an exosomal lipid and/or an exosomal peptide.

The different components of an ASO (i.e., anchoring moieties, linkers and linker combinations, and ASOs) can be linked by amide, ester, ether, thioether, disulfide, phosphoramidate, phosphotriester, phosphorodithioate, methyl phosphonate, phosphodiester, or phosphorothioate linkages or, alternatively any or other linkage.

In some aspects, the different components of an ASO can be linker using bifunctional linkers (i.e., linkers containing two functional groups), such as N-succinimidyl-3-(2-pyridyldithio)propionate, N-4-maleimide butyric acid, S-(2-pyridyldithio)cysteamine, iodoacetoxysuccinimide, N-(4-maleimidebutyloxy) succinimide, N-[5-(3'-maleimide propylamide)-1-carboxypentyl]iminodiacetic acid, N-(5-aminopentyl)-iminodiacetic acid, and the like.

### III.A.1. Anchoring moieties

Suitable anchoring moieties capable of anchoring an ASO to the surface of an EV, e.g., an exosome, comprise for example sterols (e.g., cholesterol), lipids, lysophospholipids, fatty acids, or fat-soluble vitamins, as described in detail below.

In some aspects, the anchoring moiety can be a lipid. A lipid anchoring moiety can be any lipid known in the art, *e.g.,* palmitic acid or glycosylphosphatidylinositols. **In** some aspects, the lipid, is a fatty acid, phosphatide, phospholipid (e.g., phosphatidyl choline, phosphatidyl serine, or phosphatidyl ethanolamine), or analogue thereof (e.g. phophatidylcholine, lecithin, phosphatidylethanolamine, cephalin, or phosphatidylserine or analogue or portion thereof, such as a partially hydrolyzed portion thereof).

Generally, anchoring moieties are chemically attached. However, an anchoring moiety can be attached to an ASO enzymatically. In some aspects, in the possible to attach an anchoring moiety to an ASO via modification of cell culture conditions. For example, by using a culture medium where myristic acid is limiting, some other fatty acids including shorter-chain and unsaturated, can be attached to an N-terminal glycine. For example, in BK channels, myristate has been reported to be attached posttranslationally to internal serine/threonine or tyrosine residues via a hydroxyester linkage.

The anchoring moiety can be conjugated to an ASO directly or indirectly via a linker combination, at any chemically feasible location, e.g., at the 5' and/or 3' end of the ASO. In one aspect, the anchoring moiety is conjugated only to the 3' end of the ASO. In one aspect, the anchoring moiety is conjugated only to the 5' end of the ASO. In one aspect, the anchoring moiety is conjugated at a location which is not the 3' end or 5' end of the ASO.

Some types of membrane anchors that can be used to practice the methods of the present disclosure presented in the following table:

| *Modification* | *Modifying Group* |
|---|---|
| S-Palmitoylation | 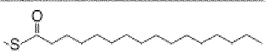 |
| N-Palmitoylation | 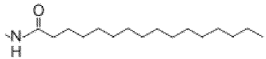 |
| N-Myristoylation | 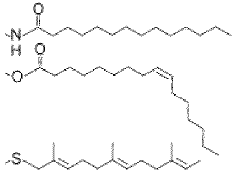 |
| O-Acylation | |
| Farnesylation | |
| Geranylgeranylation | 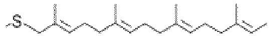 |
| Cholesterol | 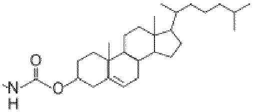 |

In some aspects, an anchoring moiety of the present disclosure can comprise two or more types of anchoring moieties disclosed herein. For example, in some aspects, an anchoring moiety can comprise two lipids, e.g., a phospholipids and a fatty acid, or two phospholipids, or two fatty acids, or a lipid and a vitamin, or cholesterol and a vitamin, etc. which taken together have 6-80 carbon atoms (i.e., an equivalent carbon number (ECN) of 6-80).

In some aspects, the combination of anchoring moieties, e.g., a combination of the lipids (e.g., fatty acids) has an ECN of 6-80, 8-80, 10-80, 12-80, 14-80, 16-80, 18-80, 20-80, 22-80, 24-80, 26-80, 28-80, 30-80, 4-76, 6-76, 8-76, 10-76, 12-76, 14-76, 16-76, 18-76, 20-76, 22-76, 24-76, 26-76, 28-76, 30-76, 6-72, 8-72, 10-72, 12-72, 14-72, 16-72, 18-72, 20-72, 22-72, 24-72, 26-72, 28-72, 30-72, 6-68, 8-68, 10-68, 12-68, 14-68, 16-68, 18-68, 20-68, 22-68, 24-68, 26-68, 28-68, 30-68, 6-64, 8-64, 10-64, 12-64, 14-64, 16-64, 18-64, 20-64, 22-64, 24-64, 26-64, 28-64, 30-64, 6-60, 8-60, 10-60, 12-56, 14-56, 16-56, 18-56, 20-56, 22-56, 24-56, 26-56, 28-56, 30-56, 6-52, 8-52, 10-52, 12-52, 14-52, 16-52, 18-52, 20-52, 22-52, 24-52, 26-52, 28-52, 30-52, 6-48, 8-48, 10-48, 12-48, 14-48, 16-48, 18-48, 20-48, 22-48, 24-48, 26-48, 28-48, 30-48, 6-44, 8-44, 10-44, 12-44, 14-44, 16-44, 18-44, 20-44, 22-44, 24-44, 26-44, 28-44, 30-44, 6-40, 8-40, 10-40, 12-40, 14-40, 16-40, 18-40, 20-40, 22-40, 24-40, 26-40, 28-40, 30-40, 6-36, 8-36, 10-36, 12-36, 14-36, 16-36, 18-36, 20-36, 22-36, 24-36, 26-36, 28-36, 30-36, 6-32, 8-32, 10-32, 12-32, 14-32, 16-32, 18-32, 20-32, 22-32, 24-32, 26-32, 28-32, or 30-32.

### III.A.1.a. Cholesterol and other sterols

In some aspects, the anchoring moiety comprises a sterol, steroid, hopanoid, hydroxysteroid, secosteroid, or analog thereof with lipophilic properties. In some aspects, the anchoring moiety comprises a sterol, such as a phytosterol, mycosterol, or zoosterol. Exemplary zoosterols include cholesterol and 24S-hydroxycholesterol; exemplary phytosterols include ergosterol (mycosterol), campesterol, sitosterol, and stigmasterol. In some aspects, the sterol is selected from ergosterol, 7-dehydrocholesterol, cholesterol, 24S-hydroxycholesterol, lanosterol, cycloartenol, fucosterol, saringosterol, campesterol, β-sitosterol, sitostanol, coprostanol, avenasterol, or stigmasterol. Sterols may be found either as free sterols, acylated (sterol esters), alkylated (steryl alkyl ethers), sulfated (sterol sulfate), or linked to a glycoside moiety (steryl glycosides), which can be itself acylated (acylated sterol glycosides).

In some aspects, the anchoring moiety comprises a steroid. In some aspects, the steroid is selected from dihydrotestosterone, uvaol, hecigenin, diosgenin, progesterone, or cortisol.

For example, sterols may be conjugated to the ASO directly or via a linker combination at the available -OH group of the sterol. Exemplary sterols have the general skeleton shown below:

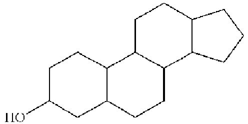

As a further example, ergosterol has the structure below:

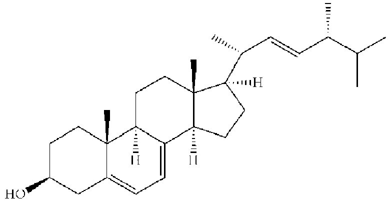

Cholesterol has the structure below:

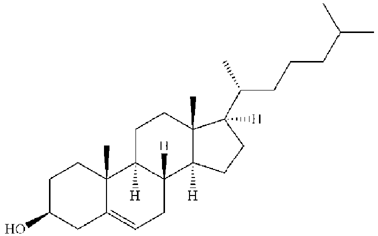

Accordingly, in some embodiments, the free -OH group of a sterol or steroid is used to conjugate the ASO directly or via a linker combination, to the sterol (e.g., cholesterol) or steroid.

### III.A.1.b. Fatty acids

In some aspects, the anchoring moiety is a fatty acid. In some aspects, the fatty acid is a short-chain, medium-chain, or long-chain fatty acid. In some aspects, the fatty acid is a saturated fatty acid. In some aspects, the fatty acid is an unsaturated fatty acid. In some aspects, the fatty acid is a monounsaturated fatty acid. In some aspects, the fatty acid is a polyunsaturated fatty acid, such as an omega-3 or omega-6 fatty acid.

In some aspects, the lipid, e.g., fatty acid, has a C₂-C₆₀ chain. In some embodiments, the lipid, e.g., fatty acid, has a C₂-C₂₈ chain. In some aspects, the fatty acid, has a C₂-C₄₀ chain. In some aspects, the fatty acid, has a C₂-C₁₂ or C₄-C₁₂ chain. In some aspects, the fatty acid, has a C₄-C₄₀ chain. In some aspects, the fatty acid, has a C₄-C₄₀, C₂-C₃₈, C₂-C₃₆, C₂-C₃₄, C₂-C₃₂, C₂-C₃₀, C₄-C₃₀, C₂-C₂₈, C₄-C₂₈, C₂- C₂₆, C₄-C₂₆, C₂-C₂₄, C₄-C₂₄, C₆-C₂₄, C₈-C₂₄, C₁₀-C₂₄, C₂-C₂₂, C₄-C₂₂, C₆-C₂₂, C₈-C₂₂, C₁₀-C₂₂, C₂-C₂₀, C₄-C₂₀, C₆-C₂₀, C₈-C₂₀, C₁₀-C₂₀, C₂-C₁₈, C₄-C₁₈, C₆-C₁₈, C₈-C₁₈, C₁₀-C₁₈, C₁₂-C₁₈, C₁₄-C₁₈, C₁₆-C₁₈, C₂-C₁₆, C₄-C₁₆, C₆-C₁₆, C₈-C₁₆, C₁₀-C₁₆, C₁₂-C₁₆, C₁₄-C₁₆, C₂-C₁₅, C₄-C₁₅, C₆-C₁₅, C₈-C₁₅, C₉-C₁₅, C₁₀-C₁₅, C₁₁-C₁₅, C₁₂-C₁₅, C₁₃-C₁₅, C₂-C₁₄, C₄-C₁₄, C₆-C₁₄, C₈-C₁₄, C₉-C₁₄, C₁₀-C₁₄, C₁₁-C₁₄, C₁₂-C₁₄, C₂-C₁₃, C₄-C₁₃, C₆-C₁₃, C₇-C₁₃, C₈-C₁₃, C₉-C₁₃, C₁₀-C₁₃, C₁₀-C₁₃, C₁₁-C₁₃, C₂-C₁₂, C₄-C₁₂, C₆-C₁₂, C₇-C₁₂, C₈-C₁₂, C₉-C₁₂, C₁₀-C₁₂, C₂-C₁₁, C₄-C₁₁, C₆-C₁₁, C₇-C₁₁, C₈-C₁₁, C₉-C₁₁, C₂-C₁₀, C₄-C₁₀, C₂-C₉, C₄-C₉, C₂-C₈, C₂-C₇, C₄-C₇, C₂-C₆, or C₄-C₆, chain. In some aspects, the fatty acid, has a C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂, C₃₃, C₃₄, C₃₅, C₃₆, C₃₇, C₃₈, C₃₉, C₄₀, C₄₁, C₄₂, C₄₃, C₄₄, C₄₅, C₄₆, C₄₇, C₄₈, C₄₉, C₅₀, C₅₁, C₅₂, C₅₃, C₅₄, C₅₅, C₅₆, C₅₇, C₅₈, C₅₉, or C₆₀ chain.

In some aspects, the anchoring moiety comprises two fatty acids, each of which is independently selected from a fatty acid having a chain with any one of the foregoing ranges or numbers of carbon atoms. In some aspects, one of the fatty acids is independently a fatty acid with a C6-C21 chain and one is independently a fatty acid with a C12-C36 chain. In some embodiments, each fatty acid independently has a chain of 11, 12, 13, 14, 15, 16, or 17 carbon atoms.

Suitable fatty acids include saturated straight-chain fatty acids, saturated branched fatty acids, unsaturated fatty acids, hydroxy fatty acids, and polycarboxylic acids. In some aspects, such fatty acids have up to 32 carbon atoms.

Examples of useful saturated straight-chain fatty acids include those having an even number of carbon atoms, such as butyric acid, caproic acid, caprylic acid, capric acid, lauric acid, myristic acid, palmitic acid, stearic acid, arachic acid, behenic acid, lignoceric acid, hexacosanoic acid, octacosanoic acid, triacontanoic acid and n-dotriacontanoic acid, and those having an odd number of carbon atoms, such as propionic acid, n-valeric acid, enanthic acid, pelargonic acid, hendecanoic acid, tridecanoic acid, pentadecanoic acid, heptadecanoic acid, nonadecanoic acid, heneicosanoic acid, tricosanoic acid, pentacosanoic acid, and heptacosanoic acid.

Examples of suitable saturated branched fatty acids include isobutyric acid, isocaproic acid, isocaprylic acid, isocapric acid, isolauric acid, 11-methyldodecanoic acid, isomyristic acid, 13-methyl-tetradecanoic acid, isopalmitic acid, 15-methyl-hexadecanoic acid, isostearic acid, 17-methyloctadecanoic acid, isoarachic acid, 19-methyl-eicosanoic acid, α-ethylhexanoic acid, α-hexyldecanoic acid, α-heptylundecanoic acid, 2-decyltetradecanoic acid, 2-undecyltetradecanoic acid, 2-decylpentadecanoic acid, 2-undecylpentadecanoic acid, and Fine oxocol 1800 acid (product of Nissan Chemical Industries, Ltd.). Suitable saturated odd-carbon branched fatty acids include anteiso fatty acids terminating with an isobutyl group, such as 6-methyl-octanoic acid, 8-methyl-decanoic acid, 10-methyl-dodecanoic acid, 12-methyl-tetradecanoic acid, 14-methyl-hexadecanoic acid, 16-methyl-octadecanoic acid, 18-methyl-eicosanoic acid, 20-methyl-docosanoic acid, 22-methyl-tetracosanoic acid, 24-methyl-hexacosanoic acid, and 26-methyloctacosanoic acid.

Examples of suitable unsaturated fatty acids include 4-decenoic acid, caproleic acid, 4-dodecenoic acid, 5-dodecenoic acid, lauroleic acid, 4-tetradecenoic acid, 5-tetradecenoic acid, 9-tetradecenoic acid, palmitoleic acid, 6-octadecenoic acid, oleic acid, 9-octadecenoic acid, 11-octadecenoic acid, 9-eicosenoic acid, cis-11-eicosenoic acid, cetoleic acid, 13-docosenoic acid, 15-tetracosenoic acid, 17-hexacosenoic acid, 6,9,12,15-hexadecatetraenoic acid, linoleic acid, linolenic acid, α-eleostearic acid, β-eleostearic acid, punicic acid, 6,9,12,15-octadecatetraenoic acid, parinaric acid, 5,8,11,14-eicosatetraenoic acid, 5,8,11,14,17-eicosapentaenoic acid, 7,10,13,16,19-docosapentaenoic acid, 4,7,10,13,16,19-docosahexaenoic acid, and the like.

Examples of suitable hydroxy fatty acids include α-hydroxylauric acid, α-hydroxymyristic acid, α-hydroxypalmitic acid, α-hydroxystearic acid, ω-hydroxylauric acid, α-hydroxyarachic acid, 9-hydroxy-12-octadecenoic acid, ricinoleic acid, α-hydroxybehenic acid, 9-hydroxy-trans-10,12-octadecadienic acid, kamolenic acid, ipurolic acid, 9,10-dihydroxystearic acid, 12-hydroxystearic acid and the like.

Examples of suitable polycarboxylic acids include oxalic acid, malonic acid, succinic acid, glutaric acid, adipic acid, pimelic acid, suberic acid, azelaic acid, sebacic acid, D,L-malic acid, and the like.

In some aspects, each fatty acid is independently selected from propionic acid, butyric acid, valeric acid, caproic acid, enanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, heneicosylic acid, behenic acid, tricosylic acid, lignoceric acid, pentacosylic acid, cerotic acid, heptacosylic acid, montanic acid, nonacosylic acid, melissic acid, henatriacontylic acid, lacceroic acid, psyllic acid, geddic acid, ceroplastic acid, hexatriacontylic acid, heptatriacontanoic acid, or octatriacontanoic acid.

In some aspects, each fatty acid is independently selected from α-linolenic acid, stearidonic acid, eicosapentaenoic acid, docosahexaenoic acid, linoleic acid, gamma-linoleic acid, dihomo-gamma-linoleic acid, arachidonic acid, docosatetraenoic acid, palmitoleic acid, vaccenic acid, paullinic acid, oleic acid, elaidic acid, gondoic acid, eurcic acid, nervonic acid, mead acid, adrenic acid, bosseopentaenoic acid, ozubondo acid, sardine acid, herring acid, docosahexaenoic acid, or tetracosanolpentaenoic acid, or another monounsaturated or polyunsaturated fatty acid.

In some aspects, one or both of the fatty acids is an essential fatty acid. In view of the beneficial health effects of certain essential fatty acids, the therapeutic benefits of disclosed therapeutic-loaded exosomes may be increased by including such fatty acids in the therapeutic agent. In some aspects, the essential fatty acid is an n-6 or n-3 essential fatty acid selected from the group consisting of linolenic acid, gamma-linolenic acid, dihomo-gamma-linolenic acid, arachidonic acid, adrenic acid, docosapentaenoic n-6 acid, alpha-linolenic acid, stearidonic acid, the 20:4n-3 acid, eicosapentaenoic acid, docosapentaenoic n-3 acid, or docosahexaenoic acid.

In some aspects, each fatty acid is independently selected from all-cis-7,10,13-hexadecatrienoic acid, α-linolenic acid, stearidonic acid, eicosatrienoic acid, eicosatetraenoic acid, eicosapentaenoic acid (EPA), docosapentaenoic acid, docosahexaenoic acid (DHA), tetracosapentaenoic acid, tetracosahexaenoic acid, or lipoic acid. In other aspects, the fatty acid is selected from eicosapentaenoic acid, docosahexaenoic acid, or lipoic acid. Other examples of fatty acids include all-cis-7,10,13-hexadecatrienoic acid, α-linolenic acid (ALA or all-cis-9,12,15-octadecatrienoic acid), stearidonic acid (STD or all-cis-6,9,12,15-octadecatetraenoic acid), eicosatrienoic acid (ETE or all-cis-11,14,17-eicosatrienoic acid), eicosatetraenoic acid (ETA or all-cis-8,11,14,17-eicosatetraenoic acid), eicosapentaenoic acid (EPA), docosapentaenoic acid (DPA, clupanodonic acid or all-cis-7,10,13,16,19-docosapentaenoic acid), docosahexaenoic acid (DHA or all-cis-4,7,10,13,16,19-docosahexaenoic acid), tetracosapentaenoic acid (all-cis-9,12,15,18,21-docosahexaenoic acid), or tetracosahexaenoic acid (nisinic acid or all-cis-6,9,12,15,18,21-tetracosenoic acid). In some aspects, the fatty acid is a medium-chain fatty acid such as lipoic acid.

Fatty acid chains differ greatly in the length of their chains and may be categorized according to chain length, e.g. as short to very long. Short-chain fatty acids (SCFA) are fatty acids with chains of about five or less carbons (e.g. butyric acid). In some aspects, the fatty acid is a SCFA. Medium-chain fatty acids (MCFA) include fatty acids with chains of about 6-12 carbons, which can form medium-chain triglycerides. In some aspects, the fatty acid is a MCFA. Long-chain fatty acids (LCFA) include fatty acids with chains of 13-21 carbons. In some aspects, the fatty acid is a LCFA. In some aspects, the fatty acid is a LCFA. Very long chain fatty acids (VLCFA) include fatty acids with chains of 22 or more carbons, such as 22-60, 22-50, or 22-40 carbons. In some aspects, the fatty acid is a VLCFA.

### III.A.1.c. Phospholipids

In some aspects, the anchoring moiety comprises a phospholipid. Phospholipids are a class of lipids that are a major component of all cell membranes. They can form lipid bilayers because of their amphiphilic characteristic. The structure of the phospholipid molecule generally consists of two hydrophobic fatty acid "tails" and a hydrophilic "head" consisting of a phosphate group. For example, a phospholipid can be a lipid according to the following formula: 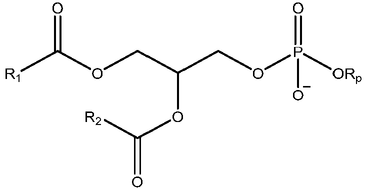 in which Rₚ represents a phospholipid moiety and R₁ and R₂ represent fatty acid moieties with or without unsaturation that may be the same or different.

A phospholipid moiety may be selected, for example, from the non-limiting group consisting of phosphatidyl choline, phosphatidyl ethanolamine, phosphatidyl glycerol, phosphatidyl serine, phosphatidic acid, 2 lysophosphatidyl choline, and a sphingomyelin.

Particular phospholipids may facilitate fusion to a lipid bilayer, e.g., the lipid bilayer of an exosomal membrane. For example, a cationic phospholipid may interact with one or more negatively charged phospholipids of a membrane. Fusion of a phospholipid to a membrane may allow one or more elements of a lipid-containing composition to bind to the membrane or to pass through the membrane.

A fatty acid moiety may be selected, for example, from the non-limiting group consisting of lauric acid, myristic acid, myristoleic acid, palmitic acid, palmitoleic acid, stearic acid, oleic acid, linoleic acid, alpha-linolenic acid, erucic acid, phytanoic acid, arachidic acid, arachidonic acid, eicosapentaenoic acid, behenic acid, docosapentaenoic acid, and docosahexaenoic acid.

The phospholipids using as anchoring moieties in the present disclosure can be natural or non-natural phospholipids. Non-natural phospholipid species including natural species with modifications and substitutions including branching, oxidation, cyclization, and alkynes are also contemplated. For example, a phospholipid may be functionalized with or cross-linked to one or more alkynes (e.g., an alkenyl group in which one or more double bonds is replaced with a triple bond). Under appropriate reaction conditions, an alkyne group may undergo a copper-catalyzed cycloaddition upon exposure to an azide.

Phospholipids include, but are not limited to, glycerophospholipids such as phosphatidylcholines, phosphatidylethanolamines, phosphatidylserines, phosphatidylinositols, phosphatidy glycerols, and phosphatidic acids.

Examples of phospholipids that can be used in the anchoring moieties disclosed herein include
- ***Phosphatidylethanolamines:*** E.g., dilauroylphosphatidyl ethanolamine, dimyristoylphosphatidyl ethanolamine, dipalmitoylphosphatidyl ethanolamine, distearoylphosphatidyl ethanolamine, dioleoylphosphatidyl ethanolamine, 1-palmitoyl-2-oleylphosphatidyl ethanolamine, 1-oleyl-2-palmitoylphosphatidyl ethanolamine, and dierucoylphosphatidyl ethanolamine;
- ***Phosphatidyl glycerols:*** E.g., dilauroylphosphatidyl glycerol, dimyristoylphosphatidyl glycerol, dipalmitoylphosphatidyl glycerol, distearoylphosphatidyl glycerol, dioleoylphosphatidyl glycerol, 1-palmitoyl-2-oleyl-phosphatidyl glycerol, 1-oleyl-2-palmitoyl-phosphatidyl glycerol, and dierucoylphosphatidyl glycerol;
- ***Phosphatidyl serines:*** E.g., such as dilauroylphosphatidyl serine, dimyristoylphosphatidyl serine, dipalmitoylphosphatidyl serine, distearoylphosphatidyl serine, dioleoylphosphatidyl serine, 1-palmitoyl-2-oleyl-phosphatidyl serine, 1-oleyl-2-palmitoyl-phosphatidyl serine, and dierucoylphosphatidyl serine;
- ***Phosphatidic acids:*** E.g., dilauroylphosphatidic acid, dimyristoylphosphatidic acid, dipalmitoylphosphatidic acid, distearoylphosphatidic acid, dioleoylphosphatidic acid, 1-palmitoyl-2-oleylphosphatidic acid, 1-oleyl-2-palmitoyl-phosphatidic acid, and dierucoylphosphatidic acid; and,
- ***Phosphatidyl inositols:*** E.g., dilauroylphosphatidyl inositol, dimyristoylphosphatidyl inositol, dipalmitoylphosphatidyl inositol, distearoylphosphatidyl inositol, dioleoylphosphatidyl inositol, 1-palmitoyl-2-oleyl-phosphatidyl inositol, 1-oleyl-2-palmitoyl-phosphatidyl inositol, and dierucoylphosphatidyl inositol.
Phospholipids may be of a symmetric or an asymmetric type. As used herein, the term "symmetric phospholipid" includes glycerophospholipids having matching fatty acid moieties and sphingolipids in which the variable fatty acid moiety and the hydrocarbon chain of the sphingosine backbone include a comparable number of carbon atoms. As used herein, the term "asymmetric phospholipid" includes lysolipids, glycerophospholipids having different fatty acid moieties (e.g., fatty acid moieties with different numbers of carbon atoms and/or unsaturations (e.g., double bonds)), and sphingolipids in which the variable fatty acid moiety and the hydrocarbon chain of the sphingosine backbone include a dissimilar number of carbon atoms (e.g., the variable fatty acid moiety include at least two more carbon atoms than the hydrocarbon chain or at least two fewer carbon atoms than the hydrocarbon chain).

In some aspects, the anchoring moiety comprises at least one symmetric phospholipid. Symmetric phospholipids may be selected from the non-limiting group consisting of
1,2-dipropionyl-*sn*-glycero-3-phosphocholine (03:0 PC),
1,2-dibutyryl-*sn*-glycero-3-phosphocholine (04:0 PC),
1,2-dipentanoyl-*sn*-glycero-3-phosphocholine (05:0 PC),
1,2-dihexanoyl-*sn*-glycero-3-phosphocholine (06:0 PC),
1,2-diheptanoyl-*sn*-glycero-3-phosphocholine (07:0 PC),
1,2-dioctanoyl-*sn*-glycero-3-phosphocholine (08:0 PC),
1,2-dinonanoyl-*sn*-glycero-3-phosphocholine (09:0 PC),
1,2-didecanoyl-*sn*-glycero-3-phosphocholine (10:0 PC),
1,2-diundecanoyl-*sn*-glycero-3-phosphocholine (11:0 PC, DUPC),
1,2-dilauroyl-*sn*-glycero-3-phosphocholine (12:0 PC),
1,2-ditridecanoyl-*sn*-glycero-3-phosphocholine (13:0 PC),
1,2-dimyristoyl-*sn*-glycero-3-phosphocholine (14:0 PC, DMPC),
1,2-dipentadecanoyl-*sn*-glycero-3-phosphocholine (15:0 PC),
1,2-dipalmitoyl-*sn*-glycero-3-phosphocholine (16:0 PC, DPPC),
1,2-diphytanoyl-*sn*-glycero-3-phosphocholine (4ME 16:0 PC),
1,2-diheptadecanoyl-*sn*-glycero-3-phosphocholine (17:0 PC),
1,2-distearoyl-*sn*-glycero-3-phosphocholine (18:0 PC, DSPC),
1,2-dinonadecanoyl-*sn*-glycero-3-phosphocholine (19:0 PC),
1,2-diarachidoyl-*sn*-glycero-3-phosphocholine (20:0 PC),
1,2-dihenarachidoyl-*sn*-glycero-3-phosphocholine (21:0 PC),
1,2-dibehenoyl-*sn*-glycero-3-phosphocholine (22:0 PC),
1,2-ditricosanoyl-*sn*-glycero-3-phosphocholine (23:0 PC),
1,2-dilignoceroyl-*sn*-glycero-3-phosphocholine (24:0 PC),
1,2-dimyristoleoyl-*sn*-glycero-3-phosphocholine (14:1 (Δ9-Cis) PC),
1,2-dimyristelaidoyl-*sn*-glycero-3-phosphocholine (14:1 (Δ9-Trans) PC),
1,2-dipalmitoleoyl-*sn*-glycero-3-phosphocholine (16:1 (Δ9-Cis) PC),
1,2-dipalmitelaidoyl-*sn*-glycero-3-phosphocholine (16:1 (Δ9-Trans) PC),
1,2-dipetroselenoyl-*sn*-glycero-3-phosphocholine (18:1 (Δ6-Cis) PC),
1,2-dioleoyl-*sn*-glycero-3-phosphocholine (18:1 (Δ9-Cis) PC, DOPC),
1,2-dielaidoyl-*sn*-glycero-3-phosphocholine (18:1 (Δ9-Trans) PC),
1,2-dilinoleoyl-*sn*-glycero-3-phosphocholine (18:2 (Cis) PC, DLPC),
1,2-dilinolenoyl-*sn*-glycero-3-phosphocholine (18:3 (Cis) PC, DLnPC),
1,2-dieicosenoyl-*sn*-glycero-3-phosphocholine (20:1 (Cis) PC),
1,2-diarachidonoyl-*sn*-glycero-3-phosphocholine (20:4 (Cis) PC, DAPC),
1,2-dierucoyl-*sn*-glycero-3-phosphocholine (22:1 (Cis) PC),
1,2-didocosahexaenoyl-*sn*-glycero-3-phosphocholine (22:6 (Cis) PC, DHAPC),
1,2-dinervonoyl-*sn*-glycero-3-phosphocholine (24:1 (Cis) PC),
1,2-dihexanoyl-*sn*-glycero-3-phosphoethanolamine (06:0 PE),
1,2-dioctanoyl-*sn*-glycero-3-phosphoethanolamine (08:0 PE),
1,2-didecanoyl-*sn*-glycero-3-phosphoethanolamine (10:0 PE),
1,2-dilauroyl-*sn*-glycero-3-phosphoethanolamine (12:0 PE),
1,2-dimyristoyl-*sn*-glycero-3-phosphoethanolamine (14:0 PE),
1,2-dipentadecanoyl-*sn*-glycero-3-phosphoethanolamine (15:0 PE),
1,2-dipalmitoyl-*sn*-glycero-3-phosphoethanolamine (16:0 PE),
1,2-diphytanoyl-*sn*-glycero-3-phosphoethanolamine (4ME 16:0 PE),
1,2-diheptadecanoyl-*sn*-glycero-3-phosphoethanolamine (17:0 PE),
1,2-distearoyl-*sn*-glycero-3-phosphoethanolamine (18:0 PE, DSPE),
1,2-dipalmitoleoyl-*sn*-glycero-3-phosphoethanolamine (16:1 PE),
1,2-dioleoyl-*sn*-glycero-3-phosphoethanolamine (18:1 (Δ9-Cis) PE, DOPE),
1,2-dielaidoyl-*sn*-glycero-3-phosphoethanolamine (18:1 (Δ9-Trans) PE),
1,2-dilinoleoyl-*sn*-glycero-3-phosphoethanolamine (18:2 PE, DLPE),
1,2-dilinolenoyl-*sn*-glycero-3-phosphoethanolamine (18:3 PE, DLnPE),
1,2-diarachidonoyl-*sn*-glycero-3-phosphoethanolamine (20:4 PE, DAPE),
1,2-didocosahexaenoyl-*sn*-glycero-3-phosphoethanolamine (22:6 PE, DHAPE),
1,2-di-O-octadecenyl-*sn*-glycero-3-phosphocholine (18:0 Diether PC),
1,2-dioleoyl-*sn*-glycero-3-phospho-rac-(1-glycerol) sodium salt (DOPG), and any combination thereof.

In some aspects, the anchoring moiety comprises at least one symmetric phospholipid selected from the non-limiting group consisting of DLPC, DMPC, DOPC, DPPC, DSPC, DUPC, 18:0 Diether PC, DLnPC, DAPC, DHAPC, DOPE, 4ME 16:0 PE, DSPE, DLPE,DLnPE, DAPE, DHAPE, DOPG, and any combination thereof.

In some aspects, the anchoring moiety comprises at least one asymmetric phospholipid. Asymmetric phospholipids may be selected from the non-limiting group consisting of
1-myristoyl-2-palmitoyl-*sn*-glycero-3-phosphocholine (14:0-16:0 PC, MPPC),
1-myristoyl-2-stearoyl-*sn*-glycero-3-phosphocholine (14:0-18:0 PC, MSPC),
1-palmitoyl-2-acetyl-*sn*-glycero-3-phosphocholine (16:0-02:0 PC),
1-palmitoyl-2-myristoyl-*sn*-glycero-3-phosphocholine (16:0-14:0 PC, PMPC),
1-palmitoyl-2-stearoyl-*sn*-glycero-3-phosphocholine (16:0-18:0 PC, PSPC),
1-palmitoyl-2-oleoyl-*sn*-glycero-3-phosphocholine (16:0-18:1 PC, POPC),
1-palmitoyl-2-linoleoyl-*sn*-glycero-3-phosphocholine (16:0-18:2 PC, PLPC),
1-palmitoyl-2-arachidonoyl-*sn*-glycero-3-phosphocholine (16:0-20:4 PC),
1-palmitoyl-2-docosahexaenoyl-*sn*-glycero-3-phosphocholine (14:0-22:6 PC),
1-stearoyl-2-myristoyl-*sn*-glycero-3-phosphocholine (18:0-14:0 PC, SMPC),
1-stearoyl-2-palmitoyl-*sn*-glycero-3-phosphocholine (18:0-16:0 PC, SPPC),
1-stearoyl-2-oleoyl-*sn*-glycero-3-phosphocholine (18:0-18:1 PC, SOPC),
1-stearoyl-2-linoleoyl-*sn*-glycero-3-phosphocholine (18:0-18:2 PC),
1-stearoyl-2-arachidonoyl-*sn*-glycero-3-phosphocholine (18:0-20:4 PC),
1-stearoyl-2-docosahexaenoyl-*sn-*glycero-3-phosphocholine (18:0-22:6 PC),
1-oleoyl-2-myristoyl-*sn*-glycero-3-phosphocholine (18:1-14:0 PC, OMPC),
1-oleoyl-2-palmitoyl-*sn*-glycero-3-phosphocholine (18:1-16:0 PC, OPPC),
1-oleoyl-2-stearoyl-*sn*-glycero-3-phosphocholine (18:1-18:0 PC, OSPC),
1-palmitoyl-2-oleoyl-*sn*-glycero-3-phosphoethanolamine (16:0-18:1 PE, POPE),
1-palmitoyl-2-linoleoyl-*sn*-glycero-3-phosphoethanolamine (16:0-18:2 PE),
1-palmitoyl-2-arachidonoyl-*sn*-glycero-3-phosphoethanolamine (16:0-20:4 PE),
1-palmitoyl-2-docosahexaenoyl-*sn*-glycero-3-phosphoethanolamine (16:0-22:6 PE),
1-stearoyl-2-oleoyl-*sn*-glycero-3-phosphoethanolamine (18:0-18:1 PE),
1-stearoyl-2-linoleoyl-*sn*-glycero-3-phosphoethanolamine (18:0-18:2 PE),
1-stearoyl-2-arachidonoyl-*sn*-glycero-3-phosphoethanolamine (18:0-20:4 PE),
1-stearoyl-2-docosahexaenoyl-*sn*-glycero-3-phosphoethanolamine (18:0-22:6 PE),
1-oleoyl-2-cholesterylhemisuccinoyl-*sn*-glycero-3-phosphocholine (OChemsPC), and
any combination thereof.

To provide more remarkable nuclease resistance, cellular uptake efficiency, and a more remarkable RNA interference effect, phosphatidylethanolamines may be used as anchoring moieties, for example, dimyristoylphosphatidyl ethanolamine, dipalmitoylphosphatidyl ethanolamine, 1-palmitoyl-2-oleyl-phosphatidyl ethanolamine, and dioleoylphosphatidyl ethanolamine.

The binding site of lipid (e.g., a phospholipid) and a linker combination or BAM, e.g., an ASO, may be suitably selected according to the types of lipid and linker or ASO. Any position other than hydrophobic groups of the lipid may be linked to the linker or ASO by a chemical bond. For example, when using a phosphatidylethanolamine, the linkage may be made by forming an amide bond, etc. between the amino group of phosphatidylethanolamine and the linker or ASO. When using a phosphatidylglycerol, the linkage may be made by forming an ester bond, an ether bond, etc. between the hydroxyl group of the glycerol residue and the linker or ASO. When using a phosphatidylserine, the linkage may be made by forming an amide bond or an ester bond, etc. between the amino group or carboxyl group of the serine residue and the linker or ASO. When using a phosphatidic acid, the linkage may be made by forming a phosphoester bond, etc. between the phosphate residue and the linker or ASO. When using a phosphatidylinositol, the linkage may be made by forming an ester bond, an ether bond, etc. between the hydroxyl group of the inositol residue and the linker or ASO.

### III.A.1.d. Lysolipids (e.g., lysophospholipids)

In some aspects, the anchoring moiety comprises a lysolipid, e.g., a lysophospholipid. Lysolipids are derivatives of a lipid in which one or both fatty acyl chains have been removed, generally by hydrolysis. Lysophospholipids are derivatives of a phospholipid in which one or both fatty acyl chains have been removed by hydrolysis.

In some aspects, the anchoring moiety comprises any of the phospholipids disclosed above, in which one or both acyl chains have been removed via hydrolysis, and therefore the resulting lysophospholipid comprises one or no fatty acid acyl chain.

In some aspects, the anchoring moiety comprises a lysoglycerophospholipid, a lysoglycosphingoliopid, a lysophosphatidylcholine, a lysophosphatidylethanolamine, a lysophosphatidylinositol, or a lysophosphatidylserine.

In some aspect, the anchoring moiety comprises a lysolipid selected from the non-limiting group consisting of
1-hexanoyl-2-hydroxy-*sn*-glycero-3-phosphocholine (06:0 Lyso PC),
1-heptanoyl-2-hydroxy-*sn*-glycero-3-phosphocholine (07:0 Lyso PC),
1-octanoyl-2-hydroxy-*sn*-glycero-3-phosphocholine (08:0 Lyso PC),
1-nonanoyl-2-hydroxy-*sn*-glycero-3-phosphocholine (09:0 Lyso PC),
1-decanoyl-2-hydroxy-*sn*-glycero-3-phosphocholine (10:0 Lyso PC),
1-undecanoyl-2-hydroxy-*sn*-glycero-3-phosphocholine (11:0 Lyso PC),
1-lauroyl-2-hydroxy-*sn*-glycero-3-phosphocholine (12:0 Lyso PC),
1-tridecanoyl-2-hydroxy-*sn*-glycero-3-phosphocholine (13:0 Lyso PC),
1-myristoyl-2-hydroxy-*sn*-glycero-3-phosphocholine (14:0 Lyso PC),
1-pentadecanoyl-2-hydroxy-*sn*-glycero-3-phosphocholine (15:0 Lyso PC),
1-palmitoyl-2-hydroxy-*sn*-glycero-3-phosphocholine (16:0 Lyso PC),
1-heptadecanoyl-2-hydroxy-*sn*-glycero-3-phosphocholine (17:0 Lyso PC),
1-stearoyl-2-hydroxy-*sn*-glycero-3-phosphocholine (18:0 Lyso PC),
1-oleoyl-2-hydroxy-*sn*-glycero-3-phosphocholine (18:1 Lyso PC),
1-nonadecanoyl-2-hydroxy-*sn*-glycero-3-phosphocholine (19:0 Lyso PC),
1-arachidoyl-2-hydroxy-*sn*-glycero-3-phosphocholine (20:0 Lyso PC),
1-behenoyl-2-hydroxy-*sn*-glycero-3-phosphocholine (22:0 Lyso PC),
1-lignoceroyl-2-hydroxy-*sn*-glycero-3-phosphocholine (24:0 Lyso PC),
1-hexacosanoyl-2-hydroxy-*sn*-glycero-3-phosphocholine (26:0 Lyso PC),
1-myristoyl-2-hydroxy-*sn*-glycero-3-phosphoethanolamine (14:0 Lyso PE),
1-palmitoyl-2-hydroxy-*sn*-glycero-3-phosphoethanolamine (16:0 Lyso PE),
1-stearoyl-2-hydroxy-*sn*-glycero-3-phosphoethanolamine (18:0 Lyso PE),
1-oleoyl-2-hydroxy-*sn*-glycero-3-phosphoethanolamine (18:1 Lyso PE),
1-hexadecyl-*sn*-glycero-3-phosphocholine (C16 Lyso PC), and
any combination thereof.

### III.A.1.e. Vitamins

In some aspects, the anchoring moiety comprises a lipophilic vitamin, e.g., folic acid, vitamin A, vitamin E, or vitamin K

In some aspects, the anchoring moiety comprises vitamin A. Vitamin A is a group of unsaturated nutritional organic compounds that includes retinol, retinal, retinoic acid, and several provitamin A carotenoids (most notably beta-carotene). In some aspects, the anchoring moiety comprises retinol. In some aspects, the anchoring moiety comprises a retinoid. Retinoids are a class of chemical compounds that are vitamers of vitamin A or are chemically related to it. In some aspects, the anchoring moiety comprises a first generation retinoid (e.g., retinol, tretinoin, isotreatinoin, or alitretinoin), a second-generation retinoid (e.g., etretinate or acitretin), a third-generation retinoid (e.g., adapalene, bexarotene, or tazarotene), or any combination thereof.

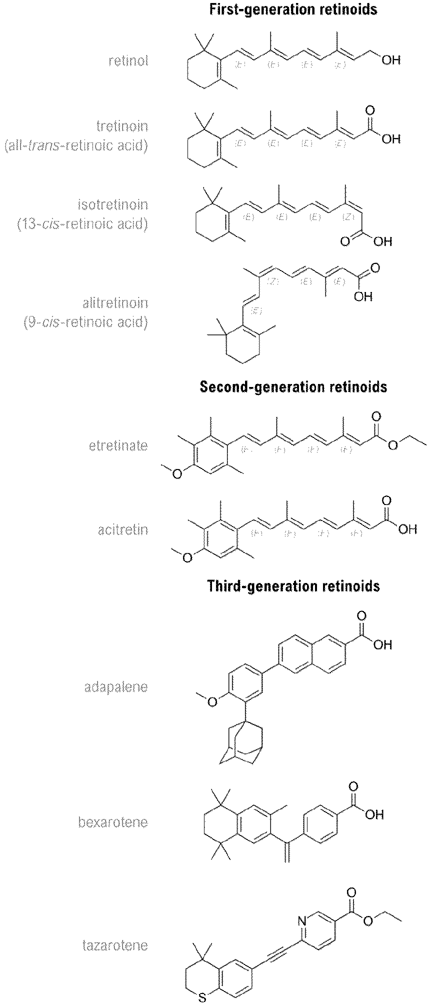

In some aspects, the anchoring moiety comprises vitamin E. Tocopherols are a class of methylated phenols many of which have vitamin E activity. Thus, in some aspects, the anchoring moiety comprises alpha-tocopherol, beta-tocopherol, gamma-tocopherol, delta-tocopherol, or a combination thereof. 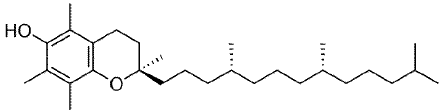 Alpha tocopherol 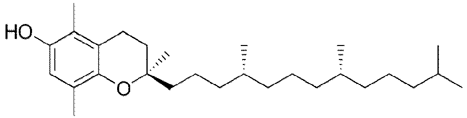 Beta tocopherol 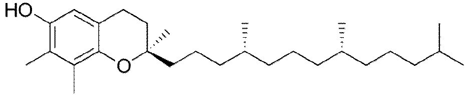 Gamma tocopherol 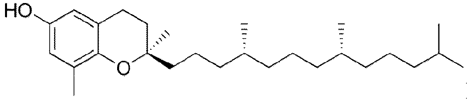 Delta tocopherol

Tocotrienols also have vitamin E activity. The critical chemical structural difference between tocotrienols and tocopherols is that tocotrienols have unsaturated isoprenoid side chain with three carbon-carbon double bonds versus saturated side chains for tocopherols. In some aspects, the anchoring moiety comprises alpha-tocotrienol, beta-tocotrienol, gamma- tocotrienol, delta-tocotrienol, or a combination thereof. Tocotrienols can be represented by the formula below 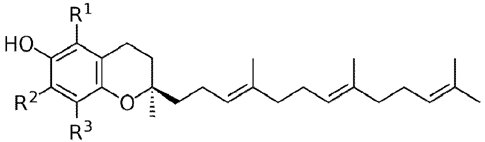
alpha(α)-Tocotrienol: R1 = Me, R2 = Me, R3 = Me;
beta(β)-Tocotrienol: R1 = Me, R2 = H, R3= Me;
gamma(γ)-Tocotrienol: R1 = H, R2 = Me, R3= Me;
delta(δ)-Tocotrienol: R1 = H, R2 = H, R3= Me.

In some aspects, the anchoring moiety comprises vitamin K. Chemically, the vitamin K family comprises 2-methyl-1.4-naphthoquinone (3-) derivatives. Vitamin K includes two natural vitamers: vitamin K₁ and vitamin K₂. The structure of vitamin K₁ (also known as phytonadione, phylloquinone, or (E)-phytonadione) is marked by the presence of a phytyl group. The structures of vitamin K₂ (menaquinones) are marked by the polyisoprenyl side chain present in the molecule that can contain six to 13 isoprenyl units. Thus, vitamin K₂ consists of a number of related chemical subtypes, with differing lengths of carbon side chains made of isoprenoid groups of atoms. MK-4 is the most common form of vitamin K₂. Long chain forms, such as MK-7, MK-8 and MK-9 are predominant in fermented foods. Longer chain forms of vitamin K₂ such as MK-10 to MK-13 are synthesized by bacteria, but they are not well absorbed and have little biological function. In addition to the natural forms of vitamin K, there is a number of synthetic forms of vitamin K such as vitamin K₃ (menadione; 2-methylnaphthalene-1,4-dione), vitamin K₄, and vitamin K₅.

Accordingly, in some aspects, the anchoring moiety comprises vitamin K₁, K₂ (e.g., MK-4, MK-5, MK-6, MK-7, MK-8, MK-9, MK-10, MK-11, MK-12, or MK-13), K₃, K₄, K₅, or any combination thereof.

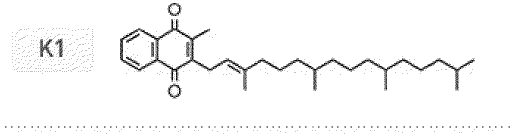

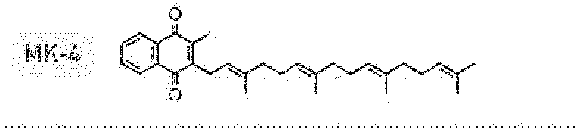

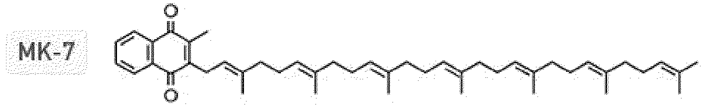

### III.A.2. Linker combinations

In some aspects, an ASO is linked to a hydrophobic membrane anchoring moiety disclosed herein via a linker combination, which can comprise any combination of cleavable and/or non-cleavable linkers. The main function of a linker combination is to provide the optimal spacing between the anchoring moiety or moieties and the BAM target. For example, in the case of an ASO, the linker combination should reduce steric hindrances and position the ASO so it can interact with a target nucleic acid, e.g., a mRNA or a miRNA.

Linkers may be susceptible to cleavage ("cleavable linker") thereby facilitating release of the biologically active molecule. Thus, in some aspects, a linker combination disclosed herein can comprise a cleavable linker. Such cleavable linkers may be susceptible, for example, to acid-induced cleavage, photo-induced cleavage, peptidase-induced cleavage, esterase-induced cleavage, and disulfide bond cleavage, at conditions under which the biologically active molecule remains active. Alternatively, linkers may be substantially resistant to cleavage ("non-cleavable linker"). In some aspects, the cleavable linker comprises a spacer. In some aspects the spacer is PEG.

In some aspects, a linker combination comprises at least 2, at least 3, at least 4, at least 5, or at least 6 or more different linkers disclosed herein. In some aspects, linkers in a linker combination can be linked by an ester linkage (e.g., phosphodiester or phosphorothioate ester).

In some aspects, the linker is direct bond between an anchoring moiety and a BAM, e.g., an ASO.

### III.A.2.a. Non-cleavable linkers

In some aspects, the linker combination comprises a "non-cleavable liker. " Non-cleavable linkers are any chemical moiety capable of linking two or more components of a modified biologically active molecule of the present disclosure (e.g., a biologically active molecule and an anchoring moiety; a biologically active molecule and a cleavable linker; an anchoring moiety and a cleavable linker) in a stable, covalent manner and does not fall off under the categories listed above for cleavable linkers. Thus, non-cleavable linkers are substantially resistant to acid-induced cleavage, photo-induced cleavage, peptidase-induced cleavage, esterase-induced cleavage and disulfide bond cleavage.

Furthermore, non-cleavable refers to the ability of the chemical bond in the linker or adjoining to the linker to withstand cleavage induced by an acid, photolabile-cleaving agent, a peptidase, an esterase, or a chemical or physiological compound that cleaves a disulfide bond, at conditions under which a cyclic dinucleotide and/or the antibody does not lose its activity. In some aspects, the biologically active molecule is attached to the linker via another linker, e.g., a self-immolative linker.

In some aspects, the linker combination comprises a non-cleavable linker comprising, e.g., tetraethylene glycol (TEG), hexaethylene glycol (HEG), polyethylene glycol (PEG), succinimide, or any combination thereof. In some aspects, the non-cleavable linker comprises a spacer unit to link the biologically active molecule to the non-cleavable linker.

In some aspects, one or more non-cleavable linkers comprise smaller units (e.g., HEG, TEG, glycerol, C2 to C12 alkyl, and the like) linked together. In one aspect, the linkage is an ester linkage (e.g., phosphodiester or phosphorothioate ester) or other linkage.

### III.A.2.b. Ethylene Glycols (HEG, TEG, PEG)

In some aspects, the linker combination comprises a non-cleavable linker, wherein the non-cleavable linker comprises a polyethylene glycol (PEG) characterized by a formula R³-(O-CH₂-CH₂)ₙ- or R³-(0-CH₂-CH₂)ₙ-O- with R³ being hydrogen, methyl or ethyl and n having a value from 2 to 200. In some aspects, the linker comprises a spacer, wherein the spacer is PEG.

In some aspects, the PEG linker is an oligo-ethylene glycol, e.g., diethylene glycol, triethylene glycol, tetra ethylene glycol (TEG), pentaethylene glycol, or a hexaethylene glycol (HEG) linker.

In some aspects, n has a value of 2, 3, 4, 5, 6, 7, 8, 9, 10, 11, 12, 13, 14, 15, 16, 17,18,19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 189, 181, 182, 183, 184, 185, 186, 187, 188, 189, 190, 191, 192, 193, 194, 195, 196, 197, 198, 199, or 200.

In some aspects, n is between 2 and 10, between 10 and 20, between 20 and 30, between 30 and 40, between 40 and 50, between 50 and 60, between 60 and 70, between 70 and 80, between 80 and 90, between 90 and 100, between 100 and 110, between 110 and 120, between 120 and 130, between 130 and 140, between 140 and 150, between 150 and 160, between 160 and 170, between 170 and 180, between 180 and 190, or between 190 and 200.

In some specific aspects, n has a value from 3 to 200, from 3 to 20, from 10 to 30, or from 9 to 45.

In some aspects, the PEG is a branched PEG. Branched PEGs have three to ten PEG chains emanating from a central core group.

In certain embodiments, the PEG moiety is a monodisperse polyethylene glycol. In the context of the present disclosure, a monodisperse polyethylene glycol (mdPEG) is a PEG that has a single, defined chain length and molecular weight. mdPEGs are typically generated by separation from the polymerization mixture by chromatography. In certain formulae, a monodisperse PEG moiety is assigned the abbreviation mdPEG.

In some aspects, the PEG is a Star PEG. Star PEGs have 10 to 100 PEG chains emanating from a central core group.

In some aspects, the PEG is a Comb PEGs. Comb PEGs have multiple PEG chains normally grafted onto a polymer backbone.

In certain aspects, the PEG has a molar mass between 100 g/mol and 3000 g/mol, particularly between 100 g/mol and 2500 g/mol, more particularly of approx. 100 g/mol to 2000 g/mol. In certain aspects, the PEG has a molar mass between 200 g/mol and 3000 g/mol, particularly between 300 g/mol and 2500 g/mol, more particularly of approx. 400 g/mol to 2000 g/mol.

In some aspects, the PEG is PEG₁₀₀, PEG₂₀₀, PEG₃₀₀, PEG₄₀₀, PEG₅₀₀, PEG₆₀₀, PEG₇₀₀, PEG₈₀₀, PEG₉₀₀, PEG₁₀₀₀, PEG₁₁₀₀, PEG₁₂₀₀, PEG₁₃₀₀, PEG₁₄₀₀, PEG₁₅₀₀, PEG₁₆₀₀, PEG₁₇₀₀, PEG₁₈₀₀, PEG₁₉₀₀, PEG₂₀₀₀, PEG₂₁₀₀, PEG₂₂₀₀, PEG₂₃₀₀, PEG₂₄₀₀, PEG₂₅₀₀, PEG₁₆₀₀, PEG₁₇₀₀, PEG₁₈₀₀, PEG₁₉₀₀, PEG₂₀₀₀, PEG₂₁₀₀, PEG₂₂₀₀, PEG₂₃₀₀, PEG₂₄₀₀, PEG₂₅₀₀, PEG₂₆₀₀, PEG₂₇₀₀, PEG₂₈₀₀, PEG₂₉₀₀, or PEG₃₀₀₀. In one particular aspect, the PEG is PEG₄₀₀. In another particular aspect, the PEG is PEG2000.

In some aspects, a linker combination of the present disclosure can comprise several PEG linkers, e.g., a cleavable linker flanked by PEG, HEG, or TEG linkers.

In some aspects, the linker combination comprises (HEG)n and/or (TEG)n, wherein n is an integer between 1 and 50, and each unit is connected, e.g., via a phosphate ester linker, a phosphorothioate ester linkage, or a combination thereof.

### III.A.2.c. Glycerol and Polyglycerols (PG)

In some aspects, the linker combination comprises a non-cleavable linker comprising a glycerol unit or a polyglycerol (PG) described by the formula ((R₃-O-(CH₂-CHOH-CH₂O)ₙ-) with R3 being hydrogen, methyl or ethyl, and n having a value from 3 to 200. In some aspects, n has a value from 3 to 20. In some aspects, n has a value from 10 to 30.

In some aspects, the PG linker is a diglycerol, triglycerol, tetraglycerol (TG), pentaglycerol, or a hexaglycerol (HG) linker.

In some aspects, n has a value of 2, 3, 4, 5, 6, 7, 8, 9, 10, 11, 12, 13, 14, 15, 16, 17,18,19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 189, 181, 182, 183, 184, 185, 186, 187, 188, 189, 190, 191, 192, 193, 194, 195, 196, 197, 198, 199, or 200.

In some aspects, n is between 2 and 10, between 10 and 20, between 20 and 30, between 30 and 40, between 40 and 50, between 50 and 60, between 60 and 70, between 70 and 80, between 80 and 90, between 90 and 100, between 100 and 110, between 110 and 120, between 120 and 130, between 130 and 140, between 140 and 150, between 150 and 160, between 160 and 170, between 170 and 180, between 180 and 190, or between 190 and 200.

In some alternatives of these embodiments, n has a value from 9 to 45. In some aspects, the heterologous moiety is a branched polyglycerol described by the formula (R³-O-(CH₂-CHOR⁵-CH₂-O)ₙ-) with R⁵ being hydrogen or a linear glycerol chain described by the formula (R³-O-(CH₂-CHOH-CH₂-O)ₙ-) and R³ being hydrogen, methyl or ethyl. In some aspects, the heterologous moiety is a hyperbranched polyglycerol described by the formula (R³-O-(CH₂-CHOR⁵-CH₂-O)ₙ-) with R⁵ being hydrogen or a glycerol chain described by the formula (R³-O-(CH₂-CHOR⁶-CH₂-O)ₙ-), with R⁶ being hydrogen or a glycerol chain described by the formula (R³-O-(CH₂-CHOR⁷-CH₂-O)ₙ-), with R⁷ being hydrogen or a linear glycerol chain described by the formula (R³-O-(CH₂-CHOH-CH₂-O)ₙ-) and R³ being hydrogen, methyl or ethyl. Hyperbranched glycerol and methods for its synthesis are described in Oudshorn et al. (2006) Biomaterials 27:5471-5479; Wilms et al. (20100 Acc. Chem. Res. 43, 129-41, and references cited therein.

In certain aspects, the PG has a molar mass between 100 g/mol and 3000 g/mol, particularly between 100 g/mol and 2500 g/mol, more particularly of approx. 100 g/mol to 2000 g/mol. In certain aspects, the PG has a molar mass between 200 g/mol and 3000 g/mol, particularly between 300 g/mol and 2500 g/mol, more particularly of approx. 400 g/mol to 2000 g/mol.

In some aspects, the PG is PG₁₀₀, PG₂₀₀, PG₃₀₀, PG₄₀₀, PG₅₀₀, PG₆₀₀, PG₇₀₀, PG₈₀₀, PG₉₀₀, PG₁₀₀₀, PG₁₁₀₀, PG₁₂₀₀, PG₁₃₀₀, PG₁₄₀₀, PG₁₅₀₀, PG₁₆₀₀, PG₁₇₀₀, PG₁₈₀₀, PG₁₉₀₀, PG₂₀₀₀, PG₂₁₀₀, PG₂₂₀₀, PG₂₃₀₀, PG₂₄₀₀, PG₂₅₀₀, PG₁₆₀₀, PG₁₇₀₀, PG₁₈₀₀, PG₁₉₀₀, PG₂₀₀₀, PG₂₁₀₀, PG₂₂₀₀, PG₂₃₀₀, PG₂₄₀₀, PG₂₅₀₀, PG₂₆₀₀, PG₂₇₀₀, PG₂₈₀₀, PG₂₉₀₀, ₒᵣ PG₃₀₀₀. In one particular aspect, the PG is PG₄₀₀. In another particular aspect, the PG is PG₂₀₀₀.

In some aspects, the linker combination comprises (glycerol)n, and/or (HG)n and/or (TG)n, wherein n is an integer between 1 and 50, and each unit is connected, e.g., via a phosphate ester linker, a phosphorothioate ester linkage, or a combination thereof.

### III.A.2.d. Aliphatic (Alkyl) linkers

In some aspects, the linker combination comprises at least one aliphatic (alkyl) linker, e.g., propyl, butyl, hexyl , or C2-C12 alkyl, such as C2-C10 alkyl or C2-C6 alkyl.

In some aspects, the linker combination comprises an alkyl chain, e.g., an unsubstituted alkyl. In some aspects, the linker combination comprises an substituted or unsubstituted alkenyl, substituted or unsubstituted alkynyl, arylalkyl, arylalkenyl, arylalkynyl, heteroarylalkyl, heteroarylalkenyl, heteroarylalkynyl, heterocyclylalkyl, heterocyclylalkenyl, heterocyclylalkynyl, Aryl, heteroaryl, heterocyclyl, cycloalkyl, cycloalkenyl, alkylarylalkyl, alkylarylalkenyl, alkylarylalkynyl, alkenylarylalkyl, alkenyl Reyl alkenyl, alkenyl aryl alkynyl, alkynyl aryl alkyl, alkynyl aryl alkenyl, alkynyl aryl alkynyl, alkyl heteroaryl alkyl, alkyl heteroaryl alkyl, alkyl heteroaryl alkenyl, alkyl heteroaryl alkynyl, alkenyl heteroaryl alkyl, alkenyl heteroaryl alkenyl, alkenyl heteroaryl alkynyl, alkynyl Heteroarylalkyl, alkynylheteroarylalkenyl, alkynylheteroarylalkynyl, alkylheterocyclylalkyl, alkylheterocyclylalkenyl, alkylheterocyclylalkynyl, alkenylheterocyclylalkyl, alkenylheterocyclylalkenyl, or alkenylheterocyclylalkynyl.

Optionally these components are substituted. Substituents include alcohol, alkoxy (such as methoxy, ethoxy, and propoxy), straight or branched chain alkyl (such as C1-C12 alkyl), amine, aminoalkyl (such as amino C1-C12 alkyl), phosphoramidite, phosphate, phosphoramidate, phosphorodithioate, thiophosphate, hydrazide, hydrazine, halogen, (such as F, Cl, Br, or I), amide, alkylamide (such as amide C1-C12 alkyl), carboxylic acid, carboxylic ester, carboxylic anhydride, carboxylic acid halide, ether, sulfonyl halide, imidate ester, isocyanate, isothiocyanate, haloformate, carboduimide adduct, aldehydes, ketone, sulfhydryl, haloacetyl, alkyl halide, alkyl sulfonate, C(=O)CH=CHC(=O) (maleimide), thioether, cyano, sugar (such as mannose, galactose, and glucose), α,β-unsaturated carbonyl, alkyl mercurial, or α,β-unsaturated sulfone.

The term "alkyl," by itself or as part of another substituent, means, unless otherwise stated, a straight or branched chain hydrocarbon radical having the number of carbon atoms designated (*e.g*., C₁-C₁₀ means one to ten carbon atoms). Typically, an alkyl group will have from 1 to 24 carbon atoms, for example having from 1 to 10 carbon atoms, from 1 to 8 carbon atoms or from 1 to 6 carbon atoms. A "lower alkyl" group is an alkyl group having from 1 to 4 carbon atoms. The term "alkyl" includes di- and multivalent radicals. For example, the term "alkyl" includes "alkylene" wherever appropriate, *e.g*., when the formula indicates that the alkyl group is divalent or when substituents are joined to form a ring. Examples of alkyl radicals include, but are not limited to, methyl, ethyl, *n*-propyl, *iso*-propyl, *n*-butyl, *tert*-butyl, *iso*-butyl, *sec*-butyl, as well as homologs and isomers of, for example, *n*-pentyl, *n*-hexyl, *n*-heptyl and *n*-octyl.

The term "alkylene" by itself or as part of another substituent means a divalent (diradical) alkyl group, wherein alkyl is defined herein. "Alkylene" is exemplified, but not limited, by -CH₂CH₂CH₂CH₂-. Typically, an "alkylene" group will have from 1 to 24 carbon atoms, for example, having 10 or fewer carbon atoms (*e.g.,* 1 to 8 or 1 to 6 carbon atoms). A "lower alkylene" group is an alkylene group having from 1 to 4 carbon atoms.

The term "alkenyl" by itself or as part of another substituent refers to a straight or branched chain hydrocarbon radical having from 2 to 24 carbon atoms and at least one double bond. A typical alkenyl group has from 2 to 10 carbon atoms and at least one double bond. In one embodiment, alkenyl groups have from 2 to 8 carbon atoms or from 2 to 6 carbon atoms and from 1 to 3 double bonds. Exemplary alkenyl groups include vinyl, 2-propenyl, 1-but-3-enyl, crotyl, 2-(butadienyl), 2,4-pentadienyl, 3-(1,4-pentadienyl), 2-isopentenyl, 1-pent-3-enyl, 1-hex-5-enyl and the like.

The term "alkynyl" by itself or as part of another substituent refers to a straight or branched chain, unsaturated or polyunsaturated hydrocarbon radical having from 2 to 24 carbon atoms and at least one triple bond. A typical "alkynyl" group has from 2 to 10 carbon atoms and at least one triple bond. In one aspect of the disclosure, alkynyl groups have from 2 to 6 carbon atoms and at least one triple bond. Exemplary alkynyl groups include prop-1-ynyl, prop-2-ynyl (*i.e*., propargyl), ethynyl and 3-butynyl.

The terms "alkoxy," "alkylamino" and "alkylthio" (or thioalkoxy) are used in their conventional sense, and refer to alkyl groups that are attached to the remainder of the molecule via an oxygen atom, an amino group, or a sulfur atom, respectively.

The term "heteroalkyl," by itself or in combination with another term, means a stable, straight or branched chain hydrocarbon radical consisting of the stated number of carbon atoms (*e.g*., C₂-C₁₀, or C₂-C₈) and at least one heteroatom chosen , *e.g.,* from N, O, S, Si, B and P (in one embodiment, N, O and S), wherein the nitrogen, sulfur and phosphorus atoms are optionally oxidized, and the nitrogen atom(s) are optionally quaternized. The heteroatom(s) is/are placed at any interior position of the heteroalkyl group. Examples of heteroalkyl groups include, but are not limited to, -CH₂-CH₂-O-CH₃, -CH₂-CH₂-NH-CH₃, -CH₂-CH₂-N(CH₃)-CH₃, -CH₂-S-CH₂-CH₃, - CH₂-CH₂-S(O)-CH₃, -CH₂-CH₂-S(O)₂-CH₃, -CH=CH-O-CH₃, -CH₂-Si(CH₃)₃, -CH₂-CH=N-OCH₃, and -CH=CH-N(CH₃)-CH₃. Up to two heteroatoms can be consecutive, such as, for example, -CH₂-NH-OCH₃ and -CH₂-O-Si(CH₃)₃.

Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. Typically, a heteroalkyl group will have from 3 to 24 atoms (carbon and heteroatoms, excluding hydrogen) (3- to 24-membered heteroalkyl). In another example, the heteroalkyl group has a total of 3 to 10 atoms (3- to 10-membered heteroalkyl) or from 3 to 8 atoms (3- to 8-membered heteroalkyl). The term "heteroalkyl" includes "heteroalkylene" wherever appropriate, *e.g*., when the formula indicates that the heteroalkyl group is divalent or when substituents are joined to form a ring.

The term "cycloalkyl" by itself or in combination with other terms, represents a saturated or unsaturated, non-aromatic carbocyclic radical having from 3 to 24 carbon atoms, for example, having from 3 to 12 carbon atoms (*e.g*., C₃-C₈ cycloalkyl or C₃-C₆ cycloalkyl). Examples of cycloalkyl include, but are not limited to, cyclopropyl, cyclobutyl, cyclopentyl, cyclohexyl, cycloheptyl, 1-cyclohexenyl, 3-cyclohexenyl, cycloheptyl and the like. The term "cycloalkyl" also includes bridged, polycyclic (*e.g*., bicyclic) structures, such as norbornyl, adamantyl and bicyclo[2.2.1]heptyl. The "cycloalkyl" group can be fused to at least one (*e.g.,* 1 to 3) other ring selected from aryl (*e.g.,* phenyl), heteroaryl (*e.g.,* pyridyl) and non-aromatic (*e.g.,* carbocyclic or heterocyclic) rings. When the "cycloalkyl" group includes a fused aryl, heteroaryl or heterocyclic ring, then the "cycloalkyl" group is attached to the remainder of the molecule via the carbocyclic ring.

The term "heterocycloalkyl," "heterocyclic," "heterocycle," or "heterocyclyl," by itself or in combination with other terms, represents a carbocyclic, non-aromatic ring (*e.g.,* 3- to 8-membered ring and for example, 4-, 5-, 6- or 7-membered ring) containing at least one and up to 5 heteroatoms selected from, *e.g.,* N, O, S, Si, B and P (for example, N, O and S), wherein the nitrogen, sulfur and phosphorus atoms are optionally oxidized, and the nitrogen atom(s) are optionally quaternized (*e.g*., from 1 to 4 heteroatoms selected from nitrogen, oxygen and sulfur), or a fused ring system of 4- to 8-membered rings, containing at least one and up to 10 heteroatoms (*e.g*., from 1 to 5 heteroatoms selected from N, O and S) in stable combinations known to those of skill in the art. Exemplary heterocycloalkyl groups include a fused phenyl ring. When the "heterocyclic" group includes a fused aryl, heteroaryl or cycloalkyl ring, then the "heterocyclic" group is attached to the remainder of the molecule via a heterocycle. A heteroatom can occupy the position at which the heterocycle is attached to the remainder of the molecule.

Exemplary heterocycloalkyl or heterocyclic groups of the present disclosure include morpholinyl, thiomorpholinyl, thiomorpholinyl S-oxide, thiomorpholinyl S,S-dioxide, piperazinyl, homopiperazinyl, pyrrolidinyl, pyrrolinyl, imidazolidinyl, tetrahydropyranyl, piperidinyl, tetrahydrofuranyl, tetrahydrothienyl, piperidinyl, homopiperidinyl, homomorpholinyl, homothiomorpholinyl, homothiomorpholinyl S,S-dioxide, oxazolidinonyl, dihydropyrazolyl, dihydropyrrolyl, dihydropyrazolyl, dihydropyridyl, dihydropyrimidinyl, dihydrofuryl, dihydropyranyl, tetrahydrothienyl S-oxide, tetrahydrothienyl S,S-dioxide, homothiomorpholinyl S-oxide, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

By "aryl" is meant a 5-, 6- or 7-membered, aromatic carbocyclic group having a single ring (*e.g.,* phenyl) or being fused to other aromatic or non-aromatic rings (*e.g.,* from 1 to 3 other rings). When the "aryl" group includes a non-aromatic ring (such as in 1,2,3,4-tetrahydronaphthyl) or heteroaryl group then the "aryl" group is bonded to the remainder of the molecule via an aryl ring (*e.g.,* a phenyl ring). The aryl group is optionally substituted (*e.g.,* with 1 to 5 substituents described herein). In one example, the aryl group has from 6 to 10 carbon atoms. Non-limiting examples of aryl groups include phenyl, 1-naphthyl, 2-naphthyl, quinoline, indanyl, indenyl, dihydronaphthyl, fluorenyl, tetralinyl, benzo[d][1,3]dioxolyl or 6,7,8,9-tetrahydro-5H-benzo[a]cycloheptenyl. In one embodiment, the aryl group is selected from phenyl, benzo[d][1,3]dioxolyl and naphthyl. The aryl group, in yet another embodiment, is phenyl.

The term "arylalkyl" or "aralkyl" is meant to include those radicals in which an aryl group or heteroaryl group is attached to an alkyl group to create the radicals -alkyl-aryl and -alkyl-heteroaryl, wherein alkyl, aryl and heteroaryl are defined herein. Exemplary "arylalkyl" or "aralkyl" groups include benzyl, phenethyl, pyridylmethyl and the like.

By "aryloxy" is meant the group -O-aryl, where aryl is as defined herein. In one example, the aryl portion of the aryloxy group is phenyl or naphthyl. The aryl portion of the aryloxy group, in one embodiment, is phenyl.

The term "heteroaryl" or "heteroaromatic" refers to a polyunsaturated, 5-, 6- or 7-membered aromatic moiety containing at least one heteroatom (*e.g*., 1 to 5 heteroatoms, such as 1-3 heteroatoms) selected from N, O, S, Si and B (for example, N, O and S), wherein the nitrogen and sulfur atoms are optionally oxidized, and the nitrogen atom(s) are optionally quaternized. The "heteroaryl" group can be a single ring or be fused to other aryl, heteroaryl, cycloalkyl or heterocycloalkyl rings (*e.g*., from 1 to 3 other rings). When the "heteroaryl" group includes a fused aryl, cycloalkyl or heterocycloalkyl ring, then the "heteroaryl" group is attached to the remainder of the molecule via the heteroaryl ring. A heteroaryl group can be attached to the remainder of the molecule through a carbon- or heteroatom.

In one example, the heteroaryl group has from 4 to 10 carbon atoms and from 1 to 5 heteroatoms selected from O, S and N. Non-limiting examples of heteroaryl groups include pyridyl, pyrimidinyl, quinolinyl, benzothienyl, indolyl, indolinyl, pyridazinyl, pyrazinyl, isoindolyl, isoquinolyl, quinazolinyl, quinoxalinyl, phthalazinyl, imidazolyl, isoxazolyl, pyrazolyl, oxazolyl, thiazolyl, indolizinyl, indazolyl, benzothiazolyl, benzimidazolyl, benzofuranyl, furanyl, thienyl, pyrrolyl, oxadiazolyl, thiadiazolyl, triazolyl, tetrazolyl, isothiazolyl, naphthyridinyl, isochromanyl, chromanyl, tetrahydroisoquinolinyl, isoindolinyl, isobenzotetrahydrofuranyl, isobenzotetrahydrothienyl, isobenzothienyl, benzoxazolyl, pyridopyridyl, benzotetrahydrofuranyl, benzotetrahydrothienyl, purinyl, benzodioxolyl, triazinyl, pteridinyl, benzothiazolyl, imidazopyridyl, imidazothiazolyl, dihydrobenzisoxazinyl, benzisoxazinyl, benzoxazinyl, dihydrobenzisothiazinyl, benzopyranyl, benzothiopyranyl, chromonyl, chromanonyl, pyridyl-N-oxide, tetrahydroquinolinyl, dihydroquinolinyl, dihydroquinolinonyl, dihydroisoquinolinonyl, dihydrocoumarinyl, dihydroisocoumarinyl, isoindolinonyl, benzodioxanyl, benzoxazolinonyl, pyrrolyl N-oxide, pyrimidinyl N-oxide, pyridazinyl N-oxide, pyrazinyl N-oxide, quinolinyl N-oxide, indolyl N-oxide, indolinyl N-oxide, isoquinolyl N-oxide, quinazolinyl N-oxide, quinoxalinyl N-oxide, phthalazinyl N-oxide, imidazolyl N-oxide, isoxazolyl N-oxide, oxazolyl N-oxide, thiazolyl N-oxide, indolizinyl N-oxide, indazolyl N-oxide, benzothiazolyl N-oxide, benzimidazolyl N-oxide, pyrrolyl N-oxide, oxadiazolyl N-oxide, thiadiazolyl N-oxide, triazolyl N-oxide, tetrazolyl N-oxide, benzothiopyranyl S-oxide, benzothiopyranyl S,S-dioxide. Exemplary heteroaryl groups include imidazolyl, pyrazolyl, thiadiazolyl, triazolyl, isoxazolyl, isothiazolyl, imidazolyl, thiazolyl, oxadiazolyl, and pyridyl. Other exemplary heteroaryl groups include 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, pyridin-4-yl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable aryl group substituents described below.

Examples of aliphatic linkers include the following structures:

-O-CO-O-

-NH-CO-O-

-NH-CO-NH-

-NH-(CH₂)ₙ₁-

-S-(CH₂)ₙ₁-

-CO-(CH₂)ₙ₁-CO-

-CO-(CH₂)ₙ₁-NH-

-NH-(CH₂)ₙ₁-NH-

-CO-NH-(CH₂)ₙ₁-NH-CO-

-C(=S)-NH-(CH₂)ₙ₁-NH-CO-

-C(=S)-NH-(CH₂)ₙ₁-NH-C-(=S)-

-CO-O-(CH₂)ₙ₁-O-CO-

-C(=S)-O-(CH₂)ₙ₁-O-CO-

-C(=S)-O-(CH₂)ₙ₁-O-C-(=S)-

-CO-NH-(CH₂)ₙ₁-O-CO-

-C(=S)-NH-(CH₂)ₙ₁-O-CO-

-C(=S)-NH-(CH₂)ₙ₁-O-C-(=S)-

-CO-NH-(CH₂)ₙ₁-O-CO-

-C(=S)-NH-(CH₂)ₙ₁-CO-

-C(=S)-O-(CH₂)ₙ₁-NH-CO-

-C(=S)-NH-(CH₂)ₙ₁-O-C-(=S)-

-NH-(CH₂CH₂O)ₙ₂-CH(CH₂OH)-

-NH-(CH₂CH₂O)ₙ₂-CH₂-

-NH-(CH₂CH₂O)ₙ₂-CH₂-CO-

-O-(CH₂)ₙ₃-S-S-(CH₂)ₙ₄-O-P(=O)₂-

-CO-(CH₂)ₙ₃-O-CO-NH-(CH₂)ₙ₄-

-CO-(CH₂)ₙ₃-CO-NH-(CH₂)ₙ₄-

- (CH2)ₙ₁NH-

-C(O)(CH2)ₙ₁NH-

-C(O)-(CH2)ₙ₁-C(O)-

-C(O)-(CH2)ₙ₁-C(O)O-

-C(O)-O-

-C(O)-(CH2)ₙ₁-NH-C(O)-

-C(O)-(CH2)ₙ₁-

-C(O)-NH-

-C(O)-

- (CH2)ₙ₁-C(O)-

- (CH2)ₙ₁-C(O)O-

- (CH2)ₙ₁-

- (CH2)ₙ₁-NH-C(O)-

n1 is an integer between 1 and 40 (e.g., 2 to 20, or 2 to 12); n2 is an integer between 1 and 20 (e.g., 1 to 10, or 1 to 6); n3 and n4 may be the same or different, and are an integer between 1 and 20 (e.g., 1 to 10, or 1 to 6).

In some aspects, the linker combination comprises (C3)n, (C4)n, (C5)n, (C6)n, (C7)n, or (C8)n, or a combination thereof, wherein n is an integer between 1 and 50, and each unit is connected, e.g., via a phosphate ester linker, a phosphorothioate ester linkage, or a combination thereof.

### III.A.3. Cleavable linkers

In some aspects, different components of an ASO disclosed herein can be linker by a cleavable linker. The term cleavable linker refers to a linker comprising at least one linkage or chemical bond that can be broken or cleaved. As used herein, the term cleave refers to the breaking of one or more chemical bonds in a relatively large molecule in a manner that produces two or more relatively smaller molecules. Cleavage may be mediated, e.g., by a nuclease, peptidase, protease, phosphatase, oxidase, or reductase, for example, or by specific physicochemical conditions, e.g., redox environment, pH, presence of reactive oxygen species, or specific wavelengths of light.

In some aspects, the term "cleavable," as used herein, refers, e.g., to rapidly degradable linkers, such as, e.g., phosphodiester and disulfides, while the term "non-cleavable" refers, e.g., to more stable linkages, such as, e.g., nuclease-resistant phosphorothioates.

In some aspects, the cleavable linker is a dinucleotide or trinucleotide linker, a disulfide, an imine, a thioketal, a val-cit dipeptide, or any combination thereof.

In some aspects, the cleavable linker comprises valine-alanine-p-aminobenzylcarbamate or valine-citrulline-p-aminobenzylcarbamate.

### III.A.3.a. Redox cleavable linkers

In some aspects, the linker combination comprises a redox cleavable linker. As a non-limiting example, one type of cleavable linker is a redox cleavable linking group that is cleaved upon reduction or upon oxidation.

In some aspects, the redox cleavable linker contains a disulfide bond, i.e., it is a disulfide cleavable linker.

Redox cleavable linkers can be reduced, e.g., by intracellular mercaptans, oxidases, or reductases.

### III.A.3.b. Reactive Oxygen Species (ROS) cleavable linkers

In some aspects, the linker combination can comprise a cleavable linker which may be cleaved by a reactive oxygen species (ROS), such as superoxide (Of) or hydrogen peroxide (H2O2), generated, e.g., by inflammation processes such as activated neutrophils. In some aspects, the ROS cleavable linker is a thioketal cleavable linker. See, e.g., U.S. Pat. 8,354,455B2.

### III.A.3.c. pH dependent cleavable linkers

In some aspects, the linker is an "acid labile linker" comprising an acid cleavable linking group, which is a linking group that is selectively cleaved under acidic conditions (pH<7).

As a non-limiting example, the acid cleavable linking group is cleaved in an acidic environment, e.g., about 6.0, 5.5, 5.0 or less. In some aspects, the pH is about 6.5 or less. In some aspects, the linker is cleaved by an agent such as an enzyme that can act as a general acid, e.g., a peptidase (which may be substrate specific) or a phosphatase. Within cells, certain low pH organelles, such as endosomes and lysosomes, can provide a cleaving environment to the acid cleavable linking group. Although the pH of human serum is 7.4, the average pH in cells is slightly lower, ranging from about 7.1 to 7.3. Endosomes also have an acidic pH, ranging from 5.5 to 6.0, and lysosomes are about 5.0 at an even more acidic pH. Accordingly, pH dependent cleavable linkers are sometimes called endosomically labile linkers in the art.

The acid cleavable group may have the general formula -C = NN-, C (O) O, or -OC (O). In another non-limiting example, when the carbon attached to the ester oxygen (alkoxy group) is attached to an aryl group, a substituted alkyl group, or a tertiary alkyl group such as dimethyl pentyl or t-butyl, for example. Examples of acid cleavable linking groups include, but are not limited to amine, imine, amino ester, benzoic imine, diortho ester, polyphosphoester, polyphosphazene, acetal, vinyl ether, hydrazone, cis-aconitate, hydrazide, thiocarbamoyl, imizine, azidomethyl-methylmaleic anhydride, thiopropionate, a masked endosomolytic agent, a citraconyl group, or any combination thereof. Disulfide linkages are also susceptible to pH.

In some aspects, the linker comprises a low pH-labile hydrazone bond. Such acid-labile bonds have been extensively used in the field of conjugates, e.g., antibody-drug conjugates. See, for example, Zhou et al, Biomacromolecules 2011, 12, 1460-7; Yuan et al, Acta Biomater. 2008, 4, 1024-37; Zhang et al, Acta Biomater. 2007, 6, 838-50; Yang et al, J. Pharmacol. Exp. Ther. 2007, 321, 462-8; Reddy et al, Cancer Chemother. Pharmacol. 2006, 58, 229-36; Doronina et al, Nature Biotechnol. 2003, 21, 778-84.

In certain embodiments, the linker comprises a low pH-labile bond selected from the following: ketals that are labile in acidic environments (e.g., pH less than 7, greater than about 4) to form a diol and a ketone; acetals that are labile in acidic environments (e.g., pH less than 7, greater than about 4) to form a diol and an aldehyde; imines or iminiums that are labile in acidic environments (e.g., pH less than 7, greater than about 4) to form an amine and an aldehyde or a ketone; silicon-oxygen-carbon linkages that are labile under acidic condition; silicon-nitrogne (silazane) linkages; silicon-carbon linkages (e.g., arylsilanes, vinylsilanes, and allylsilanes); maleamates (amide bonds synthesized from maleic anhydride derivatives and amines); ortho esters; hydrazones; activated carboxylic acid derivatives (e.g., esters, amides) designed to undergo acid catalyzed hydrolysis); or vinyl ethers.

Further examples may be found in U.S. Pat. Nos. 9,790,494B2 and 8,137,695B2.

### III.A.3.d. Enzymatic cleavable linkers

In some aspects, the linker combination can comprise a linker cleavable by intracellular or extracellular enzymes, e.g., proteases, esterases, nucleases, amidades. The range of enzymes that can cleave a specific linker in a linker combination depends on the specific bonds and chemical structure of the linker. Accordingly, peptidic linkers can be cleaved, e.g., by peptidades, linkers containing ester linkages can be cleaved, e.g., by esterases; linkers containing amide linkages can be cleaved, e.g., by amidades; etc.

### III.A.3.e. Protease cleavable linkers

In some aspects, the linker combination comprises a protease cleavable linker, i.e., a linker that can be cleaved by an endogenous protease. Only certain peptides are readily cleaved inside or outside cells. See, e.g., Trout et al., 79 Proc. Natl. Acad. Sci. USA, 626-629 (1982) and Umemoto et al. 43 Int. J. Cancer, 677-684 (1989). Cleavable linkers can contain cleavable sites composed of α-amino acid units and peptidic bonds, which chemically are amide bonds between the carboxylate of one amino acid and the amino group of a second amino acid. Other amide bonds, such as the bond between a carboxylate and the α-amino acid group of lysine, are understood not to be peptidic bonds and are considered non-cleavable.

In some aspects, the protease-cleavable linker comprises a cleavage site for a protease, *e.g*., neprilysin (CALLA or CDlO), thimet oligopeptidase (TOP), leukotriene A4 hydrolase, endothelin converting enzymes, ste24 protease, neurolysin, mitochondrial intermediate peptidase, interstitial collagenases, collagenases, stromelysins, macrophage elastase, matrilysin, gelatinases, meprins, procollagen C- endopeptidases, procollagen N-endopeptidases, ADAMs and ADAMTs metalloproteinases, myelin associated metalloproteinases, enamelysin, tumor necrosis factor α-converting enzyme, insulysin, nardilysin, mitochondrial processing peptidase, magnolysin, dactylysin-like metalloproteases, neutrophil collagenase, matrix metallopeptidases, membrane-type matrix metalloproteinases, SP2 endopeptidase, prostate specific antigen (PSA), plasmin, urokinase, human fibroblast activation protein (FAPα), trypsin, chymotrypsins, caldecrin, pancreatic elastases, pancreatic endopeptidase, enteropeptidase, leukocyte elastase, myeloblasts, chymases, tryptase, granzyme, stratum corneum chymotryptic enzyme, acrosin, kallikreins, complement components and factors, alternative-complement pathway c3/c5 convertase, mannose- binding protein-associated serine protease, coagulation factors, thrombin, protein c, u and t-type plasminogen activator, cathepsin G, hepsin, prostasin, hepatocyte growth factor-activating endopeptidase, subtilisin/kexin type proprotein convertases, furin, proprotein convertases, prolyl peptidases, acylaminoacyl peptidase, peptidyl-glycaminase, signal peptidase, n-terminal nucleophile aminohydrolases, 20s proteasome, γ-glutamyl transpeptidase, mitochondrial endopeptidase, mitochondrial endopeptidase Ia, htra2 peptidase, matriptase, site 1 protease, legumain, cathepsins, cysteine cathepsins, calpains, ubiquitin isopeptidase T, caspases, glycosylphosphatidylinositoliprotein transamidase, cancer procoagulant, prohormone thiol protease, γ-Glutamyl hydrolase, bleomycin hydrolase, seprase, cathepsin B, cathepsin D, cathepsin L, cathepsin M, proteinase K, pepsins, chymosyn, gastricsin, renin, yapsin and/or mapsins, Prostate-Specific antigen (PSA), or any Asp-N, Glu-C, Lys-C or Arg-C proteases in general. See, e.g., Cancer Res. 77(24):7027-7037 (2017).

In some aspects, the cleavable linker component comprises a peptide comprising one to ten amino acid residues. In these aspects, the peptide allows for cleavage of the linker by a protease, thereby facilitating release of the biologically active molecule upon exposure to intracellular proteases, such as lysosomal enzymes (Doronina et al. (2003) Nat. Biotechnol. 21:778-784). Exemplary peptides include, but are not limited to, dipeptides, tripeptides, tetrapeptides, pentapeptides, and hexapeptides.

A peptide may comprise naturally-occurring and/or non-natural amino acid residues. The term "naturally-occurring amino acid" refer to Ala, Asp, Cys, Glu, Phe, Gly, His, He, Lys, Leu, Met, Asn, Pro, Gin, Arg, Ser, Thr, Val, Trp, and Tyr. "Non-natural amino acids" (i.e., amino acids do not occur naturally) include, by way of non-limiting example, homoserine, homoarginine, citrulline, phenylglycine, taurine, iodotyrosine, seleno- cysteine, norleucine ("Nle"), norvaline ("Nva"), beta-alanine, L- or D-naphthalanine, ornithine ("Om"), and the like. Peptides can be designed and optimized for enzymatic cleavage by a particular enzyme, for example, a tumor-associated protease, cathepsin B, C and D, or a plasmin protease.

Amino acids also include the D-forms of natural and non-natural amino acids. "D-" designates an amino acid having the "D" (dextrorotary) configuration, as opposed to the configuration in the naturally occurring ("L-") amino acids. Natural and non-natural amino acids can be purchased commercially (Sigma Chemical Co., Advanced Chemtech) or synthesized using methods known in the art.

Exemplary dipeptides include, but are not limited to, valine-alanine, valine-citrulline, phenylalanine-lysine, N-methyl-valine-citrulline, cyclohexylalanine-lysine, and beta-alanine-lysine. Exemplary tripeptides include, but are not limited to, glycine-valine-citrulline (gly-val-cit) and glycine-glycine-glycine (gly-gly-gly).

### III.A.3.f. Esterase cleavable linkers

Some linkers are cleaved by esterases ("esterase cleavable linkers"). Only certain esters can be cleaved by esterases and amidases present inside or outside of cells. Esters are formed by the condensation of a carboxylic acid and an alcohol. Simple esters are esters produced with simple alcohols, such as aliphatic alcohols, and small cyclic and small aromatic alcohols. Examples of ester-based cleavable linking groups include, but are not limited to, esters of alkylene, alkenylene and alkynylene groups. The ester cleavable linking group has the general formula -C (O) O- or -OC (O)-.

### III.A.3.g. Phosphatase cleavable linkers

In some aspects, a linker combination can include a phosphate-based cleavable linking group is cleaved by an agent that degrades or hydrolyzes phosphate groups. An example of an agent that cleaves intracellular phosphate groups is an enzyme such as intracellular phosphatase. Examples of phosphate-based linking groups are -O-P (O) (OR k) -O-, -O-P (S) (ORₖ) -O-, -O-P (S) (SRₖ) - O-, -S-P (O) (ORₖ) -O-, -O-P (O) (ORₖ) -S-, -S-P (O) (ORₖ) -S-, -OP ( S) (ORₖ) -S-, -SP (S) (ORₖ) -O-, -OP (O) (Rₖ) -O-, -OP (S) (Rₖ) -O- , -SP (O) (Rₖ) -O-, -SP (S) (Rₖ) -O-, -SP (O) (Rₖ) -S-, or -OP (S) (Rₖ) -S-.

In various aspects, Rₖ is any of the following: NH₂ , BH₃ , CH₃ , C₁₋₆ alkyl, C₆₋₁₀ aryl, C₁₋₆ alkoxy and C₆₋₁₀ aryl-oxy. In some aspects, C₁₋₆ alkyl and C₆₋₁₀ aryl are unsubstituted. Further non-limiting examples are -O-P (O) (OH) -O-, -O-P (S) (OH) -O-, -O-P (S) (SH) -O-, -S-P (O) (OH) -O-, -O-P (O) (OH) -S-, -S-P (O) (OH) -S-, -O-P (S) ( OH) -S-, -S-P (S) (OH) -O-, - O-P (O) (H) -O-, -O-P (S) (H) -O-, -S -P (O) (H) -O-, -SP (S) (H) -O-, -SP (O) (H) -S-, -OP (S) (H)-S-, or -O-P (O) (OH) -O-.

### III.A.3.h. Photoactivated cleavable linkers

In some aspects, the combination linker comprises a photoactivated cleavable linker, e.g., a nitrobenzyl linker or a linker comprising a nitrobenzyl reactive group.

### III.A.3.i. Self-immolative linker

In some aspects, the linker combination comprises a self-immolative linker In some aspects, the self-immolative linker in the EV (*e.g*., exosome) of the present disclosure undergoes 1,4 elimination after the enzymatic cleavage of the protease-cleavable linker. In some aspects, the self-immolative linker in the EV (*e.g*., exosome) of the present disclosure undergoes 1,6 elimination after the enzymatic cleavage of the protease-cleavable linker. In some aspects, the self-immolative linker is, e.g., a p-aminobenzyl (pAB) derivative, such as a p-aminobenzyl carbamate (pABC), a p-amino benzyl ether (PABE), a p-amino benzyl carbonate, or a combination thereof.

In certain aspects, the self-immolative linker comprises an aromatic group. In some aspects, the aromatic group is selected from the group consisting of benzyl, cinnamyl, naphthyl, and biphenyl. In some aspects, the aromatic group is heterocyclic. In other aspects, the aromatic group comprises at least one substituent. In some aspects, the at least one substituent is selected from the group consisting of F, Cl, I, Br, OH, methyl, methoxy, NO₂, NH₂, NO³⁺, NHCOCH₃, N(CH₃)₂, NHCOCF₃, alkyl, haloalkyl, C₁-C₈ alkylhalide, carboxylate, sulfate, sulfamate, and sulfonate. In other aspects, at least one C in the aromatic group is substituted with N, O, or C-R*, wherein R* is independently selected from H, F, Cl, I, Br, OH, methyl, methoxy, NO₂, NH₂, NO³⁺, NHCOCH₃, N(CH₃)₂, NHCOCF₃, alkyl, haloalkyl, C₁-C₈ alkylhalide, carboxylate, sulfate, sulfamate, and sulfonate.

In some aspects, the self-immolative linker comprises an aminobenzyl carbamate group (e.g., para-aminobenzyl carbamate), an aminobenzyl ether group, or an aminobenzyl carbonate group. In one aspect, the self-immolative linker is p-amino benzyl carbamate (pABC).

pABC is the most efficient and most widespread connector linkage for self-immolative site-specific prodrug activation (see, *e.g.,* Carl et al. J. Med. Chem. 24:479-480 (1981); WO 1981/001145; Rautio et la, Nature Reviews Drug Discovery 7:255-270 (2008); Simplicio et al., Molecules 13:519-547 (2008)).

In some aspects, the self-immolative linker connects a biologically active molecule (*e.g.,* an ASO) to a protease-cleavable substrate (e.g., Val-Cit). In specific aspects, the carbamate group of a pABC self-immolative linker is connected to an amino group of a biologically active molecule (*e.g*., ASO), and the amino group of the pABC self-immolative linker is connected to a protease-cleavable substrate.

The aromatic ring of the aminobenzyl group can optionally be substituted with one or more (*e.g*., R₁ and/or R₂) substituents on the aromatic ring, which replace a hydrogen that is otherwise attached to one of the four non-substituted carbons that form the ring. As used herein, the symbol "Rₓ" (*e.g*., R₁, R₂, R₃, R₄) is a general abbreviation that represents a substituent group as described herein.

Substituent groups can improve the self-immolative ability of the p-aminobenzyl group (Hay et al., J. Chem Soc., Perkin Trans. 1:2759-2770 (1999); see also, Sykes et al. J. Chem. Soc., Perkin Trans. 1:1601-1608 (2000)).

Self-immolative elimination can take place, e.g., via 1,4 elimination, 1,6 elimination (*e.g.,* pABC), 1,8 elimination (*e.g.,* p-amino-cinnamyl alcohol), β-elimination, cyclisation-elimination (*e.g*., 4-aminobutanol ester and ethylenediamines), cyclization/lactonization, cyclization/lactolization, *etc.* See, *e.g.,* Singh et al. Curr. Med. Chem. 15:1802-1826 (2008); Greenwald et al. J. Med. Chem. 43:475-487 (2000).

In some aspects, the self-immolative linker can comprise, e.g., cinnamyl, naphthyl, or biphenyl groups (see, *e.g.,* Blencowe et al. Polym. Chem. 2:773-790 (2011)). In some aspects, the self-immolative linker comprises a heterocyclic ring (see., *e.g.,* U.S. Patent Nos. 7,375,078; 7,754,681). Numerous homoaromatic (see, *e.g.,* Carl et al. J. Med. Chem. 24:479 (1981); Senter et al. J. Org. Chem. 55:2975 (1990); Taylor et al. J. Org. Chem. 43:1197 (1978); Andrianomenjanahary et al. Bioorg. Med. Chem. Lett. 2:1903 (1992)), and coumarin (see, *e.g*., Weinstein et al. Chem. Commun. 46:553 (2010)), furan, thiophene, thiazole, oxazole, isoxazole, pyrrole, pyrazole (see, *e.g.,* Hay et al. J. Med. Chem. 46:5533 (2003)), pyridine (see, *e.g.,* Perry-Feigenbaum et al. Org. Biomol. Chem. 7:4825 (2009)), imidazone (see, *e.g.,* Nailor et al. Bioorg. Med. Chem. Lett. Z: 1267 (1999); Hay and Denny, Tetrahedron Lett. 38:8425 (1997)), and triazole (see, *e.g*., Bertrand and Gesson, J. Org. Chem. 72:3596 (2007)) based heteroaromatic groups that are self-immolative under both aqueous and physiological conditions are known in the art. See also, U.S. Pat Nos. 7,691,962; 7,091,186; U.S. Pat. Publ. Nos. US2006/0269480; US2010/0092496; US2010/0145036; US2003/0130189; US2005/0256030)

In some aspects, a linker combination disclosed herein comprises more than one self-immolative linker in tandem, *e.g.,* two or more pABC units. See, *e.g.,* de Groot et al. J. Org. Chem. 66:8815-8830 (2001). In some aspects, a linker combination disclosed herein can comprise a self-immolative linker (*e.g.,* a p-aminobenzylalcohol or a hemithioaminal derivative of p-carboxybenzaldehyde or glyoxilic acid) linked to a fluorigenic probe (see, *e.g.,* Meyer et al. Org. Biomol. Chem. 8:1777-1780 (2010)).

Where substituent groups in the self-immolative linker s are specified by their conventional chemical formulae, written from left to right, they equally encompass the chemically identical substituents, which would result from writing the structure from right to left. For example, "-CH₂O-" is intended to also recite "-OCH₂-".

Substituent groups in self-immolative, for example, R₁ and/or R₂ substituents in a p-aminobenzyl self-immolative linker as discuss above can include, *e.g*., alkyl, alkylene, alkenyl, alkynyl, alkoxy, alkylamino, alkylthio, heteroalkyl, cycloalkyl, heterocycloalkyl, aryl, arylalkyl, aryloxy, heteroaryl, *etc.* When a compound of the present disclosure includes more than one substituent, then each of the substituents is independently chosen.

In some specific aspects, the self-immolative linker is attached to cleavable peptide linker has the following formula, the combination having the following formula:

-Aₐ-Y_{y}-

wherein each -A- is independently an amino acid unit, a is independently an integer from 1 to 12; and -Y- is a self-immolative spacer, and y is 1, or 2. In some aspects, -Aₐ- is a dipeptide, a tripeptide, a tetrapeptide, a pentapeptide, or a hexapeptide. In some aspects, -Aₐ- is selected from the group consisting of valine-alanine, valine-citrulline, phenylalanine-lysine, N-methylvaline-citrulline, cyclohexylalanine-lysine, and beta-alanine-lysine. In some aspects, -Aₐ- is valine-alanine or valine-citrulline.

In some aspects, the self-immolative linker -Y_{y}- has the following formula: 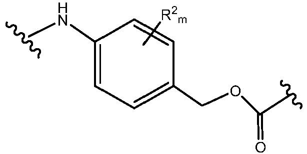 wherein each R² is independently C₁₋₈ alkyl, -O-(C₁₋₈ alkyl), halogen, nitro, or cyano; and m is an integer from 0 to 4. In some aspects, m is 0, 1, or 2. In some aspects, m is 0.

In some aspects, the cleavable linker is valine-alanine-p-aminobenzylcarbamate or valine-citrulline-p-aminobenzylcarbamate.

### III.A.4. Reactive moieties (RM)

The ASOs of the present disclosure are generated either via chemical synthesis or via chemical reaction between their components. For example, in some aspects, an anchoring moiety comprising a reactive group (e.g., maleimide) can react with an ASO comprising a maleimide-reacting group, to yield a hydrophobically modified ASO of the present disclosure, where the anchoring moiety may insert into the lipid bilayer of the membrane of an exosome, thereby attaching the ASO to the surface of the exosome.

Any component or group of components of a hydrophobically modified ASO of the present disclosure can comprise at least a RG and/or an RM, which would allow the attachment of the components through one reaction or series of reactions, to yield a hydrophobically modified ASO of the present disclosure. Exemplary synthesis schemas for the production of hydrophobically modified ASOs include:

[AM]-/RG/ + /RM/-[ASO] → [AM]-[ASO]

[AM]-/RM/ + /RG/-[ASO] → [AM]- [ASO]

[AM]-[L]-/RM/ + /RG/-[ASO] → [AM]-[L]-[ASO]

[AM]-[L]-/RG/ + /RM/-[ASO] → [AM]-[L]-[ASO]

[AM]-/RM/ + /RG/-[L]-[ASO] → [AM]-[L]-[ASO]

[AM]-/RG/ + /RM/-[L]-[ASO] → [AM]-[L]-[ASO]

[AM]-[L]-/RM/ + /RG/-[L]-[ASO] → [AM]-[L]-[L]-[ASO]

[AM]-[L]-/RG/ + /RM/-[L]-[ASO] → [AM]-[L]-[L]-[ASO]

wherein [AM] is an anchoring moiety, [ASO] is an antisense oligonucleotide, [L] is a linker or linker combination, /RM/ is a reactive moiety, and /RG/ is a reactive group. In any of the schematic representations provided, the ASO can be attached, e.g., via its 5' end or 3' end.

Exemplary synthesis schemas for the production of intermediates in the synthesis of ASOs include:

[AM]-/RM/ + /RG/-[L] → [AM]-[L]

[AM]-/RG/ + /RM/-[L] → [AM]-[L]

[L]-/RM/ + /RG/-[L] → [L]-[L]

[L]-/RG/ + /RM/-[L] → [L]-[L]

[L]-/RM/ + /RG/-[ASO] → [L]-[ASO]

[L]-/RG/ + /RM/-[ASO] → [L]-[ASO]

wherein [AM] is an anchoring moiety, [ASO] is an antisense oligonucleotide, [L] is a linker or linker combination, /RM/ is a reactive moiety, and /RG/ is a reactive group. In any of the schematic representations provided, the ASO can be attached, e.g., via its 5' end or 3' end.

In some aspects, the reactive group "/RG/" can be, e.g., an amino group, a thiol group, a hydroxyl group, a carboxylic acid group, or an azide group. Specific reactive moieties "/RM/" that can react with these reactive groups are described in more detail below.

[AM]-(/RM/)n + (/RG/-[L]-[ASO])n → [AM]-[L]-[ASO]

Any of the anchoring moieties, linker or linker combinations, or ASO disclosed herein can be conjugated to a reactive moiety, e.g., an amino reactive moiety (e.g.,. NHS-ester, p-nitrophenol, isothiocyanate, isocyanate, or aldehyde), a thiol reactive moiety (e.g., acrylate, maleimide, or pyridyl disulfide), a hydroxy reactive moiety (e.g., isothiocyanate or isocyanate), a carboxylic acid reactive moiety (e.g., epoxyde), or an azide reactive moiety (e.g., alkyne).

Exemplary reactive moieties that can be used to covalent bind two components disclosed herein (e.g., an anchoring moiety and an ASO, or an anchoring moiety and a linker, or an anchoring moiety and a linker, or two linkers, or a linker and an ASO, or a two anchoring moieties) include, e.g., N-succinimidyl-3-(2-pyridyldithio)propionate, N-4-maleimide butyric acid, S-(2-pyridyldithio)cysteamine, iodoacetoxysuccinimide, N-(4-maleimidebutyryl oxy)succinimide, N-[5-(3'-maleimide propylamide)-1-carboxypentyl]iminodiacetic acid, N-(5-aminopentyl)iminodiacetic acid, and 1'-[(2-cyanoethyl)-(N,N-diisopropyl)]-phosphoramidite). Bifunctional linkers (linkers containing two functional groups) are also usable.

In some aspects, an anchoring moiety, linker, or ASO can comprise a terminal oxyamino group, e.g., -ONH2, an hydrazino group, -NHNH2, a mercapto group (i.e., SH or thiol), or an olefin (e.g., CH=CH2). In some aspects, an anchoring moiety, linker, or ASO can comprise an electrophilic moiety, e.g., at a terminal position, e.g., an aldehyde, alkyl halide, mesylate, tosylate, nosylate, or brosylate, or an activated carboxylic acid ester, e.g. an NHS ester, a phosphoramidite, or a pentafluorophenyl ester. In some aspects, a covalent bond can be formed by coupling a nucleophilic group of a ligand, e.g., a hydroxyl, a thiol or amino group, with an electrophilic group.

The present disclosure is amenable to all manner of reactive groups and reactive moieties including but not limited to those known in the art.

The term "protecting group," as used herein, refers to a labile chemical moiety which is known in the art to protect reactive groups including without limitation, hydroxyl, amino and thiol groups, against undesired reactions during synthetic procedures. Protecting groups are typically used selectively and/or orthogonally to protect sites during reactions at other reactive sites and can then be removed to leave the unprotected group as is or available for further reactions. Protecting groups as known in the art are described generally in Greene and Wuts, Protective Groups in Organic Synthesis, 3rd edition, John Wiley & Sons, New York (1999).

Additionally, the various synthetic steps may be performed in an alternate sequence or order to give the desired compounds. Synthetic chemistry transformations and protecting group methodologies (protection and deprotection) useful in synthesizing the compounds described herein are known in the art and include, for example, those such as described in R. Larock, Comprehensive Organic Transformations, VCH Publishers (1989); T. W. Greene and P. G. M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

Solid phase synthesis known in the art may additionally or alternatively be employed. Suitable solid phase techniques, including automated synthesis techniques, are described in F. Eckstein (ed.), Oligonucleotides and Analogues, a Practical Approach, Oxford University Press, New York (1991) and Toy, P.H.; Lam, Y (ed.), Solid-Phase Organic synthesis, concepts, Strategies, and Applications, John Wiley & Sons, Inc. New Jersey (2012).

In some aspects, the reactive group can alternatively react with more than one of the reactive moieties described below.

### III.A.4.a. Amine reactive moieties

In some aspects, the reactive moiety is an amine reactive moiety. As used herein the term "amine reactive moiety" refers to a chemical groups which can react with a reactive group having an amino moiety, e.g., primary amines. Exemplary amine reactive moieties are N-hydroxysuccinimide esters (NHS-ester), p-nitrophenol, isothiocyanate, isocyanate, and aldehyde. Alternative reactive moieties that react with primary amines are also well known in the art. In some aspects, an amine reactive moiety can be attached to a terminal position of an anchoring moiety, linker combination, or ASO of the present disclosure.

In some aspects, the amine reactive moiety is a NHS-ester. Typically, a NHS-ester reactive moiety reacts with a primary amine of a reactive group to yield a stable amide bond and N-hydroxysuccinimide (NHS).

In some aspects, the amine reactive moiety is a p-nitrophenol group. Typically, a p-nitrophenol reactive moiety is an activated carbamate that reacts with a primary amine of a reactive group to yield a stable carbamate moiety and p-nitrophenol.

In some aspects, the amine reactive moiety is an isothiocyanate. Typically, a isothiocyanate reacts with a primary amine of a reactive group to yield a stable thiourea moiety.

In some aspects, the amine reactive moiety is an isocyanate. Typically, a isocyanate reacts with a primary amine of a reactive group to yield a stable urea moiety.

In some aspects, amine the reactive moiety is an aldehyde. Typically, aldehydes react with primary amines to form Schiff bases which can be further reduced to form a covalent bond through reductive amination.

### III.A.4.b. Thiol reactive moieties

In some aspects, the reactive moiety is a thiol reactive moiety. As used herein the term "thiol reactive moiety" refers to a chemical groups which can react with a reactive group having a thiol moiety (or mercapto group). Exemplary thiol reactive moieties are acrylates, maleimides, and pyridyl disulfides. Alternative reactive moieties that react with thiols are also well known in the art. In some aspects, a thiol reactive moiety can be attached to a terminal position of an anchoring moiety, linker combination, or ASO of the present disclosure.

In some aspects, the thiol reactive moiety is an acrylate. Typically, acrylates react with thiols at the carbon β to the carbonyl of the acrylate to form a stable sulfide bond.

In some aspects, the thiol reactive moiety is a maleimide. Typically, maleimides react with thiols at either of at the carbon β the to the carbonyls to form a stable sulfide bond.

In some aspects, the thiol reactive moiety is a pyridyl disulfide. Typically, pyridyl disulfides react with thiols at the sulfur atom β to the pyridyl to form a stable disulfide bond and pyridine-2-thione.

### III.A.4.c. Hydroxy reactive moieties

In some aspects, the reactive moiety is a hydroxyl reactive moiety. As used herein the term "hydroxyl reactive moiety" refers to a chemical group which can react with a reactive group having an hydroxyl moiety. Exemplary hydroxyl reactive moieties are isothiocyanates and isocyanates. Alternative reactive moieties that react with hydroxyl moieties are also well known in the art. In some aspects, a hydroxyl reactive moiety can be attached to a terminal position of an anchoring moiety, linker combination, or ASO of the present disclosure.

In some aspects, the hydroxyl reactive moiety is an isothiocyanate. Typically, an isothiocyanate reacts with a hydroxyl of a reactive group to yield a stable carbamothioate moiety.

In some aspects, amine the reactive moiety is a isocyanate. Typically, an isocyante reacts with a hydroxyl of a reactive group to yield a stable carbamate moiety.

### III.A.4.d. Carboxylic acid reactive moieties

In some aspects, the reactive moiety is a carboxylic acid reactive moiety. As used herein the term "carboxylic acid reactive moiety" refers to a chemical groups which can react with a reactive group having an carboxylic acid moiety. An exemplary carboxylic acid reactive moieties is an epoxide. Alternative reactive moieties that react with carboxylic acid moieties are also well known in the art. In some aspects, an carboxylic acid reactive moiety can be attached to a terminal position of an anchoring moiety, linker combination, or ASO of the present disclosure.

In some aspects, the carboxylic acid reactive moiety is an epoxide. Typically, an epoxide reacts with the carboxylic acid of a reactive group at either of the carbon atoms of the epoxide to form a 2-hydroxyethyl acetate moiety.

### III.A.4.e. Azide reactive moieties

In some aspects, the reactive moiety is an azide reactive moiety. As used herein the term "azide reactive moiety" refers to a chemical groups which can react with a reactive group having an azide moiety. An exemplary azide reactive moieties is an alkyne. Alternative reactive moieties that react with azide moieties are also well known in the art. In some aspects, a carboxylic acid reactive moiety can be attached to a terminal position of an anchoring moiety, linker combination, or ASO of the present disclosure.

In some aspects, the azide reactive moiety is an alkyne. Typically, an alkyne reacts with the azide of a reactive group through a 1,3-dipolar cycloaddition reaction, also referred to "click chemistry," to form a 1,2,3-triazole moiety.

### III.A.5. Specific examples and topologies

In specific aspects of the present disclosure, the linker combination consists of a linker of formula

[Alkyl linker]m-[PEG1]n-[PEG2] o

wherein m, n, and o are 0 or 1, and at least one of m, n, or o is not zero. Exemplary linker combinations according to such formula are C6-TEG-HEG, C6-HEG, C6-TEG, C6, TEG-HEG, TEG, C8-TEG-HEG, C8-HEG, C8-TEG, and C8.

In some aspects, the linker combination comprises anon-cleavable linker (e.g., TEG or HEG) in combination with one or more cleavable linkers, e.g., an enzymatic cleavable linker and a self immolative linker.

In a specific aspect, the linker combination the linker combination comprises the linker combination TEG (non-cleavable linker)-Val-Cit(cleavable linker)-pAB(self-immolative linker), as shown below

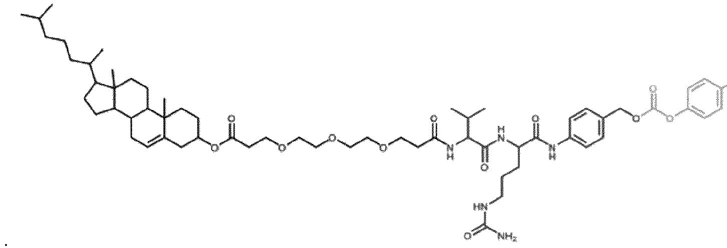

Specific combinations of anchoring moieties and linker combinations are illustrated in the tables below.

**Table 2.**

| | **Linker combination** | | |
|---|---|---|---|
| **Anchoring moiety** | **1^{st} Linker** | **2^{nd} Linker** | **3^{rd} Linker** |
| Cholesterol | C6 | TEG | HEG |
| Cholesterol | C6 | HEG | No |
| Cholesterol | C6 | TEG | No |
| Cholesterol | C6 | No | No |
| Cholesterol | TEG | HEG | No |
| Cholesterol | TEG | No | No |
| Tocopherol | C8 | TEG | HEG |
| Tocopherol | C8 | HEG | No |
| Tocopherol | C8 | TEG | No |
| Tocopherol | C8 | No | No |
| Tocopherol | TEG | HEG | No |
| Tocopherol | HEG | No | No |
| Tocopherol | TEG | No | No |
| Tocopherol | No | No | No |
| Palmitate | C6 | TEG | HEG |
| Palmitate | C6 | HEG | No |
| Palmitate | C6 | TEG | No |
| Palmitate | C6 | No | No |
| Cholesterol | TEG | Glycerol | HEG |

**Table 3.**

| **Linker Combination** | | |
|---|---|---|
| **Linker 1** | **Cleavable Linker 2** | **Linker 3** |
| C6 | Disulfide | C6 |
| | Imine | |
| None | | None |
| | Thioketal | |
| | Tri/Dinucleotide | |
| TEG | | TEG |
| | Val-Cit | |
| HEG | | HEG |
| TEG-HEG | | TEG-HEG |

Specific oligonucleotides such as ASOs of the present disclosure are exemplified below 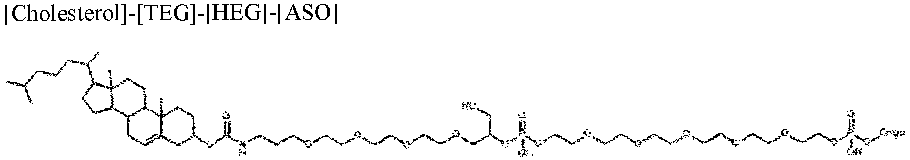 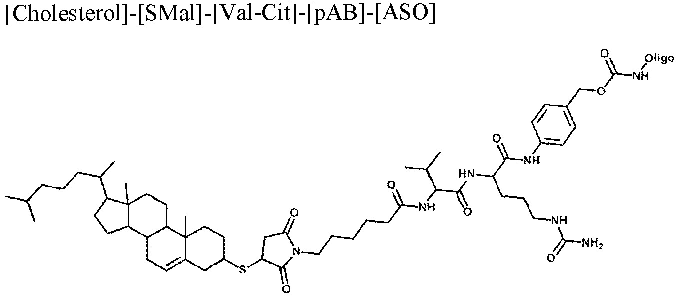 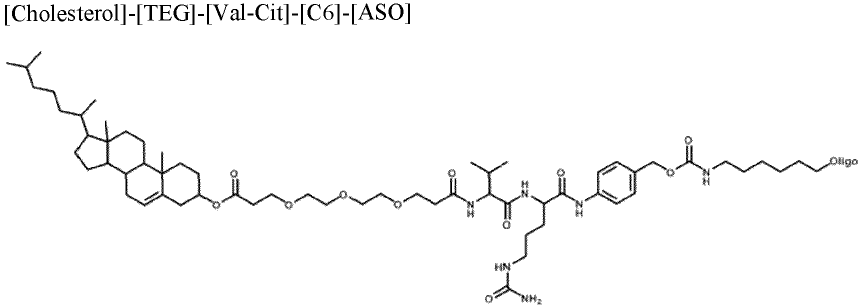 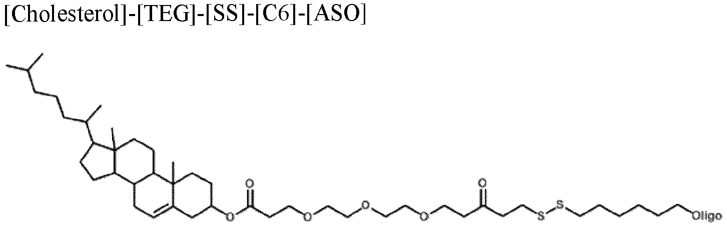 wherein [Cholesterol] is a cholesterol anchoring moiety, [TEG] is a TEG non-cleavable linker, [HEG] is a HEG non-cleavable linker, [SS] is a disulfide redox cleavable linker, [C6] is an alkyl non-cleavable linker, [SMal] is S-maleimide, [Val-Cit] is a valine-citrulline cleavable linker, [pAB] is a pAB self-immolative linker. In some aspects, an ASO of the present disclosure has a structure according to the exemplary structures provided above, in which one or more components has been replaced by a component in the same class as those depicted in the example. For example, the [cholesterol] anchoring moiety can be substituted by another anchoring moiety disclosed herein, a [TEG] can be substituted by another polymeric non-cleavable linker disclosed herein (e.g., HEG, PEG, PG), [Val-Cit] can be replaced by another peptidase cleavable linker, or [pAB] can be substituted by another self-immolative linker.

### III.B. Scaffold Moieties

One or more scaffold moieties can be expressed in the EVs. In some aspects, one or more scaffold moieties are used to anchor an ASO to the EV of the present disclosure. In other aspects, one or more scaffold moieties are used to anchor a protein or a molecule to the EVs in addition to the ASOs. Therefore, an EV of the present disclosure comprises an anchoring moiety linking an ASO and a scaffold moiety linking a protein or a molecule, e.g., a targeting moiety. In some aspects, the ASO is linked to the scaffold moiety. In some aspects, the EV comprises more than one scaffold moiety. In some aspects, a first ASO is linked to a first scaffold moiety and a second ASO is linked to a second scaffold moiety. In some aspects, the first scaffold moiety and the second scaffold moiety are the same type of scaffold moiety, e.g., the first and second scaffold moieties are both a Scaffold X protein. In some aspects, the first scaffold moiety and the second scaffold moiety are different types of scaffold moiety, *e.g*., the first scaffold moiety is a Scaffold Y protein and the second scaffold moiety is a Scaffold X protein. In some aspects, the first scaffold moiety is a Scaffold Y, disclosed herein. In some aspects, the first scaffold moiety is a Scaffold X, disclosed herein. In some aspects, the second scaffold moiety is a Scaffold Y, disclosed herein. In some aspects, the second scaffold moiety is a Scaffold X, disclosed herein.

In some aspects, the EV comprises one or more scaffold moieties, which are capable of anchoring an ASO to the EV, *e.g.,* exosome, (*e.g.,* either on the luminal surface or on the exterior surface). In certain aspects, the scaffold moiety is a polypeptide ("scaffold protein"). In certain aspects, the scaffold protein comprises an exosome protein or a fragment thereof. In other aspects, scaffold moieties are non-polypeptide moieties. In some aspects, scaffold proteins include various membrane proteins, such as transmembrane proteins, integral proteins and peripheral proteins, enriched on the exosome membranes. They can include various CD proteins, transporters, integrins, lectins, and cadherins. In certain aspects, a scaffold moiety (*e.g*., scaffold protein) comprises Scaffold X. In other aspects, a scaffold moiety (*e.g*., exosome protein) comprises Scaffold Y. In further aspects, a scaffold moiety (*e.g*., exosome protein) comprises both a Scaffold X and a Scaffold Y.

### III.B.1. Scaffold X-Engineered EVs, e.g., Exosomes

In some aspects, EVs, *e.g*., exosomes, of the present disclosure comprise a membrane modified in its composition. For example, their membrane compositions can be modified by changing the protein, lipid, or glycan content of the membrane.

In some aspects, the surface-engineered EVs, *e.g*., exosomes, are generated by chemical and/or physical methods, such as PEG-induced fusion and/or ultrasonic fusion. In other aspects, the surface-engineered EVs, *e.g*., exosomes, are generated by genetic engineering. EVs, *e.g*., exosomes, produced from a genetically-modified producer cell or a progeny of the genetically-modified cell can contain modified membrane compositions. In some aspects, surface-engineered EVs, *e.g.,* exosomes, have scaffold moiety (*e.g.,* exosome protein, *e.g.,* Scaffold X) at a higher or lower density (*e.g*., higher number) or include a variant or a fragment of the scaffold moiety.

For example, surface (*e.g*., Scaffold X)-engineered EVs, can be produced from a cell (*e.g*., HEK293 cells) transformed with an exogenous sequence encoding a scaffold moiety (*e.g.,* exosome proteins, *e.g.,* Scaffold X) or a variant or a fragment thereof. EVs including scaffold moiety expressed from the exogenous sequence can include modified membrane compositions.

Various modifications or fragments of the scaffold moiety can be used for the aspects of the present disclosure. For example, scaffold moiety modified to have enhanced affinity to a binding agent can be used for generating surface-engineered EV that can be purified using the binding agent. Scaffold moieties modified to be more effectively targeted to EVs and/or membranes can be used. Scaffold moieties modified to comprise a minimal fragment required for specific and effective targeting to exosome membranes can be also used.

Scaffold moieties can be engineered to be expressed as a fusion molecule, *e.g*., fusion molecule of Scaffold X to an ASO. For example, the fusion molecule can comprise a scaffold moiety disclosed herein (*e.g*., Scaffold X, *e.g*., PTGFRN, BSG, IGSF2, IGSF3, IGSF8, ITGB1, ITGA4, SLC3A2, ATP transporter, or a fragment or a variant thereof) linked to an ASO.

In some aspects, the surface (*e.g*., Scaffold X)-engineered EVs described herein demonstrate superior characteristics compared to EVs known in the art. For example, surface (*e.g*., Scaffold X)-engineered contain modified proteins more highly enriched on their surface than naturally occurring EVs or the EVs produced using conventional exosome proteins. Moreover, the surface (*e.g*., Scaffold X)-engineered EVs of the present disclosure can have greater, more specific, or more controlled biological activity compared to naturally occurring EVs or the EVs produced using conventional exosome proteins.

In some aspects, the Scaffold X comprises Prostaglandin F2 receptor negative regulator (the PTGFRN polypeptide). The PTGFRN protein can be also referred to as CD9 partner 1 (CD9P-1), Glu-Trp-Ile EWI motif-containing protein F (EWI-F), Prostaglandin F2-alpha receptor regulatory protein, Prostaglandin F2-alpha receptor-associated protein, or CD315. The full length amino acid sequence of the human PTGFRN protein (Uniprot Accession No. Q9P2B2) is shown at Table 4 as SEQ ID NO: 301. The PTGFRN polypeptide contains a signal peptide (amino acids 1 to 25 of SEQ ID NO: 301), the extracellular domain (amino acids 26 to 832 of SEQ ID NO: 301), a transmembrane domain (amino acids 833 to 853 of SEQ ID NO: 301), and a cytoplasmic domain (amino acids 854 to 879 of SEQ ID NO: 301). The mature PTGFRN polypeptide consists of SEQ ID NO: 301 without the signal peptide, *i.e.,* amino acids 26 to 879 of SEQ ID NO: 301. In some aspects, a PTGFRN polypeptide fragment useful for the present disclosure comprises a transmembrane domain of the PTGFRN polypeptide. In other aspects, a PTGFRN polypeptide fragment useful for the present disclosure comprises the transmembrane domain of the PTGFRN polypeptide and (i) at least five, at least 10, at least 15, at least 20, at least 25, at least 30, at least 40, at least 50, at least 70, at least 80, at least 90, at least 100, at least 110, at least 120, at least 130, at least 140, at least 150 amino acids at the N terminus of the transmembrane domain, (ii) at least five, at least 10, at least 15, at least 20, or at least 25 amino acids at the C terminus of the transmembrane domain, or both (i) and (ii).

In some aspects, the fragments of PTGFRN polypeptide lack one or more functional or structural domains, such as IgV.

In other aspects, the Scaffold X comprises an amino acid sequence at least about 70%, at least about 75%, at least about 80%, at least about 85%, at least about 90%, at least about 95%, at least about 96%, at least about 97%, at least about 98%, at least about 99%, or about 100% identical to amino acids 26 to 879 of SEQ ID NO: 301. In other aspects, the Scaffold X comprises an amino acid sequence at least about at least about 70%, at least about 75%, at least about 80%, at least about 85%, at least about 90%, at least about 95%, at least about 96%, at least about 97%, at least about 98%, at least about 99%, or about 100% identical to SEQ ID NO: 302. In other aspects, the Scaffold X comprises the amino acid sequence of SEQ ID NO: 302, except one amino acid mutation, two amino acid mutations, three amino acid mutations, four amino acid mutations, five amino acid mutations, six amino acid mutations, or seven amino acid mutations. The mutations can be a substitution, an insertion, a deletion, or any combination thereof. In some aspects, the Scaffold X comprises the amino acid sequence of SEQ ID NO: 302 and 1 amino acid, two amino acids, three amino acids, four amino acids, five amino acids, six amino acids, seven amino acids, eight amino acids, nine amino acids, ten amino acids, 11 amino acids, 12 amino acids, 13 amino acids, 14 amino acids, 15 amino acids, 16 amino acids, 17 amino acids, 18 amino acids, 19 amino acids, or 20 amino acids or longer at the N terminus and/or C terminus of SEQ ID NO: 302.

In other aspects, the Scaffold X comprises an amino acid sequence at least about at least about 70%, at least about 75%, at least about 80%, at least about 85%, at least about 90%, at least about 95%, at least about 96%, at least about 97%, at least about 98%, at least about 99%, or about 100% identical to SEQ ID NO: 301, 302, 303, 304, 305, 306, 307, 308, 309, 310, 311, 312, 313, 314, 315, 316, 317, or 318. In other aspects, the Scaffold X comprises the amino acid sequence of SEQ ID NO: 301, 302, 303, 304, 305, 306, 307, 308, 309, 310, 311, 312, 313, 314, 315, 316, 317, or 318, except one amino acid mutation, two amino acid mutations, three amino acid mutations, four amino acid mutations, five amino acid mutations, six amino acid mutations, or seven amino acid mutations. The mutations can be a substitution, an insertion, a deletion, or any combination thereof. In some aspects, the Scaffold X comprises the amino acid sequence of SEQ ID NO: 301, 302, 303, 304, 305, 306, 307, 308, 309, 310, 311, 312, 313, 314, 315, 316, 317, or 318 and 1 amino acid, two amino acids, three amino acids, four amino acids, five amino acids, six amino acids, seven amino acids, eight amino acids, nine amino acids, ten amino acids, 11 amino acids, 12 amino acids, 13 amino acids, 14 amino acids, 15 amino acids, 16 amino acids, 17 amino acids, 18 amino acids, 19 amino acids, or 20 amino acids or longer at the N terminus and/or C terminus of SEQ ID NO: 301, 302, 303, 304, 305, 306, 307, 308, 309, 310, 311, 312, 313, 314, 315, 316, 317, or 318.

**Table 4. Exemplary Scaffold X Protein Sequences**

| Protein | Sequence |
|---|---|
| The PTGFRN Protein (SEQ ID NO: 301) | MGRLASRPLLLALLSLALCRGRVVRVPTATLVRVVGTELVIPCNVSDYDGP SEQNFDWSFSSLGSSFVELASTWEVGFPAQLYQERLQRGEILLRRTANDAV ELHIKNVQPSDQGHYKCSTPSTDATVQGNYEDTVQVKVLADSLHVGPSARP PPSLSLREGEPFELRCTAASASPLHTHLALLWEVHRGPARRSVLALTHEGR FHPGLGYEQRYHSGDVRLDTVGSDAYRLSVSRALSADQGSYRCIVSEWIAE QGNWQEIQEKAVEVATVVIQPSVLRAAVPKNVSVAEGKELDLTCNITTDRA DDVRPEVTWSFSRMPDSTLPGSRVLARLDRDSLVHSSPHVALSHVDARSYH LLVRDVSKENSGYYYCHVSLWAPGHNRSWHKVAEAVSSPAGVGVTWLEPDY QVYLNASKVPGFADDPTELACRVVDTKSGEANVRFTVSWYYRMNRRSDNVV |
| | TSELLAVMDGDWTLKYGERSKQRAQDGDFIFSKEHTDTFNFRIQRTTEEDR GNYYCVVSAWTKQRNNSWVKSKDVFSKPVNIFWALEDSVLVVKARQPKPFF AAGNTFEMTCKVSSKNIKSPRYSVLIMAEKPVGDLSSPNETKYIISLDQDS VVKLENWTDASRVDGVVLEKVQEDEFRYRMYQTQVSDAGLYRCMVTAWSPV RGSLWREAATSLSNPIEIDFQTSGPIFNASVHSDTPSVIRGDLIKLFCIIT VEGAALDPDDMAFDVSWFAVHSFGLDKAPVLLSSLDRKGIVTTSRRDWKSD LSLERVSVLEFLLQVHGSEDQDFGNYYCSVTPWVKSPTGSWQKEAEIHSKP VFITVKMDVLNAFKYPLLIGVGLSTVIGLLSCLIGYCSSHWCCKKEVQETR RERRRLMSMEMD |
| The PTGFRN protein Fragment (SEQ ID NO: 302) | GPIFNASVHSDTPSVIRGDLIKLFCIITVEGAALDPDDMAFDVSWFAVHSF GLDKAPVLLSSLDRKGIVTTSRRDWKSDLSLERVSVLEFLLQVHGSEDQDF GNYYCSVTPWVKSPTGSWQKEAEIHSKPVFITVKMDVLNAFKYPLLIGVGL STVIGLLSCLIGYCSSHWCCKKEVQETRRERRRLMSMEM 687-878 of SEQ ID NO: 301 |

In other aspects, the Scaffold X comprises an amino acid sequence at least about at least about 70%, at least about 75%, at least about 80%, at least about 85%, at least about 90%, at least about 95%, at least about 96%, at least about 97%, at least about 98%, at least about 99%, or about 100% identical to SEQ ID NO: 319, 320, 321, 322, 323, 323, or 325. In other aspects, the Scaffold X comprises the amino acid sequence of SEQ ID NO: 319, 320, 321, 322, 323, 323, or 325, except one amino acid mutation, two amino acid mutations, three amino acid mutations, four amino acid mutations, five amino acid mutations, six amino acid mutations, or seven amino acid mutations. The mutations can be a substitution, an insertion, a deletion, or any combination thereof. In some aspects, the Scaffold X comprises the amino acid sequence of SEQ ID NO: 319, 320, 321, 322, 323, 323, or 325 and 1 amino acid, two amino acids, three amino acids, four amino acids, five amino acids, six amino acids, seven amino acids, eight amino acids, nine amino acids, ten amino acids, 11 amino acids, 12 amino acids, 13 amino acids, 14 amino acids, 15 amino acids, 16 amino acids, 17 amino acids, 18 amino acids, 19 amino acids, or 20 amino acids or longer at the N terminus and/or C terminus of SEQ ID NO: 319, 320, 321, 322, 323, 323, or 325.

In some aspects, a Scaffold X comprises Basigin (the BSG protein), represented by SEQ ID NO: 303. The BSG protein is also known as 5F7, Collagenase stimulatory factor, Extracellular matrix metalloproteinase inducer (EMMPRIN), Leukocyte activation antigen M6, OK blood group antigen, Tumor cell-derived collagenase stimulatory factor (TCSF), or CD147. The Uniprot number for the human BSG protein is P35613. The signal peptide of the BSG protein is amino acid 1 to 21 of SEQ ID NO: 303. Amino acids 138-323 of SEQ ID NO: 303 is the extracellular domain, amino acids 324 to 344 is the transmembrane domain, and amino acids 345 to 385 of SEQ ID NO: 303 is the cytoplasmic domain.

In other aspects, the Scaffold X comprises an amino acid sequence at least about 70%, at least about 75%, at least about 80%, at least about 85%, at least about 90%, at least about 95%, at least about 96%, at least about 97%, at least about 98%, at least about 99%, or about 100% identical to amino acids 22 to 385 of SEQ ID NO: 303. In some aspects, the fragments of BSG polypeptide lack one or more functional or structural domains, such as IgV, *e.g*., amino acids 221 to 315 of SEQ ID NO: 303. In other aspects, the Scaffold X comprises an amino acid sequence at least about at least about 70%, at least about 75%, at least about 80%, at least about 85%, at least about 90%, at least about 95%, at least about 96%, at least about 97%, at least about 98%, at least about 99%, or about 100% identical to SEQ ID NO: 326, 327, or 328. In other aspects, the Scaffold X comprises the amino acid sequence of SEQ ID NO: 326, 327, or 328, except one amino acid mutation, two amino acid mutations, three amino acid mutations, four amino acid mutations, five amino acid mutations, six amino acid mutations, or seven amino acid mutations. The mutations can be a substitution, an insertion, a deletion, or any combination thereof. In some aspects, the Scaffold X comprises the amino acid sequence of SEQ ID NO: 326, 327, or 328 and 1 amino acid, two amino acids, three amino acids, four amino acids, five amino acids, six amino acids, seven amino acids, eight amino acids, nine amino acids, ten amino acids, 11 amino acids, 12 amino acids, 13 amino acids, 14 amino acids, 15 amino acids, 16 amino acids, 17 amino acids, 18 amino acids, 19 amino acids, or 20 amino acids or longer at the N terminus and/or C terminus of SEQ ID NO: 326, 327, or 328.

In some aspects, a Scaffold X comprises Immunoglobulin superfamily member 8 (IgSF8 or the IGSF8 protein), which is also known as CD81 partner 3, Glu-Trp-Ile EWI motif-containing protein 2 (EWI-2), Keratinocytes-associated transmembrane protein 4 (KCT-4), LIR-D1, Prostaglandin regulatory-like protein (PGRL) or CD316. The full length human IGSF8 protein is accession no. Q969P0 in Uniprot and is shown as SEQ ID NO: 304 herein. The human IGSF8 protein has a signal peptide (amino acids 1 to 27 of SEQ ID NO: 304), an extracellular domain (amino acids 28 to 579 of SEQ ID NO: 304), a transmembrane domain (amino acids 580 to 600 of SEQ ID NO: 304), and a cytoplasmic domain (amino acids 601 to 613 of SEQ ID NO: 304).

In other aspects, the Scaffold X comprises an amino acid sequence at least about 70%, at least about 75%, at least about 80%, at least about 85%, at least about 90%, at least about 95%, at least about 96%, at least about 97%, at least about 98%, at least about 99%, or about 100% identical to amino acids 28 to 613 of SEQ ID NO: 304. In some aspects, the IGSF8 protein lack one or more functional or structural domains, such as IgV. In other aspects, the Scaffold X comprises an amino acid sequence at least about at least about 70%, at least about 75%, at least about 80%, at least about 85%, at least about 90%, at least about 95%, at least about 96%, at least about 97%, at least about 98%, at least about 99%, or about 100% identical to SEQ ID NO: 330, 331, 332, or 333. In other aspects, the Scaffold X comprises the amino acid sequence of SEQ ID NO: 330, 331, 332, or 333, except one amino acid mutation, two amino acid mutations, three amino acid mutations, four amino acid mutations, five amino acid mutations, six amino acid mutations, or seven amino acid mutations. The mutations can be a substitution, an insertion, a deletion, or any combination thereof. In some aspects, the Scaffold X comprises the amino acid sequence of SEQ ID NO: 330, 331, 332, or 333 and 1 amino acid, two amino acids, three amino acids, four amino acids, five amino acids, six amino acids, seven amino acids, eight amino acids, nine amino acids, ten amino acids, 11 amino acids, 12 amino acids, 13 amino acids, 14 amino acids, 15 amino acids, 16 amino acids, 17 amino acids, 18 amino acids, 19 amino acids, or 20 amino acids or longer at the N terminus and/or C terminus of SEQ ID NO: 330, 331, 332, or 333.

In some aspects, a Scaffold X for the present disclosure comprises Immunoglobulin superfamily member 3 (IgSF3 or the IGSF3 protein), which is also known as Glu-Trp-Ile EWI motif-containing protein 3 (EWI-3), and is shown as the amino acid sequence of SEQ ID NO: 309. The human IGSF3 protein has a signal peptide (amino acids 1 to 19 of SEQ ID NO: 309), an extracellular domain (amino acids 20 to 1124 of SEQ ID NO: 309), a transmembrane domain (amino acids 1125 to 1145 of SEQ ID NO: 309), and a cytoplasmic domain (amino acids 1146 to 1194 of SEQ ID NO: 309).

In other aspects, the Scaffold X comprises an amino acid sequence at least about 70%, at least about 75%, at least about 80%, at least about 85%, at least about 90%, at least about 95%, at least about 96%, at least about 97%, at least about 98%, at least about 99%, or about 100% identical to amino acids 28 to 613 of SEQ ID NO: 309. In some aspects, the IGSF3 protein lack one or more functional or structural domains, such as IgV.

In some aspects, a Scaffold X for the present disclosure comprises Integrin beta-1 (the ITGB1 protein), which is also known as Fibronectin receptor subunit beta, Glycoprotein IIa (GPIIA), VLA-4 subunit beta, or CD29, and is shown as the amino acid sequence of SEQ ID NO: 305. The human ITGB1 protein has a signal peptide (amino acids 1 to 20 of SEQ ID NO: 305), an extracellular domain (amino acids 21 to 728 of SEQ ID NO: 305), a transmembrane domain (amino acids 729 to 751 of SEQ ID NO: 305), and a cytoplasmic domain (amino acids 752 to 798 of SEQ ID NO: 305).

In other aspects, the Scaffold X comprises an amino acid sequence at least about 70%, at least about 75%, at least about 80%, at least about 85%, at least about 90%, at least about 95%, at least about 96%, at least about 97%, at least about 98%, at least about 99%, or about 100% identical to amino acids 21 to 798 of SEQ ID NO: 305. In some aspects, the ITGB1 protein lack one or more functional or structural domains, such as IgV.

In other aspects, the Scaffold X comprises the ITGA4 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 306 without the signal peptide (amino acids 1 to 33 of SEQ ID NO: 306). In some aspects, the ITGA4 protein lacks one or more functional or structural domains, such as IgV.

In other aspects, the Scaffold X comprises the SLC3A2 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 307 without the signal peptide. In some aspects, the SLC3A2 protein lacks one or more functional or structural domains, such as IgV.

In other aspects, the Scaffold X comprises the ATP1A1 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 310 without the signal peptide. In some aspects, the ATP1A1 protein lacks one or more functional or structural domains, such as IgV.

In other aspects, the Scaffold X comprises the ATP1A2 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 311 without the signal peptide. In some aspects, the ATP1A2 protein lacks one or more functional or structural domains, such as IgV.

In other aspects, the Scaffold X comprises the ATP1A3 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 312 without the signal peptide. In some aspects, the ATP1A3 protein lacks one or more functional or structural domains, such as IgV.

In other aspects, the Scaffold X comprises the ATP1A4 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 313 without the signal peptide. In some aspects, the ATP1A4 protein lacks one or more functional or structural domains, such as IgV.

In other aspects, the Scaffold X comprises the ATP2B1 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 314 without the signal peptide. In some aspects, the ATP2B1 protein lacks one or more functional or structural domains, such as IgV.

In other aspects, the Scaffold X comprises the ATP2B2 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 315 without the signal peptide. In some aspects, the ATP2B2 protein lacks one or more functional or structural domains, such as IgV.

In other aspects, the Scaffold X comprises the ATP2B3 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 316 without the signal peptide. In some aspects, the ATP2B3 protein lacks one or more functional or structural domains, such as IgV.

In other aspects, the Scaffold X comprises the ATP2B4 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 317 without the signal peptide. In some aspects, the ATP2B4 protein lacks one or more functional or structural domains, such as IgV.

In other aspects, the Scaffold X comprises the IGSF2 protein, which comprises an amino acid sequence at least about 70%, at least about 75%, at least about 80%, at least about 85%, at least about 90%, at least about 95%, at least about 96%, at least about 97%, at least about 98%, at least about 99%, or about 100% identical to SEQ ID NO: 318 without the signal peptide. In some aspects, the IGSF2 protein lacks one or more functional or structural domains, such as IgV.

Non-limiting examples of other Scaffold X proteins can be found at US Patent No. US10195290B1, issued Feb. 5, 2019.

In some aspects, the sequence encodes a fragment of the scaffold moiety lacking at least 5, 10, 50, 100, 200, 300, 400, 500, 600, 700, or 800 amino acids from the N-terminus of the native protein. In some aspects, the sequence encodes a fragment of the scaffold moiety lacking at least 5, 10, 50, 100, 200, 300, 400, 500, 600, 700, or 800 amino acids from the C-terminus of the native protein. In some aspects, the sequence encodes a fragment of the scaffold moiety lacking at least 5, 10, 50, 100, 200, 300, 400, 500, 600, 700, or 800 amino acids from both the N-terminus and C-terminus of the native protein. In some aspects, the sequence encodes a fragment of the scaffold moiety lacking one or more functional or structural domains of the native protein.

In some aspects, the scaffold moieties, *e.g*., Scaffold X, *e.g.,* a PTGFRN protein, are linked to one or more heterologous proteins. The one or more heterologous proteins can be linked to the N-terminus of the scaffold moieties. The one or more heterologous proteins can be linked to the C-terminus of the scaffold moieties. In some aspects, the one or more heterologous proteins are linked to both the N-terminus and the C-terminus of the scaffold moieties. In some aspects, the heterologous protein is a mammalian protein. In some aspects, the heterologous protein is a human protein.

In some aspects, Scaffold X can be used to link any moiety, *e.g*., an ASO, to the luminal surface and on the exterior surface of the EV, *e.g*., exosome, at the same time. For example, the PTGFRN polypeptide can be used to link an ASO inside the lumen (*e.g*., on the luminal surface) in addition to the exterior surface of the EV, *e.g*., exosome. Therefore, in certain aspects, Scaffold X can be used for dual purposes, *e.g*., an ASO on the luminal surface and an ASO on the exterior surface of the EV, *e.g*., exosome. In some aspects, Scaffold X is a scaffold protein that is capable of anchoring the ASO on the luminal surface of the EV and/or on the exterior surface of the EV.

### III.B.2. Scaffold Y-Engineered EVs, e.g., Exosomes

In some aspects, EVs, e.g., exosomes, of the present disclosure comprise an internal space (*i.e*., lumen) that is different from that of the naturally occurring EVs. For example, the EV can be changed such that the composition in the luminal surface of the EV, *e.g*., exosome has the protein, lipid, or glycan content different from that of the naturally-occurring exosomes.

In some aspects, engineered EVs, *e.g*., exosomes, can be produced from a cell transformed with an exogenous sequence encoding a scaffold moiety (*e.g.,* exosome proteins, *e.g*., Scaffold Y) or a modification or a fragment of the scaffold moiety that changes the composition or content of the luminal surface of the EV, *e.g.,* exosome. Various modifications or fragments of the exosome protein that can be expressed on the luminal surface of the EV, *e.g*., exosome, can be used for the aspects of the present disclosure.

In some aspects, the exosome proteins that can change the luminal surface of the EVs, *e.g*., exosomes, include, but are not limited to, the myristoylated alanine rich Protein Kinase C substrate (MARCKS) protein, the myristoylated alanine rich Protein Kinase C substrate like 1 (MARCKSL1) protein, the brain acid soluble protein 1 (BASP1) protein, or any combination thereof.

In some aspects, Scaffold Y comprises the MARCKS protein (Uniprot accession no. P29966). The MARCKS protein is also known as protein kinase C substrate, 80 kDa protein, light chain. The full-length human MARCKS protein is 332 amino acids in length and comprises a calmodulin-binding domain at amino acid residues 152-176. In some aspects, Scaffold Y comprises the MARCKSL1 protein (Uniprot accession no. P49006). The MARCKSL1 protein is also known as MARCKS-like protein 1, and macrophage myristoylated alanine-rich C kinase substrate. The full-length human MARCKSL1 protein is 195 amino acids in length. The MARCKSL1 protein has an effector domain involved in lipid-binding and calmodulin-binding at amino acid residues 87-110. In some aspects, the Scaffold Y comprises the BASP1 protein (Uniprot accession number P80723). The BASP1 protein is also known as 22 kDa neuronal tissue-enriched acidic protein or neuronal axonal membrane protein NAP-22. The full-length human BASP1 protein sequence (isomer 1) is 227 amino acids in length. An isomer produced by an alternative splicing is missing amino acids 88 to 141 from SEQ ID NO: 403 (isomer 1). Table 5 provides the full-length sequences for the exemplary Scaffold Y disclosed herein (*i.e*., the MARCKS, MARCKSL1, and BASP1 proteins).

**Table 5. Exemplary Scaffold Y Protein Sequences**

| Protein | Sequence |
|---|---|
| The MARCKS protein (SEQ ID NO: 401) | MGAQFSKTAAKGEAAAERPGEAAVASSPSKANGQENGHVKVNGDASPAA AESGAKEELQANGSAPAADKEEPAAAGSGAASPSAAEKGEPAAAAAPEA GASPVEKEAPAEGEAAEPGSPTAAEGEAASAASSTSSPKAEDGATPSPS NETPKKKKKRFSFKKSFKLSGFSFKKNKKEAGEGGEAEAPAAEGGKDEA AGGAAAAAAEAGAASGEQAAAPGEEAAAGEEGAAGGDPQEAKPQEAAVA PEKPPASDETKAAEEPSKVEEKKAEEAGASAAACEAPSAAGPGAPPEQE AAPAEEPAAAAASSACAAPSQEAQPECSPEAPPAEAAE |
| The MARCKSL1 protein (SEQ ID NO: 402) | MGSQSSKAPRGDVTAEEAAGASPAKANGQENGHVKSNGDLSPKGEGESPPVNGT DEAAGATGDAIEPAPPSQGAEAKGEVPPKETPKKKKKFSFKKPFKLSGLSFKRN RKEGGGDSSASSPTEEEQEQGEIGACSDEGTAQEGKAAATPESQEPQAKGAEAS AASEEEAGPQATEPSTPSGPESGPTPASAEQNE |
| The BASP1 protein (SEQ ID NO: 403) | MGGKLSKKKKGYNVNDEKAKEKDKKAEGAATEEEGTPKESEPQAAAEPA EAKEGKEKPDQDAEGKAEEKEGEKDAAAAKEEAPKAEPEKTEGAAEAKA EPPKAPEQEQAAPGPAAGGEAPKAAEAAAAPAESAAPAAGEEPSKEEGE PKKTEAPAAPAAQETKSDGAPASDSKPGSSEAAPSSKETPAATEAPSST PKAQGPAASAEEPKPVEAPAANSDQTVTVKE |

The mature BASP1 protein sequence is missing the first Met from SEQ ID NO: 403 and thus contains amino acids 2 to 227 of SEQ ID NO: 403. Similarly, the mature MARCKS and MARCKSL1 proteins also lack the first Met from SEQ ID NOs: 401 and 402, respectively. Accordingly, the mature MARCKS protein contains amino acids 2 to 332 of SEQ ID NO: 401. The mature MARCKSL1 protein contains amino acids 2 to 227 of SEQ ID NO: 402.

In other aspects, Scaffold Y useful for the present disclosure comprises an amino acid sequence at least about 70%, at least about 75%, at least about 80%, at least about 85%, at least about 90%, at least about 95%, at least about 96%, at least about 97%, at least about 98%, at least about 99%, or about 100% identical to amino acids 2 to 227 of SEQ ID NO: 403. In other aspects, the Scaffold Y comprises an amino acid sequence at least about at least about 70%, at least about 75%, at least about 80%, at least about 85%, at least about 90%, at least about 95%, at least about 96%, at least about 97%, at least about 98%, at least about 99%, or about 100% identical to any one of SEQ ID NOs: 404-567. In other aspects, a Scaffold Y useful for the present disclosure comprises the amino acid sequence of SEQ ID NO: 403, except one amino acid mutation, two amino acid mutations, three amino acid mutations, four amino acid mutations, five amino acid mutations, six amino acid mutations, or seven amino acid mutations. In other aspects, a Scaffold Y useful for the present disclosure comprises the amino acid sequence of SEQ ID NO: 403 without Met at amino acid residue 1 of the SEQ ID NO: 403, except one amino acid mutation, two amino acid mutations, three amino acid mutations, four amino acid mutations, five amino acid mutations, six amino acid mutations, or seven amino acid mutations. The mutations can be a substitution, an insertion, a deletion, or any combination thereof. In some aspects, a Scaffold Y useful for the present disclosure comprises the amino acid sequence of any one of SEQ ID NOs: 404-567 and 1 amino acid, two amino acids, three amino acids, four amino acids, five amino acids, six amino acids, seven amino acids, eight amino acids, nine amino acids, ten amino acids, 11 amino acids, 12 amino acids, 13 amino acids, 14 amino acids, 15 amino acids, 16 amino acids, 17 amino acids, 18 amino acids, 19 amino acids, or 20 amino acids or longer at the N terminus and/or C terminus of SEQ ID NOs: 404-567.

In some aspects, the protein sequence of any of SEQ ID NOs: 404-567 is sufficient to be a Scaffold Y for the present disclosure (*e.g*., scaffold moiety linked to an ASO).

In some aspects, a Scaffold Y useful for the present disclosure comprises a peptide with the GXKLSKKK, where X is alanine or any other amino acid (SEQ ID NO: 404). In some aspects, an EV, *e.g.,* exosome, comprises a peptide with sequence of (G)(π)(ξ)(Φ/π)(S/A/G/N)(+)(+), wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). In further aspects, an exosome described herein (e.g., engineered exosome) comprises a peptide with sequence of (G)(π)(X)(Φ/π)(π)(+)(+), wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). See Aasland et al., FEBS Letters 513 (2002) 141-144 for amino acid nomenclature.

In other aspects, the Scaffold X comprises an amino acid sequence at least about 70%, at least about 75%, at least about 80%, at least about 85%, at least about 90%, at least about 95%, at least about 96%, at least about 97%, at least about 98%, at least about 99%, or about 100% identical to any one of SEQ ID NO: 404-567.

Scaffold Y-engineered EVs, *e.g*., exosomes described herein can be produced from a cell transformed with a sequence set forth in SEQ ID NOs: 404-567.

In some aspects, the Scaffold Y protein useful for the present disclosure comprises an "N-terminus domain" (ND) and an "effector domain"(ED), wherein the ND and/or the ED are associated with the luminal surface of the EV, *e.g.,* an exosome. In some aspects, the Scaffold Y protein useful for the present disclosure comprises an intracellular domain, a transmembrane domain, and an extracellular domain; wherein the intracellular domain comprises an "N-terminus domain" (ND) and an "effector domain" (ED), wherein the ND and/or the ED are associated with the luminal surface of the EV, *e.g.,* an exosome. As used herein the term "associated with" refers to the interaction between a scaffold protein with the luminal surface of the EV, *e.g*., and exosome, that does not involve covalent linking to a membrane component. For example, the scaffolds useful for the present disclosure can be associated with the luminal surface of the EV, *e.g*., via a lipid anchor (*e.g*., myristic acid), and/or a polybasic domain that interacts electrostatically with the negatively charged head of membrane phospholipids. In other aspects, the Scaffold Y protein comprises an N-terminus domain (ND) and an effector domain (ED), wherein the ND is associated with the luminal surface of the EV and the ED are associated with the luminal surface of the EV by an ionic interaction, wherein the ED comprises at least two, at least three, at least four, at least five, at least six, or at least seven contiguous basic amino acids, *e.g*., lysines (Lys), in sequence.

In other aspects, the Scaffold Y protein comprises an N-terminus domain (ND) and an effector domain (ED), wherein the ND is associated with the luminal surface of the EV, *e.g.,* exosome, and the ED is associated with the luminal surface of the EV by an ionic interaction, wherein the ED comprises at least two, at least three, at least four, at least five, at least six, or at least seven contiguous basic amino acids, *e.g*., lysines (Lys), in sequence.

In some aspects, the ND is associated with the luminal surface of the EV, *e.g.,* an exosome, via lipidation, *e.g*., via myristoylation. In some aspects, the ND has Gly at the N terminus. In some aspects, the N-terminal Gly is myristoylated.

In some aspects, the ED is associated with the luminal surface of the EV, *e.g*., an exosome, by an ionic interaction. In some aspects, the ED is associated with the luminal surface of the EV, *e.g.,* an exosome, by an electrostatic interaction, in particular, an attractive electrostatic interaction.

In some aspects, the ED comprises (i) a basic amino acid (*e.g*., lysine), or (ii) two or more basic amino acids (*e.g*., lysine) next to each other in a polypeptide sequence. In some aspects, the basic amino acid is lysine (Lys; K), arginine (Arg, R), or Histidine (His, H). In some aspects, the basic amino acid is (Lys)n, wherein n is an integer between 1 and 10.

In other aspects, the ED comprises at least a lysine and the ND comprises a lysine at the C terminus if the N terminus of the ED is directly linked to lysine at the C terminus of the ND, *i.e.,* the lysine is in the N terminus of the ED and is fused to the lysine in the C terminus of the ND. In other aspects, the ED comprises at least two lysines, at least three lysines, at least four lysines, at least five lysines, at least six lysines, or at least seven lysines when the N terminus of the ED is linked to the C terminus of the ND by a linker, e.g., one or more amino acids.

In some aspects, the ED comprises K, KK, KKK, KKKK (SEQ ID NO: 405), KKKKK (SEQ ID NO: 406), R, RR, RRR, RRRR (SEQ ID NO: 407); RRRRR (SEQ ID NO: 408), KR, RK, KKR, KRK, RKK, KRR, RRK, (K/R)(K/R)(K/R)(K/R) (SEQ ID NO: 409), (K/R)(K/R)(K/R)(K/R)(K/R) (SEQ ID NO: 410), or any combination thereof. In some aspects, the ED comprises KK, KKK, KKKK (SEQ ID NO: 405), KKKKK (SEQ ID NO: 406), or any combination thereof. In some aspects, the ND comprises the amino acid sequence as set forth in G:X2:X3:X4:X5:X6, wherein G represents Gly; wherein ":" represents a peptide bond; wherein each of the X2 to the X6 independently represents an amino acid; and wherein the X6 represents a basic amino acid. In some aspects, the X6 amino acid is selected is selected from the group consisting of Lys, Arg, and His. In some aspects, the X5 amino acid is selected from the group consisting of Pro, Gly, Ala, and Ser. In some aspects, the X2 amino acid is selected from the group consisting of Pro, Gly, Ala, and Ser. In some aspects, the X4 is selected from the group consisting of Pro, Gly, Ala, Ser, Val, Ile, Leu, Phe, Trp, Tyr, Gln, and Met.

In some aspects, the Scaffold Y protein comprises an N-terminus domain (ND) and an effector domain (ED), wherein the ND comprises the amino acid sequence as set forth in G:X2:X3:X4:X5:X6, wherein G represents Gly; wherein ":" represents a peptide bond; wherein each of the X2 to the X6 is independently an amino acid; wherein the X6 comprises a basic amino acid, and wherein the ED is linked to X6 by a peptide bond and comprises at least one lysine at the N terminus of the ED.

In some aspects, the ND of the Scaffold Y protein comprises the amino acid sequence of G:X2:X3:X4:X5:X6, wherein G represents Gly; ":" represents a peptide bond; the X2 represents an amino acid selected from the group consisting of Pro, Gly, Ala, and Ser; the X3 represents any amino acid; the X4 represents an amino acid selected from the group consisting of Pro, Gly, Ala, Ser,Val, Ile, Leu, Phe, Trp, Tyr, Gln, and Met; the X5 represents an amino acid selected from the group consisting of Pro, Gly, Ala, and Ser; and the X6 represents an amino acid selected from the group consisting of Lys, Arg, and His.

In some aspects, the X3 amino acid is selected from the group consisting of Asn, Gln, Ser, Thr, Asp, Glu, Lys, His, and Arg.

In some aspects, the ND and ED are joined by a linker. In some aspects, the linker comprises one or more amino acids. In some aspects, the term "linker" refers to a peptide or polypeptide sequence (*e.g*., a synthetic peptide or polypeptide sequence) or to a non-polypeptide, *e.g.,* an alkyl chain. In some aspects, two or more linkers can be linked in tandem. Generally, linkers provide flexibility or prevent/ameliorate steric hindrances. Linkers are not typically cleaved; however, in certain aspects, such cleavage can be desirable. Accordingly, in some aspects a linker can comprise one or more protease-cleavable sites, which can be located within the sequence of the linker or flanking the linker at either end of the linker sequence. When the ND and ED are joined by a linker, the ED comprise at least two lysines, at least three lysines, at least four lysines, at least five lysines, at least six lysines, or at least seven lysines.

In some aspects, the linker is a peptide linker. In some aspects, the peptide linker can comprise at least about two, at least about three, at least about four, at least about five, at least about 10, at least about 15, at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 amino acids.

In some aspects, the linker is a glycine/serine linker. In some aspects, the peptide linker is glycine/serine linker according to the formula [(Gly)n-Ser]m where n is any integer from 1 to 100 and m is any integer from 1 to 100. In other aspects, the glycine/serine linker is according to the formula [(Gly)x-Sery]z wherein x in an integer from 1 to 4, y is 0 or 1, and z is an integer from 1 to 50. In some aspects, the peptide linker comprises the sequence Gn, where n can be an integer from 1 to 100. In some aspects, the peptide linker can comprise the sequence (GlyAla)n, wherein n is an integer between 1 and 100. In other aspects, the peptide linker can comprise the sequence (GlyGlySer)n, wherein n is an integer between 1 and 100.

In some aspects, the peptide linker is synthetic, *i.e.,* non-naturally occurring. In one aspect, a peptide linker includes peptides (or polypeptides) (*e.g*., natural or non-naturally occurring peptides) which comprise an amino acid sequence that links or genetically fuses a first linear sequence of amino acids to a second linear sequence of amino acids to which it is not naturally linked or genetically fused in nature. For example, in one aspect the peptide linker can comprise non-naturally occurring polypeptides which are modified forms of naturally occurring polypeptides (*e.g.,* comprising a mutation such as an addition, substitution or deletion).

In other aspects, the peptide linker can comprise non-naturally occurring amino acids. In yet other aspects, the peptide linker can comprise naturally occurring amino acids occurring in a linear sequence that does not occur in nature. In still other aspects, the peptide linker can comprise a naturally occurring polypeptide sequence.

The present disclosure also provides an isolated extracellular vesicle (EV), *e.g.,* an exosome, comprising an ASO linked to a Scaffold Y protein, wherein the Scaffold Y protein comprises ND-ED, wherein: ND comprises G:X2:X3:X4:X5:X6; wherein: G represents Gly; ":" represents a peptide bond; X2 represents an amino acid selected from the group consisting of Pro, Gly, Ala, and Ser; X3 represents any amino acid; X4 represents an amino acid selected from the group consisting of Pro, Gly, Ala, Ser,Val, Ile, Leu, Phe, Trp, Tyr, Glu, and Met; X5 represents an amino acid selected from the group consisting of Pro, Gly, Ala, and Ser; X6 represents an amino acid selected from the group consisting of Lys, Arg, and His; "-" represents an optional linker; and ED is an effector domain comprising (i) at least two contiguous lysines (Lys), which is linked to the X6 by a peptide bond or one or more amino acids or (ii) at least one lysine, which is directly linked to the X6 by a peptide bond.

In some aspects, the X2 amino acid is selected from the group consisting of Gly and Ala. In some aspects, the X3 amino acid is Lys. In some aspects, the X4 amino acid is Leu or Glu. In some aspects, the X5 amino acid is selected from the group consisting of Ser and Ala. In some aspects, the X6 amino acid is Lys. In some aspects, the X2 amino acid is Gly, Ala, or Ser; the X3 amino acid is Lys or Glu; the X4 amino acid is Leu, Phe, Ser, or Glu; the X5 amino acid is Ser or Ala; and X6 amino acid is Lys. In some aspects, the "-" linker comprises a peptide bond or one or more amino acids.

In some aspects, the ED in the scaffold protein comprises Lys (K), KK, KKK, KKKK (SEQ ID NO: 405), KKKKK (SEQ ID NO: 406), Arg (R), RR, RRR, RRRR (SEQ ID NO: 407); RRRRR (SEQ ID NO: 408), KR, RK, KKR, KRK, RKK, KRR, RRK, (K/R)(K/R)(K/R)(K/R) (SEQ ID NO: 409), (K/R)(K/R)(K/R)(K/R)(K/R) (SEQ ID NO: 410), or any combination thereof.

In some aspects, the Scaffold Y protein comprises an amino acid sequence selected from the group consisting of (i) GGKLSKK (SEQ ID NO: 411), (ii) GAKLSKK (SEQ ID NO: 412), (iii) GGKQSKK (SEQ ID NO: 413), (iv) GGKLAKK (SEQ ID NO: 414), or (v) any combination thereof.

**In** some aspects, the ND in the Scaffold Y protein comprises an amino acid sequence selected from the group consisting of (i) GGKLSK (SEQ ID NO: 415), (ii) GAKLSK (SEQ ID NO: 416), (iii) GGKQSK (SEQ ID NO: 417), (iv) GGKLAK (SEQ ID NO: 418), or (v) any combination thereof and the ED in the scaffold protein comprises K, KK, KKK, KKKG (SEQ ID NO: 419), KKKGY (SEQ ID NO: 420), KKKGYN (SEQ ID NO: 421), KKKGYNV (SEQ ID NO: 422), KKKGYNVN (SEQ ID NO: 423), KKKGYS (SEQ ID NO: 424), KKKGYG (SEQ ID NO: 425), KKKGYGG (SEQ ID NO: 426), KKKGS (SEQ ID NO: 427), KKKGSG (SEQ ID NO: 428), KKKGSGS (SEQ ID NO: 429), KKKS (SEQ ID NO: 430), KKKSG (SEQ ID NO: 431), KKKSGG (SEQ ID NO: 432), KKKSGGS (SEQ ID NO: 433), KKKSGGSG (SEQ ID NO: 434), KKSGGSGG (SEQ ID NO: 435), KKKSGGSGGS (SEQ ID NO: 436), KRFSFKKS (SEQ ID NO: 437).

**In** some aspects, the polypeptide sequence of a Scaffold Y protein useful for the present disclosure consists of an amino acid sequence selected from the group consisting of (i) GGKLSKK (SEQ ID NO: 411), (ii) GAKLSKK (SEQ ID NO: 412), (iii) GGKQSKK (SEQ ID NO: 413), (iv) GGKLAKK (SEQ ID NO: 414), or (v) any combination thereof.

**In** some aspects, the Scaffold Y protein comprises an amino acid sequence selected from the group consisting of (i) GGKLSKKK (SEQ ID NO: 438), (ii) GGKLSKKS (SEQ ID NO: 439), (iii) GAKLSKKK (SEQ ID NO: 440), (iv) GAKLSKKS (SEQ ID NO: 441), (v) GGKQSKKK (SEQ ID NO: 442), (vi) GGKQSKKS (SEQ ID NO: 443), (vii) GGKLAKKK (SEQ ID NO: 444), (viii) GGKLAKKS (SEQ ID NO: 445), and (ix) any combination thereof.

**In** some aspects, the polypeptide sequence of a Scaffold Y protein useful for the present disclosure consists of an amino acid sequence selected from the group consisting of (i) GGKLSKKK (SEQ ID NO: 438), (ii) GGKLSKKS (SEQ ID NO: 439), (iii) GAKLSKKK (SEQ ID NO: 440), (iv) GAKLSKKS (SEQ ID NO: 441), (v) GGKQSKKK (SEQ ID NO: 442), (vi) GGKQSKKS (SEQ ID NO: 443), (vii) GGKLAKKK (SEQ ID NO: 444), (viii) GGKLAKKS (SEQ ID NO: 445), and (ix) any combination thereof.

**In** some aspects, the Scaffold Y protein is at least about 8, at least about 9, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 26, at least about 27, at least about 28, at least about 29, at least about 30, at least 31, at least about 32, at least about 33, at least about 34, at least about 35, at least about 36, at least about 37, at least about 38, at least about 39, at least about 39, at least about 40, at least about 41, at least about 42, at least about 43, at least about 44, at least about 50, at least about 46, at least about 47, at least about 48, at least about 49, at least about 50, at least about 55, at least about 60, at least about 65, at least about 70, at least about 75, at least about 80, at least 85, at least about 90, at least about 95, at least about 100, at least about 105, at least about 110, at least about 115, at least about 120, at least about 125, at least about 130, at least about 135, at least about 140, at least about 145, at least about 150, at least about 155, at least about 160, at least about 165, at least about 170, at least about 175, at least about 180, at least about 185, at least about 190, at least about 195, at least about 200, at least about 205, at least about 210, at least about 215, at least about 220, at least about 225, at least about 230, at least about 235, at least about 240, at least about 245, at least about 250, at least about 255, at least about 260, at least about 265, at least about 270, at least about 275, at least about 280, at least about 285, at least about 290, at least about 295, at least about 300, at least about 305, at least about 310, at least about 315, at least about 320, at least about 325, at least about 330, at least about 335, at least about 340, at least about 345, or at least about 350 amino acids in length.

**In** some aspects, the Scaffold Y protein is between about 5 and about 10, between about 10 and about 20, between about 20 and about 30, between about 30 and about 40, between about 40 and about 50, between about 50 and about 60, between about 60 and about 70, between about 70 and about 80, between about 80 and about 90, between about 90 and about 100, between about 100 and about 110, between about 110 and about 120, between about 120 and about 130, between about 130 and about 140, between about 140 and about 150, between about 150 and about 160, between about 160 and about 170, between about 170 and about 180, between about 180 and about 190, between about 190 and about 200, between about 200 and about 210, between about 210 and about 220, between about 220 and about 230, between about 230 and about 240, between about 240 and about 250, between about 250 and about 260, between about 260 and about 270, between about 270 and about 280, between about 280 and about 290, between about 290 and about 300, between about 300 and about 310, between about 310 and about 320, between about 320 and about 330, between about 330 and about 340, or between about 340 and about 350 amino acids in length.

**In** some aspects, the Scaffold Y protein comprises (i) GGKLSKKKKGYNVN (SEQ ID NO: 446), (ii) GAKLSKKKKGYNVN (SEQ ID NO: 447), (iii) GGKQSKKKKGYNVN (SEQ ID NO: 448), (iv) GGKLAKKKKGYNVN (SEQ ID NO: 449), (v) GGKLSKKKKGYSGG (SEQ ID NO: 450), (vi) GGKLSKKKKGSGGS (SEQ ID NO: 451), (vii) GGKLSKKKKSGGSG (SEQ ID NO: 452), (viii) GGKLSKKKSGGSGG (SEQ ID NO: 853), (ix) GGKLSKKSGGSGGS (SEQ ID NO: 484), (x) GGKLSKSGGSGGSV (SEQ ID NO: 855), or (xi) GAKKSKKRFSFKKS (SEQ ID NO: 456).

In some aspects, the polypeptide sequence of a Scaffold Y protein useful for the present disclosure consists of (i) GGKLSKKKKGYNVN (SEQ ID NO: 446), (ii) GAKLSKKKKGYNVN (SEQ ID NO: 447), (iii) GGKQSKKKKGYNVN (SEQ ID NO: 448), (iv) GGKLAKKKKGYNVN (SEQ ID NO: 449), (v) GGKLSKKKKGYSGG (SEQ ID NO: 450), (vi) GGKLSKKKKGSGGS (SEQ ID NO: 451), (vii) GGKLSKKKKSGGSG (SEQ ID NO: 452), (viii) GGKLSKKKSGGSGG (SEQ ID NO: 453), (ix) GGKLSKKSGGSGGS (SEQ ID NO: 454), (x) GGKLSKSGGSGGSV (SEQ ID NO: 455), or (xi) GAKKSKKRFSFKKS (SEQ ID NO: 456).

Non-limiting examples of the Scaffold Y protein useful for the present disclosure are disclosed herein. In some aspects, the Scaffold Y protein comprises an amino acid sequence selected from SEQ ID NOs: 411, 438, 446, and 455-567. In some aspects, the Scaffold Y protein consists of an amino acid sequence selected from SEQ ID NOs: 411, 438, 446, and 455-567.

In some aspects, the Scaffold Y protein useful for the present disclosure does not contain an N-terminal Met. In some aspects, the Scaffold Y protein comprises a lipidated amino acid, *e.g.,* a myristoylated amino acid, at the N-terminus of the scaffold protein, which functions as a lipid anchor. In some aspects, the amino acid residue at the N-terminus of the scaffold protein is Gly. The presence of an N-terminal Gly is an absolute requirement for N-myristoylation. In some aspects, the amino acid residue at the N-terminus of the scaffold protein is synthetic. In some aspects, the amino acid residue at the N-terminus of the scaffold protein is a glycine analog, *e.g.,* allylglycine, butylglycine, or propargylglycine.

### III.C. Targeting Moiety

In some aspects, the EV, *e.g.,* exosome, comprises a targeting moiety, *e.g.,* an exogenous targeting moiety. In some aspects, the exogenous targeting moiety comprises a peptide, an antibody or an antigen-binding fragment thereof, a chemical compound, an RNA aptamer, or any combination thereof. In some aspects, the targeting moiety comprises a microprotein, a designed ankyrin repeat protein (darpin), an anticalin, an adnectin, an aptamer, a peptide mimetic molecule, a natural ligand for a receptor, a camelid nanobody, or any combination thereof. In some aspects, the exogenous targeting moiety comprises a full-length antibody, a single domain antibody, a heavy chain only antibody (VHH), a single chain antibody, a shark heavy chain only antibody (VNAR), an scFv, a Fv, a Fab, a Fab', a F(ab')2, or any combination thereof. In some aspects, the antibody is a single chain antibody.

In some aspects, the targeting moiety targets the exosome to the liver, heart, lungs, brain, kidneys, central nervous system, peripheral nervous system, muscle, bone, joint, skin, intestine, bladder, pancreas, lymph nodes, spleen, blood, bone marrow, or any combination thereof. In some aspects, the targeting moiety targets the exosome to a tumor cell, dendritic cell, T cell, B cell, macrophage, neuron, hepatocyte, Kupffer cell, a myeloid-lineage cell (e.g., neutrophil, maonocyte, macrophage, or an MDSC *(e.g.,* a monocytic MDSC or a granulocytic MDSC)), hematopoietic stem cell, or any combination thereof.

In some aspects, the targeting moiety is linked to the EV, *e.g.,* the exosome, by a scaffold protein. In some aspects, the scaffold protein is any scaffold protein disclosed herein. In some aspects, the scaffold protein is a Scaffold X. In some aspects, the scaffold protein is a Scaffold Y.

### III.D. Linkers

As described *supra,* extracellular vesicles (EVs) of the present disclosure (*e.g.,* exosomes and nanovesicles) can comprises one or more linkers that link a molecule of interest (*e.g.,* an ASO) to the EVs (*e.g.,* to the exterior surface or on the luminal surface). In some aspects, an ASO is linked to the EVs directly or via a scaffold moiety (e.g., Scaffold X or Scaffold Y). In certain aspects, the ASO is linked to the scaffold moiety by a linker. In certain aspects, the ASO is linked to the second scaffold moiety by a linker.

In certain aspects, an ASO is linked to the exterior surface of an exosome via Scaffold X. In further aspects, an ASO is linked to the luminal surface of an exosome via Scaffold X or Scaffold Y. The linker can be any chemical moiety known in the art.

As used herein, the term "linker" refers to a peptide or polypeptide sequence (e.g., a synthetic peptide or polypeptide sequence) or to a non-polypeptide, e.g., an alkyl chain. In some aspects, two or more linkers can be linked in tandem. When multiple linkers are present, each of the linkers can be the same or different. Generally, linkers provide flexibility or prevent/ameliorate steric hindrances. Linkers are not typically cleaved; however, in certain aspects, such cleavage can be desirable. Accordingly, in some aspects, a linker can comprise one or more protease-cleavable sites, which can be located within the sequence of the linker or flanking the linker at either end of the linker sequence.

In some aspects, the linker is a peptide linker. In some aspects, the peptide linker can comprise at least about two, at least about three, at least about four, at least about five, at least about 10, at least about 15, at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 amino acids. v

**In** some aspects, the peptide linker is synthetic, *i.e.,* non-naturally occurring. In one aspect, a peptide linker includes peptides (or polypeptides) (*e.g.,* natural or non-naturally occurring peptides) which comprise an amino acid sequence that links or genetically fuses a first linear sequence of amino acids to a second linear sequence of amino acids to which it is not naturally linked or genetically fused in nature. For example, in one aspect the peptide linker can comprise non-naturally occurring polypeptides which are modified forms of naturally occurring polypeptides *(e.g.,* comprising a mutation such as an addition, substitution or deletion).

Linkers can be susceptible to cleavage ("cleavable linker") thereby facilitating release of the biologically active molecule (e.g., an ASO).

**In** some aspects, the linker is a "reduction-sensitive linker." In some aspects, the reduction-sensitive linker contains a disulfide bond. In some aspects, the linker is an "acid labile linker." In some aspects, the acid labile linker contains hydrazone. Suitable acid labile linkers also include, for example, a cis-aconitic linker, a hydrazide linker, a thiocarbamoyl linker, or any combination thereof.

**In** some aspects, the linker comprises a non-cleavable linker.

**In** some aspects, the linker comprises acrylic phosphoramidite (e.g., ACRYDITE^{™}), adenylation, azide (NHS Ester), digoxigenin (NHS Ester), cholesterol-TEG, I-LINKER^{™}, an amino modifier (e.g., amino modifier C6, amino modifier C12, amino modifier C6 dT, or Uni-Link^{™} amino modifier), alkyne, 5' Hexynyl, 5-Octadiynyl dU, biotinylation (e.g., biotin, biotin (Azide), biotin dT, biotin-TEG, dual biotin, PC biotin, or desthiobiotin), thiol modification (thiol modifier C3 S-S, dithiol or thiol modifier C6 S-S), or any combination thereof.

**In** some aspects, the linker comprises a terpene such as nerolidol, farnesol, limonene, linalool, geraniol, carvone, fenchone, or menthol; a lipid such as palmitic acid or myristic acid; cholesterol; oleyl; retinyl; cholesteryl residues; cholic acid; adamantane acetic acid; 1-pyrene butyric acid; dihydrotestosterone; 1,3-Bis-O(hexadecyl)glycerol; geranyloxyhexyl group; hexadecylglycerol; borneol; 1,3-propanediol; heptadecyl group; O3-(oleoyl)lithocholic acid; O3-(oleoyl)cholenic acid; dimethoxytrityl; phenoxazine, a maleimide moiety, a glucorinidase type, a CL2A-SN38 type, folic acid; a carbohydrate; vitamin A; vitamin E; vitamin K, or any combination thereof.

### III.E. Modified EVs Comprising Tropism Moieties

In some aspects, an EV, e.g., exosome, disclosed herein can be surface engineered to adjust its properties, e.g., biodistribution, e.g., via incorporation of immuno-affinity ligands or cognate receptor ligands. For example, EV, e.g., exosomes, disclosed herein can be surface engineered to direct them to a specific cellular type, e.g., Schwann cells, sensory neurons, motor neurons, meningeal macrophages, or a tumor cell, or can be surface engineered to enhance their migration to a specific compartment, e.g., to the CNS (in order to improve intrathecal compartment retention) or to a tumor microenvironment.

In some aspects, an EV, e.g., exosome, comprises (i) an ASO disclosed herein and (ii) a bio-distribution modifying agent or targeting moiety. In some aspects, the bio-distribution modifying agent or targeting moiety comprises a single-domain antigen-biding moiety, e.g., a VHH and/or a vNAR. As used here, the terms "bio-distribution modifying agent" and "targeting moiety" are used interchangeably and refer to an agent that can modify the distribution of extracellular vesicles (*e.g.,* exosomes, nanovesicles) *in vivo* or *in vitro* (*e.g.,* in a mixed culture of cells of different varieties). In some aspects, the targeting moiety alters the tropism of the EV (e.g., exosome), i.e., the target moiety is a "tropism moiety". As used herein, the term "tropism moiety" refers to a targeting moiety that when expressed on an EV (e.g., exosome) alters and/or enhances the natural movement of the EV. For example, in some aspects, a tropism moiety can promote the EV (e.g., exosome) to be taken up by a particular cell, tissue, or organ.

EVs, *e.g.,* exosomes, exhibit preferential uptake in discrete cell types and tissues, and their tropism can be directed by adding proteins to their surface that interact with receptors on the surface of target cells. The tropism moiety can comprise a biological molecule, such as a protein, a peptide, a lipid, or a carbohydrate, or a synthetic molecule. For example, in some aspects the tropism moiety can comprise an affinity ligand, *e.g.,* an antibody (such as an anti-CD19 nanobody, an anti-CD22 nanobody, an anti-CLEC9A nanobody, or an anti-CD3 nanobody), a VHH domain, a phage display peptide, a fibronectin domain, a camelid nanobody, and/or a vNAR. In some aspects, the tropism moiety can comprise, e.g., a synthetic polymer (e.g., PEG), a natural ligand/molecule (e.g., CD40L, albumin, CD47, CD24, CD55, CD59), and/or a recombinant protein (e.g., XTEN).

In some aspects, a tropism moiety can increase uptake of the EV, e.g., an exosome, by a cell. In some aspects, the tropism moiety that can increase uptake of the EV, e.g., an exosome, by a cell comprises a lymphocyte antigen 75 (also known as DEC205 or CD205), C-type lectin domain family 9 member A (CLEC9A), C-type lectin domain family 6 (CLEC6), C-type lectin domain family 4 member A (also known as DCIR or CLEC4A), Dendritic Cell-Specific Intercellular adhesion molecule-3-Grabbing Non-integrin (also known as DC-SIGN or CD209), lectin-type oxidized LDL receptor 1(LOX-1), macrophage receptor with collagenous structure (MARCO), C-type lectin domain family 12 member A (CLEC12A), C-type lectin domain family 10 member A (CLEC10A), DC-asialoglycoprotein receptor (DC-ASGPR), DC immunoreceptor 2 (DCIR2), Dectin-1, macrophage mannose receptor (MMR), BDCA-2 (CD303, CLEC4C), Dectin-2, BST-2 (CD317), Langerin, CD206, CD11b, CD11c, CD123, CD304, XCR1, AXL, SIGLEC 6, CD209, SIRPA, CX3CR1, GPR182, CD14, CD16, CD32, CD34, CD38, CD10, anti-CD3 antibody, or any combination thereof.

In some aspects, when tropism to the central nervous system is desired, an EV, e.g., exosome, of the present disclosure can comprise a tissue or cell-specific target ligand, which increases EV, e.g., exosome, tropism to a specific central nervous system tissue or cell. In some aspects, the cell is a glial cell. In some aspects, the glial cell is an oligodendrocyte, an astrocyte, an ependymal cell, a microglia cell, a Schwann cell, a satellite glial cell, an olfactory ensheathing cell, or a combination thereof. In some aspects, the cell is a neural stem cell. In some aspects, the cell-specific target ligand, which increases EV, e.g., exosome, tropism to a Schwann cells binds to a Schwann cell surface marker such as Myelin Basic Protein (MBP), Myelin Protein Zero (P0), P75NTR, NCAM, PMP22, or any combination thereof. In some aspects, the cell-specific tropism moiety comprises an antibody or an antigen-binding portion thereof, an aptamer, or an agonist or antagonist of a receptor expressed on the surface of the Schwann cell.

In some aspects, the bio-distribution modifying agent or targeting moiety comprises an antigen-binding moiety that binds an antigen expressed on a tumor cell. In some aspects, the bio-distribution modifying agent or targeting moiety comprises an antigen-binding moiety that binds an antigen expressed in a tumor microenvironment. In some aspects, the bio-distribution modifying agent or targeting moiety comprises an antigen-binding moiety that binds mesothelin. Any antigen-binding moiety known in the art that is capable of binding mesothelin can be used in the EVs disclosed herein. In some aspects, bio-distribution modifying agent or targeting moiety comprises an antigen-binding moiety that binds CD33. Any antigen-binding moiety known in the art that is capable of binding CD33 can be used in the EVs disclosed herein. In certain aspects, the antigen-binding moiety that binds CD33 is selected from the anti-CD33 binding moieties disclosed in US Patent No. 5,877,296.

In principle, the EVs, e.g., exosomes of the present disclosure comprising at least one tropism moiety that can direct the EV, e.g., exosome, to a specific target cell or tissue (e.g., a cell in the CNS or a Schwann cell in peripheral nerves) can be administered using any suitable administration method known in the art (e.g., intravenous injection or infusion) since the presence of the tropism moiety (alone or in combination with the presence of an antiphagocytic signal such as CD47 and the use of a specific administration route) will induce a tropism of the EVs, e.g., exosomes, towards the desired target cell or tissue.

In certain aspects, the tropism moiety is linked, e.g., chemically linked via a maleimide moiety, to a scaffold moiety, e.g., a Scaffold X protein or a fragment thereof, on the exterior surface of the EV, e.g., exosome. Tropism can be further improved by the attachment of an anti-phagocytic signal (e.g., CD47 and/or CD24), a half-life extension moiety (e.g., albumin or PEG), or any combination thereof to the external surface of an EV, e.g., exosome of the present disclosure. In certain aspects, the anti-phagocytic signal is linked, e.g., chemically linked via a maleimide moiety, to a scaffold moiety, e.g., a Scaffold X protein or a fragment thereof, on the exterior surface of the EV, e.g., exosome.

Pharmacokinetics, biodistribution, and in particular tropism and retention in the desired tissue or anatomical location can also be accomplished by selecting the appropriate administration route (e.g., intrathecal administration or intraocular administration to improve tropism to the central nervous system).

In some aspects, the EV, e.g., exosome, comprises at least two different tropism moieties. In some aspects, the EV, e.g., exosome, comprises three different tropism moieties. In some aspects, the EV, e.g., exosome, comprises four different tropism moieties. In some aspects, the EV, e.g., exosome, comprises five or more different tropism moieties. In some aspects, one or more of the tropism moieties increases uptake of the EV, e.g., exosome, by a cell. In some aspects, each tropism moiety is attached to a scaffold moiety, e.g., a Scaffold X protein or a fragment thereof. In some aspects, multiple tropism moieties can be attached to the same scaffold moiety, e.g., a Scaffold X protein or a fragment thereof. In some aspects, several tropism moieties can be attached in tandem to a scaffold moiety, e.g., a Scaffold X protein or a fragment thereof. In some aspects, a tropism moiety disclosed herein or a combination thereof is attached to a scaffold moiety, e.g., a Scaffold X protein or a fragment thereof, via a linker or spacer. In some aspects, a linker or spacer or a combination thereof is interposed between two tropism moieties disclosed herein.

Non-limiting examples of tropism moieties capable of directing EVs, e.g., exosomes, of the present disclosure to different nervous system cell types are disclosed below.

### III.E.1. Tropism moieties targeting Schwann cells

In some aspects, a tropism moiety can target a Schwann cell. In some aspects, the tropism moiety that directs an EV, e.g., exosome, disclosed herein to a Schwann cell targets, e.g., a transferrin receptor (TfR), apolipoprotein D (ApoD), Galectin 1 (LGALS1), Myelin proteolipid protein (PLP), Glypican 1, or Syndecan 3. In some aspects, the tropism moiety directing an EV, e.g., exosome, of the present disclosure to a Schwann cell is a transferrin, or a fragment, variant or derivative thereof.

In some aspects, a tropism moiety of the present disclosure targets a transferrin receptor (TfR). Transferrin receptors, e.g., TfR1 or TfR2, are carrier proteins for transferrin. Transferrin receptors import iron by internalizing the transferrin-ion complex through receptor-mediated endocytosis.

TfR1 (see, e.g., UniProt P02786 TFR1_Human) or transferrin receptor 1 (also known as cluster of differentiation 71 or CD71) is expressed on the endothelial cells of the blood-brain barrier (BBB). TfR1 is known to be expressed in a variety of cells such as red blood cells, monocytes, hepatocytes, intestinal cells, and erythroid cells, and is upregulated in rapidly dividing cells such as tumor cells (non small cell lung cancer, colon cancer, and leukemia) as well as in tissue affected by disorders such as acute respiratory distress syndrome (ARDS). TfR2 is primarily expressed in liver and erythroid cells, is found to a lesser extent in lung, spleen and muscle, and has a 45% identity and 66% similarity with TfR1. TfR1 is a transmembrane receptor that forms a homodimer of 760 residues with disulfide bonds and a molecular weight of 90 kDa. Affinity for transferrin varies between the two receptor types, with the affinity for TfR1 being at least 25-30 fold higher than that of TfR2.

Binding to TfR1 allows the transit of large molecules, e.g., antibodies, into the brain. Some TfR1-targeting antibodies have been shown to cross the blood-brain barrier, without interfering with the uptake of iron. Amongst those are the mouse anti rat-TfR antibody OX26 and the rat anti mouse-TfR antibody 8D3. The affinity of the antibody-TfR interaction is important to determine the success of transcytotic transport over endothelial cells of the BBB. Monovalent TfR interaction favors BBB transport due to altered intracellular sorting pathways. Avidity effects of bivalent interactions redirecting transport to the lysosome. Also, reducing TfR binding affinity directly promote dissociation from the TfR which increase brain parenchymal exposure of the TfR binding antibody. See, e.g., U.S. Patent No. 8,821,943. Accordingly, in some aspects, a tropism moiety of the present disclosure can comprise a ligand that can target TfR, e.g., target TfR1, such as transferrin, or an antibody or other binding molecule capable of specifically binding to TfR. In some aspects, the antibody targeting a transferrin receptor is a low affinity anti-transferring receptor antibody (see, e.g., US20190202936A1).

In some aspects, the tropism moiety comprises all or a portion (e.g., a binding portion) of a ligand for a transferrin receptor, for example a human transferrin available in GenBank as Accession numbers NM001063, XM002793, XM039847, NM002343 or NM013900, among others, or a variant, fragment, or derivative thereof.

In some aspects, the tropism moiety comprises a transferrin-receptor-targeting moiety, i.e., a targeting moiety directed to a transferrin receptor. Suitable transferrin-receptor-targeting moieties include a transferrin or transferrin variant, such as, but not limited to, a serum transferrin, lacto transferrin (lactoferrin) ovotransferrin, or melanotransferrin. Transferrins are a family of nonheme iron-binding proteins found in vertebrates, including serum transferrins, lacto transferrins (lactoferrins), ovotransferrins, and melanotransferrins. Serum transferrin is a glycoprotein with a molecular weight of about 80 kDa, comprising a single polypeptide chain with two N-linked polysaccharide chains that are branched and terminate in multiple antennae, each with terminal sialic acid residues. There are two main domains, the N domain of about 330 amino acids, and the C domain of about 340 amino acids, each of which is divided into two subdomains, N1 and N2, and C1 and C2. Receptor binding of transferrin occurs through the C domain, regardless of glycosylation.

In some aspects, the tropism moiety is a serum transferrin or transferrin variant such as, but not limited to a hexasialo transferrin, a pentasialo transferrin, a tetrasialo transferrin, a trisialo transferrin, a disialo transferrin, a monosialo transferrin, or an asialo transferrin, or a carbohydrate-deficient transferrin (CDT) such as an asialo, monosialo or disialo transferrin, or a carbohydrate-free transferrin (CFT) such as an asialo transferrin. In some aspects, the tropism moiety is a transferrin variant having the N-terminal domain of transferrin, the C-terminal domain of transferrin, the glycosylation of native transferrin, reduced glycosylation as compared to native (wild-type) transferrin, no glycosylation, at least two N terminal lobes of transferrin, at least two C terminal lobes of transferrin, at least one mutation in the N domain, at least one mutation in the C domain, a mutation wherein the mutant has a weaker binding avidity for transferrin receptor than native transferrin, and/or a mutation wherein the mutant has a stronger binding avidity for transferrin receptor than native transferrin, or any combination of the foregoing.

In some aspects, the tropism moiety targeting a transferrin receptor comprises an anti-trasferrin receptor variable new antigen receptor (vNAR), e.g., a binding domain with a general motif structure (FW1-CDR1-FW2-3-CDR3-FW4). See, e.g., U.S. 2017-0348416. vNARs are key component of the adaptive immune system of sharks. At only 11 kDa, these single-domain structures are the smallest IgG-like proteins in the animal kingdom and provide an excellent platform for molecular engineering and biologics drug discovery. vNAR attributes include high affinity for target, ease of expression, stability, solubility, multi-specificity, and increased potential for solid tissue penetration. See Ubah et al. Biochem. Soc. Trans. (2018) 46(6):1559-1565.

In some aspects, the tropism moiety comprises a vNAR domain capable of specifically binding to TfR1, wherein the vNAR domain comprises or consists essentially of a vNAR scaffold with any one CDR1 peptide in Table 1 of U.S. 2017-0348416 in combination with any one CDR3 peptide in Table 1 of U.S. 2017-0348416.

In some aspects, a tropism moiety of the present disclosure targets ApoD. Unlike other lipoproteins, which are mainly produced in the liver, apolipoprotein D is mainly produced in the brain, cerebellum, and peripheral nerves. ApoD is 169 amino acids long, including a secretion peptide signal of 20 amino acids. It contains two glycosylation sites (aspargines 45 and 78) and the molecular weight of the mature protein varies from 20 to 32 kDa. ApoD binds steroid hormones such as progesterone and pregnenolone with a relatively strong affinity, and to estrogen with a weaker affinity. Arachidonic acid (AA) is an ApoD ligand with a much better affinity than that of progesterone or pregnenolone. Other ApoD ligands include E-3-methyl-2-hexenoic acid, retinoic acid, sphingomyelin and sphingolipids. Accordingly, in some aspects, a tropism moiety of the present disclosure comprises a ligand that can target ApoD, e.g., an antibody or other binding molecule capable of specifically binding to ApoD.

In some aspects, a tropism moiety of the present disclosure targets Galectin 1. The galectin-1 protein is 135 amino acids in length. Accordingly, in some aspects, a tropism moiety of the present disclosure comprises a ligand that can target Galectin 1, e.g., an antibody or other binding molecule capable of specifically binding to Galectin 1.

In some aspects, a tropism moiety of the present disclosure targets PLP. PLP is the major myelin protein from the CNS. It plays an important role in the formation or maintenance of the multilamellar structure of myelin. The myelin sheath is a multi-layered membrane, unique to the nervous system that functions as an insulator to greatly increase the efficiency of axonal impulse conduction. PLP is a highly conserved hydrophobic protein of 276 to 280 amino acids which contains four transmembrane segments, two disulfide bonds and which covalently binds lipids (at least six palmitate groups in mammals). Accordingly, in some aspects, a tropism moiety of the present disclosure comprises a ligand that can target PLP, e.g., an antibody or other binding molecule capable of specifically binding to PLP.

**In** some aspects, a tropism moiety of the present disclosure targets Glypican 1. Accordingly, in some aspects, a tropism moiety of the present disclosure comprises a ligand that can target Glypican 1, e.g., an antibody or other binding molecule capable of specifically binding to Glypican 1. In some aspects, a tropism moiety of the present disclosure targets Syndecan 3. Accordingly, in some aspects, a tropism moiety of the present disclosure comprises a ligand that can target Syndecan 3, e.g., an antibody or other binding molecule capable of specifically binding.

### III.E.2. Tropism moieties targeting sensory neurons

**In** some aspects, a tropism moiety disclosed herein can direct an EV, e.g., exosome, disclosed herein to a sensory neuron. In some aspects, the tropism moiety that directs an EV, e.g., exosome, disclosed herein to a sensory neuron targets a Trk receptor, e.g., TrkA, TrkB, TrkC, or a combination thereof.

Trk (tropomyosin receptor kinase) receptors are a family of tyrosine kinases that regulates synaptic strength and plasticity in the mammalian nervous system. The common ligands of Trk receptors are neurotrophins, a family of growth factors critical to the functioning of the nervous system. The binding of these molecules is highly specific. Each type of neurotrophin has different binding affinity toward its corresponding Trk receptor. Accordingly, in some aspects, the tropism moiety directing an EV, e.g., exosome, disclosed herein to a sensory neuron, comprises a neurotrophin.

Neurotrophins bind to Trk receptors as homodimers. Accordingly, in some aspects, the tropism moiety comprises at least two neurotrophins disclosed herein, e.g., in tandem. In some aspects, the tropism moiety comprises at least two neurotrophins disclosed herein, e.g., in tandem, that are attached to a scaffold protein, for example, Protein X, via a linker. In some aspects, the linker connecting the scaffold protein, e.g., Protein X, to the neurotrophin (e.g., a neurotrophin homodimer) has a length of at least 10 amino acids. In some aspects, the linker connecting the scaffold protein, e.g., Protein X, to the neurotrophin (e.g., a neurotrophin homodimer) has a length of at least about 25 amino acids, about 30 amino acids, about 35 amino acids, about 40 amino acids, about 45 amino acids, or about 50 amino acids.

**In** some aspects, the neurotrophin is a neurotrophin precursor, i.e., a proneurotrophin, which is later cleaved to produce a mature protein.

Nerve growth factor (NGF) is the first identified and probably the best characterized member of the neurotrophin family. It has prominent effects on developing sensory and sympathetic neurons of the peripheral nervous system. Brain-derived neurotrophic factor (BDNF) has neurotrophic activities similar to NGF, and is expressed mainly in the CNS and has been detected in the heart, lung, skeletal muscle and sciatic nerve in the periphery (Leibrock, J. et al., Nature, 341:149-152 (1989)). Neurotrophin-3 (NT-3) is the third member of the NGF family and is expressed predominantly in a subset of pyramidal and granular neurons of the hippocampus, and has been detected in the cerebellum, cerebral cortex and peripheral tissues such as liver and skeletal muscles (Ernfors, P. et al., Neuron 1: 983-996 (1990)). Neurotrophin-4 (also called NT-415) is the most variable member of the neurotrophin family. Neurotrophin-6 (NT-5) was found in teleost fish and binds to p75 receptor.

In some aspects, the neurotrophin targeting TrkB comprises, e.g., NT-4 or BDNF, or a fragment, variant, or derivative thereof. In some aspects, the neurotrophin targeting TrkA comprises, e.g., NGF or a fragment, variant, or derivative thereof. In some aspects, the neurotrophin targeting TrkC comprises, e.g., NT-3 or a fragment, variant, or derivative thereof.

In some aspects, the tropism moiety comprises brain derived neurotrophic factor (BDNF). In some aspects, the BDNF is a variant of native BDNF, such as a two amino acid carboxyl-truncated variant. In some aspects, the tropism moiety comprises the full-length 119 amino acid sequence of BDNF (HSDPARRGELSVCDSISEWVTAADKKTAVDMSGGTVTVLEKVPVSKGQLKQYFYETKCNPM GYTKEGCRGIDKRHWNSQCRTTQSYVRALTMDSKKRIGWRFIRIDTSCVCTLTIKRGR; SEQ ID NO: 161). In some aspects, a one amino-acid carboxy-truncated variant of BDNF is utilized (amino acids 1-118 of SEQ ID NO: 161).

In some aspects, the tropism moiety comprises a carboxy-truncated variant of the native BDNF, e.g., a variant in which 1, 2, 3, 4, 5, 6, 7, 8, 9, 10, or more than 10 amino acids are absent from the carboxy-terminus of the BDNF. BDNF variants include the complete 119 amino acid BDNF, the 117 or 118 amino acid variant with a truncated carboxyl terminus, variants with a truncated amino terminus, or variants with up to about 20%, about 30, or about 40% change in amino acid composition, as long as the protein variant still binds to the TrkB receptor with high affinity.

In some aspects, the tropism moiety comprises a two amino-acid carboxy-truncated variant of BDNF (amino acids 1-117 of SEQ ID NO: 161). In some aspects, the tropism moiety comprises a three amino-acid carboxy-truncated variant of BDNF (amino acids 1-116 of SEQ ID NO: 161). In some aspects, the tropism moiety comprises a four amino-acid carboxy-truncated variant of BDNF (amino acids 1-115 of SEQ ID NO: 161). In some aspects, the tropism moiety comprises a five amino-acid carboxy-truncated variant of BDNF (amino acids 1-114 of SEQ ID NO: 161). In some aspects, the tropism moiety comprises a BDNF that is at least about 60%, at least about 65%, at least about 70%, at least about 75%, at least about 80%, at least about 85%, at least about 90%, at least about 95%, at least about 99%, or about 100% identical with the sequence of SEQ ID NO: 161, or a truncated version thereof, e.g., the 117 or 118 amino acid variant with a one- or two-amino acid truncated carboxyl terminus, or variants with a truncated amino terminus. See, e.g., U.S. Pat. No. 8,053,569B2.

In some aspects, the tropism moiety comprises nerve growth factor (NGF). In some aspects, the NGF is a variant of native NGF, such as a truncated variant. In some aspects, the tropism moiety comprises the 26-kDa beta subunit of protein, the only component of the 7S NGF complex that is biologically active. In some aspects, the tropism moiety comprises the full-length 120 amino acid sequence of beta NGF (SSSHPIFHRGEFSVCDSVSVWVGDKTTATDIKGKEVMVLGEVNINNSVFKQYFFETKCRDPNP VDSGCRGIDSKHWNSYCTTTHTFVKALTMDGKQAAWRFIRIDTACVCVLSRKAVRRA; SEQ ID NO: 162). In some aspects, the tropism moiety comprises a carboxy-truncated variant of the native NGF, e.g., a variant in which 1, 2, 3, 4, 5, 6, 7, 8, 9, 10, or more than 10 amino acids are absent from the carboxy-terminus of NGF. NGF variants include the complete 120 amino acid NGF, the shorter amino acid variants with a truncated carboxyl terminus, variants with a truncated amino terminus, or variants with up to about 20%, about 30%, or about 40% change in amino acid composition, as long as the tropism moiety still binds to the TrkB receptor with high affinity. In some aspects, the tropism moiety comprises an NGF that is at least about 60%, at least about 65%, at least about 70%, at least about 75%, at least about 80%, at least about 85%, at least about 90%, at least about 95%, at least about 99%, or about 100% identical with the sequence of SEQ ID NO: 162, or a truncated version thereof.

In some aspects, the tropism moiety comprises neurotrophin-3 (NT-3). In some aspects, the NT-3 is a variant of native NT-3, such as a truncated variant. In some aspects, the tropism moiety comprises the full-length 119 amino acid sequence of NT-3 (YAEHKSHRGEYSVCDSESLWVTDKSSAIDIRGHQVTVLGEIKTGNSPVKQYFYETRCKEARPV KNGCRGIDDKHWNSQCKTSQTYVRALTSENNKLVGWRWIRIDTSCVCALSRKIGRT; SEQ ID NO: 163). In some aspects, the tropism moiety comprises a carboxy-truncated variant of the native NT-3, e.g., a variant in which 1, 2, 3, 4, 5, 6, 7, 8, 9, 10, or more than 10 amino acids are absent from the carboxy-terminus of NT-3. NT-3 variants include the complete 119 amino acid NT-3, the shorter amino acid variants with a truncated carboxyl terminus, variants with a truncated amino terminus, or variants with up to about 20%, about 30%, or about 40% change in amino acid composition, as long as the tropism moiety still binds to the TrkC receptor with high affinity. In some aspects, the tropism moiety comprises an NT-3 that is at least about 60%, at least about 65%, at least about 70%, at least about 75%, at least about 80%, at least about 85%, at least about 90%, at least about 95%, at least about 99%, or about 100% identical with the sequence of SEQ ID NO: 163, or a truncated version thereof.

In some aspects, the tropism moiety comprises neurotrophin-4 (NT-4). In some aspects, the NT-4 is a variant of native NT-4, such as a truncated variant. In some aspects, the tropism moiety comprises the full-length 130 amino acid sequence of NT-4 (GVSETAPASRRGELAVCDAVSGWVTDRRTAVDLRGREVEVLGEVPAAGGSPLRQYFFETRCK ADNAEEGGPGAGGGGCRGVDRRHWVSECKAKQSYVRALTADAQGRVGWRWIRIDTACVCTL LSRTGRA; SEQ ID NO: 164). In some aspects, the tropism moiety comprises a carboxy-truncated variant of the native NT-4, e.g., a variant in which 1, 2, 3, 4, 5, 6, 7, 8, 9, 10, or more than 10 amino acids are absent from the carboxy-terminus of NT-4. NT-4 variants include the complete 130 amino acid NT-4, the shorter amino acid variants with a truncated carboxyl terminus, variants with a truncated amino terminus, or variants with up to about 20%, about 30%, or about 40% change in amino acid composition, as long as the tropism moiety still binds to the TrkB receptor with high affinity. In some aspects, the tropism moiety comprises an NT-4 that is at least about 60%, at least about 65%, at least about 70%, at least about 75%, at least about 80%, at least about 85%, at least about 90%, at least about 95%, at least about 99%, or about 100% identical with the sequence of SEQ ID NO: 164, or a truncated version thereof.

Structure/function relationship studies of NGF and NGF-related recombinant molecules demonstrated that mutations in NGF region 25-36, along with other β-hairpin loop and non-loop regions, significantly influenced NGF/NGF-receptor interactions (Ibanez et al., EMBO J., 10, 2105-2110, (1991)). Small peptides derived from this region have been demonstrated to mimic NGF in binding to Mock receptor and affecting biological responses (LeSauteur et al. J. Biol. Chem. 270, 6564-6569, 1995). Dimers of cyclized peptides corresponding to β-loop regions of NGF were found to act as partial NGF agonists in that they had both survival-promoting and NGF-inhibiting activity while monomer and linear peptides were inactive (Longo et al., J. Neurosci. Res., 48, 1-17, 1997). Accordingly, in some aspects, a tropism moiety of the present disclosure comprises such peptides.

Cyclic peptides have also been designed and synthesized to mimic the β-loop regions of NGF, BDNF, NT3 and NT-4/5. Certain monomers, dimers or polymers of these cyclic peptides can have a three-dimensional structure, which binds to neurotrophin receptors under physiological conditions. All of these structural analogs of neurotrophins that bind to nerve cell surface receptors and are internalized can serve as the binding agent B of the compound according to the present disclosure to deliver the conjugated therapeutic moiety TM to the nervous system. Accordingly, in some aspects, a tropism moiety of the present disclosure comprises such cyclic peptides or combinations thereof.

In some aspects, antibodies against nerve cell surface receptors that are capable of binding to the receptors and being internalized can also serve as tropism moieties binding to a Trk receptor. For example, monoclonal antibody (MAb) 5C3 is specific for the NGF docking site of the human p140 TrkA receptor, with no cross-reactivity with human TrkB receptor. MAb 5C3 and its Fab mimic the effects of NGF in vitro, and image human Trk-A positive tumors in vivo (Kramer et al., Eur. J. Cancer, 33, 2090-2091, (1997)). Molecular cloning, recombination, mutagenesis and modeling studies of Mab 5C3 variable region indicated that three or less of its complementarity determining regions (CDRs) are relevant for binding to TrkA. Assays with recombinant CDRs and CDR-like synthetic polypeptides demonstrated that they had agonistic bioactivities similar to intact Mab 5C3. Monoclonal antibody MC192 against p75 receptor has also been demonstrated to have neurotrophic effects. Therefore, these antibodies and their functionally equivalent fragments can also serve as tropism moieties of the present disclosure.

In some aspects, peptidomimetics that are synthesized by incorporating unnatural amino acids or other organic molecules can also serve tropism moieties of the present disclosure.

Other neurotrophins are known in the art. Accordingly, in some aspects, the target moiety comprises a neurotrophin selected from the group consisting of fibroblast growth factor (FGF)-2 and other FGFs, erythropoietin (EPO), hepatocyte growth factor (HGF), epidermal growth factor (EGF), transforming growth factor (TGF)-a, TGF-(3, vascular endothelial growth factor (VEGF), interleukin-1 receptor antagonist (IL- lra), ciliary neurotrophic factor (CNTF), glial-derived neurotrophic factor (GDNF), neurturin, platelet-derived growth factor (PDGF), heregulin, neuregulin, artemin, persephin, interleukins, granulocyte-colony stimulating factor (CSF), granulocyte-macrophage-CSF, netrins, cardiotrophin-1, hedgehogs, leukemia inhibitory factor (LIF), midlcine, pleiotrophin, bone morphogenetic proteins (BMPs), netrins, saposins, semaphorins, and stem cell factor (SCF).

In some aspects, the tropism moiety directing an EV, e.g., exosome, disclosed herein to a sensory neuron, comprises a varicella zoster virus (VZV) peptide.

### III.E.3. Tropism moieties targeting motor neurons

In some aspects, a tropism moiety disclosed herein can direct an EV, e.g., exosome, disclosed herein to a motor neuron. In some aspects, the tropism moiety that directs an EV, e.g., exosome, disclosed herein to a motor comprises a Rabies Virus Glycoprotein (RVG) peptide, a Targeted Axonal Import (TAxI) peptide, a P75R peptide, or a Tet-C peptide.

In some aspects, the tropism moiety comprises a Rabies Virus Glycoprotein (RVG) peptide. See, e.g., U.S. Pat. App. Publ. 2014-00294727. In some aspects, the RVG peptide comprises amino acid residues 173-202 of the RVG (YTIWMPENPRPGTPCDIFTNSRGKRASNG; SEQ ID NO: 601) or a variant, fragment, or derivative thereof. In some aspects, the tropism moiety is a fragment of SEQ ID NO: 601. Such a fragment of SEQ ID NO: 601 can have, for example, 1, 2, 3, 4, 5, 6, 7, 8, 9, or 10 amino acids deleted from the N-terminal and/or the C-terminal of SEQ ID NO: 601. A functional fragment derived from SEQ ID NO: 601 can be identified by sequentially deleting N- and/or C-terminal amino acids from SEQ ID NO: 601 and assessing the function of the resulting peptide fragment, such as function of the peptide fragment to bind acetylcholine receptor and/or ability to transmit through the blood brain barrier. In some aspects, the tropism moiety comprises a fragment of SEQ ID NO: 601 28, 27, 26, 25, 24, 23, 22, 21, 20, 19, 18, 17, 16 or 15 amino acids in length. In some aspects, the tropism moiety comprises a fragment of SEQ ID NO: 601 less than 15 peptides in length.

A "variant" of a RGV peptide, for example SEQ ID NO: 601, is meant to refer to a molecule substantially similar in structure and function, i.e., where the function is the ability to pass or transit through the BBB, to either the entire molecule, or to a fragment thereof. A variant of an RVG peptide can contain a mutation or modification that differs from a reference amino acid in SEQ ID NO: 601. In some aspects, a variant of SEQ ID NO: 601 is a fragment of SEQ ID NO: 601 as disclosed herein. In some aspects, an RVG variant can be a different isoform of SEQ ID NO: 601 or can comprise different isomer amino acids. Variants can be naturally-occurring, synthetic, recombinant, or chemically modified polynucleotides or polypeptides isolated or generated using methods well known in the art. RVG variants can include conservative or non-conservative amino acid changes. See, e.g., U.S. Pat. No. 9,757,470.

In some aspects, the tropism moiety comprises a Targeted Axonal Import (TAxI) peptide. In some aspects, the TAxI peptide is cyclized TAxI peptide of sequence SACQSQSQMRCGGG (SEQ ID NO: 602). See, e.g., Sellers et al. (2016) Proc. Natl. Acad. Sci. USA 113:2514-2519, and U.S. Pat. No. 9,056,892. TAxI transport peptides as described herein may be of any length. Typically, the transport peptide will be between 6 and 50 amino acids in length, more typically between 10 and 20 amino acids in length. In some aspects, the TAxI transport peptide comprises the amino acid sequence QSQSQMR (SEQ ID NO: 603), ASGAQAR (SEQ ID NO: 604), PF, or TSTAPHLRLRLTSR (SEQ ID NO: 605). Optionally, the TAxI transport peptide further includes a flanking sequence to facilitate incorporation into a delivery construct or carrier, e.g., a linker. In one aspect, the peptide is flanked with cysteines. In some aspects, the TAxI transport peptide further comprises additional sequence selected to facilitate delivery into nuclei. For example, a peptide that facilitates nuclear delivery is a nuclear localizing signal (NLS). Typically, this signal consists of a few short sequences of positively charged lysines or arginines, such as PPKKRKV (SEQ ID NO: 606). In one aspect, the NLS has the amino acid sequence PKKRKV (SEQ ID NO: 607).

In some aspects, a tropism moiety of the present disclosure comprises a peptide BBB shuttle having a sequence selected from SEQ ID NOs: 608-627 (below) and any combination thereof. See, e.g., Oller-Salvia et al. (2016) Chem. Soc. Rev. 45, 4690-4707, and Jafari et al. (2019) Expert Opinion on Drug Delivery 16:583-605.

| SEQ ID NO | Peptide | Sequence |
|---|---|---|
| 608 | Angiopep-2 | TFFYGGSRGKRNNFKTEEY-*OH* |
| 609 | ApoB (3371-3409) | SSVIDALQYKLEGTTRLTRK-RGLKLATALSLSNKFVEGS |
| 610 | ApoE (159-167)₂ | (LRKLRKRLL)₂ |
| 611 | Peptide-22 | *Ac*-C(&)MPRLRGC(&)-*NH₂* |
| 612 | THR | THRPPMWSPVWP-*NH₂* |
| 613 | THR retro-enantio | pwvpswmpprht*-NH₂* |
| 614 | CRT | C(&)RTIGPSVC(&) |
| 615 | Leptin30 | YQQILTSMPSRNVIQISND-LENLRDLLHVL |
| 616 | RVG29 | YTIWMPENPRPGTPCDIFT-NSRGKRASNG-*OH* |
| 617 | ^{D}CDX | GreirtGraerwsekf-OH |
| 618 | Apamin | C(&₁)NC(&₂)KAPETALC(&₁)-AR-RC(&₂)QQH-*NH₂* |
| 619 | MiniAp-4 | [Dap](&)KAPETALD(&) |
| 620 | GSH | γ-L-glutamyl-CG-OH |
| 621 | G23 | HLNILSTLWKYRC |
| 622 | g7 | GFtGFLS(*O*-β-Glc)-*NH₂* |
| 623 | TGN | TGNYKALHPHNG |
| 624 | TAT (47-57) | YGRKKRRQRRR-*NH₂* |
| 625 | SynB1 | RGGRLSYSRRRFSTSTGR |
| 626 | Diketopiperazines | &(*N*-MePhe)-(*N*-MePhe)Diketo-piperazines |
| 627 | PhPro | (Phenylproline)₄-*NH₂* |

Nomenclature for cyclic peptides (&) is adapted to the 3-letter amino acid code from the one described by Spengler et al-. Pept. Res., 2005, 65, 550-555

### [Dap] stands for diaminopropionic acid.

### III.F. Anti-phagocytic Signal

Clearance of administered EVs, e.g., exosomes, by the body's immune system can reduce the efficacy of an administered EV, e.g., exosome, therapy. In some aspects, the surface of the EV, e.g., exosome, is modified to limit or block uptake of the EV, e.g., exosome, by cells of the immune system, e.g., macrophages. In some aspects, the surface of the EV, e.g., exosome, is modified to express one or more surface antigen that inhibits uptake of the EV, e.g., exosome, by a macrophage. In some aspects, the surface antigen is associated with the exterior surface of the EV, (e.g., exosome).

Surface antigens useful in the present disclosure include, but are not limited to, antigens that label a cell as a "self" cell. In some aspects, the surface antigen comprises an anti-phagocytic signal. In some aspects, the anti-phagocytic signal is selected from CD47, CD24, a fragment thereof, and any combination thereof. In certain aspects, the anti-phagocytic signal comprises CD24, e.g., human CD24. In some aspects, the anti-phagocytic signal comprises a fragment of CD24, e.g., human CD24. In certain aspects, the EV, e.g., exosome, is modified to express CD47 or a fragment thereof on the exterior surface of the EV, e.g., exosome.

CD47, also referred to as leukocyte surface antigen CD47 and integrin associated protein (IAP), as used herein, is a transmembrane protein that is found on many cells in the body. CD47 is often referred to as the "don't eat me" signal, as it signals to immune cells, in particular myeloid cells, that a particular cell expressing CD47 is not a foreign cell. CD47 is the receptor for SIRPA, binding to which prevents maturation of immature dendritic cells and inhibits cytokine production by mature dendritic cells. Interaction of CD47 with SIRPG mediates cell-cell adhesion, enhances superantigen-dependent T-cell-mediated proliferation and costimulates T-cell activation. CD47 is also known to have a role in both cell adhesion by acting as an adhesion receptor for THBS1 on platelets, and in the modulation of integrins. CD47 also plays an important role in memory formation and synaptic plasticity in the hippocampus (by similarity). In addition, CD47 can play a role in membrane transport and/or integrin dependent signal transduction, prevent premature elimination of red blood cells, and be involved in membrane permeability changes induced following virus infection.

In some aspects, an EV, e.g., exosome, disclosed herein is modified to express a human CD47 on the surface of the EV, e.g., exosome. The canonical amino acid sequence for human CD47 and various known isoforms (UniProtKB - Q08722) are provided herein as SEQ ID NOs: 629-632. In some aspects, the EV, e.g., exosome, is modified to express a polypeptide comprising the amino acid sequence set forth in SEQ ID NO: 629 or a fragment thereof. In some aspects, the EV, e.g., exosome, is modified to express a polypeptide comprising the amino acid sequence set forth in SEQ ID NO: 630 or a fragment thereof. In some aspects, the EV, e.g., exosome, is modified to express a polypeptide comprising the amino acid sequence set forth in SEQ ID NO: 631 or a fragment thereof. In some aspects, the EV, e.g., exosome, is modified to express a polypeptide comprising the amino acid sequence set forth in SEQ ID NO: 632 or a fragment thereof.

In some aspects, the EV, e.g., exosome, is modified to express full length CD47 on the surface of the EV, *e.g.,* exosome. In some aspects, the EV, *e.g.,* exosome, is modified to express a fragment of CD47 on the surface of the EV, e.g., exosome, wherein the fragment comprises the extracellular domain of CD47, e.g., human CD47. Any fragment of CD47 that retains an ability to block and/or inhibit phagocytosis by a macrophage can be used in the EVs, e.g., exosomes, disclosed herein. In some aspects, the fragment comprises amino acids 19 to about 141 of the canonical human CD47 sequence (e.g., amino acids 19-141 of SEQ ID NO 629). In some aspects, the fragment comprises amino acids 19 to about 135 of the canonical human CD47 sequence (e.g., amino acids 19-135 of SEQ ID NO 629). In some aspects, the fragment comprises amino acids 19 to about 130 of the canonical human CD47 sequence (e.g., amino acids 19-130 of SEQ ID NO 629). In some aspects, the fragment comprises amino acids 19 to about 125 of the canonical human CD47 sequence (e.g., amino acids 19-125 of SEQ ID NO 629).

In some aspects, the EV, e.g., exosome, is modified to express a polypeptide having at least about 70%, at least about 75%, at least about 80%, at least about 85%, at least about 90%, at least about 95%, at least about 96%, at least about 97%, at least about 98%, or at least about 99% sequence identity to amino acids 19 to about 141 of the canonical human CD47 sequence (e.g., amino acids 19-141 of SEQ ID NO 629). In some aspects, the EV, e.g., exosome, is modified to express a polypeptide having at least about 70%, at least about 75%, at least about 80%, at least about 85%, at least about 90%, at least about 95%, at least about 96%, at least about 97%, at least about 98%, or at least about 99% sequence identity to amino acids 19 to about 135 of the canonical human CD47 sequence (e.g., amino acids 19-135 of SEQ ID NO 629). In some aspects, the EV, e.g., exosome, is modified to express a polypeptide having at least about 70%, at least about 75%, at least about 80%, at least about 85%, at least about 90%, at least about 95%, at least about 96%, at least about 97%, at least about 98%, or at least about 99% sequence identity to amino acids 19 to about 130 of the canonical human CD47 sequence (e.g., amino acids 19-130 of SEQ ID NO 629). In some aspects, the EV, e.g., exosome, is modified to express a polypeptide having at least about 70%, at least about 75%, at least about 80%, at least about 85%, at least about 90%, at least about 95%, at least about 96%, at least about 97%, at least about 98%, or at least about 99% sequence identity to amino acids 19 to about 125 of the canonical human CD47 sequence (e.g., amino acids 19-125 of SEQ ID NO 629).

In some aspects, the CD47 or the fragment thereof is modified to increase the affinity of CD47 and its ligand SIRPα. In some aspects, the fragment of CD47 comprises a Velcro-CD47 (see, e.g., Ho et al., JBC 290:12650-63 (2015)). In some aspects, the Velcro-CD47 comprises a C15S substitution relative to the wild-type human CD47 sequence (SEQ ID NO: 629).

In some aspects, the EV, e.g., exosome, comprises a CD47 or a fragment thereof expressed on the surface of the EV, e.g., exosome, at a level that is higher than an unmodified EV, e.g., exosome. In some aspects, the CD47 or the fragment thereof is fused with a scaffold protein. Any scaffold protein disclosed herein can be used to express the CD47 or the fragment thereof on the surface of the EV, e.g., exosome. In some aspects, the EV, e.g., exosome, is modified to express a fragment of CD47 fused to the N-terminus of a Scaffold X protein. In some aspects, the EV, e.g., exosome, is modified to express a fragment of CD47 fused to the N-terminus of PTGFRN.

In some aspects, the EV, e.g., exosome, comprises at least about 20 molecules, at least about 30 molecules, at least about 40, at least about 50, at least about 75, at least about 100, at least about 125, at least about 150, at least about 200, at least about 250, at least about 300, at least about 350, at least about 400, at least about 450, at least about 500, at least about 750, or at least about 1000 molecules of CD47 on the surface of the EV, e.g., exosome. In some aspects, the EV, e.g., exosome, comprises at least about 20 molecules of CD47 on the surface of the EV, e.g., exosome. In some aspects, the EV, e.g., exosome, comprises at least about 30 molecules of CD47 on the surface of the EV, e.g., exosome. In some aspects, the EV, e.g., exosome, comprises at least about 40 molecules of CD47 on the surface of the EV, e.g., exosome. In some aspects, the EV, e.g., exosome, comprises at least about 50 molecules of CD47 on the surface of the EV, e.g., exosome. In some aspects, the EV, e.g., exosome, comprises at least about 100 molecules of CD47 on the surface of the EV, e.g., exosome. In some aspects, the EV, e.g., exosome, comprises at least about 200 molecules of CD47 on the surface of the EV, e.g., exosome. In some aspects, the EV, e.g., exosome, comprises at least about 300 molecules of CD47 on the surface of the EV, e.g., exosome. In some aspects, the EV, e.g., exosome, comprises at least about 400 molecules of CD47 on the surface of the EV, e.g., exosome. In some aspects, the EV, e.g., exosome, comprises at least about 500 molecules of CD47 on the surface of the EV, e.g., exosome. In some aspects, the EV, e.g., exosome, comprises at least about 1000 molecules of CD47 on the surface of the EV, e.g., exosome.

In some aspects, expression CD47 or a fragment thereof on the surface of the EV, e.g., exosome, results in decreased uptake of the EV, e.g., exosome, by myeloid cells as compared to an EV, e.g., exosome, not expressing CD47 or a fragment thereof. In some aspects, uptake by myeloid cells of the EV, e.g., exosome, expressing CD47 or a fragment thereof is decreased by at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, or at least about 95%, relative to uptake by myeloid cells of EVs, e.g., exosomes, that do not express CD47 or a fragment thereof.

In some aspects, expression CD47 or a fragment thereof on the surface of the EV, e.g., exosome, results in decreased localization of the EV, e.g., exosome, to the liver, as compared to an EV, e.g., exosome, not expressing CD47 or a fragment thereof. In some aspects, localization to the liver of EVs, e.g., exosomes, expressing CD47 or a fragment thereof is decreased by at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, or at least about 95%, relative to the localization to the liver of EVs, e.g., exosomes, not expressing CD47 or a fragment thereof.

In some aspects, the in vivo half-life of an EV, e.g., exosome, expressing CD47 or a fragment thereof is increased relative to the in vivo half-life of an EV, e.g., exosome, that does not express CD47 or a fragment thereof. In some aspects, the in vivo half-life of an EV, e.g., exosome, expressing CD47 or a fragment thereof is increased by at least about 1.5-fold, at least about 2-fold, at least about 2.5-fold, at least about 3-fold, at least about 3.5-fold, at least about 4-fold, at least about 4.5-fold, at least about 5-fold, at least about 6-fold, at least about 7-fold, at least about 8-fold, at least about 9-fold, or at least about 10-fold, relative to the in vivo half-life of an EV, e.g., exosome, that does not express CD47 or a fragment thereof.

In some aspects, an EV, e.g., exosome, expressing CD47 or a fragment thereof has an increased retention in circulation, e.g., plasma, relative to the retention of an EV, e.g., exosome, that does not express CD47 or a fragment thereof in circulation, e.g., plasma. In some aspects, retention in circulation, e.g., plasma, of an EV, e.g., exosome, expressing CD47 or a fragment thereof is increased by at least about 1.5-fold, at least about 2-fold, at least about 2.5-fold, at least about 3-fold, at least about 3.5-fold, at least about 4-fold, at least about 4.5-fold, at least about 5-fold, at least about 6-fold, at least about 7-fold, at least about 8-fold, at least about 9-fold, or at least about 10-fold, relative to the retention in circulation, e.g., plasma, of an EV, e.g., exosome, that does not express CD47 or a fragment thereof.

In some aspects, an EV, e.g., exosome, expressing CD47 or a fragment thereof has an altered biodistribution when compared with an exosome that does not express CD47 or a fragment. In some aspects, the altered biodistribution leads to increased uptake into endothelial cells, T cells, or increased accumulation in various tissues, including, but not limited to skeletal muscle, cardiac muscle, diaphragm, kidney, bone marrow, central nervous system, lungs, cerebral spinal fluid (CSF), or any combination thereof.

### IV. Producer Cell for Production of Engineered Exosomes

EVs, e.g., exosomes, of the present disclosure can be produced from a cell grown *in vitro* or a body fluid of a subject. When exosomes are produced from *in vitro* cell culture, various producer cells, e.g., HEK293 cells, CHO cells, and MSCs, can be used. In certain aspects, a producer cell is not a dendritic cell, macrophage, B cell, mast cell, neutrophil, Kupffer-Browicz cell, cell derived from any of these cells, or any combination thereof.

Human embryonic kidney 293 cells, also often referred to as HEK 293, HEK-293, 293 cells, or less precisely as HEK cells, are a specific cell line originally derived from human embryonic kidney cells grown in tissue culture.

HEK 293 cells were generated in 1973 by transfection of cultures of normal human embryonic kidney cells with sheared adenovirus 5 DNA in Alex van der Eb's laboratory in Leiden, the Netherlands. The cells were cultured and transfected by adenovirus. Subsequent analysis has shown that the transformation was brought about by inserting ~4.5 kilobases from the left arm of the viral genome, which became incorporated into human chromosome 19.

A comprehensive study of the genomes and transcriptomes of HEK 293 and five derivative cell lines compared the HEK 293 transcriptome with that of human kidney, adrenal, pituitary and central nervous tissue. The HEK 293 pattern most closely resembled that of adrenal cells, which have many neuronal properties.

HEK 293 cells have a complex karyotype, exhibiting two or more copies of each chromosome and with a modal chromosome number of 64. They are described as hypotriploid, containing less than three times the number of chromosomes of a haploid human gamete. Chromosomal abnormalities include a total of three copies of the X chromosome and four copies of chromosome 17 and chromosome 22.

Variants of HEK293 cells useful to produce EVs include, but are not limited to, HEK 293F, HEK 293FT, and HEK 293T.

The producer cell can be genetically modified to comprise exogenous sequences encoding an ASO to produce EVs described herein. The genetically-modified producer cell can contain the exogenous sequence by transient or stable transformation. The exogenous sequence can be transformed as a plasmid. In some aspects, the exogenous sequence is a vector. The exogenous sequences can be stably integrated into a genomic sequence of the producer cell, at a targeted site or in a random site. In some aspects, a stable cell line is generated for production of lumen-engineered exosomes.

The exogenous sequences can be inserted into a genomic sequence of the producer cell, located within, upstream (5'-end) or downstream (3'-end) of an endogenous sequence encoding an exosome protein. Various methods known in the art can be used for the introduction of the exogenous sequences into the producer cell. For example, cells modified using various gene editing methods (e.g., methods using a homologous recombination, transposon-mediated system, loxP-Cre system, CRISPR/Cas9 or TALEN) are within the scope of the present disclosure.

The exogenous sequences can comprise a sequence encoding a scaffold moiety disclosed herein or a fragment or variant thereof. An extra copy of the sequence encoding a scaffold moiety can be introduced to produce an exosome described herein (e.g., having a higher density of a scaffold moiety on the surface or on the luminal surface of the EV, e.g., exosome). An exogenous sequence encoding a modification or a fragment of a scaffold moiety can be introduced to produce a lumen-engineered and/or surface-engineered exosome containing the modification or the fragment of the scaffold moiety.

In some aspects, a producer cell can be modified, e.g., transfected, with one or more vectors encoding a scaffold moiety linked to an ASO.

In some aspects, EVs, *e.g.,* exosomes, of the present disclosure *(e.g.,* surface-engineered and/or lumen-engineered exosomes) can be produced from a cell transformed with a sequence encoding a full-length, mature scaffold moiety disclosed herein or a scaffold moiety linked to an ASO. Any of the scaffold moieties described herein can be expressed from a plasmid, an exogenous sequence inserted into the genome or other exogenous nucleic acid, such as a synthetic messenger RNA (mRNA).

### V. Pharmaceutical Compositions

Provided herein are pharmaceutical compositions comprising an EV, e.g., exosome, of the present disclosure having the desired degree of purity, and a pharmaceutically acceptable carrier or excipient, in a form suitable for administration to a subject. Pharmaceutically acceptable excipients or carriers can be determined in part by the particular composition being administered, as well as by the particular method used to administer the composition. Accordingly, there is a wide variety of suitable formulations of pharmaceutical compositions comprising a plurality of extracellular vesicles. *(See, e.g.,* Remington's Pharmaceutical Sciences, Mack Publishing Co., Easton, Pa. 21st ed. (2005)). The pharmaceutical compositions are generally formulated sterile and in full compliance with all Good Manufacturing Practice (GMP) regulations of the U.S. Food and Drug Administration.

In some aspects, a pharmaceutical composition comprises one or more therapeutic agents and an exosome described herein. In certain aspects, the EVs, e.g., exosomes, are co-administered with one or more additional therapeutic agents in a pharmaceutically acceptable carrier. In some aspects, the ASO and the one or more additional therapeutic agents for the present disclosure can be administered in the same EV. In other aspects, the ASO and the one or more additional therapeutic agents for the present disclosure are administered in different EVs. For example, the present disclosure includes a pharmaceutical composition comprising an EV comprising an ASO and an EV comprising an additional therapeutic agent. In some aspects, the pharmaceutical composition comprising the EV, e.g., exosome, is administered prior to administration of the additional therapeutic agent(s). In other aspects, the pharmaceutical composition comprising the EV, e.g., exosome, is administered after the administration of the additional therapeutic agent(s). In further aspects, the pharmaceutical composition comprising the EV, e.g., exosome, is administered concurrently with the additional therapeutic agent(s).

Acceptable carriers, excipients, or stabilizers are nontoxic to recipients (e.g., animals or humans) at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

Examples of carriers or diluents include, but are not limited to, water, saline, Ringer's solutions, dextrose solution, and 5% human serum albumin. The use of such media and compounds for pharmaceutically active substances is well known in the art. Except insofar as any conventional media or compound is incompatible with the extracellular vesicles described herein, use thereof in the compositions is contemplated. Supplementary therapeutic agents can also be incorporated into the compositions. Typically, a pharmaceutical composition is formulated to be compatible with its intended route of administration. The EVs, e.g., exosomes, can be administered by parenteral, topical, intravenous, oral, subcutaneous, intra-arterial, intradermal, transdermal, rectal, intracranial, intraperitoneal, intranasal, intratumoral, intramuscular route or as inhalants. In certain aspects, the pharmaceutical composition comprising exosomes is administered intravenously, *e.g.* by injection. The EVs, *e.g.,* exosomes, can optionally be administered in combination with other therapeutic agents that are at least partly effective in treating the disease, disorder or condition for which the EVs, e.g., exosomes, are intended.

Solutions or suspensions can include the following components: a sterile diluent such as water, saline solution, fixed oils, polyethylene glycols, glycerine, propylene glycol or other synthetic solvents; antibacterial compounds such as benzyl alcohol or methyl parabens; antioxidants such as ascorbic acid or sodium bisulfite; chelating compounds such as ethylenediaminetetraacetic acid (EDTA); buffers such as acetates, citrates or phosphates, and compounds for the adjustment of tonicity such as sodium chloride or dextrose. The pH can be adjusted with acids or bases, such as hydrochloric acid or sodium hydroxide. The preparation can be enclosed in ampoules, disposable syringes or multiple dose vials made of glass or plastic.

Pharmaceutical compositions suitable for injectable use include sterile aqueous solutions (if water soluble) or dispersions and sterile powders. For intravenous administration, suitable carriers include physiological saline, bacteriostatic water, Cremophor EL^{™} (BASF, Parsippany, N.J.) or phosphate buffered saline (PBS). The composition is generally sterile and fluid to the extent that easy syringeability exists. The carrier can be a solvent or dispersion medium containing, *e.g.,* water, ethanol, polyol *(e.g.,* glycerol, propylene glycol, and liquid polyethylene glycol, and the like), and suitable mixtures thereof. The proper fluidity can be maintained, e.g., by the use of a coating such as lecithin, by the maintenance of the required particle size in the case of dispersion and by the use of surfactants. Prevention of the action of microorganisms can be achieved by various antibacterial and antifungal compounds, e.g., parabens, chlorobutanol, phenol, ascorbic acid, thimerosal, and the like. If desired, isotonic compounds, e.g., sugars, polyalcohols such as manitol, sorbitol, and sodium chloride can be added to the composition. Prolonged absorption of the injectable compositions can be brought about by including in the composition a compound which delays absorption, e.g., aluminum monostearate and gelatin.

Sterile injectable solutions can be prepared by incorporating the EVs, e.g., exosomes, in an effective amount and in an appropriate solvent with one or more ingredients enumerated herein or known in the art, as desired. Generally, dispersions are prepared by incorporating the EVs, e.g., exosomes, into a sterile vehicle that contains a basic dispersion medium and any desired other ingredients. In the case of sterile powders for the preparation of sterile injectable solutions, methods of preparation are vacuum drying and freeze-drying that yield a powder of the active ingredient plus any additional desired ingredient from a previously sterile-filtered solution thereof. The EVs, e.g., exosomes, can be administered in the form of a depot injection or implant preparation which can be formulated in such a manner to permit a sustained or pulsatile release of the EV, e.g., exosome.

Systemic administration of compositions comprising exosomes can also be by transmucosal means. For transmucosal administration, penetrants appropriate to the barrier to be permeated are used in the formulation. Such penetrants are generally known in the art, and include, *e.g.,* for transmucosal administration, detergents, bile salts, and fusidic acid derivatives. Transmucosal administration can be accomplished through the use of, *e.g.,* nasal sprays.

In certain aspects the pharmaceutical composition comprising EVs, e.g., exosomes is administered intravenously into a subject that would benefit from the pharmaceutical composition. In certain other aspects, the composition is administered to the lymphatic system, *e.g.,* by intralymphatic injection or by intranodal injection (*see e.g.,* Senti et al., PNAS 105(46): 17908 (2008)), or by intramuscular injection, by subcutaneous administration, by intratumoral injection, by direct injection into the thymus, or into the liver.

In certain aspects, the pharmaceutical composition comprising exosomes is administered as a liquid suspension. In certain aspects, the pharmaceutical composition is administered as a formulation that is capable of forming a depot following administration. In certain preferred aspects, the depot slowly releases the EVs, e.g., exosomes, into circulation, or remains in depot form.

Typically, pharmaceutically-acceptable compositions are highly purified to be free of contaminants, are biocompatible and not toxic, and are suited to administration to a subject. If water is a constituent of the carrier, the water is highly purified and processed to be free of contaminants, e.g., endotoxins.

The pharmaceutically-acceptable carrier can be lactose, dextrose, sucrose, sorbitol, mannitol, starch, gum acacia, calcium phosphate, alginates, gelatin, calcium silicate, microcrystalline cellulose, polyvinylpyrrolidone, cellulose, water, syrup, methyl cellulose, methylhydroxy benzoate, propylhydroxy benzoate, talc, magnesium stearate, and/or mineral oil, but is not limited thereto. The pharmaceutical composition can further include a lubricant, a wetting agent, a sweetener, a flavor enhancer, an emulsifying agent, a suspension agent, and/or a preservative.

In some aspects, the pharmaceutical compositions described herein comprise a pharmaceutically acceptable salt. In some aspects, the pharmaceutically acceptable salt comprises a sodium salt, a potassium salt, an ammonium salt, or any combination thereof.

The pharmaceutical compositions described herein comprise the EVs, e.g., exosomes, described herein and optionally an additional pharmaceutically active or therapeutic agent. The additional therapeutic agent can be a biological agent, a small molecule agent, or a nucleic acid agent. In some aspects, the additional therapeutic agent is an additional STAT6 antagonist. In some aspects, the STAT6 antagonist is any STAT6 antagonist disclosed herein. In some aspects, the additional STAT6 antagonist is an anti-STAT6 antibody. In some aspects, the additional STAT6 antagonist is a small molecule. In some aspects, the additional STAT6 antagonist is a small molecule.

In some aspects, the additional STAT6 antagonist comprises an ASO. In some aspects, the additional STAT6 antagonist comprises any ASO described herein.

Dosage forms are provided that comprise a pharmaceutical composition comprising the EVs, e.g., exosomes, described herein. In some aspects, the dosage form is formulated as a liquid suspension for intravenous injection. In some aspects, the dosage form is formulated as a liquid suspension for intratumoral injection.

In certain aspects, the preparation of exosomes is subjected to radiation, *e.g.,* X rays, gamma rays, beta particles, alpha particles, neutrons, protons, elemental nuclei, UV rays in order to damage residual replication-competent nucleic acids.

**In** certain aspects, the preparation of exosomes is subjected to gamma irradiation using an irradiation dose of more than 1, 5, 10, 15, 20, 25, 30, 35, 40, 50, 60, 70, 80, 90, 100, or more than 100 kGy.

**In** certain aspects, the preparation of exosomes is subjected to X-ray irradiation using an irradiation dose of more than 0.1, 0.5, 1, 5, 10, 15, 20, 25, 30, 35, 40, 50, 60, 70, 80, 90, 100, 200, 300, 400, 500, 600, 700, 800, 900, 1000, 2000, 3000, 4000, 5000, 6000, 7000, 8000, 9000, 10000, or greater than 10000 mSv.

### VI. Kits

Also provided herein are kits comprising one or more exosomes described herein. **In** some aspects, provided herein is a pharmaceutical pack or kit comprising one or more containers filled with one or more of the ingredients of the pharmaceutical compositions described herein, such as one or more exosomes provided herein, optional an instruction for use. **In** some aspects, the kits contain a pharmaceutical composition described herein and any prophylactic or therapeutic agent, such as those described herein. **In** some aspects, the kit further comprises instructions to administer the EV according to any method disclosed herein. **In** some aspects, the kit is for use in the treatment of a disease or condition associated with hematopoiesis. **In** some aspects, the kit is a diagnostic kit.

### VII. Methods of Producing EVs

**In** some aspects, the present disclosure is also directed to methods of producing EVs described herein. **In** some aspects, the method comprises: obtaining the EV, e.g., exosome from a producer cell, wherein the producer cell contains one or more components of the EV, e.g., exosome *(e.g.,* an ASO); and optionally isolating the obtained EV, *e.g.,* exosome. **In** some aspects, the method comprises: modifying a producer cell by introducing one or more components of an EV disclosed herein *(e.g.,* an ASO); obtaining the EV, *e.g.,* exosome, from the modified producer cell; and optionally isolating the obtained EV, *e.g.,* exosome. **In** further aspects, the method comprises: obtaining an EV from a producer cell; isolating the obtained EV; and modifying the isolated EV. **In** certain aspects, the method further comprises formulating the isolated EV into a pharmaceutical composition.

### VII.A. Methods of Modifying a Producer Cell

As described *supra,* in some aspects, a method of producing an EV comprises modifying a producer cell with one or more moieties (*e.g*., an ASO). In certain aspects, the one or more moieties comprise an ASO. In some aspects, the one or more moieties further comprise a scaffold moiety disclosed herein (e.g., Scaffold X or Scaffold Y).

In some aspects, the producer cell can be a mammalian cell line, a plant cell line, an insect cell line, a fungi cell line, or a prokaryotic cell line. In certain aspects, the producer cell is a mammalian cell line. Non-limiting examples of mammalian cell lines include: a human embryonic kidney (HEK) cell line, a Chinese hamster ovary (CHO) cell line, an HT-1080 cell line, a HeLa cell line, a PERC-6 cell line, a CEVEC cell line, a fibroblast cell line, an amniocyte cell line, an epithelial cell line, a mesenchymal stem cell (MSC) cell line, and combinations thereof. In certain aspects, the mammalian cell line comprises HEK-293 cells, BJ human foreskin fibroblast cells, fHDF fibroblast cells, AGE.HN^{®} neuronal precursor cells, CAP^{®} amniocyte cells, adipose mesenchymal stem cells, RPTEC/TERT1 cells, or combinations thereof. In some aspects, the producer cell is a primary cell. In certain aspects, the primary cell can be a primary mammalian cell, a primary plant cell, a primary insect cell, a primary fungi cell, or a primary prokaryotic cell.

In some aspects, the producer cell is not an immune cell, such as an antigen presenting cell, a T cell, a B cell, a natural killer cell (NK cell), a macrophage, a T helper cell, or a regulatory T cell (Treg cell). In other aspects, the producer cell is not an antigen presenting cell (e.g., dendritic cells, macrophages, B cells, mast cells, neutrophils, Kupffer-Browicz cell, or a cell derived from any such cells).

In some aspects, the one or more moieties can be a transgene or mRNA, and introduced into the producer cell by transfection, viral transduction, electroporation, extrusion, sonication, cell fusion, or other methods that are known to the skilled in the art.

In some aspects, the one or more moieties is introduced to the producer cell by transfection. In some aspects, the one or more moieties can be introduced into suitable producer cells using synthetic macromolecules, such as cationic lipids and polymers (Papapetrou et al., Gene Therapy 12: S118-S130 (2005)). In some aspects, the cationic lipids form complexes with the one or more moieties through charge interactions. In some of these aspects, the positively charged complexes bind to the negatively charged cell surface and are taken up by the cell by endocytosis. In some other aspects, a cationic polymer can be used to transfect producer cells. In some of these aspects, the cationic polymer is polyethylenimine (PEI). In certain aspects, chemicals such as calcium phosphate, cyclodextrin, or polybrene, can be used to introduce the one or more moieties to the producer cells. The one or more moieties can also be introduced into a producer cell using a physical method such as particle-mediated transfection, "gene gun", biolistics, or particle bombardment technology (Papapetrou et al., Gene Therapy 12: S118-S130 (2005)). A reporter gene such as, for example, beta-galactosidase, chloramphenicol acetyltransferase, luciferase, or green fluorescent protein can be used to assess the transfection efficiency of the producer cell.

In certain aspects, the one or more moieties are introduced to the producer cell by viral transduction. A number of viruses can be used as gene transfer vehicles, including moloney murine leukemia virus (MMLV), adenovirus, adeno-associated virus (AAV), herpes simplex virus (HSV), lentiviruses, and spumaviruses. The viral mediated gene transfer vehicles comprise vectors based on DNA viruses, such as adenovirus, adeno-associated virus and herpes virus, as well as retroviral based vectors.

In certain aspects, the one or more moieties are introduced to the producer cell by electroporation. Electroporation creates transient pores in the cell membrane, allowing for the introduction of various molecules into the cell. In some aspects, DNA and RNA as well as polypeptides and non-polypeptide therapeutic agents can be introduced into the producer cell by electroporation.

In certain aspects, the one or more moieties introduced to the producer cell by microinjection. In some aspects, a glass micropipette can be used to inject the one or more moieties into the producer cell at the microscopic level.

In certain aspects, the one or more moieties are introduced to the producer cell by extrusion.

In certain aspects, the one or more moieties are introduced to the producer cell by sonication. In some aspects, the producer cell is exposed to high intensity sound waves, causing transient disruption of the cell membrane allowing loading of the one or more moieties.

In certain aspects, the one or more moieties are introduced to the producer cell by cell fusion. In some aspects, the one or more moieties are introduced by electrical cell fusion. In other aspects, polyethylene glycol (PEG) is used to fuse the producer cells. In further aspects, sendai virus is used to fuse the producer cells.

In some aspects, the one or more moieties are introduced to the producer cell by hypotonic lysis. In such aspects, the producer cell can be exposed to low ionic strength buffer causing them to burst allowing loading of the one or more moieties. In other aspects, controlled dialysis against a hypotonic solution can be used to swell the producer cell and to create pores in the producer cell membrane. The producer cell is subsequently exposed to conditions that allow resealing of the membrane.

In some aspects, the one or more moieties are introduced to the producer cell by detergent treatment. In certain aspects, producer cell is treated with a mild detergent which transiently compromises the producer cell membrane by creating pores allowing loading of the one or more moieties. After producer cells are loaded, the detergent is washed away thereby resealing the membrane.

**In** some aspects, the one or more moieties introduced to the producer cell by receptor mediated endocytosis. In certain aspects, producer cells have a surface receptor which upon binding of the one or more moieties induces internalization of the receptor and the associated moieties.

**In** some aspects, the one or more moieties are introduced to the producer cell by filtration. In certain aspects, the producer cells and the one or more moieties can be forced through a filter of pore size smaller than the producer cell causing transient disruption of the producer cell membrane and allowing the one or more moieties to enter the producer cell.

**In** some aspects, the producer cell is subjected to several freeze thaw cycles, resulting in cell membrane disruption allowing loading of the one or more moieties.

### VII.B. Methods of Modifying EV, e.g., Exosome

**In** some aspects, a method of producing an EV, e.g., exosome, comprises modifying the isolated EV by directly introducing one or more moieties into the EVs. In certain aspects, the one or more moieties comprise an ASO. In some aspects, the one or more moieties comprise a scaffold moiety disclosed herein (e.g., Scaffold X or Scaffold Y).

**In** certain aspects, the one or more moieties are introduced to the EV by transfection. In some aspects, the one or more moieties can be introduced into the EV using synthetic macromolecules such as cationic lipids and polymers (Papapetrou et al., Gene Therapy 12: S118-S130 (2005)). In certain aspects, chemicals such as calcium phosphate, cyclodextrin, or polybrene, can be used to introduce the one or more moieties to the EV.

**In** certain aspects, the one or more moieties are introduced to the EV by electroporation. In some aspects, EVs are exposed to an electrical field which causes transient holes in the EV membrane, allowing loading of the one or more moieties.

**In** certain aspects, the one or more moieties are introduced to the EV by microinjection. In some aspects, a glass micropipette can be used to inject the one or more moieties directly into the EV at the microscopic level.

**In** certain aspects, the one or more moieties are introduced to the EV by extrusion.

**In** certain aspects, the one or more moieties are introduced to the EV by sonication. In some aspects, EVs are exposed to high intensity sound waves, causing transient disruption of the EV membrane allowing loading of the one or more moieties.

**In** some aspects, one or more moieties can be conjugated to the surface of the EV. Conjugation can be achieved chemically or enzymatically, by methods known in the art.

**In** some aspects, the EV comprises one or more moieties that are chemically conjugated. Chemical conjugation can be accomplished by covalent bonding of the one or more moieties to another molecule, with or without use of a linker. The formation of such conjugates is within the skill of artisans and various techniques are known for accomplishing the conjugation, with the choice of the particular technique being guided by the materials to be conjugated. In certain aspects, polypeptides are conjugated to the EV. In some aspects, non-polypeptides, such as lipids, carbohydrates, nucleic acids, and small molecules, are conjugated to the EV.

**In** some aspects, the one or more moieties are introduced to the EV by hypotonic lysis. In such aspects, the EVs can be exposed to low ionic strength buffer causing them to burst allowing loading of the one or more moieties. In other aspects, controlled dialysis against a hypotonic solution can be used to swell the EV and to create pores in the EV membrane. The EV is subsequently exposed to conditions that allow resealing of the membrane.

**In** some aspects, the one or more moieties are introduced to the EV by detergent treatment. In certain aspects, extracellular vesicles are treated with a mild detergent which transiently compromises the EV membrane by creating pores allowing loading of the one or more moieties. After EVs are loaded, the detergent is washed away thereby resealing the membrane.

**In** some aspects, the one or more moieties are introduced to the EV by receptor mediated endocytosis. In certain aspects, EVs have a surface receptor which upon binding of the one or more moieties induces internalization of the receptor and the associated moieties.

**In** some aspects, the one or more moieties are introduced to the EV by mechanical firing. In certain aspects, extracellular vesicles can be bombarded with one or more moieties attached to a heavy or charged particle such as gold microcarriers. In some of these aspects, the particle can be mechanically or electrically accelerated such that it traverses the EV membrane.

**In** some aspects, extracellular vesicles are subjected to several freeze thaw cycles, resulting in EV membrane disruption allowing loading of the one or more moieties.

### VII.C. Methods of Isolating EV, e.g., Exosome

**In** some aspects, methods of producing EVs disclosed herein comprises isolating the EV from the producer cells. In certain aspects, the EVs released by the producer cell into the cell culture medium. It is contemplated that all known manners of isolation of EVs are deemed suitable for use herein. For example, physical properties of EVs can be employed to separate them from a medium or other source material, including separation on the basis of electrical charge (e.g., electrophoretic separation), size *(e.g.,* filtration, molecular sieving, *etc*.), density (*e.g.,* regular or gradient centrifugation), Svedberg constant (e.g., sedimentation with or without external force, *etc*.). Alternatively, or additionally, isolation can be based on one or more biological properties, and include methods that can employ surface markers (e.g., for precipitation, reversible binding to solid phase, FACS separation, specific ligand binding, non-specific ligand binding, affinity purification *etc*.).

Isolation and enrichment can be done in a general and non-selective manner, typically including serial centrifugation. Alternatively, isolation and enrichment can be done in a more specific and selective manner, such as using EV or producer cell-specific surface markers. For example, specific surface markers can be used in immunoprecipitation, FACS sorting, affinity purification, and magnetic separation with bead-bound ligands.

**In** some aspects, size exclusion chromatography can be utilized to isolate the EVs. Size exclusion chromatography techniques are known in the art. Exemplary, non-limiting techniques are provided herein. In some aspects, a void volume fraction is isolated and comprises the EVs of interest. Further, in some aspects, the EVs can be further isolated after chromatographic separation by centrifugation techniques (of one or more chromatography fractions), as is generally known in the art. In some aspects, for example, density gradient centrifugation can be utilized to further isolate the extracellular vesicles. In certain aspects, it can be desirable to further separate the producer cell-derived EVs from EVs of other origin. For example, the producer cell-derived EVs can be separated from non-producer cell-derived EVs by immunosorbent capture using an antigen antibody specific for the producer cell.

**In** some aspects, the isolation of EVs can involve combinations of methods that include, but are not limited to, differential centrifugation, size-based membrane filtration, immunoprecipitation, FACS sorting, and magnetic separation.

The practice of the present disclosure will employ, unless otherwise indicated, conventional techniques of cell biology, cell culture, molecular biology, transgenic biology, microbiology, recombinant DNA, and immunology, which are within the skill of the art. Such techniques are explained fully in the literature. *See,* for example, Sambrook et al., ed. (1989) Molecular Cloning A Laboratory Manual (2nd ed.; Cold Spring Harbor Laboratory Press); Sambrook et al., ed. (1992) Molecular Cloning: A Laboratory Manual, (Cold Springs Harbor Laboratory, NY); D. N. Glover ed., (1985) DNA Cloning, Volumes I and II; Gait, ed. (1984) Oligonucleotide Synthesis; Mullis et al. U.S. Pat. No. 4,683,195; Hames and Higgins, eds. (1984) Nucleic Acid Hybridization; Hames and Higgins, eds. (1984) Transcription And Translation; Freshney (1987) Culture Of Animal Cells (Alan R. Liss, Inc.); Immobilized Cells And Enzymes (IRL Press) (1986); Perbal (1984) A Practical Guide To Molecular Cloning; the treatise, Methods In Enzymology (Academic Press, Inc., N.Y.); Miller and Calos eds. (1987) Gene Transfer Vectors For Mammalian Cells, (Cold Spring Harbor Laboratory); Wu et al., eds., Methods In Enzymology, Vols. 154 and 155; Mayer and Walker, eds. (1987) Immunochemical Methods In Cell And Molecular Biology (Academic Press, London); Weir and Blackwell, eds., (1986) Handbook Of Experimental Immunology, Volumes I-IV; Manipulating the Mouse Embryo, Cold Spring Harbor Laboratory Press, Cold Spring Harbor, N.Y., (1986); ); Crooke, Antisense drug Technology: Principles, Strategies and Applications, 2nd Ed. CRC Press (2007) and in Ausubel et al. (1989) Current Protocols in Molecular Biology (John Wiley and Sons, Baltimore, Md.).

### VIII. Methods of Use

In certain aspects, the present disclosure provides an EV (e.g., exosome) disclosed herein, comprising an ASO of the present disclosure, for use in the treatment of a cancer in a subject. As described herein, ASOs useful for the present disclosure can specifically hybridize to one or more regions of a *STAT6* transcript *(e.g.,* pre-mRNA or mRNA), resulting in reduction and/or inhibition of STAT6 protein expression in a cell. Accordingly, EVs (e.g., exosomes) comprising such an ASO (e.g., EVs disclosed herein) can be useful for preventing and/or treating any disease or disorder associated with increased expression of a STAT6 protein.

A disease or disorder that can be treated comprises a cancer. In certain aspects, the cancer is associated with increased expression of a STAT6 protein. Non-limiting examples of cancers that can be treated with the present disclosure include a colorectal cancer, lung cancer (e.g., non-small cell lung cancer (NSCLC)), pancreatic cancer (e.g., pancreatic ductal adenocarcinoma (PDAC)), leukemia, uterine cancer, ovarian cancer, bladder cancer, bile duct cancer, gastric cancer, or any combination thereof. In some aspects, the cancer is selected from colon adenocarcinoma, rectum adenocarcinoma, pancreatic adenocarcinoma, pancreatic ductal adenocarcinoma (PDAC), ovarian serous cystadenocarcinoma, acute myelid leukemia, testicular germ cell tumors, lung adenocarcinoma, brain lower grade glioma, glioblastoma multiforme, uveal melanoma, thyroid carcinoma, uterine corpus endometrial carcinoma, uterine carcinosarcoma, pheochromocytoma, paraganglioma, and any combiniation thereof. In certain aspects, the cancer is a myeloid-rich cancer. In some aspects, the cancer comprises a liver cancer. In some aspects, the cancer comprises hepatocellular cancer (HCC). In some aspects, the cancer comprises pancreatic ductal adenocarcinoma (PDAC), in some aspects, the cancer comprises colorectal carcinoma (CRC). In some aspects, the cancer comprises ovarian cancer. In some aspects, the cancer comprises leptomeningeal cancer.

When administered to a subject with a cancer, in certain aspects, EVs (e.g., exosome) of the present disclosure can up-regulate an immune response and enhance the tumor targeting of the subject's immune system. In some aspects, the cancer being treated is characterized by infiltration of leukocytes (T-cells, B-cells, macrophages, dendritic cells, monocytes) into the tumor microenvironment, or so-called "hot tumors" or "inflammatory tumors". In some aspects, the cancer being treated is characterized by low levels or undetectable levels of leukocyte infiltration into the tumor microenvironment, or so-called "cold tumors" or "non-inflammatory tumors". In some aspects, an EV is administered in an amount and for a time sufficient to convert a "cold tumor" into a "hot tumor", *i.e.,* said administering results in the infiltration of leukocytes (such as T-cells) into the tumor microenvironment. In certain aspects, cancer comprises bladder cancer, cervical cancer, renal cell cancer, testicular cancer, colorectal cancer, lung cancer, head and neck cancer, and ovarian, lymphoma, liver cancer, glioblastoma, melanoma, myeloma, leukemia, pancreatic cancers, or combinations thereof. In other term, "distal tumor" or "distant tumor" refers to a tumor that has spread from the original (or primary) tumor to distant organs or distant tissues, e.g., lymph nodes. In some aspects, the EVs of the disclosure treats a tumor after the metastatic spread.

In some aspects, the EVs (e.g., exosomes) are administered intravenously to the circulatory system of the subject. In some aspects, the EVs are infused in suitable liquid and administered into a vein of the subject.

In some aspects, the EVs (e.g., exosomes) are administered intra-arterially to the circulatory system of the subject. In some aspects, the EVs are infused in suitable liquid and administered into an artery of the subject.

In some aspects, the EVs (e.g., exosomes) are administered to the subject by intrathecal administration. In some aspects, the EVs (e.g., exosomes) are administered by intrathecal administration, followed by application of a mechanical convective force to the torso. See, e.g., Verma et al., Alzheimer's Dement. 12:e12030 (2020)). As such, certain aspects of the present disclosure are directed to methods of administering an EV, e.g., an exosome, to a subject in need thereof, comprising administering the EV, e.g., exosome, to the subject by intrathecal injection, followed by applying a mechanical convective force to the torso of the subject. In some aspects, the mechanical convective force is achieved using a high frequency chest wall or lumbothoracic oscillating respiratory clearance device *(e.g.,* a Smart Vest or Smart Wrap, ELECTROMED INC, New Prague, MN, USA). In some aspects, the mechanical convective force, e.g., the oscillating vest, facilitates spread of the intrathecally dosed EVs, e.g., exosomes, further down the nerve thus allowing for better EV, e.g., exosome, delivery to nerves.

In some aspects, the intra- and trans-compartmental biodistribution of exosomes can be manipulated by exogenous extracorporeal forces acting upon a subject after compartmental delivery of exosomes. This includes the application of mechanical convection, for example by way of applying percussion, vibration, shaking, or massaging of a body compartment or the entire body. Following intrathecal dosing for example, the application of chest wall vibrations by several means including an oscillating mechanical jacket can spread the biodistribution of exosomes along the neuraxis or along cranial and spinal nerves, which can be helpful in the treatment of nerve disorders by drug carrying exosomes.

In some aspects, the application of external mechanical convective forces via an oscillating jacket or other similar means can be used to remove exosomes and other material from the cerebrospinal fluid of the intrathecal space and out to the peripheral circulation. This aspect can help remove endogenous toxic exosomes and other deleterious macromolecules such as beta-amyloid, tau, alpha-synuclein, TDP43, neurofilament and excessive cerebrospinal fluid from the intrathecal space to the periphery for elimination.

In some aspects, exosomes delivered via the intracebroventricular route can be made to translocate throughout the neuraxis by simultaneously incorporating a lumbar puncture and allowing for ventriculo-lumbar perfusion wherein additional fluid is infused into the ventricles after exosome dosing, while allowing the existing neuraxial column of CSF to exit is the lumbar puncture. Ventriculo-lumbar perfusion can allow ICV dosed exosome to spread along the entire neuraxis and completely cover the subarachoid space in order to treat leptomeningeal cancer and other diseases.

In some aspects, the application of external extracorporeal focused ultrasound, thermal energy (heat) or cold may be used to manipulate the compartmental pharmacokinetics and drug release properties of exosomes engineered to be sensitive to these phenomena.

In some aspects, the intracompartmental behavior and biodistribution of exosomes engineered to contain paramagnetic material can be manipulated by the external application of magnets or a magnetic field.

In some aspects, the EVs are administered *via* an injection into the spinal canal, or into the subarachnoid space so that it reaches the cerebrospinal fluid (CSF).

In some aspects, the EVs (e.g., exosomes) are administered intratumorally into one or more tumors of the subject.

In some aspects, the EVs (e.g., exosomes) are administered to the subject by intranasal administration. In some aspects, the EVs can be insufflated through the nose in a form of either topical administration or systemic administration. In certain aspects, the EVs are administered as nasal spray.

In some aspects, the EVs (e.g., exosomes) are administered to the subject by intraperitoneal administration. In some aspects, the EVs are infused in suitable liquid and injected into the peritoneum of the subject. In some aspects, the intraperitoneal administration results in distribution of the EVs to the lymphatics. In some aspects, the intraperitoneal administration results in distribution of the EVs to the thymus, spleen, and/or bone marrow. In some aspects, the intraperitoneal administration results in distribution of the EVs to one or more lymph nodes. In some aspects, the intraperitoneal administration results in distribution of the EVs to one or more of the cervical lymph node, the inguinal lymph node, the mediastinal lymph node, or the sternal lymph node. In some aspects, the intraperitoneal administration results in distribution of the EVs to the pancreas.

In some aspects, the EVs, e.g., exosomes, are administered to the subject by periocular administration. In some aspects, the s are injected into the periocular tissues. Periocular drug administration includes the routes of subconjunctival, anterior sub-Tenon's, posterior sub-Tenon's, and retrobulbar administration.

### VIII.A. Methods of Treating a Brain Tumor

Certain aspects of the present disclosure are directed to an EV as defined in the claims for use in a method of treating a brain tumor in a subject in need thereof. In some aspects, the method comprises administering to the subject a therapeutically effective amount of an EV, *e.g.,* exosome, comprising an ASO, as disclosed herein. In some aspects, the EV, *e.g.,* exosome, is capable of targeted delivery of a therapeutic agent, e.g., an ASO, as disclosed herein, to the CNS to treat the brain tumor. In some aspects, the EV, e.g., exosome, is capable of up-regulating an immune response in the subject, thereby enhancing the subject's immune response against the neuroimmunological disorder. In some aspects, the composition is administered intratumorally or intrathecally to the subject.

Also provided herein are methods of preventing metastasis of a brain tumor in a subject. The method comprises administering to the subject a therapeutically effective amount of the compositions disclosed herein, wherein the composition is capable of preventing a brain tumor at one location in the subject from promoting the growth of one or more tumors at another location in the subject. In some aspects, the composition is administered intratumorally or intrathecally in a first tumor in one location, and the composition administered in a first tumor prevents metastasis of one or more tumors at a second location.

**In** some aspects, administering an EV, e.g., exosome, disclosed herein inhibits and/or reduces growth of a brain tumor in a subject. In some aspects, the growth of a brain tumor (e.g., tumor volume or weight) is reduced by at least about 5%, at least about 10%, at least about 20%, at least about 30%, at least about 40%, at least about 50%, at least about 60%, at least about 70%, at least about 80%, at least about 90%, or about 100% compared to a reference (e.g., tumor volume in a corresponding subject after administration of an EV, e.g., exosome, without the ASO).

As used herein, the term "brain tumor" refers to an abnormal growth of cells within the brain (e.g., within the meninges). Brain tumors can be categorized as primary or secondary brain tumor. "Primary brain tumor" refers to brain tumors that originate within the brain. "Secondary brain tumor" refers to brain tumors that are the result of cancer cells originating at primary sites outside the brain that have metastasized *(i.e.,* spread) to the brain. Unless specified otherwise, the term brain tumor can refer to both primary and secondary brain tumors.

**In** some aspects, a brain tumor that can be treated with the present disclosure comprises an acoustic neuroma, choroid plexus carcinoma, craniopharyngioma, embryonal tumor, glioma, medulloblastoma, meningioma, pediatric brain tumor, pineoblastoma, pituitary tumor, or combinations thereof.

**In** certain aspects, a brain tumor that can be treated with the present disclosure comprises a glioma. As used herein, the term "glioma" refers to a type of tumor that starts in the glial cells of the brain or the spine. In some aspects, a glioma can be classified by specific type of cells with which they share histological features. Accordingly, a glioma that can be treated with EVs (e.g., exosomes) disclosed herein can be classified as an ependymoma (ependymal cells), astrocytoma (astrocytes), oligodendroglioma (oligodendrocytes), brainstem glioma (e.g., diffuse intrinsic pontine glioma), optic nerve glioma, mixed glioma, oligoastrocytoma, or any combination thereof. In certain aspects, an astrocytoma comprises glioblastoma multiforme (GBM).

Gliomas disclosed herein can be further categorized according to their grade, which is determined by pathologic evaluation of the tumor. In some aspects, the neuropathological evaluation and diagnostics of brain tumor specimens is performed according to WHO Classification of Tumours of the Central Nervous System. In some aspects, a glioma that can be treated with the present disclosure comprises a low-grade glioma. A "low-grade glioma" [WHO grade II] are well-differentiated (not anaplastic) and tend to exhibit benign tendencies and portend a better prognosis for the patient. However, in some aspects, low-grade gliomas can have a uniform rate of recurrence and increase in grade over time, so should be classified as malignant. In some aspects, a glioma that can be treated comprises a high grade glioma. A "high-grade glioma" [WHO grades III-IV] gliomas are undifferentiated or anaplastic and are malignant and carry a worse prognosis. Of numerous grading systems in use, the most common is the World Health Organization (WHO) grading system for astrocytoma, under which tumors are graded from I (least advanced disease-best prognosis) to IV (most advanced disease-worst prognosis). Non-limiting examples of high-grade gliomas include anaplastic astrocytomas and glioblastoma multiforme.

In some aspects, an EV (e.g., exosome) disclosed herein can be used to treat a glioma grade I, grade II, grade III, grade IV, or combinations thereof, as determined under the WHO grading system. In certain aspects, an EV (e.g., exosome) disclosed herein can be used to treat any type of gliomas.

In some aspects, the glioma treatable by the present methods is a diffuse intrinsic pontine glioma (DIPG), a type of brainstem glioma. Diffuse intrinsic pontine glioma primarily affects children, usually between the ages of 5 and 7. The median survival time with DIPG is under 12 months. Surgery to attempt tumor removal is usually not possible or advisable for DIPG. By their very nature, these tumors invade diffusely throughout the brain stem, growing between normal nerve cells.

In other aspects, the glioma treatable by the present methods is an IDH1 and IDH2-mutated glioma. Patients with glioma carrying mutations in either IDH1 or IDH2 have a relatively favorable survival, compared with patients with glioma with wild-type IDH1/2 genes. In WHO grade III glioma, IDH1/2-mutated glioma have a median prognosis of ~3.5 years, whereas IDH1/2 wild-type glioma perform poor with a median overall survival of 1.5 years. In glioblastoma, the difference is larger.

In some aspects, a neuroimmunological disorder that can be treated with the present disclosure comprises a neoplastic meningitis. As used herein, "neoplastic meningitis" refers to a tumor which has spread from the original tumor site into the dural and leptomeninges, which are thin tissue membranes covering the brain and spinal cord. In some aspects, connective tissue nerve sheaths that extend from the meninges onto and into nerves can also become involved. Neoplastic meningitis is also known as carcinomatous meningitis, leptomeningeal carcinoma, leptomeningeal carcinomatosis, leptomeningeal metastasis, leptomeningeal disease (LMD), leptomeningeal cancer, meningeal carcinomatosis, and meningeal metastasis. In certain aspects, a neoplastic meningitis is caused by leukemia. In some aspects, a neoplastic meningitis is caused by melanoma, breast, lung, gastrointestinal cancer, or combinations thereof. In certain aspects, a neoplastic meningitis is caused by a glioma.

The following examples are offered by way of illustration and not by way of limitation.

### EXAMPLES

### Example 1: In vitro analysis of mRNA and/or protein reduction

Exemplary ASOs disclosed herein were designed to specifically target the *STAT6* transcript (FIG. 1). The disclosed ASOs will be tested for their ability to knockdown *STAT6* mRNA and/or STAT6 protein expression in reporter cell lines containing a human *STAT6* coding sequence upstream of reporter. STAT6-specific siRNA will be used as positive control.

Briefly, the reporter cell lines expressing STAT6 will be grown in cell culture media and seeded onto a 96 well plate. Then, the cells will be treated with different concentrations of EVs (e.g., exosomes) comprising one or more ASOs disclosed herein ("EV-ASO"). Methods for producing such EVs are provided elsewhere in the present disclosure. Approximately 3 days after EV-ASO treatment, the cells will be harvested and RNA and/or protein will be purified from the cells. Then, the *STAT6* mRNA and/or STAT6 protein expression levels in the cells will be quantified using assays such as, qPCR and Western blot.

A lead ASO will be selected first by using *in silico* selection based on alternative transcript cross reactivity, species cross reactivity, specificity for gene of interest, presence of SNPs within ASO, length of ASO, location diversity, toxic motifs, and predicted binding affinity. Next, the ASOs will be screened for the ability to knock down (by at least 50% at 2 nM, and less than 20% knock down of GAPDH at 20 nM) target gene expression in cell lines transfected with the target sequence *(STAT6* mRNA). ASOs will then be assayed for target gene knock down potency in primary macrophages from at least two donors. Housekeeping gene expression stability, diversity of sequence location, and expression of predicted off-targets after treatment will also be observed. Optimal ASOs having the highest reprogramming activity (gene expression changes, cytokine production, T cell suppression) in primary macrophages will be selected as the lead ASOs.

### Example 2: Construction of an Exosome

To generate exosomes described herein, human embryonic kidney (HEK) cell line (e.g., HEK293SF) will be used. The cells will be stably transfected with Scaffold X, Scaffold Y, and/or anchoring moiety linked to an agent of interest.

Upon transfection, HEK cells will be grown to high density in chemically defined medium for 7 days. Conditioned cell culture media will be then collected and centrifuged at 300 - 800 x g for 5 minutes at room temperature to remove cells and large debris. Media supernatant will be supplemented with 1000 U/L BENZONASE^{®} and incubated at 37 °C for 1 hour in a water bath. Supernatant will be collected and centrifuged at 16,000 x g for 30 minutes at 4 °C to remove residual cell debris and other large contaminants. Supernatant will then be ultracentrifuged at 133,900 x g for 3 hours at 4 °C to pellet the exosomes. Supernatant will be discarded and any residual media will be aspirated from the bottom of the tube. The pellet will be resuspended in 200 - 1000 µL PBS (-Ca -Mg).

To further enrich exosome populations, the pellet will be processed via density gradient purification (sucrose or OPTIPREP^{™}).

The gradient will be spun at 200,000 x g for 16 hours at 4 °C in a 12 mL Ultra-Clear (344059) tube placed in a SW 41 Ti rotor to separate the exosome fraction.

The exosome layer will then be gently removed from the top layer and diluted in ~32.5 mL PBS in a 38.5 mL Ultra-Clear (344058) tube and ultracentrifuged again at 133,900 x g for 3 hours at 4 °C to pellet the purified exosomes. The resulting pellet will be resuspended in a minimal volume of PBS (~200 µL) and stored at 4 °C.

For OPTIPREP^{™} gradient, a 3-tier sterile gradient will be prepared with equal volumes of 10%, 30%, and 45% OPTIPREP^{™} in a 12 mL Ultra-Clear (344059) tube for a SW 41 Ti rotor. The pellet will be added to the OPTIPREP^{™} gradient and ultracentrifuged at 200,000 x g for 16 hours at 4 °C to separate the exosome fraction. The exosome layer will then be gently collected from the top ~3 mL of the tube.

The exosome fraction will be diluted in ~32 mL PBS in a 38.5 mL Ultra-Clear (344058) tube and ultracentrifuged at 133,900 x g for 3 hours at 4 °C to pellet the purified exosomes. The pelleted exosomes will then be resuspended in a minimal volume of PBS (~200 µL) and stored at 4°C until ready to be used.

### Example 3: ASO Design

Mouse and human ASOs were designed to target *STAT6* (Gene ID No. 6778) expression. Target sequences were selected using the reference sequences NM_001178078.2 for human *STAT6* and NM_009284.2 for mouse *STAT6.* A list of possible ASOs were generated for each gene by tilling of ASOs across the entire length of the nascent transcript. ASOs having 15, 16, 17, 18, 19, or 20 nucleobases in length were generated.

ASOs were prioritized based on the following properties: must hit all splice forms; low self-dimerization energy (on-target activity); no GGGG motif (can cause synthesis issues); less than 3 CpG dinucleotides in the oligo (potential immunostimulation); less than 8 bases of palindromic sequence (potential dimerization & immunostimulation); more than 2 mismatch and no more than 17 contiguous bases in an off-target hit to any gene, including known miRNA and lncRNA, and both nascent and mature transcripts; no overlap with repetitive sequences; and no overlap with SNPs of greater than or equal to 0.01 MAF in the general population. Additional criteria included Predicted species cross reactivity (e.g., human, cyno, rhesus, rat, mouse transcripts); and an off target (OT) filter less than or equal to 3 mismatch (mm) in mature transcripts, less than or equal to 3 mm in lnc transcripts, less than or equal to 3 mm in miRNAs, and less than or equal to 3 mm in nascent transcripts.

### Example 4: ASO Loading on Exosomes

ASOs targeting STAT6 were loaded onto exosomes that overexpressed PTGFRN as shown in FIG. 2A. Mice were treated intravenously with a single dose of 2E11 exosomes loaded with a reporter ASO ("exo ASO") or with a single dose of free reporter ASO ("free ASO"). One hour following administration, increased exo ASO uptake was observed in CD11b⁺ dendritic cells, monocytes, and mMDSCs in the blood (FIG. 2B); Kupffer cells in the liver (FIG. 2C); red pulp macs, monocytes, and mMDSCs in the spleen (FIG. 2D); and dendritic cells and mMDSCs in tumor tissue (FIGs. 2E-2F), as evidenced by MFI, relative to the localization of free ASO. Uptake of exo ASO was also higher in bone marrow (FIGs. 2G-2H) as compared to uptake of free ASO and negative controls (FIGs. 2I-2L). FIG. 2O show enhanced uptake of exoASO by M2 and M0 macrophage uptake as compared to free ASO. FIG. 2P shows expression of PTGFRN cognate receptors in glioblastoma (GBM) in various cell types across five targets, with the highest expression present in myeloid cells.

### Example 5: Exo-STAT6-ASO Are Capable of Repolarizing M2 Macrophages

Primary human macrophages were polarized with IL4/IL10/TGFβ treatment and treated with increasing concentrations of Exo-STAT6-ASO (*see* FIG. 2A). *In vitro* treatment of primary human macrophages with Exo-STAT6-ASO induces dose-dependent knockdown of *STAT6* (FIG. 3A), respectively, as well as the downregulation of an M2 macrophage gene, CD163 (FIG. 3B). Potency was found to be slightly higher using the Exo-ASOs as compared to the free ASOs. Various M2 genes were downregulated and various M1 genes were upregulated following treatment with Exo-STAT6-ASO (FIGs. 4A-4J). In addition, production of various cytokines was upregulated and downregulated after lipopolysaccharide (LPS) stimulation and treatment with Exo-STAT6-ASO (FIGs. 4K-4N).

BALB/c mice bearing CT26 subcutaneous tumors were treated with a single intratumoral dose of either exo ASO or free ASO. One hour following administration, exo ASO uptake was observed in tumor cells, macrophages, myeloid-derived suppressor cells, and dendritic cells (FIGs. 2L-2M).

### Example 6: Exo-STAT6-ASO Target Gene Knockdown in CD11b cells.

*In vivo,* the primary recipient cell for Exo-STAT6-ASO is CD11b cells. To further measure the uptake and known-down efficiency of the Exo-ASOs, mice were treated with Exo-STAT6-ASO and sacrificed. CD11b⁺ cells were then isolated and enriched (FIGs. 5A-5F). Though not the endpoint, tumor volume was significantly lower in Exo-STAT6-ASO treated mice relative to mice treated with a scramble Exo-ASO control, and mice treated with Exo-STAT6-ASO tended to have smaller tumors than mice treated with STAT6 free ASO (FIG. 5G). Exo-ASO target gene knockdown was more pronounced in the CD11b-enriched cells than non-enriched cells following treatment with Exo-STAT6-ASO (FIG. 6A). In addition, Exo-ASOs were effective at reducing Arg1 expression to a greater extent in CD11b-enriched cells than non-enriched cells (FIG. 6B).

CD11b-enriched cells treated with Exo-STAT6-ASO also showed macrophage reprogramming as evidenced by upregulation of various M1 genes and downregulation of various M2 genes (FIGs. 7A-7V).

### Example 7: Treatment of Fibrosis Using Exo-STAT6-ASO.

Excessive M2 macrophage activation leads to the continuous production of TGFβ and growth factors that promote proliferation of myofibroblasts, activation of EMT/EndoMT, and extracellular matrix deposition. M2 macrophages represent a break point between wound healing and exacerbation of pro-fibrotic process. To test whether Exo-STAT6-ASO could be used to treat fibrosis in a subject, primary human M2 macrophages were polarized with IL-13/TGFβ treatment, which are drivers of fibrosis. Cells were then exposed to increasing concentrations of free STAT6 ASO, Exo-STAT6-ASO, or scrambled ASO (FIGs. 8A and 8B); and assayed for expression of *STAT6* (FIG. 8A) or expression of *TGFβ1* (FIG. 8B).

To test the feasibility of Exo-ASO delivery *in vivo* using intra-nasal administration, 6-weak old mice were treated with bleomycin to induce pulmonary fibrosis. Two weeks later, mice were administered Exo-ASO-Cy5 intranasally, and the mice were sacrificed 4 hours post-administration. Bleomycin induced mice administered Exo-ASO-Cy5 showed increased total flux of Cy5 relative to naïve mice administered Exo-ASO-Cy5 ("IN naïve") and relative to naïve and treated mice administered a PBS negative control ("-C") (FIG. 9).

Exosome uptake was observed by lung macrophages and lung capillary endothelial cells in both normal lung and induced-pulmonary fibrosis lungs tissue (FIGs. 10A-10H and 11A-11H).

### Example 8: Treatment of a Hepatocarcinoma Mouse-Model Using Exo-STAT6-ASO.

Hepa1-6 mice will be used to test the *in vivo* efficacy of Exo-STAT6-ASO for treating a tumor. The Hepa1-6 line is an orthotopic mouse model of hepatocarcinoma. Samples were obtained from CRO and analyzed by in situ hybridization for expression of *STAT6* (FIGs. 12A-12B and 13A-13F).

### Example 9: Treatment of a Colon Carcinoma Mouse Model using Exo-STAT6-ASO

CT26 tumor cells were implanted subcutaneously into the flanks of BALB/c mice. Eight days after implantation mice were treated intratumorally with exo-STAT6-ASO or free STAT6 ASO or intraperitoneally with an anti-PD1 antibody or an anti-CSF1R antibody (n=10 mice per group). As controls, two groups of mice were treated with either exoASO-scramble or PBS. Analysis of the geometric means of the tumor volumes shows that mice treated with exo-STAT6-ASO had very little tumor growth through 30 days post-implantation (FIG. 17B). In contrast, mice treated with free STAT6 ASO developed tumors similar in size to the PBS and exoASO-Scramble control treated mice.

### Example 10: Treatment of a Glioblasoma Multiforme Mouse Model using Exo-STAT6-ASO

Glioblasoma Multiforme (GBM) carrying mice will be treated intratumorally with exo-STAT6-ASO or free STAT6 ASO. As controls, two groups of mice will be treated with either exoASO-scramble or PBS. The geometric means of the tumor volumes will be measured through at least 30 days post-implantation. Macrophage localization and marker gene expression will be monitored, including the occurrence of tumor-infiltrating macrophages.

### Example 11: Treatment of a Leptomeningeal Cancer Disease (LMD) Mouse Model using Exo-STAT6-ASO

Leptomeningeal Cancer Disease (LMD) mice will be treated intratumorally with exo-STAT6-ASO or free STAT6 ASO. As controls, two groups of mice will be treated with either exoASO-scramble or PBS. The geometric means of the tumor volumes will be measured through at least 30 days post-implantation. Macrophage localization and marker gene expression will be monitored, including the occurrence of tumor-infiltrating macrophages.

### Example 12: In Vitro Analysis of Exo-STAT6-ASO Dose Duration

To determine the dose duration of exo-STAT6, human M2 macrophages from two donors were treated with either exo-STAT6-ASO or free STAT6 ASO. Knock down of STAT6 mRNA and protein levels (KD) were analyzed at 24, 48, 72, 120, and 168 hours. STAT6 gene expression was normalized to a housekeeping gene. Maximum knock down of STAT6 mRNA was observed between day 3 and day 5 (FIGs. 21A-21G). There were no changes in cell viability at any timepoint (data not shown). Reduction of STAT6 mRNA levels was dose dependent, with the highest exo-STAT6-ASO dose of 2.5 µM maintaining 85% mRNA knock down at 7 days (FIGs. 22A-22B).

Protein knock down was similarly dose dependent (FIG. 23A), and the highest level of protein knock down was observed as 70% reduction at day 7 following exo-STAT6-ASO (FIG. 23B). STAT6 protein levels were normalized to untreated, b-Actin housekeeping shows no changes across groups and timepoints

The enhanced potency of exo-STAT6-ASO as compared to free STAT6 ASO was observed at all time points except 24 hours (FIGs. 21A-21E). The data were consistent across the two donors.

These results show that the durability of mRNA and protein knock down is longer than typically measured (48 hours). Functional effects, such as reprogramming, may be stronger at later timepoints.

### Example 13: In Vivo Analysis of Exo-STAT6-ASO Dose Duration

To determine the dose duration of exo-STAT6 *in vitro,* exo-STAT6-ASO or free STAT6 ASO will be intratumorrally administered to CT26 model mice. STAT6 mRNA and protein levels will be assayed at various time points over several days, e.g., 7 or more days. The dose of administered exo-STAT6-ASO will be titrated. Mouse and cyno exo-STAT6-ASO constructs will be tested.

### Example 14: In vivo Induction of Pro-Inflammatory M1 Markers Following ExoASO Treatment

YUMM1.7 tumors were dosed with intratumoral microinjections of exo-STAT6-ASO, free STAT6 ASO, or an exoASO scramble using the Comparative In Vivo Oncology (CIVO^{®}) Platform by Presage Biosciences. Mice were euthanized 24 hours after one single dose, (n=6 mice per group). Expression of TNFα (FIGs. 24A-24C, open triangle), CD11b (FIGs. 24D-24F, open triangle), iNOS (FIGs. 24G-24I, open triangle), and F4/80 (FIGs. 24J-24L, open triangle) is shown by *In situ* hybridization for tumor cells treated with exoASO scramble (FIGs. 24A, 24D, 24G, and 24J), free STAT6 ASO (FIGs. 24B, 24E, 24H, and 24K), and exo-STAT6-ASO (FIGs. 24C, 24F, 24I, and 24L). Each panel row is a different injection site on the same tumor. Fluorescent tracking marker (FTM; arrows) denotes the injection site. These data show robust induction of pro-inflammatory M1 markers following intratumoral injection of exo-STAT6-ASO.

### Example 15: In Vivo Efficacy of Exo-STAT6-ASO Administered to Mice by Intraperitoneal Injection

A Pancreatic ductal adenocarcinoma mouse model was used to test the *in vivo* efficacy of intraperitoneal administration Exo-STAT6-ASO. Tumor-bearing mice were administered a dose of (i) 2E11 modified exosomes, comprising Scaffold X and loaded with Exo-STAT6-ASO, (ii) gemcitabine (gem; standard of care), (iii) scrambled ASO (Scr), or (iv) PBS, on days 15, 18, and 20 post inoculation (FIGs. 25A-25D). Samples were randomized by a first ultrasound at D14. A second ultrasound and takedown occurred at D22. These preliminary results show that mice treated with exo-STAT6-ASO monotherapy had smaller tumor by size (FIGs. 25B-25C) and weight (FIG. 25D) as compared to negative controls (PBS and Scr) and standard of care treatment (Gem).

Survival was also observed in tumor-bearing mice treated with exo-STAT6-ASO monotherapy. Mice were recruited by a second surgery and tumor measurement. Mice were administered six doses total (TIWx2) of (i) Exo-STAT6-ASP or (ii) Scramble exoASO, doesd at 2E11 (7ug) of exoASO/injection, or four doses (DIWx2) 100mg/kg gemcitabine over 2 weeks (FIG. 26A). Mice were then monitored for survival. Mice treated with exo-STAT-6 ASO had improved survival relative to PBS and exo-Scramble controls (FIGs. 26B-26D), and there was no difference observed in percent survival of mice treated with exo-STAT6-ASO monotherapy as compared to mice treated with gemcitabine (FIGs. 26B-26C).

## Claims

1. An extracellular vesicle capable of reducing STAT6 protein or mRNA expression in a human cell expressing STAT6, wherein the extracellular vesicle comprises an antisense oligonucleotide (ASO) which comprises a contiguous nucleotide sequence of 10 to 30 nucleotides in length that is complementary to a nucleic acid sequence within a *STAT6* transcript (SEQ ID NO: 1 or SEQ ID NO: 3), wherein the ASO comprises the nucleotide sequence set forth in SEQ ID NO: 91.

2. The extracellular vesicle of claim 1, wherein the ASO is a gapmer, a mixmer, or a totalmer.

3. The extracellular vesicle of claim 1 or 2, wherein the ASO comprises one or more nucleoside analogs.

4. The extracellular vesicle of claim 3, wherein one or more of the nucleoside analogs is a sugar modified nucleoside.

5. The extracellular vesicle of any one of claims 1 to 4, which further comprises
(i) an anchoring moiety; and/or
(ii) an exogenous targeting moiety.

6. The extracellular vesicle of claim 5, wherein the anchoring moiety comprises sterol, GM1, a lipid, a vitamin, a small molecule, a peptide, or a combination thereof.

7. The extracellular vesicle of claim 5, wherein the anchoring moiety comprises cholesterol.

8. The extracellular vesicle of any one of claims 5 to 7, wherein the ASO is linked to the anchoring moiety.

9. The extracellular vesicle of any one of claims 5 to 8, wherein the ASO is linked to the anchoring moiety by a linker, wherein the linker:
(i) comprises a polypeptide;
(ii) comprises a non-polypeptide moiety;
(iii) comprises ethylene glycol;
(iv) comprises HEG, TEG, PEG, or any combination thereof;
(v) comprises acrylic phosphoramidite (e.g,. ACRYDITE^{™}), adenylation, azide (NHS Ester), digoxigenin (NHS Ester), cholesterol-TEG, I-LINKER^{™}, an amino modifier (e.g., amino modifier C6, amino modifier C12, amino modifier C6 dT, or Uni-Link^{™} amino modifier), alkyne, 5' Hexynyl, 5-Octadiynyl dU, biotinylation (e.g., biotin, biotin (Azide), biotin dT, biotin-TEG, dual biotin, PC biotin, or desthiobiotin), thiol modification (thiol modifier C3 S-S, dithiol or thiol modifier C6 S-S), or any combination thereof;
(vi) comprises a cleavable linker;
(vii) comprises valine-alanine-p-aminobenzylcarbamate or valine-citrulline-p-aminobenzylcarbamate; or
(viii) comprises (a) a maleimide moiety and (b) valine-alanine-p-aminobenzylcarbamate or valine-citrulline-p-aminobenzylcarbamate.

10. The extracellular vesicle of any one of claims 1 to 9, wherein the EV is an exosome.

11. A pharmaceutical composition comprising the extracellular vesicle of any one of claims 1 to 10 and a pharmaceutically acceptable diluent, carrier, salt, or adjuvant.

12. The extracellular vesicle of any one of claims 1 to 10 or the pharmaceutical composition of claim 11, for use in the treatment of a cancer.

## Patentansprüche

1. Extrazelluläres Vesikel, das in der Lage ist, die Expression von STAT6-Protein oder STAT6-mRNA in einer STAT6 exprimierenden menschlichen Zelle zu verringern, wobei das extrazelluläre Vesikel ein Antisense-Oligonukleotid (ASO) umfasst, das eine zusammenhängende Nukleotidsequenz mit einer Länge von 10 bis 30 Nukleotiden umfasst, die zu einer Nukleinsäuresequenz innerhalb eines *STAT6*-Transkripts komplementär ist (SEQ ID NO: 1 oder SEQ ID NO: 3), wobei das ASO die in SEQ ID NO: 91 angegebene Nukleotidsequenz umfasst.

2. Extrazelluläres Vesikel nach Anspruch 1, wobei das ASO ein Gapmer, ein Mixmer oder ein Totalmer ist.

3. Extrazelluläres Vesikel nach Anspruch 1 oder 2, wobei das ASO ein oder mehrere Nukleosidanaloga umfasst.

4. Extrazelluläres Vesikel nach Anspruch 3, wobei es sich bei einem oder mehreren der Nukleosidanaloga um ein zuckermodifiziertes Nukleosid handelt.

5. Extrazelluläres Vesikel nach einem der Ansprüche 1 bis 4, das ferner umfasst
(i) eine Verankerungseinheit; und/oder
(ii) eine exogene Targeting-Einheit.

6. Extrazelluläres Vesikel nach Anspruch 5, wobei die Verankerungseinheit Sterol, GM1, ein Lipid, ein Vitamin, ein kleines Molekül, ein Peptid oder eine Kombination davon umfasst.

7. Extrazelluläres Vesikel nach Anspruch 5, wobei die Verankerungseinheit Cholesterin umfasst.

8. Extrazelluläres Vesikel nach einem der Ansprüche 5 bis 7, wobei das ASO an die Verankerungseinheit gebunden ist.

9. Extrazelluläres Vesikel nach einem der Ansprüche 5 bis 8, wobei das ASO über einen Linker an die Verankerungseinheit gebunden ist, wobei der Linker:
(i) ein Polypeptid umfasst;
(ii) eine Nicht-Polypeptid-Einheit umfasst;
(iii) Ethylenglykol umfasst;
(iv) HEG, TEG, PEG oder eine beliebige Kombination davon umfasst;
(v) Acrylphosphoramidit (z. B. Acrydite^{™}), Adenylierung, Azid (NHS-Ester), Digoxigenin (NHS-Ester), Cholesterin-TEG, I-LINKER^{™}, einen Aminomodifikator (z. B. Aminomodifikator C6, Aminomodifikator C12, Aminomodifikator C6 dT oder Uni-Link^{™}-Aminomodifikator), Alkin, 5'-Hexinyl, 5-Octadiynyl-dU, Biotinylierung (z. B. Biotin, Biotin (Azid), BiotindT, Biotin-TEG, Dual-Biotin, PC-Biotin oder Desthiobiotin), Thiolmodifikation (Thiolmodifikator C3 S-S, Dithiol oder Thiolmodifikator C6 S-S) oder eine beliebige Kombination davon umfasst;
(vi) einen spaltbaren Linker umfasst;
(vii) Valin-Alanin-p-Aminobenzylcarbamat oder Valin-Citrullin-p-Aminobenzylcarbamat umfasst; oder
(viii) (a) eine Maleimideinheit und (b) Valin-Alanin-p-Aminobenzylcarbamat oder Valin-Citrullin-p-Aminobenzylcarbamat umfasst.

10. Extrazelluläres Vesikel nach einem der Ansprüche 1 bis 9, wobei das extrazelluläre Vesikel ein Exosom ist.

11. Pharmazeutische Zusammensetzung, umfassend das extrazelluläre Vesikel nach einem der Ansprüche 1 bis 10 und ein pharmazeutisch verträgliches Verdünnungsmittel, Trägermittel, Salz oder Adjuvans.

12. Extrazelluläres Vesikel nach einem der Ansprüche 1 bis 10 oder pharmazeutische Zusammensetzung nach Anspruch 11 zur Verwendung bei der Behandlung einer Krebserkrankung.

## Revendications

1. Vésicule extracellulaire capable de réduire l'expression d'une protéine ou d'un ARNm de STAT6 dans une cellule humaine exprimant STAT6, dans laquelle la cellule extracellulaire comprend un oligonucléotide antisens (OAS) qui comprend une séquence nucléotidique contiguë de 10 à 30 nucléotides de longueur qui est complémentaire d'une séquence d'acide nucléique au sein d'un produit de transcription *STAT6* (SEQ ID NO : 1 ou SEQ ID NO : 3), dans laquelle l'OAS comprend la séquence nucléotidique telle que définie dans la SEQ ID NO : 91.

2. Vésicule extracellulaire selon la revendication 1, dans laquelle l'OAS est un gapmère, un mixmère, ou un totalmère.

3. Vésicule extracellulaire selon la revendication 1 ou 2, dans laquelle l'OAS comprend un ou plusieurs analogues nucléosidiques.

4. Vésicule extracellulaire selon la revendication 3, dans laquelle une ou plusieurs des analogues nucléosidiques est un nucléoside à sucre modifié.

5. Vésicule extracellulaire selon l'une quelconque des revendications 1 à 4, qui comprend en outre
(i) un groupement d'ancrage ; et/ou
(ii) un groupement de ciblage exogène.

6. Vésicule extracellulaire selon la revendication 5, dans laquelle le groupement d'ancrage comprend du stérol, GM1, un lipide, une vitamine, une petite molécule, un peptide, ou une combinaison de ceux-là.

7. Vésicule extracellulaire selon la revendication 5, dans laquelle le groupement d'ancrage comprend du cholestérol.

8. Vésicule extracellulaire selon l'une quelconque des revendications 5 à 7, dans laquelle l'OAS est lié au groupement d'ancrage.

9. Vésicule extracellulaire selon l'une quelconque des revendications 5 à 8, dans laquelle l'OAS est lié au groupement d'ancrage par un segment de liaison, dans laquelle le segment de liaison :
(i) comprend un polypeptide ;
(ii) comprend un groupement non-polypeptidique ;
(iii) comprend de l'éthylène glycol ;
(iv) comprend du HEG, du TEG, du PEG, ou toute combinaison de ceux-là ;
(v) comprend un phosphoramidite acrylique (par exemple, ACRYDITE^{™}), un produit d'adénylation, (ester de NHS), une digoxigénine (ester de NHS), du cholestérol-TEG, I-LINKER^{™}, un modificateur amino (par exemple, un modificateur amino C6, un modificateur amino C12, un modificateur amino C6 dT, ou un modificateur amino Uni-Link^{™}), un alcyne, un 5' hexynyle, un 5-octadiynyle dU, un produit de biotinylation (par exemple, une biotine, une biotine (azotue), une biotine dT, une biotine-TEG, une double biotine, une PC biotine, ou une desthiobiotine), un produit de modification de thiol (un modificateur de thiol C3 S-S, un dithiol ou un modificateur de thiol C6 S-S), ou toute combinaison de ceux-là ;
(vi) comprend un segment de liaison clivable ;
(vii) comprend un valine-alanine-p-aminobenzylcarbamate ou un valine-citrulline-p-aminobenzylcarbamate ; ou
(viii) comprend (a) un groupement maléimide et (b) un valine-alanine-p-aminobenzylcarbamate ou un valine-citrulline-p-aminobenzylcarbamate.

10. Vésicule extracellulaire selon l'une quelconque des revendications 1 à 9, dans laquelle la VE est un exosome.

11. Composition pharmaceutique comprenant la vésicule extracellulaire selon l'une quelconque des revendications 1 à 10 et un diluant, un support, un sel, ou un adjuvant pharmaceutiquement acceptable.

12. Vésicule extracellulaire selon l'une quelconque des revendications 1 à 10 ou composition pharmaceutique selon la revendication 11 destinée à être utilisée dans le traitement d'un cancer.
